# EUROPEAN PATENT APPLICATION

(11) **EP 4 509 494 A1**
(43) Date of publication of application: **19.02.2025**
(21) Application number: 23795603.2
(22) Date of filing: 28.04.2023
(51) Int. Cl.: C07D 205/04, C07D 213/81, C07D 213/82, C07D 239/80, A61P 31/14, A61K 31/44, A61K 31/505, A61K 31/166, A61K 31/18, A61K 31/17

(54) **PLPRO PROTEIN INHIBITOR, AND PREPARATION METHOD AND APPLICATION THEREOF**

(30) Priority: 29.04.2022 CN 202210474109; 25.08.2022 CN 202211026573
(71) Applicant: Tsinghua University, Haidian District, Beijing 100084 (CN)
(72) Inventor: HE, Wei, Beijing 100084 (CN); WU, Cai, Beijing 100084 (CN); LI, Bin, Beijing 100084 (CN); LI, Haoxiang, Beijing 100084 (CN); ZHENG, Jiqing, Beijing 100084 (CN); WAN, Shizhang, Beijing 100084 (CN); XU, Liqian, Beijing 100084 (CN); WANG, Meijing, Beijing 100084 (CN); LIU, Kai, Beijing 100084 (CN); XU, Chenhao, Beijing 100084 (CN); LIANG, E, Beijing 100084 (CN); LIU, Lei, Beijing 100084 (CN)
(74) Representative: HGF
(86) International application number: PCT/CN2023/091481
(87) International publication number: WO 2023/208171

(57) **Abstract**

Disclosed in the present invention are a PLpro protease inhibitor, and a preparation method and application thereof. The PLpro protease inhibitor can be combined with one or more pharmaceutically acceptable auxiliary materials or one or more other active ingredients to serve as a PLpro inhibitor to treat diseases caused by or diseases related to virus infection. The auxiliary material can be a carrier, a diluent, an adhesive, a lubricant, a wetting agent, etc. The protease inhibitor has high inhibitory activity, and can be used for broad-spectrum antivirals, especially for coronaviruses, for example, HCoV-229E, HCoV-OC43, HCoV-NL63, HCoV-HKU, SARS-CoV, MERS-CoV, SARS-CoV-2, etc., in particular SARS-CoV, MERS-CoV, SARS-CoV-2.

## Description

### TECHNICAL FIELD

The present invention relates to the technical field of medicines, and particularly relates to a PLPro protein inhibitor, and a preparation method therefor and use thereof.

### BACKGROUND

PLpro is one of two key proteases for cutting polyproteins ppla and pplab expressed by a host cell translation mechanism (PLpro is responsible for cutting nsp1, nsp2, and nsp3), and can cut the modification of Lys48-linked polyubiquitin and ubiquitin-like molecule interferon-stimulated gene 15 (ISG15) with high activity to realize immune escape. When PLpro is inhibited, a reduction in viral load and recovery of the host's innate immune system can be achieved. Due to its multiple roles in viral replication and host cell control, PLpro is considered as a potential antiviral target.

### SUMMARY

The present invention provides a compound, or a pharmaceutically acceptable salt, stereoisomer, ester, prodrug, solvate and deuterated compound thereof, the compound having the following structure: 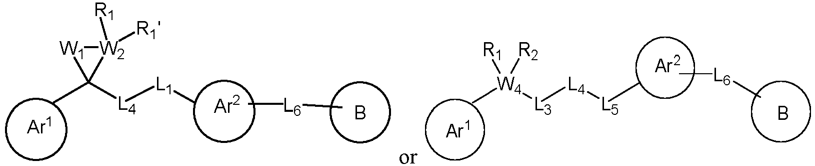 wherein
Ar¹ is substituted naphthyl, or substituted or unsubstituted non-naphthyl aryl;
Ar² is aryl or heteroaryl;
B is selected from: heterocyclyl, -S(O)ₜNR₁₅, halogen, and -NH₂;
W₁ is selected from: 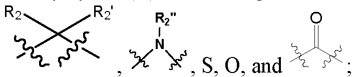
W₂ is selected from: C, N, and O; when W₂ is N, R₁' is absent; when W₂ is O, R₁ and R₁' are absent;
W₄ is absent or selected from: C or S; when W₄ is absent, R₁ and R₂ are absent;
R₁, R₁', R₂, and R₂' are independently selected from: H, D, (=O), -C₁-C₆ alkyl, -X, -CH₂X, -CHX₂, -CX₃, -OH, -NH₂, -COOH, and -OC₁-C₆ alkyl;
R₂" is selected from: H, C₁-C₆ alkyl, -OH, -(C₁-C₆ alkylene)-COOR₂₁, -(C₁-C₆ alkylene)-OR₂₁, and -(C₁-C₆ alkylene)-CONR₂₁R₂₂;
R₃ is selected from: H or C₁-C₆ alkyl;
L₁ is absent or selected from: C₁-C₆ alkylene, -CO-, -SO₂-, 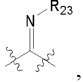 or -N(R₃)-;
L₃ and L₅ are absent or independently selected from: alkylene, heteroalkylene, cycloalkylene, heterocyclylene, and carbonyl, which can be optionally substituted;
L₄ is selected from: -NR₁₅C(O)-, -NR₁₅S(O)ₜ-, -C(O)-, -C(O)O-, -NR₁₅-, -C(O)NR₁₅-, -S(O)ₜNR₁₅-, 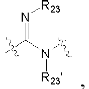 and 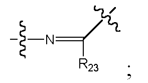
L₆ is absent or selected from: C₁-C₆ alkylene, -SO₂-, -NR₁₅C(O)-, -NR₁₅S(O)ₜ-, -C(O)-, -C(O)O-, -NR₁₅-, -C(O)NR₁₅-, - S(O)ₜNR₁₅-, and 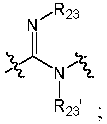
R₁₅ is selected from: H, D, alkyl, cycloalkyl, cycloalkylalkyl, aryl, aralkyl, heterocyclyl, heterocyclylalkyl, hydroxy, and alkoxy; alternatively, R₁₅, together with the nitrogen atom linked thereto and L₃ or L₅, forms heterocyclyl, which can be optionally substituted;
t is 1 or 2;
R₂₁ is H or C₁-C₆ alkyl;
R₂₂ is H or C₁-C₆ alkyl;
R₂₃ or R_{23'} is selected from H or C₁-C₆ alkyl; and
X is selected from F, Cl, Br, and I.

In an embodiment of the present invention, L₆ may be

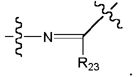

In an embodiment of the present invention, L₆ may be

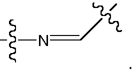

Preferably, the substituted naphthyl is selected from: 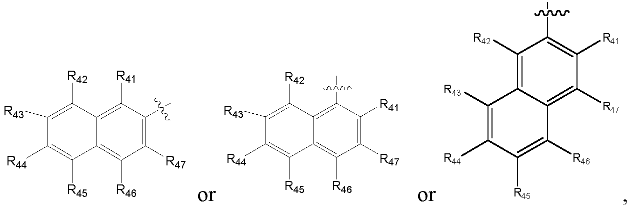
wherein R₄₁, R₄₂, R₄₃, R₄₄, R₄₅, R₄₆, and R₄₇ represent substituents on the ring and are independently selected from: H, D, C₁-C₆ alkyl, C₁-C₆ haloalkyl, C₃-C₆ cycloalkyl, aryl, aralkyl, heterocyclyl, heterocyclylalkyl, halogen, -Si(C₁-C₆ alkyl), -NO₂-R_{L}-COR', -R_{L}-C(O)OR', -R_{L}-C(O)NR'R", -R_{L}-CH=NR', -R_{L}-CN, -R_{L}-OR', -R_{L}-OC(O)R', -R_{L}-S(O)ₜ-NR'R", -R_{L}-S(O)ₜ-R', - R_{L}-NR'R", -R_{L}-NR'C(O)R", -R_{L}-NR'S(O)ₜR", -NR'-R_{L}-NR'R", -R_{L}-NO₂, and -R_{L}-N=CR'R", which can be optionally substituted, and R₄₁, R₄₂, R₄₃, R₄₄, R₄₅, R₄₆, and R₄₇ are not simultaneously H;
t is 1 or 2;
R_{L} is absent or selected from: C₁-C₆ alkylene, C₃-C₆ heteroalkylene, C₃-C₆ cycloalkylene, C₃-C₆ heterocyclylene, -NR₄C(O)-, -NR₄S(O)ₜ-, -C(O)-, -C(O)O-, -NR₄-, -C(O)NR₄-, and -S(O)ₜNR₄-, which can be optionally substituted;
R' and R" are independently selected from: H, D, C₁-C₆ alkyl, C₁-C₆ haloalkyl, C₃-C₆ cycloalkyl, cycloalkylalkyl, aryl, aralkyl, heterocyclyl, heterocyclylalkyl, and halogen, which can be optionally substituted;
R₄ is selected from: H, D, alkyl, cycloalkyl, cycloalkylalkyl, aryl, aralkyl, heterocyclyl, heterocyclylalkyl, hydroxy, and alkoxy;
more preferably, R_{L} is absent or selected from -CH₂-, -CH₂CH₂-, and -CH(CH₃)CH₂-;
more preferably, R' and R" are selected from H, -CH₃, -CH₂CH₃, and -CH(CH₃)CH₃;
more preferably, R₄ is selected from H, -CH₃, -CH₂CH₃, and -CH(CH₃)CH₃;
more preferably, R₄₁, R₄₂, R₄₃, R₄₄, R₄₅, R₄₆, and R₄₇ are independently selected from: H, D, -CH₃, -X, -CH₂F, -CHF₂, -CF₃, - OH, -CN, -OCH₃, -OCH₂X, -OCHX₂, -OCX₃, -NH₂, -NH(C₁-C₆ alkyl), -N(C₁-C₆ alkyl)(C₁-C₆ alkyl), -NO₂, -COO(C₁-C₆ alkyl), -COOH, -CN, -Si(CH₃)₃, -NHSO₂(C₁-C₆ alkyl), -SO₂NH₂, -SO₂(C₁-C₆ alkyl), -N(C₁-C₆ alkyl)SO₂(C₁-C₆ alkyl), - SO₂NH(C₁-C₆ alkyl), and -SO₂N(C₁-C₆ alkyl)(C₁-C₆ alkyl), and R₄₁, R₄₂, R₄₃, R₄₄, R₄₅, R₄₆, and R₄₇ are not simultaneously H.

More preferably, the substituted naphthyl is selected from:

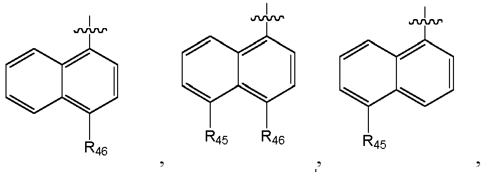

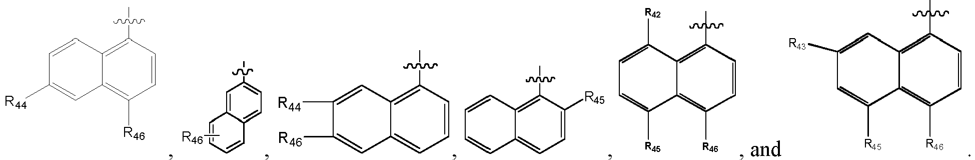

More preferably, R₄₂, R₄₃, R₄₅, and R₄₆ are independently selected from: H, -F, -D, -Br, -Cl, -I, -CH₃, -CH₂F, -CHF₂, -CF₃, - COOH, -CN, -COOCH₃, -NH₂, -NHCH₃, -N(CH₃)₂, -NO₂, -OCH₃, -OH, -TMS, -SO₂CH₃, -NHSO₂CH₃, and -SO₂NH₂.

Preferably, the non-naphthyl aryl is selected from: phenyl, substituted phenyl, 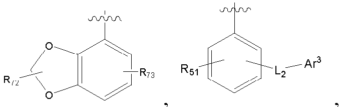 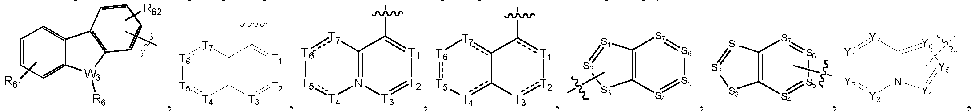 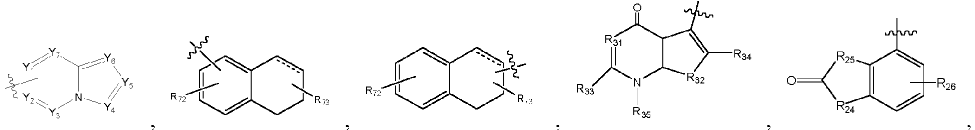 and 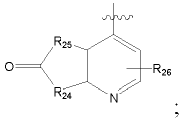
wherein, L₂ is absent or selected from: -O-, C₁-C₆ alkyl (including methyl, ethyl, propyl, isopropyl, butyl, *tert-butyl,* etc.), - CO-, -CONR₅₃-, -NR₅₃-, -NR₅₃CO-, -(C₁-C₆ alkylene)-O-, -(C₁-C₆ alkylene)-CO-, -(C₁-C₆ alkylene)-CONR₅₃-, -(C₁-C₆ alkylene)-NR₅₃-, and -(C₁-C₆ alkylene)-NR₅₃CO-;
R₅₃ is selected from H, D, or C₁-C₆ alkyl;
R₅₁ is selected from: H, D, C₁-C₆ alkyl, C₁-C₆ haloalkyl, C₃-C₆ cycloalkyl, aryl, aralkyl, heterocyclyl, heterocyclylalkyl, halogen, -Si(C₁-C₆ alkyl), -NO₂-R_{L}-COR', -R_{L}-C(O)OR', -R_{L}-C(O)NR'R", -R_{L}-CH=NR', -R_{L}-CN, -R_{L}-OR', -R_{L}-OC(O)R', - R_{L}-S(O)ₜ-NR'R", -R_{L}-S(O)ₜ-R', -R_{L}-NR'R", -R_{L}-NR'C(O)R", -R_{L}-NR'S(O)ₜR", -NR'-R_{L}-NR'R", -R_{L}-NO₂, and -R_{L}-N=CR'R", which can be optionally substituted;
t is 1 or 2;
R_{L} is absent or selected from: C₁-C₆ alkylene, C₃-C₆ heteroalkylene, C₃-C₆ cycloalkylene, C₃-C₆ heterocyclylene, -NR₄C(O)-, -NR₄S(O)ₜ-, -C(O)-, -C(O)O-, -NR₄-, -C(O)NR₄-, and -S(O)ₜNR₄-, which can be optionally substituted;
R' and R" are independently selected from: H, D, amino, C₁-C₆ alkyl, C₁-C₆ haloalkyl, C₃-C₆ cycloalkyl, cycloalkylalkyl, aryl, aralkyl, heterocyclyl, heterocyclylalkyl, and halogen, which can be optionally substituted;
preferably, R₅₁ is selected from: H, -D, -CH₃, -X, -CF₃, -OH, -OCH₃, -OCH₂X, -OCHX₂, -OCX₃, -NH₂, -NH(C₁-C₆ alkyl), - N(C₁-C₆ alkyl)(C₁-C₆ alkyl), -NO₂, -COO(C₁-C₆ alkyl), -COOH, -CN, -Si(CH₃)₃, -NHSO₂(C₁-C₆ alkyl), -SO₂NH₂, -SO₂(C₁-C₆ alkyl), -N(C₁-C₆ alkyl)SO₂(C₁-C₆ alkyl), -SO₂NH(C₁-C₆ alkyl), and -SO₂N(C₁-C₆ alkyl)(C₁-C₆ alkyl);
Ar³ is selected from substituted or unsubstituted phenyl, substituted or unsubstituted oxygen-containing five-membered or six-membered heterocyclyl, substituted or unsubstituted nitrogen-containing five-membered or six-membered heterocyclyl, and substituted or unsubstituted sulfur-containing five-membered or six-membered heterocyclyl;
preferably, Ar³ is selected from phenyl, C₁-C₆ alkyl-substituted phenyl, furyl, pyrrolyl, thienyl, pyridinyl, pyrimidinyl, thiazolyl, imidazolyl, and oxazolyl, which can be optionally substituted; more preferably, Ar³ is selected from phenyl, *tert-*butylphenyl, thienyl, and pyridinyl, which can be optionally substituted;
W₃ is selected from N or CH;
R₆ is selected from H, D, C₁-C₆ alkyl, -OH, -(C₁-C₆ alkylene)-COOR₆₁, -(C₁-C₆ alkylene)-OR₆₁, and -(C₁-C₆ alkylene)-CONR₆₁;
R₆₁ is H, D, or C₁-C₆ alkyl;
preferably, W₃ is N; R₆ is H, D, CH₃, -CH₂COOH, or -CH₂COOCH₃;
R₆₂ is selected from: H, D, C₁-C₆ alkyl, C₁-C₆ haloalkyl, C₃-C₆ cycloalkyl, aryl, aralkyl, heterocyclyl, heterocyclylalkyl, halogen, -Si(C₁-C₆ alkyl), -N₃, -B(OH)₂, -NO₂-R_{L}-COR', -R_{L}-C(O)OR', -R_{L}-C(O)NR'R", -R_{L}-CH=NR', -R_{L}-CN, -R_{L}-OR', - R_{L}-OC(O)R', -R_{L}-S(O)ₜ-NR'R", -R_{L}-S(O)ₜ-R', -R_{L}-NR'R", -R_{L}-NR'C(O)R", -R_{L}-NR'S(O)ₜR", -NR'-R_{L}-NR'R", -R_{L}-NO₂, and - R_{L}-N=CR'R", which can be optionally substituted;
T₁, T₂, T₃, T₄, T₅, T₆, and T₇ are independently selected from O, C-R₇, 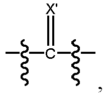 or N;
X' is N, O, and S.

In some embodiments of the present invention, when T₄ and T₅ have a single bond therebetween, T₄ and T₅ together are - CONR₈-;
T₆ and T₇ are independently selected from C-R₇ or N.

In some embodiments of the present invention, when T₆ and T₇ have a single bond therebetween, T₆ and T₇ together are - CONR₈-;
when T₁-T₇ are selected from C-R₇, each R₇ can be independently selected from: H, O, -D, -CH₃, -X, -CF₃, -OH, -OCH₃, - OCH₂X, -OCHX₂, -OCX₃, -NH₂, -NH(C₁-C₆ alkyl), -N(C₁-C₆ alkyl)(C₁-C₆ alkyl), -NO₂, -COO(C₁-C₆ alkyl), -COOH, -CN, -Si(CH₃)₃, -NHSO₂(C₁-C₆ alkyl), -SO₂NH₂, -SO₂(C₁-C₆ alkyl), -N(C₁-C₆ alkyl)SO₂(C₁-C₆ alkyl), -SO₂NH(C₁-C₆ alkyl), - SO₂N(C₁-C₆ alkyl)(C₁-C₆ alkyl), -O(C₁-C₆ alkyl)NH(C₁-C₆ alkyl), and 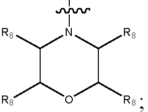
R₈ is selected from: H, D, C₁-C₆ alkyl, -(C₁-C₆ alkylene)-COOR₆₁, -(C₁-C₆ alkylene)-OR₆₁, and -(C₁-C₆ alkylene)-CONR₆₁;
S₃ is selected from: O, S, NR₉₁, and CR₉₂R₉₃;
S₁, S₂, S₄, S₅, S₆, and S₇ are independently selected from: N and CR₉₄;
wherein R₉₂, R₉₃, and R₉₄ are independently selected from: a connecting bond, H, D, C₁-C₆ alkyl, C₁-C₆ haloalkyl, C₃-C₆ cycloalkyl, aryl, aralkyl, heterocyclyl, heterocyclylalkyl, halogen, -Si(C₁-C₆ alkyl), -NO₂-R_{L}-COR', -R_{L}-C(O)OR', -R_{L}-C(O)NR'R", -R_{L}-CH=NR', -R_{L}-CN, -R_{L}-OR', -R_{L}-OC(O)R', -R_{L}-S(O)ₜ-NR'R", -R_{L}-S(O)ₜ-R', -R_{L}-NR'R", -R_{L}-NR'C(O)R", - R_{L}-NR'S(O)ₜR", -NR'-R_{L}-NR'R", -R_{L}-NO₂, -R_{L}-N=CR'R", and 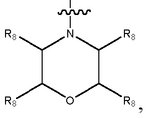 which can be optionally substituted;
preferably, R₉₂, R₉₃, and R₉₄ are independently selected from a linking bond, H, D, -CH₃, -F, -CF₃, -OH, -OCH₃, -NH₂, - NH(C₁-C₆ alkyl), -N(C₁-C₆ alkyl)(C₁-C₆ alkyl), -NO₂, -COO(C₁-C₆ alkyl), -COOH, -CN, -Si(CH₃)₃, -NHSO₂(C₁-C₆ alkyl), - SO₂NH₂, -SO₂(C₁-C₆ alkyl), -N(C₁-C₆ alkyl)SO₂(C₁-C₆ alkyl), -SO₂NH(C₁-C₆ alkyl), -SO₂N(C₁-C₆ alkyl)(C₁-C₆ alkyl), and 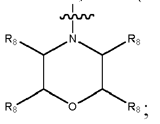
R₉₁ is selected from a linking bond, H, -D, C₁-C₆ alkyl, -OH, -(C₁-C₆ alkylene)-COOR₆₁, -(C₁-C₆ alkylene)-OR₆₁, -(C₁-C₆ alkylene)-CONR₆₁, and 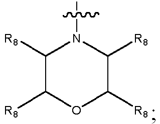
Y₁, Y₂, Y₃, Y₄, Y₅, Y₆, and Y₇ are independently selected from N or CR₁₁;
R₁₁ is selected from a linking bond, H, D, C₁-C₆ alkyl, C₁-C₆ haloalkyl, C₃-C₆ cycloalkyl, aryl, aralkyl, heterocyclyl, heterocyclylalkyl, halogen, -Si(C₁-C₆ alkyl), -NO₂-R_{L}-COR', -R_{L}-C(O)OR', -R_{L}-C(O)NR'R", -R_{L}-CH=NR', -R_{L}-CN, -R_{L}-OR', -R_{L}-OC(O)R', -R_{L}-S(O)ₜ-NR'R", -R_{L}-S(O)ₜ-R', -R_{L}-NR'R", -R_{L}-NR'C(O)R", -R_{L}-NR'S(O)ₜR", -NR'-R_{L}-NR'R", -R_{L}-NO₂, and -R_{L}-N=CR'R", which can be optionally substituted;
preferably, R₁₁ is independently selected from a linking bond, H, -D, -CH₃, -F, -CF₃, -OH, -OCH₃, -NH₂, -NH(C₁-C₆ alkyl), - N(C₁-C₆ alkyl)(C₁-C₆ alkyl), -NO₂, -COO(C₁-C₆ alkyl), -COOH, -CN, -Si(CH₃)₃, -NHSO₂(C₁-C₆ alkyl), -SO₂NH₂, -SO₂(C₁-C₆ alkyl), -N(C₁-C₆ alkyl)SO₂(C₁-C₆ alkyl), -SO₂NH(C₁-C₆ alkyl), and -SO₂N(C₁-C₆ alkyl)(C₁-C₆ alkyl);
R₇₂ and R₇₃ are independently selected from: H, -D, -CH₃, -X, -CF₃, -OH, -OCH₃, -NH₂, -NH(C₁-C₆ alkyl), -N(C₁-C₆ alkyl)(C₁-C₆ alkyl), -N₃, -B(OH)₂, -NO₂, -COO(C₁-C₆ alkyl), -COOH, -CN, -Si(CH₃)₃, -NHSO₂(C₁-C₆ alkyl), -SO₂NH₂, - SO₂(C₁-C₆ alkyl), -N(C₁-C₆ alkyl)SO₂(C₁-C₆ alkyl), -SO₂NH(C₁-C₆ alkyl), and -SO₂N(C₁-C₆ alkyl)(C₁-C₆ alkyl);
R₃₁ is N or CR₃₆; R₃₂ is NR₃₇ or -N=CR₃₈-;
R₃₅ or R₃₇ is independently selected from H, -D, C₁-C₆ alkyl, -OH, -(C₁-C₆ alkylene)-COOR₆₁, -(C₁-C₆ alkylene)-OR₆₁, and - (C₁-C₆ alkylene)-CONR₆₁;
R₃₃, R₃₄, R₃₆, and R₃₈ are independently selected from: H, -D, C₁-C₆ alkyl, C₁-C₆ haloalkyl, C₃-C₆ cycloalkyl, aryl, aralkyl, heterocyclyl, heterocyclylalkyl, halogen, -Si(C₁-C₆ alkyl)₃, -N₃, -B(OH)₂, -NO₂-R_{L}-COR', -R_{L}-C(O)OR', -R_{L}-C(O)NR'R", - R_{L}-CH=NR', -R_{L}-CN, -R_{L}-OR', -R_{L}-OC(O)R', -R_{L}-S(O)ₜ-NR'R", -R_{L}-S(O)ₜ-R', -R_{L}-NR'R", -R_{L}-NR'C(O)R", -R_{L}-NR'S(O)ₜR", -NR'-R_{L}-NR'R", -R_{L}-NO₂, and -R_{L}-N=CR'R", which can be optionally substituted;
preferably, R₃₃, R₃₄, R₃₆, and R₃₈ are independently selected from: H, -D, -CH₃, -F, -CF₃, -OH, -OCH₃, -NH₂, -NH(C₁-C₆ alkyl), -N(C₁-C₆ alkyl)(C₁-C₆ alkyl), -NO₂, -COO(C₁-C₆ alkyl), -COOH, -CN, -Si(CH₃)₃, -NHSO₂(C₁-C₆ alkyl), -SO₂NH₂, - SO₂(C₁-C₆ alkyl), -N(C₁-C₆ alkyl)SO₂(C₁-C₆ alkyl), -SO₂NH(C₁-C₆ alkyl), and -SO₂N(C₁-C₆ alkyl)(C₁-C₆ alkyl);
R₂₄ is absent or selected from: CR₂₃ and NR₂₇;
R₂₅ is selected from: CR₂₈ and NR₂₉;
R₂₃, R₂₆, and R₂₈ are independently selected from: H, -D, C₁-C₆ alkyl, C₁-C₆ haloalkyl, C₃-C₆ cycloalkyl, aryl, aralkyl, heterocyclyl, heterocyclylalkyl, halogen, -Si(C₁-C₆ alkyl)₃, -N₃, -B(OH)₂, -NO₂-R_{L}-COR', -R_{L}-C(O)OR', -R_{L}-C(O)NR'R", - R_{L}-CH=NR', -R_{L}-CN, -R_{L}-OR', -R_{L}-OC(O)R', -R_{L}-S(O)ₜ-NR'R", -R_{L}-S(O)ₜ-R', -R_{L}-NR'R", -R_{L}-NR'C(O)R", -R_{L}-NR'S(O)ₜR", -NR'-R_{L}-NR'R", -R_{L}-NO₂, and -R_{L}-N=CR'R", which can be optionally substituted;
preferably, R₂₃, R₂₆, and R₂₈ are independently selected from H, -D, -CH₃, -F, -CF₃, -OH, -OCH₃, -NH₂, -NH(C₁-C₆ alkyl), - N(C₁-C₆ alkyl)(C₁-C₆ alkyl), -NO₂, -COO(C₁-C₆ alkyl), -COOH, -CN, -Si(CH₃)₃, -NHSO₂(C₁-C₆ alkyl), -SO₂NH₂, -SO₂(C₁-C₆ alkyl), -N(C₁-C₆ alkyl)SO₂(C₁-C₆ alkyl), -SO₂NH(C₁-C₆ alkyl), and -SO₂N(C₁-C₆ alkyl)(C₁-C₆ alkyl);
R₂₇ and R₂₉ are independently selected from H, C₁-C₆ alkyl, -OH, -(C₁-C₆ alkylene)-COOR₆₁, -(C₁-C₆ alkylene)-OR₆₁, and - (C₁-C₆ alkylene)-CONR₆₁.

More preferably, the non-naphthyl aryl is 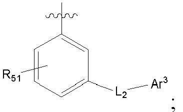 more preferably, the non-naphthyl aryl is 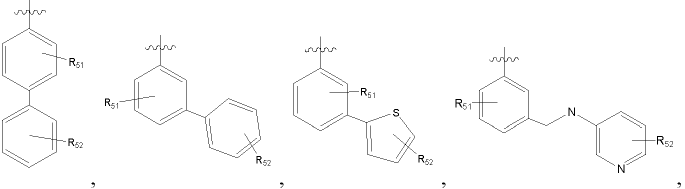 or

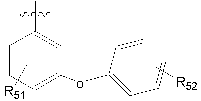

More preferably, R₅₁ and R₅₂ are independently selected from H, D, -F, -Br, -Cl, -I, -CH₃, -CH₂F, -CHF₂, -CF₃, -COOH, -CN, -COOCH₃, -NH₂, -NHCH₃, -NO₂, -OCH₃, -OH, -TMS, -SO₂CH₃, -NHSO₂CH₃, and -SO₂NH₂.

More preferably, the non-naphthyl aryl is 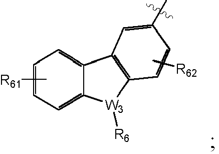 more preferably, the non-naphthyl aryl is

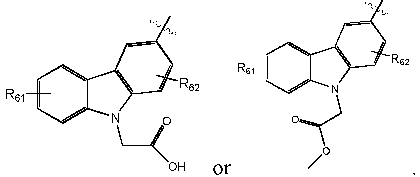

More preferably, R₆₁ and R₆₂ are independently selected from H, -D, -F, -Br, -Cl, -I, -CH₃, -CH₂F, -CHF₂, -CF₃, -COOH, - CN, -COOCH₃, -NH₂, -NHCH₃, -NO₂, -OCH₃, -OH, -TMS, -SO₂CH₃, -NHSO₂CH₃, and -SO₂NH₂.

More preferably, the non-naphthyl aryl is

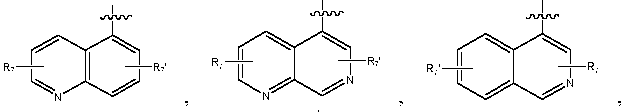

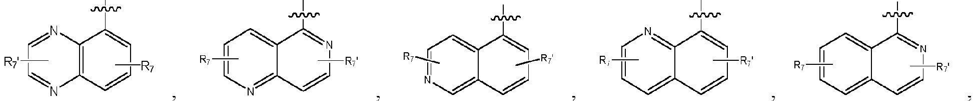

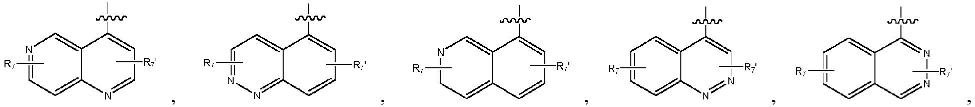

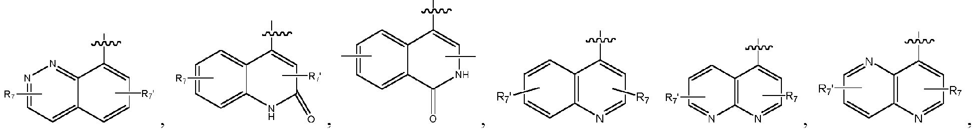

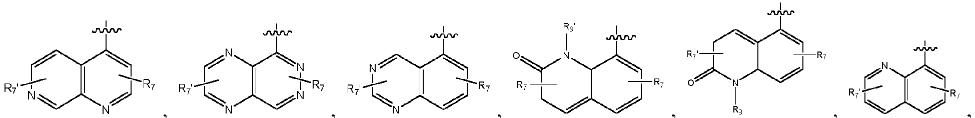

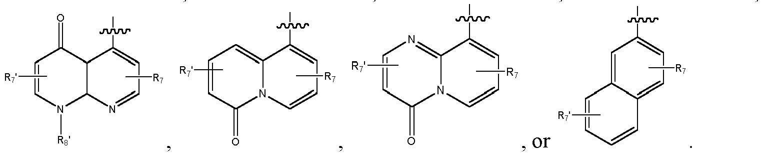

More preferably, R₇, R₇', R₇", R₇‴, R_{7α}, and R_{7β} are independently selected from H, -D, -F, -Br, -Cl, -I, -CH₃, -CH₂F, -CHF₂, -CF₃, -COOH, -CN, -COOCH₃, -NH₂, -NHCH₃, -NO₂, -OCH₃, -OH, -TMS, -SO₂CH₃, -NHSO₂CH₃, -SO₂NH₂, and substituted or unsubstituted morpholine ring, particularly preferably unsubstituted morpholine ring.

More preferably, R₈' is selected from H or methyl, ethyl, propyl, isopropyl, butyl, *tert-butyl,* and the like.

More preferably, the non-naphthyl aryl is

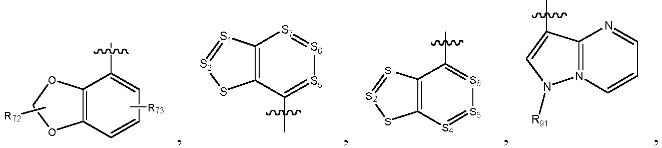

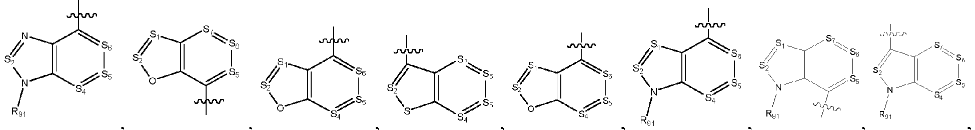

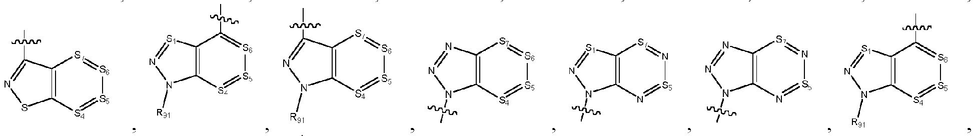

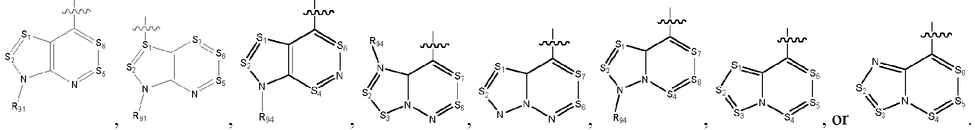

More preferably, S₁, S₂, S₄, S₅, and S₆ are CR₉₄.

More preferably, R₉₄ is selected from H, -D, -F, -Br, -Cl, -I, -CH₃, -CH₂F, -CHF₂, -CF₃, -COOH, -CN, -COOCH₃, -NH₂, - NHCH₃, -NO₂, -OCH₃, -OH, -TMS, -SO₂CH₃, -NHSO₂CH₃, and -SO₂NH₂.

Preferably, the non-naphthyl aryl is

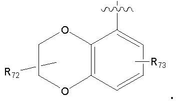

In an embodiment of the present invention, the non-naphthyl aryl is

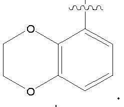

Preferably, the non-naphthyl aryl is

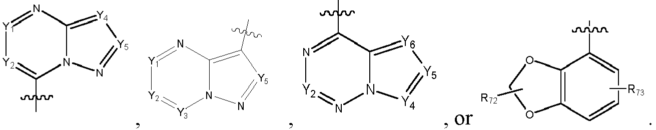

More preferably, Y₁, Y₂, Y₃, Y₄, Y₅, and Y₆ are CR₁₁.

More preferably, R₁₁ is selected from H, D, -F, -Br, -Cl, -I, -CH₃, -CH₂F, -CHF₂, -CF₃, -COOH, -CN, -COOCH₃, -NH₂, - NHCH₃, -NO₂, -OCH₃, -OH, -TMS, -SO₂CH₃, -NHSO₂CH₃, and -SO₂NH₂.

More preferably, the non-naphthyl aryl is

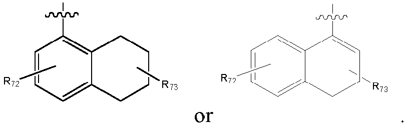

More preferably, R₇₂ and R₇₃ are selected from H, D, -F, -Br, -Cl, -I, -CH₃, -CH₂F, -CHF₂, -CF₃, -COOH, -CN, -COOCH₃, - NH₂, -NHCH₃, -NO₂, -OCH₃, -OH, -TMS, -SO₂CH₃, -NHSO₂CH₃, and -SO₂NH₂.

More preferably, the non-naphthyl aryl is

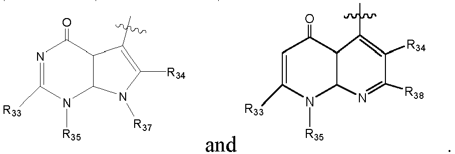

More preferably, R₃₃ is selected from: H, -D, -F, -Br, -Cl, -I, -CH₃, -CH₂F, -CHF₂, -CF₃, -COOH, -CN, -COOCH₃, -NH₂, - NHCH₃, -NO₂, -OCH₃, -OH, -TMS, -SO₂CH₃, -NHSO₂CH₃, and -SO₂NH₂.

More preferably, R₃₄, R₃₅, and R₃₇ are selected from: H, D or methyl, ethyl, propyl, isopropyl, butyl, *tert-butyl,* and the like.

Preferably, the non-naphthyl aryl is

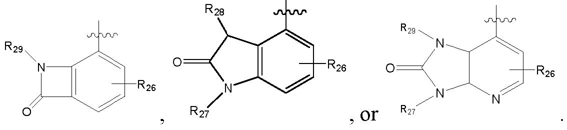

More preferably, R₂₇ and R₂₉ are independently selected from: H, D or methyl, ethyl, propyl, isopropyl, butyl, *tert-butyl,* and the like.

More preferably, R₂₆ and R₂₈ are independently selected from: H, D, -F, -Br, -Cl, -I, -CH₃, -CH₂F, -CHF₂, -CF₃, -COOH, -CN, -COOCH₃, -NH₂, -NHCH₃, -NO₂, -OCH₃, -OH, -TMS, -SO₂CH₃, -NHSO₂CH₃, and -SO₂NH₂.

Preferably, W₁ is C, and W₂ is C; alternatively, W₁ is C, and W₂ is N; alternatively, W₁ is C, and W₂ is O.

Preferably, R₁ and R₂ are independently selected from: H, (=O), C₁-3 alkyl, -COOH, -CF₃, and hydroxy; preferably, both R₁ and R₂ are H.

Preferably,

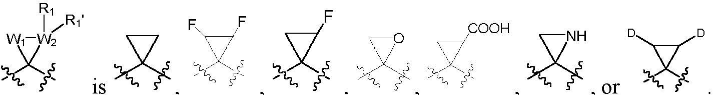

Preferably 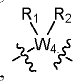 is 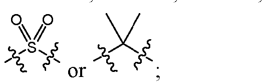 preferably, Ar² has the following structure: 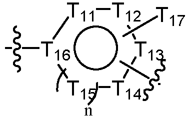 wherein
n is 0 or 1;
T₁₁-T₁₆ are independently selected from: C, N, O, and S;
T₁₇ represents one or more independent substituents on the ring selected from: H, alkyl, cycloalkyl, cycloalkylalkyl, aryl, aralkyl, heterocyclyl, heterocyclylalkyl, halogen, -R_{L}-COR', -R_{L}-C(O)OR', -R_{L}-C(O)NR'R", -R_{L}-CH=NR', -R_{L}-CN, -R_{L}-OR', -R_{L}-OC(O)R', -R_{L}-S(O)ₜ-NR'R", -R_{L}-S(O)ₜ-R', -R_{L}-NR'R", -R_{L}-NR'C(O)R", -R_{L}-NR'S(O)ₜR", -NR'-R_{L}-NR'R", -R_{L}-NO₂, and -R_{L}-N=CR'R", which can be optionally substituted;
t is 1 or 2;
R_{L} is selected from: a single bond, alkylene, heteroalkylene, cycloalkylene, heterocyclylene, -NR₄C(O)-, -NR₄S(O)ₜ, -C(O)-, -C(O)O-, -NR₄-, -C(O)NR₄-, and -S(O)ₜNR₄-, which can be optionally substituted;
R₄ is selected from: H, D, alkyl, cycloalkyl, cycloalkylalkyl, aryl, aralkyl, heterocyclyl, heterocyclylalkyl, hydroxy, and alkoxy;
R' and R" are independently selected from: H, D, alkyl, cycloalkyl, cycloalkylalkyl, aryl, aralkyl, heterocyclyl, heterocyclylalkyl, and halogen, which can be optionally substituted.

In some embodiments of the present invention, Ar² is

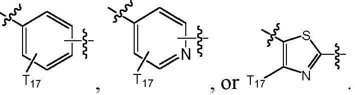

In one specific embodiment of the present invention, the compound has the following structure:

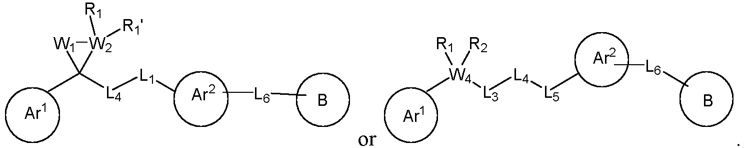

In one specific embodiment of the present invention, the compound has the following structure: 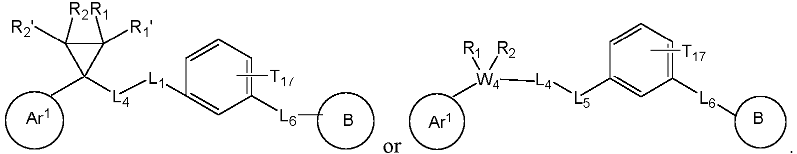 L₆ is absent or selected from: a single bond, -NH-, -N(CH₃)-, -N(CH₃)C(O)-, and -NHC(O)-.

Preferably, B has the following structure: -F, -Cl, -Br, -I, -NH₂, -S(O)ₜNR₁₅, 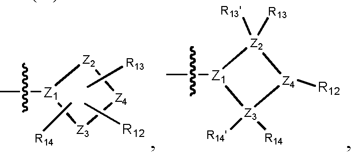 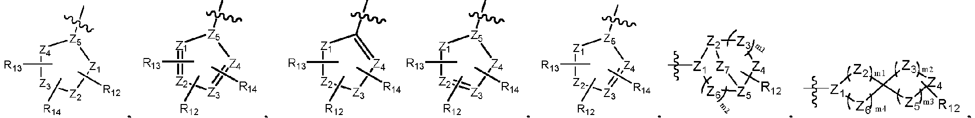 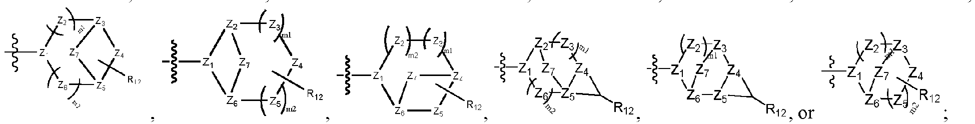
t is 1 or 2;
Z₂-Z₆ are independently selected from: C, N, O, and S;
Z₁ is selected from: C and N;
Z₇ is absent or selected from: a liking bond, C, N, O, S, and C₁-C₆ alkylene;
m1-m4 are independently selected from an integer of 0-5;
R₁₂ represents one or more independent substituents on the ring selected from: H, D, (=O), alkyl, cycloalkyl, cycloalkylalkyl, aryl, aralkyl, heterocyclyl, heterocyclylalkyl, halogen, -R_{L}-COR', -R_{L}-C(O)OR', -R_{L}-C(O)NR'R", 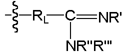 , -R_{L}-CN, -
R_{L}-OR', -R_{L}-OC(O)R', -R_{L}-S(O)ₜ-NR'R", -R_{L}-S(O)ₜ-R', -R_{L}-NR'R", -R_{L}-NR'C(O)R", -R_{L}-NR'S(O)ₜR", -NR'-R_{L}-NR'R", -R_{L}-NO₂, and -R_{L}-N=CR'R", which can be optionally substituted;
R‴ is selected from: H, alkyl, cycloalkyl, cycloalkylalkyl, aryl, aralkyl, heterocyclyl, heterocyclylalkyl, halogen, and 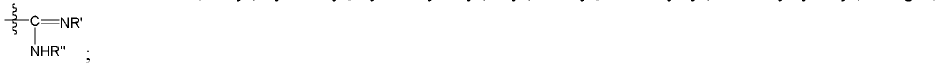
R₁₃ and R₁₃' are each independently selected from: H, D, (=O), alkyl, cycloalkyl, cycloalkylalkyl, aryl, aralkyl, heterocyclyl, heterocyclylalkyl, halogen, -N₃, -B(OH)₂, -R_{L}-COR', -R_{L}-C(O)OR', -R_{L}-C(O)NR'R", 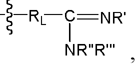 -R_{L}-CN, -R_{L}-OR', -R_{L}-OC(O)R', -R_{L}-S(O)ₜ-NR'R", -R_{L}-S(O)ₜ-R', -R_{L}-NR'R", -R_{L}-NR'C(O)R", -R_{L}-NR'S(O)ₜR", -R_{L}-N(S(O)ₜR')(S(O)ₜR"), -NR'-R_{L}-NR"R"', -R_{L}-NO₂, -R_{L}-N=CR'R", and -R_{L}-R'R", which can be optionally substituted;
R₁₄ and R₁₄' separately represent one or more independent substituents on the ring selected from: H, D, (=O), alkyl, cycloalkyl, cycloalkylalkyl, aryl, aralkyl, heterocyclyl, heterocyclylalkyl, halogen, -R_{L}-COR', -R_{L}-C(O)OR', -R_{L}-C(O)NR'R", 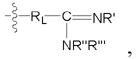 -R_{L}-CN, -R_{L}-OR', -R_{L}-OC(O)R', -R_{L}-S(O)ₜ-NR'R", -R_{L}-S(O)ₜ-R', -R_{L}-NR'R", -R_{L}-NR'C(O)R", -R_{L}-NR'S(O)ₜR", -NR'-R_{L}-NR'R", -R_{L}-NO₂, and -R_{L}-N=CR'R", which can be optionally substituted;
more preferably, B has the following structure: -F, -Cl, -Br, -I, -NH₂, -S(O)ₜNR₁₅, 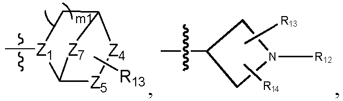 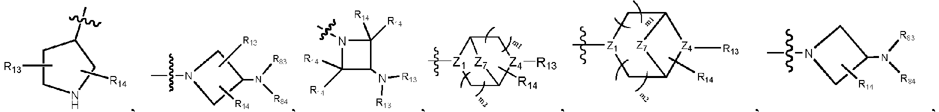 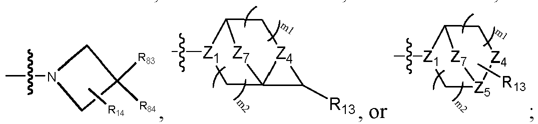
wherein, Z₁ and Z₄ are independently selected from: C, N, O, and S, and when Z₄ is O or S, R₉ is absent;
t is 1 or 2;
Z₇ is absent or selected from: a single bond, C, N, O, S, and C₁-C₃ alkylene;
m1 and m2 are independently selected from an integer of 0-5;
when Z₄ is S, R₁₃ is absent, or R₁₃ is carbonyl and R₁₄ is carbonyl;
R‴ is selected from: H, D, alkyl, cycloalkyl, cycloalkylalkyl, aryl, aralkyl, heterocyclyl, heterocyclylalkyl, halogen, and 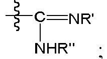
R₈₃ and R₈₄ are selected from H, D, (=O), alkyl, cycloalkyl, cycloalkylalkyl, aryl, aralkyl, heterocyclyl, heterocyclylalkyl, halogen, -R_{L}-COR', -R_{L}-C(O)OR', -R_{L}-C(O)NR'R", 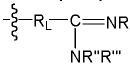 , -R_{L}-CN, -R_{L}-OR', -R_{L}-OC(O)R', -R_{L}-S(O)ₜ-NR'R", -R_{L}-S(O)ₜ-R', -R_{L}-NR'R", -R_{L}-NR'C(O)R", -R_{L}-NR'S(O)ₜR", -R_{L}-N(S(O)ₜR')(S(O)ₜR"), -NR'-R_{L}-NR"R‴, -R_{L}-NO₂, and -R_{L}-N=CR'R", which can be optionally substituted;
alternatively, R₈₃ and R₈₄, together with the N atom therebetween, form 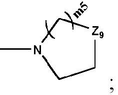
Z₉ is selected from S, NR₈₅, and O;
m₅ is selected from 1, 2, or 3;
R₈₅ is selected from H, (=O), D, alkyl, cycloalkyl, cycloalkylalkyl, aryl, aralkyl, heterocyclyl, heterocyclylalkyl, halogen, - R_{L}-COR', -R_{L}-C(O)OR', -R_{L}-C(O)NR'R", 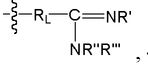 -R_{L}-CN, -R_{L}-OR', -R_{L}-OC(O)R', -R_{L}-S(O)ₜ-NR'R", -R_{L}-S(O)ₜ-R', - R_{L}-NR'R", -R_{L}-NR'C(O)R", -R_{L}-NR'S(O)ₜR", -R_{L}-N(S(O)ₜR')(S(O)ₜR"), -NR'-R_{L}-NR"R‴, -R_{L}-NO₂, and -R_{L}-N=CR'R", which can be optionally substituted.

Preferably, B has the following structure: -F, -Cl, -Br, -I, -NH₂, -S(O)ₜNR₁₅, 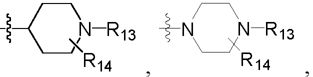 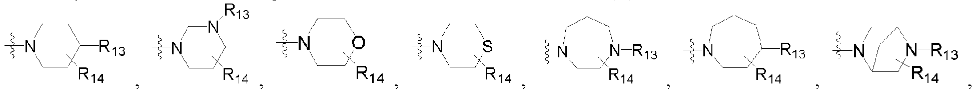 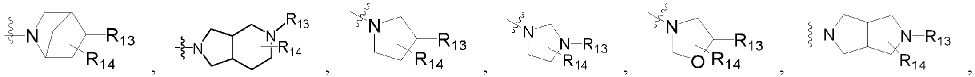 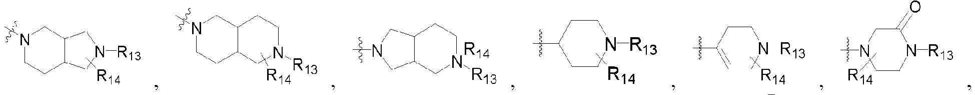 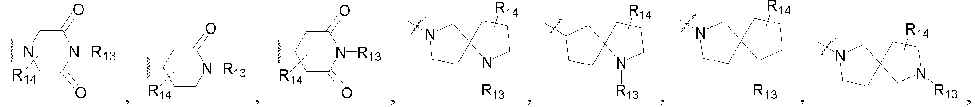 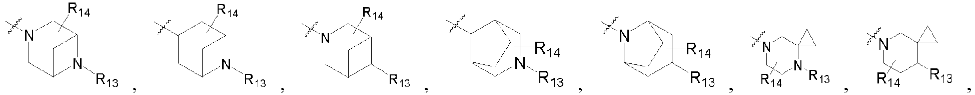 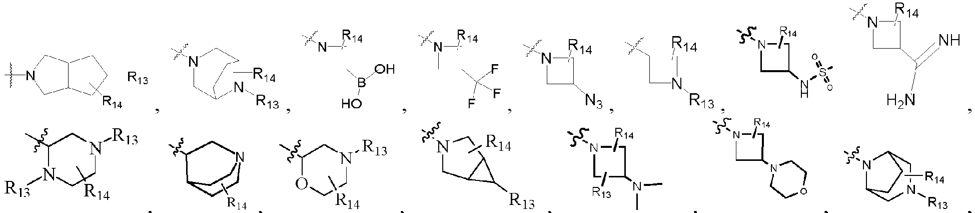 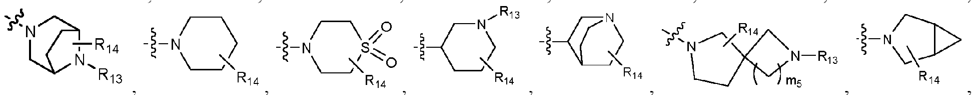 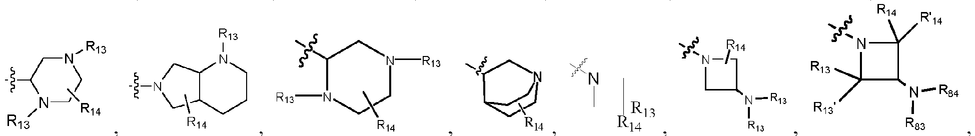 or 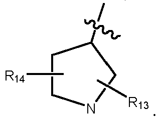 t is 1 or 2.

In some embodiments of the present invention, B is

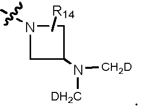

In some embodiments of the present invention, B is selected from: -F, -Cl, -Br, -I, and -NH₂.

In some embodiments of the present invention, B is

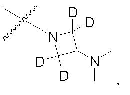

More preferably, R₁₄ and R₁₄' are each independently H or C₁-C₆ alkyl, D, amino, or

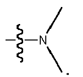

More preferably, R₁₃ and R₁₃' are each independently selected from the following structures: -H, -D, (=O), F, Cl, Br, I, methyl, ethyl, *n*-propyl, isopropyl, *n*-butyl, *tert*-butyl, -CF₃, -CH₂D, -OH, -N₃, -B(OH)₂, 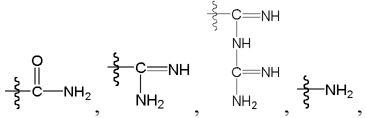 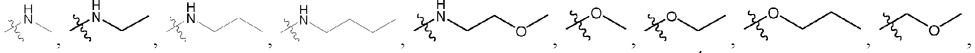 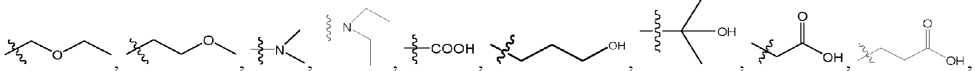 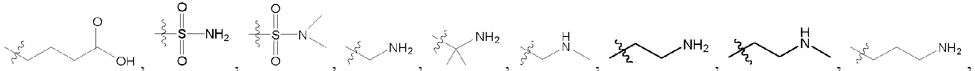 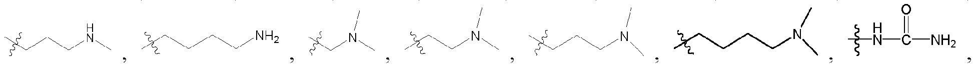 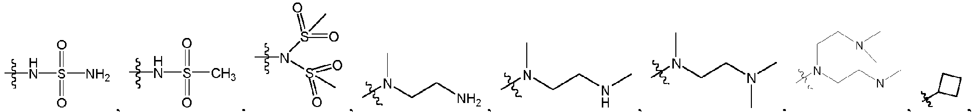 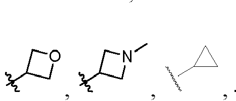 -CN,

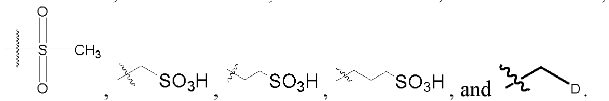

The present invention further provides the following specific compounds:

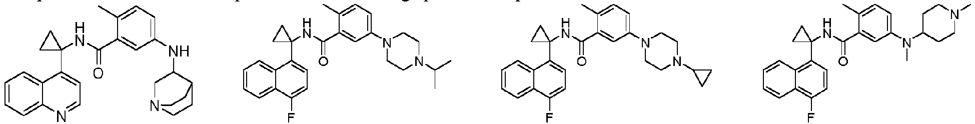

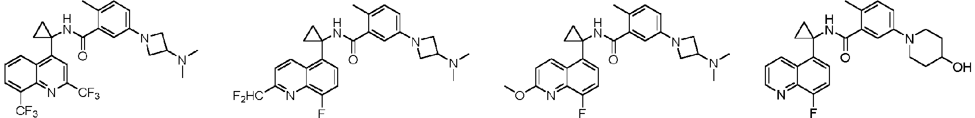

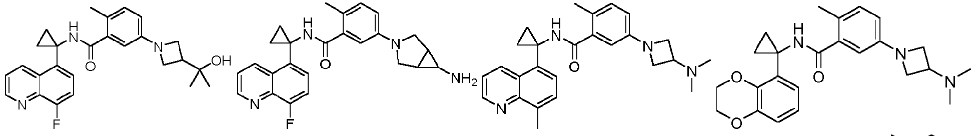

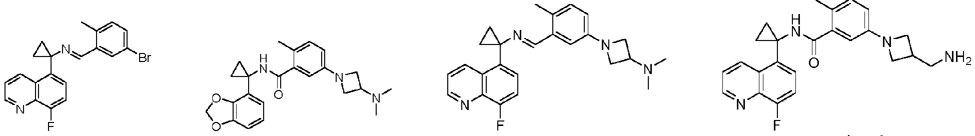

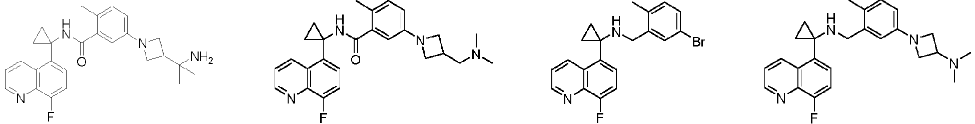

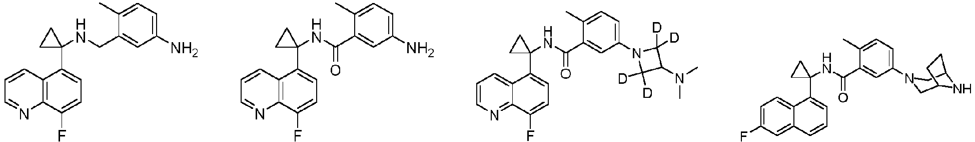

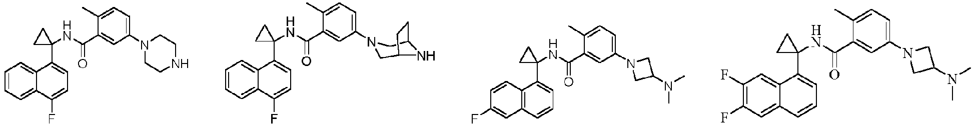

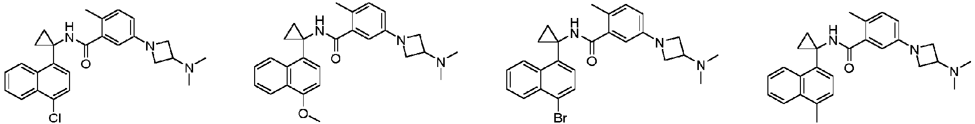

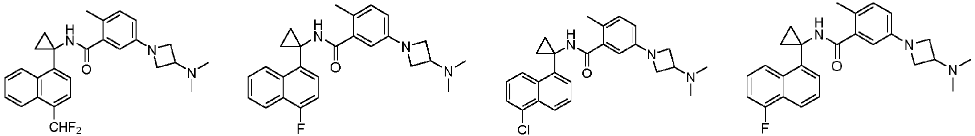

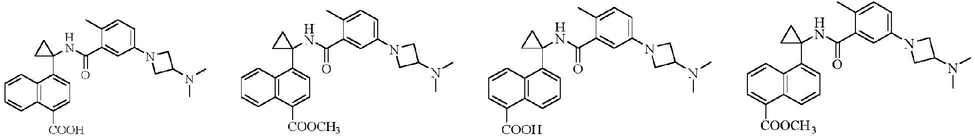

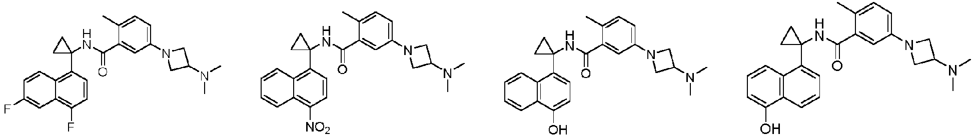

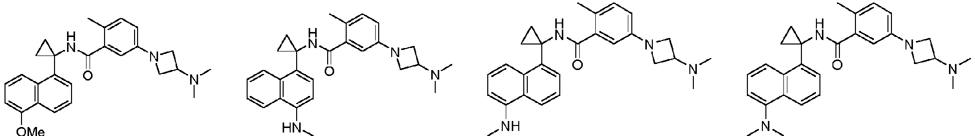

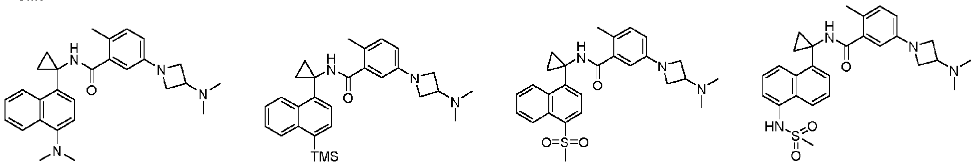

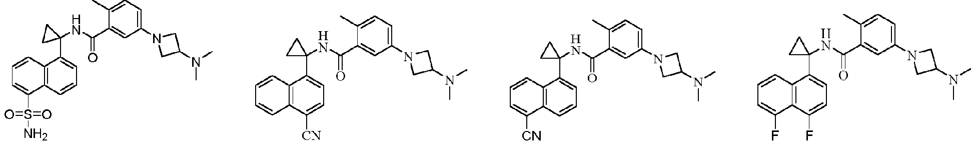

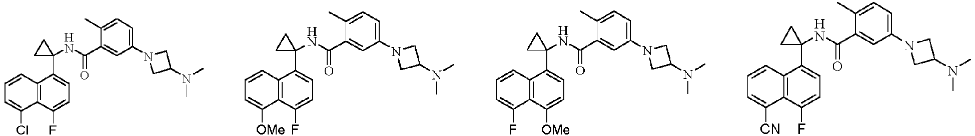

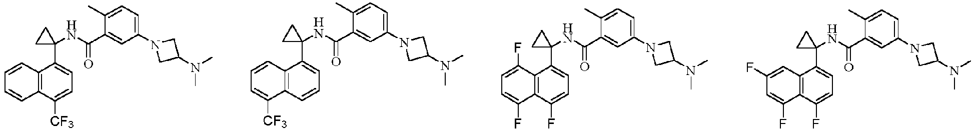

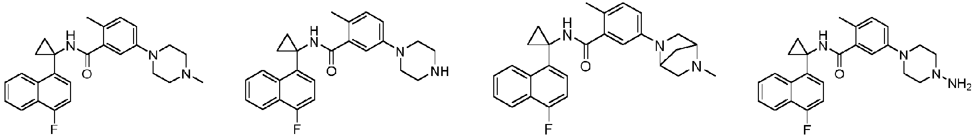

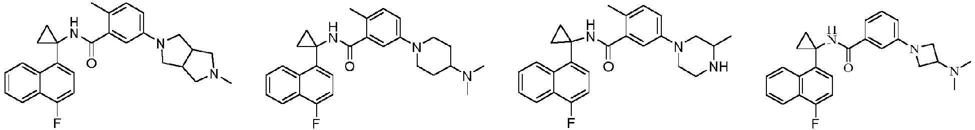

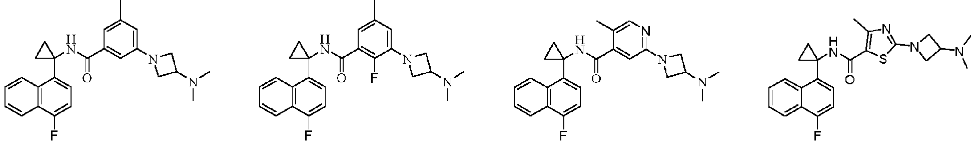

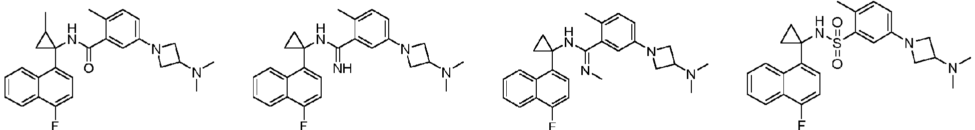

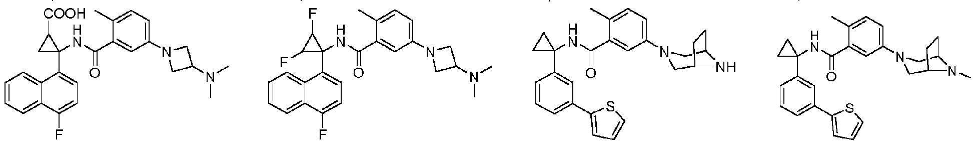

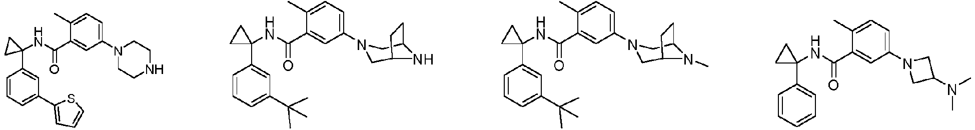

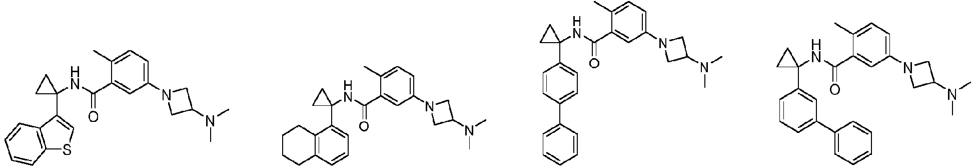

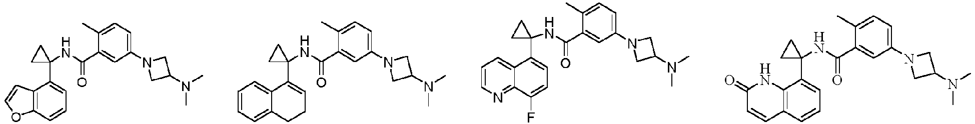

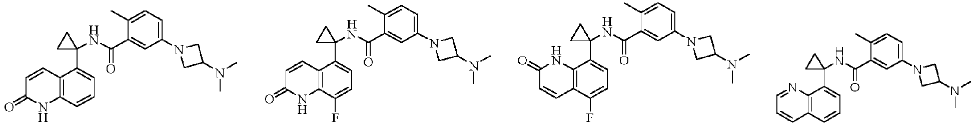

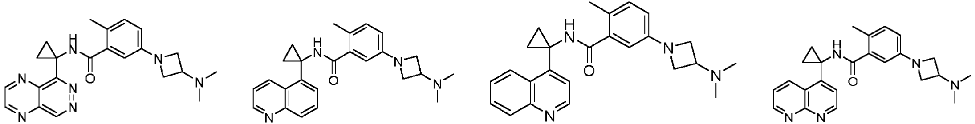

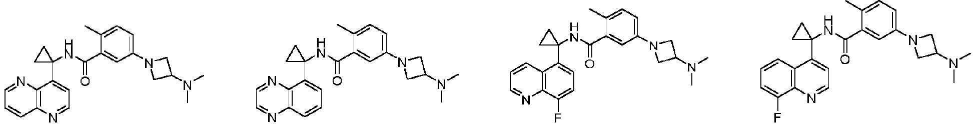

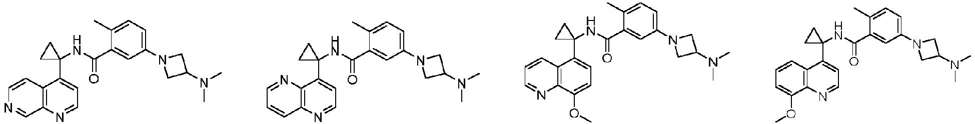

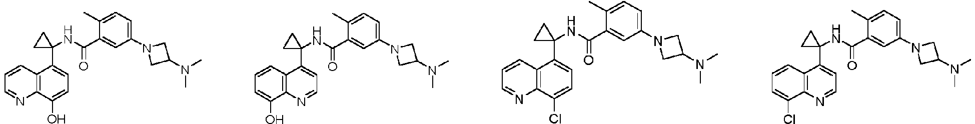

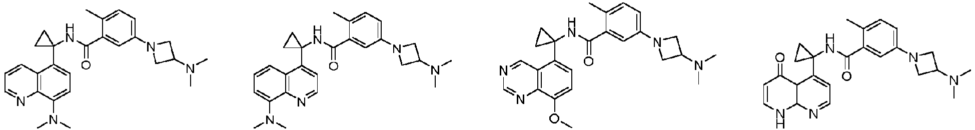

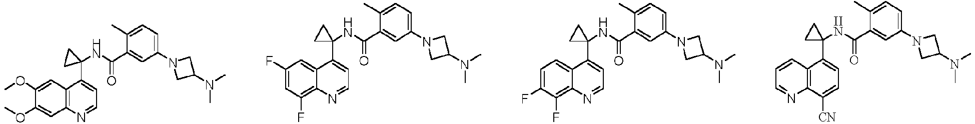

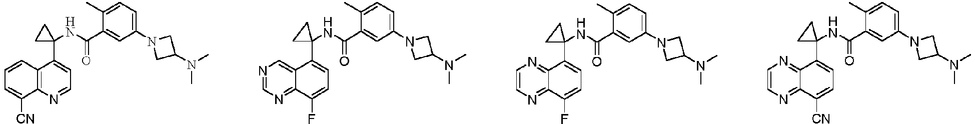

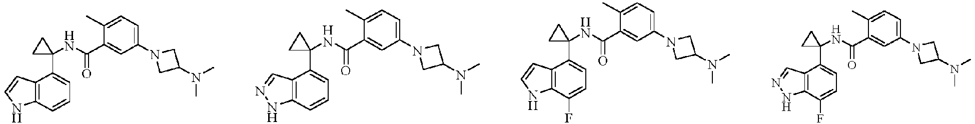

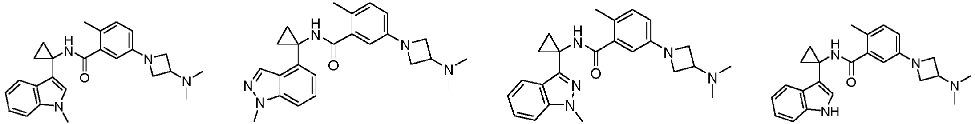

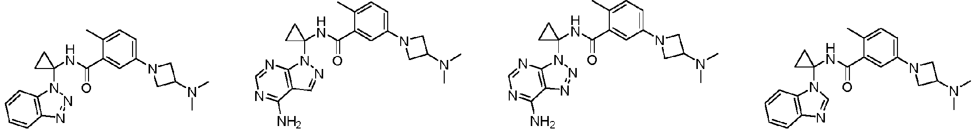

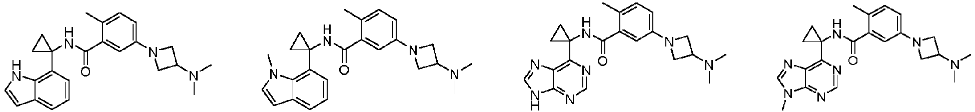

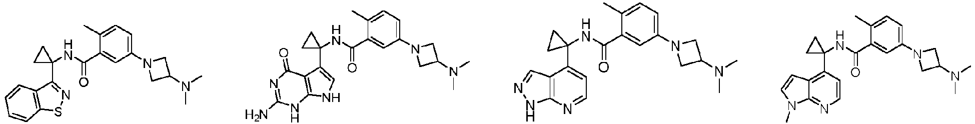

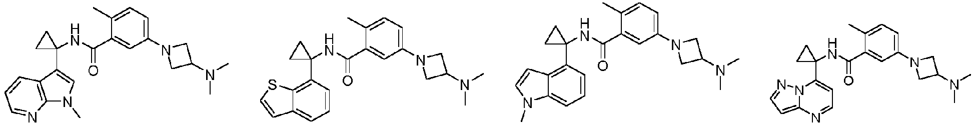

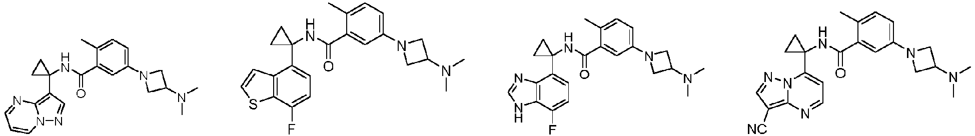

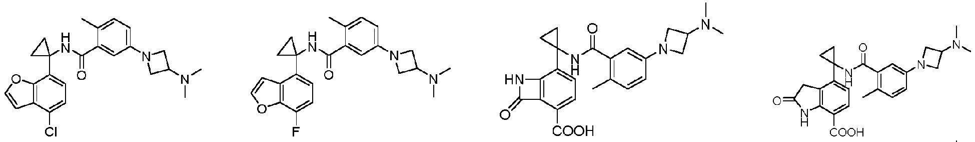

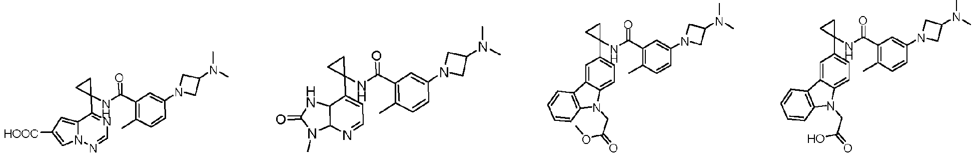

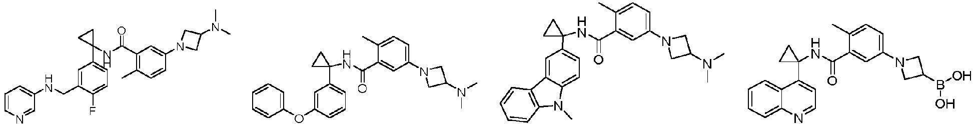

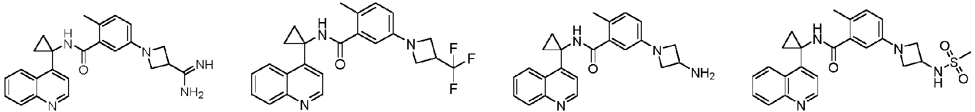

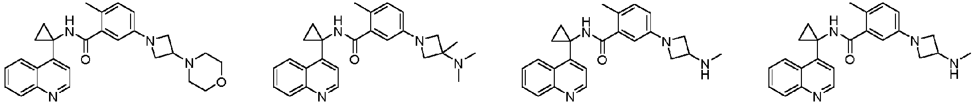

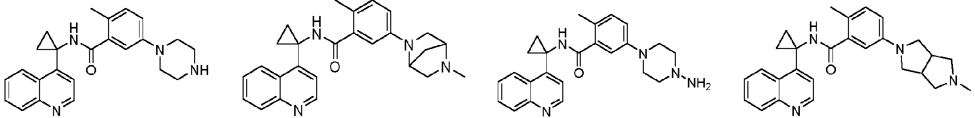

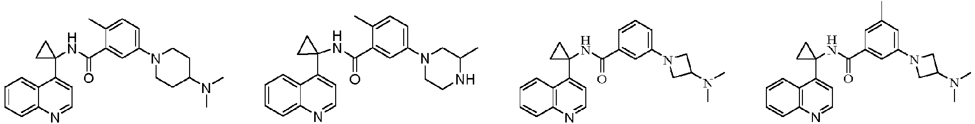

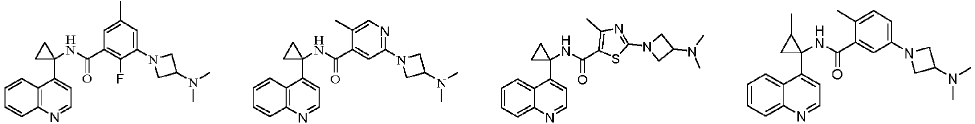

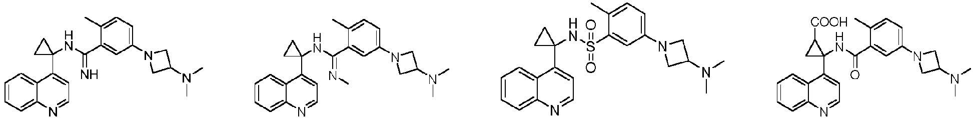

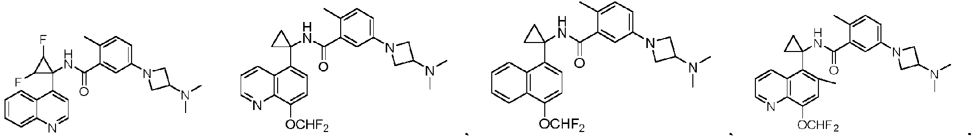

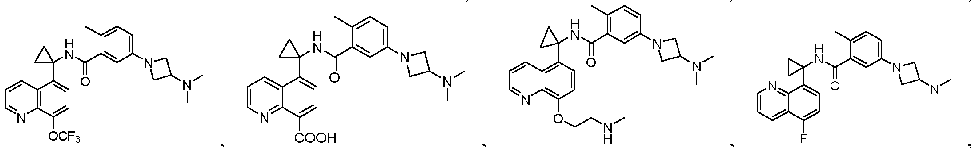

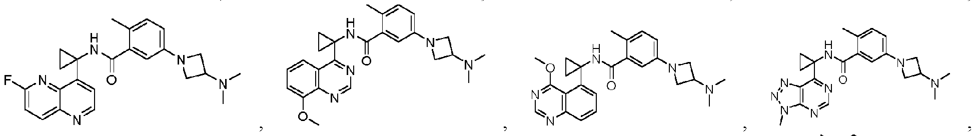

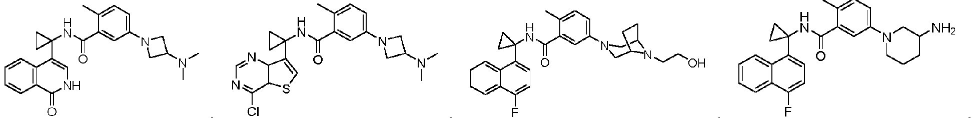

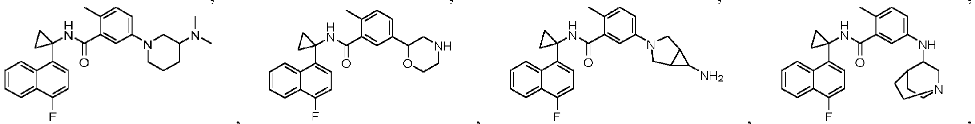

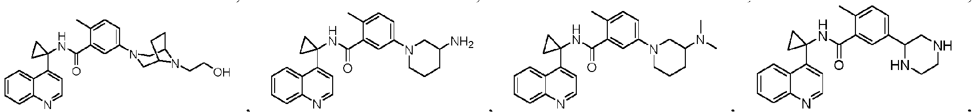

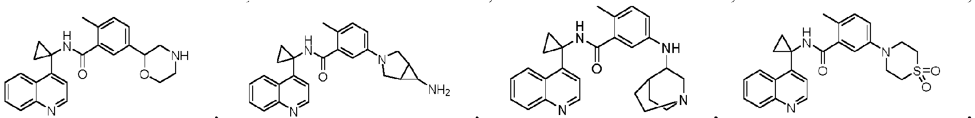

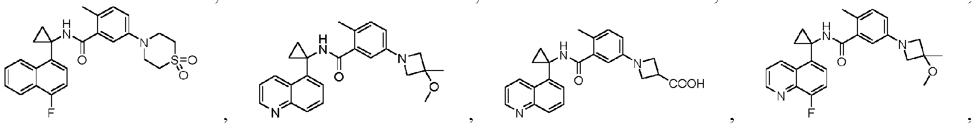

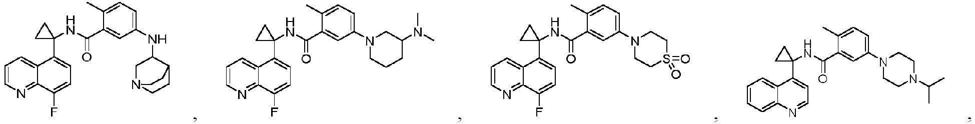

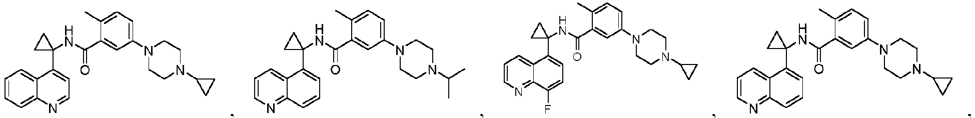

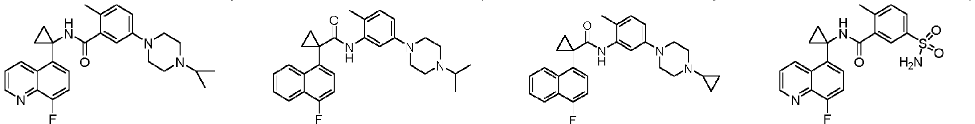

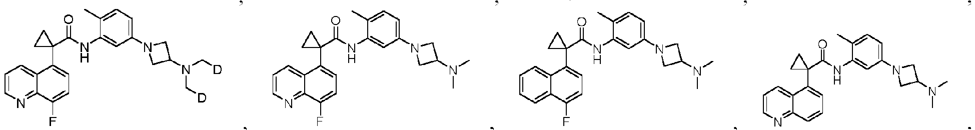

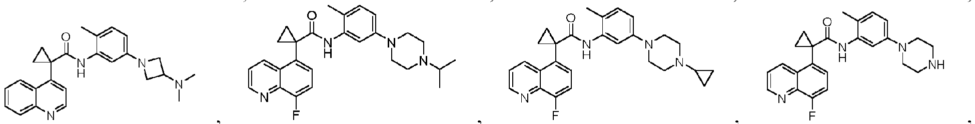

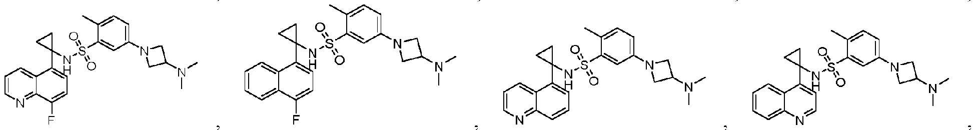

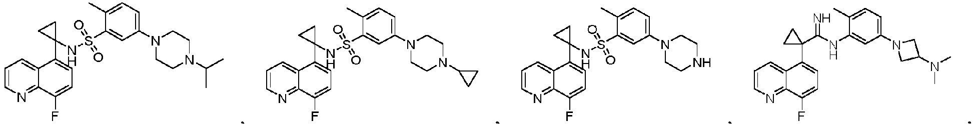

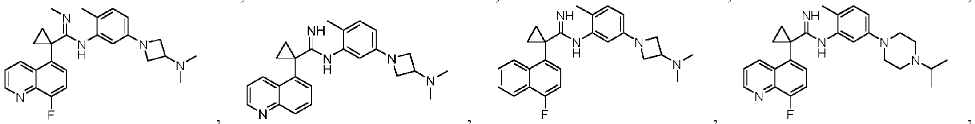

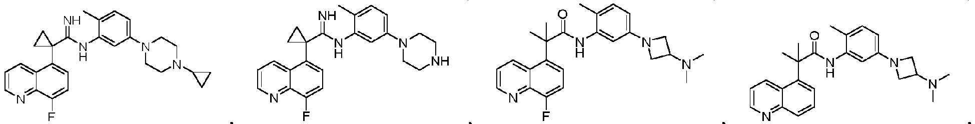

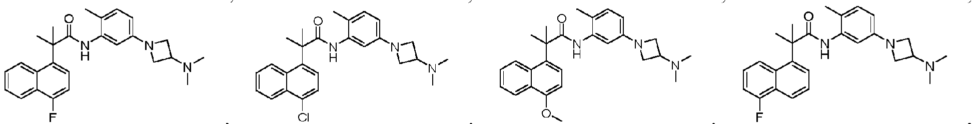

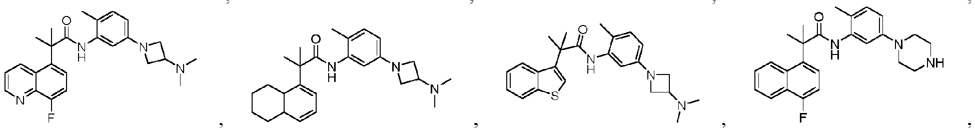

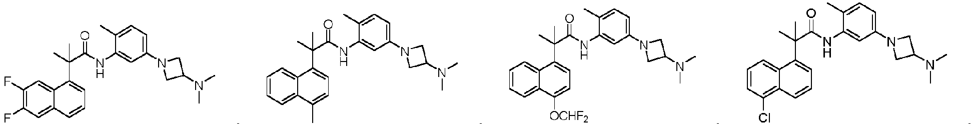

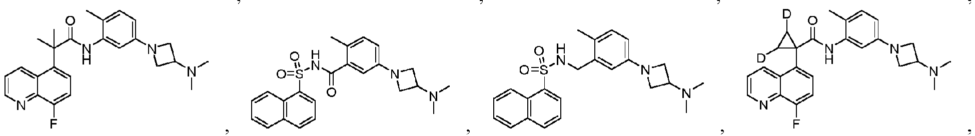

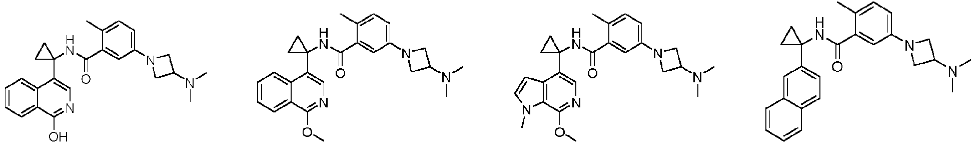

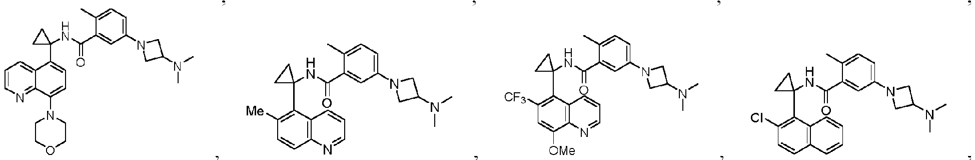

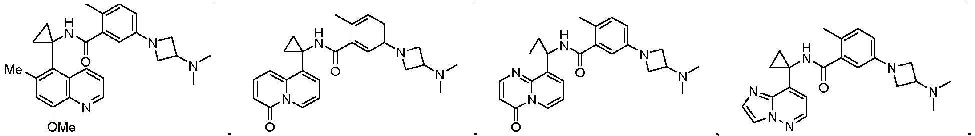

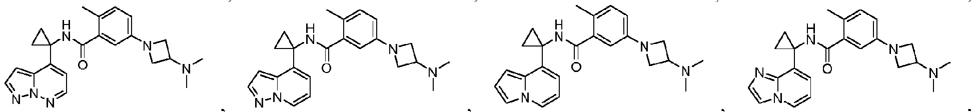

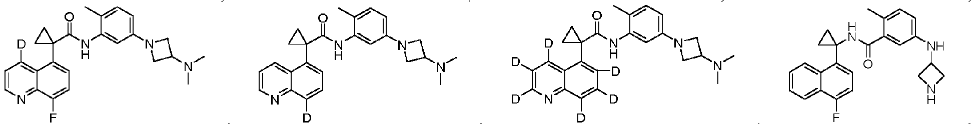

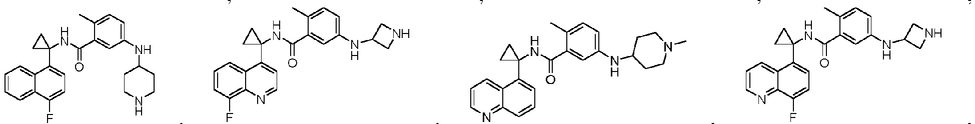

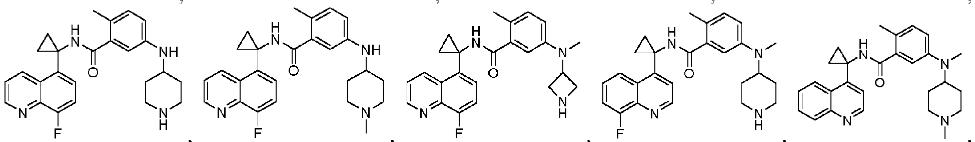

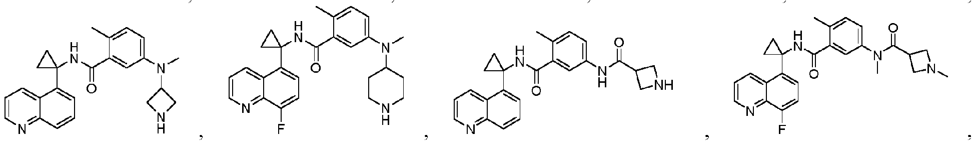

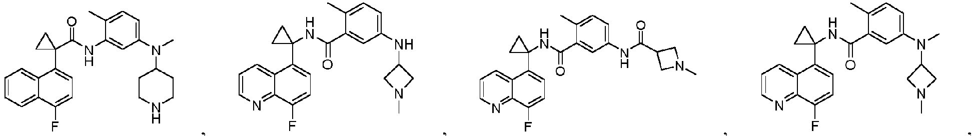

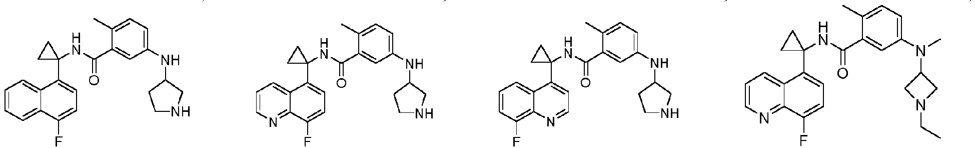

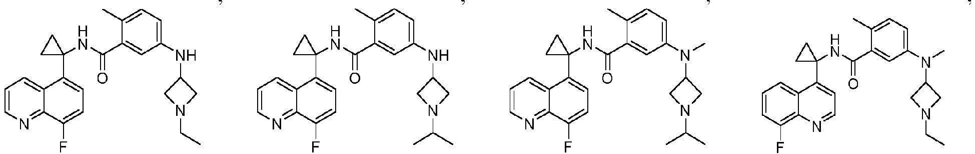

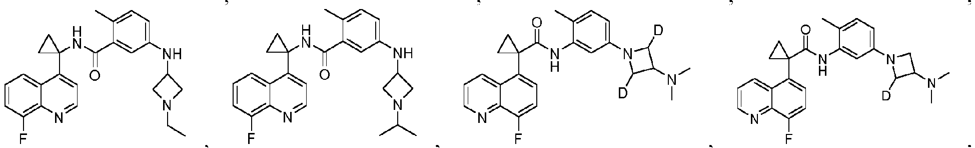

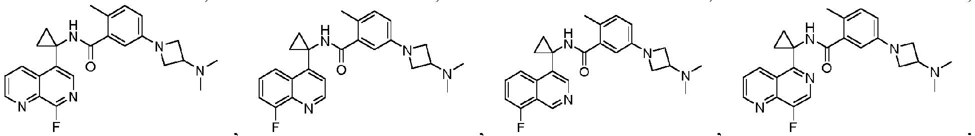

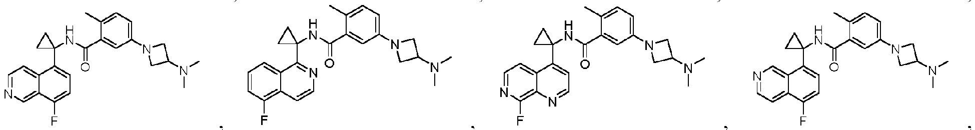

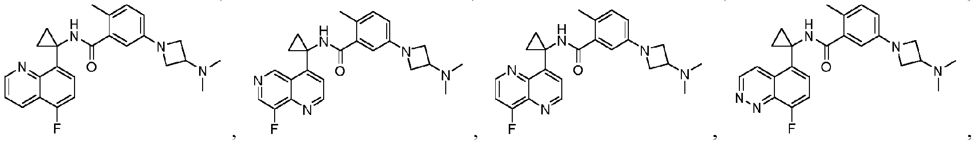

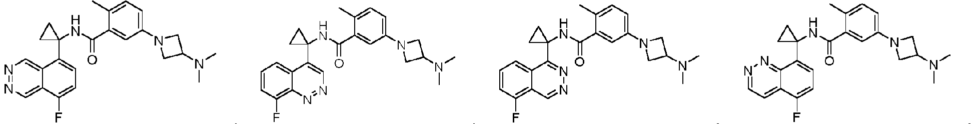

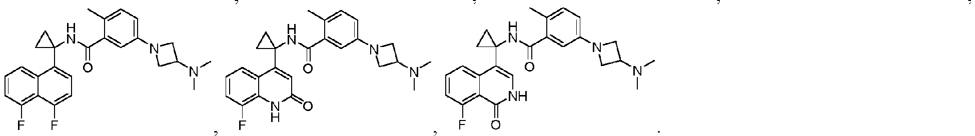

The present invention further provides a pharmaceutical composition comprising the compound or the pharmaceutically acceptable salt, the stereoisomer, the ester, the prodrug, the solvate, and the deuterated compound thereof described above, and one or more pharmaceutically acceptable auxiliary materials.

The pharmaceutical composition may further include one or more other active ingredients in combination.

For example, the auxiliary material may be a carrier, a diluent, a binder, a lubricant, a wetting agent, and the like.

The compound of the present invention may be formulated as pharmaceutical compositions in the form of syrups, elixirs, suspensions, powders, granules, tablets, capsules, lozenges, solutions, creams, ointments, lotions, gels, emulsions and the like. The pharmaceutical formulation is preferably in unit dosage form. In this form, the formulation is subdivided into unit dosages containing an appropriate amount of active ingredients. The unit dosage form may be a packaged formulation, the package containing a discrete quantity of formulation, such as tablets, capsules and powders packaged in vials or ampoules. The amount of active components of the formulation in unit dosage may be varied or adjusted from 0.001 mg to 1000 mg, depending on the specific application and the potency of active ingredients.

The present invention further provides use of the compound and the pharmaceutically acceptable salt, the stereoisomer, the ester, the prodrug, the solvate, and the deuterated compound thereof described above, and the pharmaceutical composition described above as a PLpro inhibitor, such as use as an antiviral drug.

The present invention further provides use of the compound and the pharmaceutically acceptable salt, the stereoisomer, the ester, the prodrug, the solvate, and the deuterated compound thereof described above, and the pharmaceutical composition described above in a medicament for reducing and/or inhibiting replication of a coronavirus.

The present invention further provides use of the compound and the pharmaceutically acceptable salt, the stereoisomer, the ester, the prodrug, the solvate, and the deuterated compound thereof described above, and the pharmaceutical composition described above in preparing a medicament for reducing and/or inhibiting replication of a coronavirus.

The present invention further provides use of the compound and the pharmaceutically acceptable salt, the stereoisomer, the ester, the prodrug, the solvate, and the deuterated compound thereof described above, and the pharmaceutical composition described above in preparing a medicament for preventing and/or treating a disease or condition caused by or associated with viral infection.

Specifically, in the use described above, the compound and the pharmaceutical composition have the corresponding definitions described above in the present invention.

In one embodiment of the present invention, the virus described above is a coronavirus, such as HCoV-229E, HCoV-OC43, HCoV-NL63, HCoV-HKU, SARS-CoV, MERS-CoV, SARS-CoV-2, and the like, particularly SARS-CoV, MERS-CoV, or SARS-CoV-2.

Specifically, the disease or condition described above is a disease or condition caused by or associated with coronavirus infection, such as COVID-19, SARS, MERS, and the like.

The present invention further provides a method for preventing and/or treating a disease or condition caused by or associated with viral infection, which comprises a step of administering to a subject an effective amount of the compound or the pharmaceutically acceptable salt, the stereoisomer, the ester, the prodrug, the solvate and the deuterated compound thereof described above in the present invention, or the pharmaceutical composition described above in the present invention. Specifically, in the method described above, the compound, the pharmaceutical composition, and the disease or disorder have the corresponding definitions described above in the present invention.

Particularly, the disease or condition described above is a disease or condition caused by or associated with coronavirus infection, such as COVID-19, SARS, MERS, and the like.

Specifically, the subject described above is an animal; in one embodiment of the present invention, the subject described above is a mammal, such as human, monkey, cat, dog, mouse, bat, and the like; in another embodiment of the present invention, the subject described above is a bird.

### BRIEF DESCRIPTION OF THE DRAWINGS

FIG. 1 shows the inhibition curve of compound C21.
FIG. 2 shows the inhibition curve of compound C14.
FIG. 3 shows the inhibition curve of compound C24.
FIG. 4 shows the inhibition curve of compound C16.
FIG. 5 shows the inhibition curve of compound C17.
FIG. 6 shows the inhibition curve of compound C18.
FIG. 7 shows the inhibition curve of compound C26.
FIG. 8 shows the inhibition curve of compound C75.
FIG. 9 shows the inhibition curve of compound C76.

### DETAILED DESCRIPTION

Unless otherwise defined, all scientific and technical terms used in the present invention have the same meaning as commonly understood by those skilled in the art to which the present invention relates.

The term "alkyl" refers to a hydrocarbon chain radical that is linear or branched and that does not contain unsaturated bonds, and that is linked to the rest of the molecule via a single bond. Typical alkyl groups contain 1 to 12 (e.g., 1, 2, 3, 4, 5, 6, 7, 8, 9, 10, 11, or 12) carbon atoms, such as methyl, ethyl, *n*-propyl, isopropyl, *n*-butyl, isobutyl, *tert*-butyl, *n*-pentyl, isopentyl, neopentyl, *tert*-pentyl, *n*-hexyl, isohexyl, etc. If alkyl is substituted with cycloalkyl, it is correspondingly "cycloalkylalkyl" radical, such as cyclopropylmethyl, cyclopropylethyl, cyclobutylmethyl, cyclopentylmethyl, or cyclohexylmethyl. If alkyl is substituted with aryl, it is correspondingly "aralkyl" radical, such as benzyl, benzhydryl or phenethyl. If alkyl is substituted with heterocyclyl, it is correspondingly "heterocyclylalkyl" radical. "Alkylene" generally refers to alkanediyl having two free valence bonds. Typical alkylene contains 1 to 12 (e.g., 1, 2, 3, 4, 5, 6, 7, 8, 9, 10, 11, and 12) carbon atoms, such as methylene, ethylene, propylene, butylene, and the like.

The term "alkoxy" refers to a substituent formed from a hydroxy group by substituting the hydrogen atom with alkyl, and typical alkoxy contains 1 to 12 (e.g., 1, 2, 3, 4, 5, 6, 7, 8, 9, 10, 11, or 12) carbon atoms, such as methoxy, ethoxy, propoxy, or butoxy.

The term "cycloalkyl" refers to a saturated or partially saturated (especially saturated) monocyclic or polycyclic group, which may contain 1 to 4 monocyclic and/or fused rings and 3-18 carbon atoms, preferably 3-10 (e.g., 3, 4, 5, 6, 7, 8, 9, or 10) carbon atoms, e.g., cyclopropyl, cyclobutyl, cyclopentyl, cyclohexyl, or adamantyl.

The term "aryl" refers to a monocyclic or polycyclic radical, including polycyclic radicals containing monoaryl and/or fused aryl groups, e.g., containing 1 to 3 monocyclic or fused rings and 6 to 18 (e.g., 6, 8, 10, 12, 14, 16, or 18) carbon ring atoms. Typical aryl is an aryl group containing 6 to 12 carbon ring atoms, e.g., phenyl, naphthyl, biphenyl, or indenyl. "Arylene" refers to a divalent group derived from an aromatic hydrocarbon by the removal of two hydrogen atoms.

The term "heterocyclyl" includes heteroaromatic and heteroalicyclic groups containing 1 to 3 monocyclic and/or fused rings and 3 to about 18 ring atoms. Preferred heteroaromatic groups and heteroalicyclic groups contain 5 to about 10 ring atoms. Suitable heteroaryl in the compound of the present invention contains 1, 2 or 3 heteroatoms which are selected from N, O, or S atoms. Examples of heteroaryl are, for example, but not limited to, coumarin, including 8-coumarin, quinolyl, including 8-quinolyl, isoquinolyl, pyridinyl, pyrazinyl, pyrazolyl, pyrimidinyl, furyl, pyrrolyl, thienyl, thiazolyl, isothiazolyl, triazolyl, tetrazolyl, isoxazolyl, oxazolyl, imidazolyl, indolyl, isoindolyl, indazolyl, indolizinyl, phthalazinyl, pteridinyl, purinyl, oxadiazolyl, thiadiazolyl, furazanyl, pyridazinyl, triazinyl, cinnolinyl, benzimidazolyl, benzofuranyl, benzofurazanyl, benzothiophenyl, benzothiazolyl, benzoxazolyl, quinazolinyl, quinoxalinyl, naphthyridinyl, furopyridinyl, and the like. Suitable heteroalicyclic groups in the compound of the present invention contain 1, 2 or 3 heteroatoms which are selected from N, O, or S atoms. Examples of heteroalicyclic groups are, for example, but not limited to, pyrrolidinyl, tetrahydrofuryl, dihydrofuran, tetrahydrothienyl, tetrahydrothiopyranyl, piperidinyl, morpholinyl, thiomorpholinyl, oxathianyl, piperazinyl, azetidinyl, oxetanyl, thietanyl, homopiperidinyl, oxiranyl, thiiranyl, azepinyl, oxazepanyl, diazepinyl, triazepinyl, 1,2,3,6-tetrahydropyridinyl, 2-pyrrolinyl, 3-pyrrolinyl, indolinyl, 2*H*-pyranyl, 4*H*-pyranyl, dioxanyl, 1,3-dioxolanyl, pyrazolinyl, dithianyl, dithiolyl, dihydropyranyl, dihydrothienyl, pyrazolidinyl, imidazolinyl, imidazolidinyl, 3-azabicyclo[3.1.0]hexyl, 3-azabicyclo[4.1.0]heptyl, 3*H*-indolyl, quinolizinyl, and the like.

The groups described above may be substituted at one or more available positions with one or more suitable groups such as, OR', =O, SR', SOR', SO₂R', OSO₂R', OSO₃R', NO₂, NHR', N(R')₂, =N-R', N(R')COR', N(COR')₂, N(R')SO₂R', N(R')C(=NR')N(R')R', N₃, CN, halogen, COR', COOR', OCOR', OCOOR', OCONHR', OCON(R')₂, CONHR', CON(R')₂, CON(R')OR', CON(R')SO₂R', PO(OR')₂, PO(OR')R', PO(OR')(N(R')R'), C₁-C₁₂ alkyl, C₃-C₁₀ cycloalkyl, C₂-C₁₂ alkenyl, C₂-C₁₂ alkynyl, aryl and heterocyclyl, wherein each R' group is independently selected from hydrogen, OH, NO₂, NH₂, SH, CN, halogen, COH, CO alkyl, COOH, C₁-C₁₂ alkyl, C₃-C₁₀ cycloalkyl, C₂-C₁₂ alkenyl, C₂-C₁₂ alkynyl, aryl and heterocyclyl. These groups themselves are substituted, and the substituents may be selected from the foregoing list.

"Halogen" refers to bromine, chlorine, iodine, or fluorine. Haloalkyl refers to a group in which a hydrogen atom on an alkyl group is substituted with a halogen atom (F, Cl, Br, and I), such as -CH₂Rh, -CHRh₂, and -CRh₃, wherein Rh is F, Cl, Br, or I; such as -CF₃.

The term "pharmaceutically acceptable salt" refers to an acidic or basic salt that is theoretically non-toxic, non-irritating, and non-allergic, and capable of achieving or providing clinically acceptable pharmacokinetic, absorption, distribution, and metabolic properties of pharmaceutical molecules to be capable of achieving intended purposes. The salt described in the present invention includes a pharmaceutically acceptable acid or basic salt of an acidic, basic, or amphoteric group in the compound. A list of suitable salts can be found in S.M. Birge, et al., J. Pharm. Sci., 66, 1-19 (1977).

The pharmaceutically acceptable salts of the present invention include acid addition salts and base addition salts.

The acid addition salts include, but are not limited to, salts derived from inorganic acids, such as hydrochloric acid, nitric acid, phosphoric acid, sulfuric acid, hydrobromic acid, hydroiodic acid, and phosphonic acid, and salts derived from organic acids, such as aliphatic mono-carboxylic acid and aliphatic dicarboxylic acid, phenyl-substituted alkanoic acid, hydroxyalkanoic acid, alkanedioic acids, aromatic acid, aliphatic sulfonic acid and aromatic sulfonic acid. Thus, these salts include, but are not limited to, sulfate, pyrosulfate, bisulfate, sulfite, bisulfite, nitrate, phosphate, monohydrogenphosphate, dihydrogenphosphate, metaphosphate, pyrophosphate, hydrochloride, hydrobromide, iodate, acetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, tosylate, phenylacetate, citrate, lactate, maleate, tartrate, and methanesulfonate, and salts comprising amino acids such as arginate, gluconate and galacturonate. Acid addition salts can be prepared by contacting the free base form with a sufficient amount of the desired acid to form the salt in a conventional manner. The free base form can be regenerated by contacting the salt form with a base and isolating the free base in a conventional manner.

The base addition salts according to the present invention are salts with metals or amines, such as hydroxides of alkali metals and alkaline earth metals, or with organic amines. Examples of metals used as cations include, but are not limited to, sodium, potassium, magnesium, and calcium. Examples of suitable amines include, but are not limited to, *N,N-*dibenzylethylenediamine, chloroprocaine, choline, diethanolamine, ethylenediamine(ethane-1,2-diamine), *N-*methylglucamine and procaine. Base addition salts can be prepared by contacting the free acid form with a sufficient amount of the desired base to form the salt in a conventional manner. The free acid form can be regenerated by contacting the salt form with an acid and isolating the free acid in a conventional manner.

The term "solvate" is to be understood as any form of the compound of the present invention, wherein the compound is linked to another molecule (usually a polar solvent) by a non-covalent bond, particularly including hydrate and alcoholate such as methanolate. A preferred solvate is hydrate.

The term "prodrug" is used in its broad sense and encompasses derivatives that can be converted into the compound of the present invention *in vivo.* Examples of prodrugs include, but are not limited to, derivatives and metabolites of the compound, including biohydrolyzable moieties such as biohydrolyzable amides, biohydrolyzable esters, biohydrolyzable carbamates, biohydrolyzable carbonates, biohydrolyzable ureides, and biohydrolyzable phosphate analogs. Preferably, the prodrug having a carboxyl functional group is a lower alkyl ester of a carboxylic acid. The carboxylic acid ester is readily esterified from any carboxylic acid moiety present in the molecule. Prodrugs can generally be prepared by known methods, such as those described in "Burger's Medicinal Chemistry and Drug Discovery", sixth edition (Donald J. Abraham ed., 2001, Wiley) and "Design and Applications of Prodrugs" (H. Bundgaard ed., 1985, Harwood Academic Publishers).

The term "absent" means that the linking group is a connecting bond. For example, in the 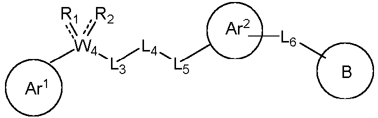 structure, the absence of L6 means that Ar² is directly linked to B. For another example, the absence of R_{L} in -R_{L}-CH=NR' means that the group is only -CH=NR'.

Any compound referred to herein is intended to represent such particular compound and certain variations or certain forms thereof. Particularly, the compounds referred to herein may have asymmetric centers and thus have different enantiomeric or diastereomeric forms. Thus, any given compound referred to herein represents any one of racemates thereof, one or more enantiomeric forms, one or more diastereomeric forms and mixtures thereof. Likewise, molecules with a double bond may also have stereoisomers or geometric isomers. Thus, in some cases, an (E)-isomer or (Z)-isomer (trans and cis isomers) may be present. If a molecule contains multiple double bonds, each double bond will have its own stereoisomerism, which may be the same with or be different from the stereoisomerisms of other double bonds of the molecule. In addition, the compounds referred to herein may have atropisomers. All stereoisomers of the compounds referred to herein, including enantiomers, diastereomers, geometric isomers, atropisomers and mixtures thereof, are within the scope of the present invention.

### Example 1:

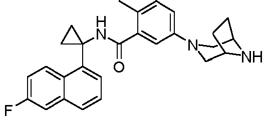

**C1:** ¹H NMR (600 MHz, DMSO-*d*₆) δ 9.23 (s, 1H), 9.07 (s, 1H), 8.74 (dd, *J* = 9.4, 5.7 Hz, 1H), 7.84 (d, *J* = 8.2 Hz, 1H), 7.78 (d, *J* = 7.0 Hz, 1H), 7.73 (dd, *J* = 10.3, 2.7 Hz, 1H), 7.54 - 7.46 (m, 2H), 6.99 (d, *J* = 8.4 Hz, 1H), 6.81 (dd, *J* = 8.4, 2.8 Hz, 1H), 6.56 (d, *J* = 2.7 Hz, 1H), 4.08 (s, 2H), 3.50 (d, *J* = 12.2 Hz, 2H), 2.99 (t, *J* = 10.3 Hz, 2H), 1.95 (dd, *J* = 8.8, 4.3 Hz, 2H), 1.89 (d, *J* = 10.1 Hz, 5H), 1.36 (d, *J* = 5.0 Hz, 2H), 1.19 (d, *J* = 5.8 Hz, 2H). ¹³C NMR (151 MHz, DMSO-*d*₆) δ 170.03, 160.14 (d, *J* = 243.6 Hz), 147.99, 138.34 (d, *J* = 18.6 Hz), 134.86, 131.30, 129.40, 128.68 (d, *J* = 8.6 Hz), 128.34, 127.64 (d,*J* = 5.2 Hz), 126.84, 125.53, 116.17, 116.00, 115.80, 113.51, 111.77 (d, *J* = 20.0 Hz), 54.19, 51.08, 34.58, 25.82, 18.27, 14.59. MS (ESI, m/z):C27H28FN3O, [M+H]+430.229.

### Example 2:

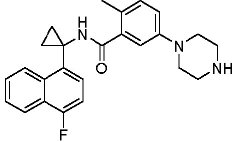

C2: ¹H NMR (600 MHz, DMSO-*d*₆) δ 9.09 - 9.06 (m, 1H), 8.73 (d, *J* = 8.5 Hz, 1H), 8.08 (d, *J* = 8.2 Hz, 1H), 7.80 (dd, *J* = 8.0, 5.6 Hz, 1H), 7.67 (dt, *J* = 26.1, 7.3 Hz, 2H), 7.29 (dd, *J* = 10.6, 7.9 Hz, 1H), 7.01 (d, *J* = 8.4 Hz, 1H), 6.90 (dd, *J* = 8.4, 2.7 Hz, 1H), 6.65 (d, *J =* 3.0 Hz, 1H), 3.25 (dt, *J* = 8.6, 4.3 Hz, 4H), 3.16 (dd, *J* = 8.8, 4.6 Hz, 4H), 1.92 (s, 3H), 1.37 (d, *J* = 5.8 Hz, 2H), 1.19 (d, *J* = 5.6 Hz, 2H). ¹³C NMR (151 MHz, DMSO-*d*₆) δ 169.93, 157.71 (d, *J* = 249.7 Hz), 148.11, 138.27, 134.47, 133.49, 131.44, 129.12 (d, *J =* 8.3 Hz), 127.36, 126.68 (d, *J* = 5.3 Hz), 125.84, 123.42 (d, *J =* 16.2 Hz), 120.85 (d, *J* = 5.4 Hz), 117.54, 115.14, 109.02 (d, *J* = 19.2 Hz), 46.17, 43.01, 34.14, 18.40, 14.58. MS (ESI, m/z):C25H26FN3O, [M+H]+404.212.

### Example 3:

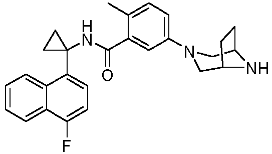

**C3:** ¹H NMR (600 MHz, DMSO-*d*₆) δ 9.03 (s, 1H), 8.72 (d, *J =* 8.4 Hz, 1H), 8.08 (d, *J =* 8.2 Hz, 1H), 7.79 (dd, *J =* 8.0, 5.6 Hz, 1H), 7.72 - 7.62 (m, 2H), 7.29 (dd, *J* = 10.6, 7.8 Hz, 1H), 6.96 (dd, *J* = 8.7, 2.7 Hz, 1H), 6.79 - 6.74 (m, 1H), 6.51 (d,*J =* 3.2 Hz, 1H), 3.84 (s, 2H), 3.40 (t, *J* = 10.2 Hz, 2H), 2.80 (d, *J =* 11.6 Hz, 2H),1.89 (s, 3H), 1.79 (d, *J =* 14.5 Hz, 4H), 1.35 (t, *J = 3.3 Hz,* 2H), 1.17 (d, *J* = 5.8 Hz, 2H). ¹³C NMR (151 MHz, DMSO-*d*₆) δ 170.17, 157.70 (d, *J* = 249.4 Hz), 148.49, 138.16, 134.49, 133.48, 131.22, 129.07 (d, *J* = 8.3 Hz), 127.33, 126.67, 125.84, 123.43 (d, *J* = 15.6 Hz), 120.85, 115.36, 113.12, 109.01 (d, *J =* 19.1 Hz), 54.13, 52.57, 34.14, 27.18, 18.26, 14.52. MS (ESI, m/z):C27H28FN3O, [M+H]+430.229.

### Example 4:

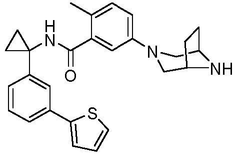

**C4:** ¹H NMR (600 MHz, DMSO-*d*₆) δ 9.46 (s, 1H), 9.01 (d, *J* = 2.7 Hz, 1H), 7.56 (dd, *J* = 12.5, 3.4 Hz, 2H), 7.52 - 7.47 (m, 2H), 7.35 (t, *J =* 7.7 Hz, 1H), 7.18 - 7.14 (m, 1H), 7.11 (dd, *J =* 8.6, 5.5 Hz, 2H), 6.90 (d, *J =* 6.0 Hz, 2H), 4.14 (s, 2H), 3.62 (d, *J =* 12.2 Hz, 2H), 3.15 (t, *J =* 10.6 Hz, 2H), 2.24 (s, 3H), 2.05 - 1.90 (m, 4H), 1.32 (s, 2H), 1.31 - 1.29 (m, 2H). ¹³C NMR (151 MHz, DMSO-*d*₆) δ 170.34, 148.14, 144.99, 137.92, 133.87, 131.56, 129.50, 128.96, 126.09, 125.89, 124.06, 123.92, 123.19, 122.63, 115.99, 113.68, 54.17, 51.10, 34.59, 25.90, 18.84, 18.79. MS (ESI, m/z):C27H29N3OS, [M+H]+444.209.

### Example 5:

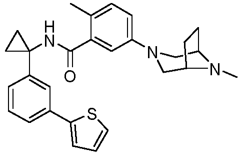

**C5:** ¹H NMR (600 MHz, DMSO-*d*₆) δ 11.09 (d, *J* = 15.0 Hz, 1H), 9.02 (s, 1H), 7.60 - 7.54 (m, 2H), 7.52 - 7.47 (m, 2H), 7.35 (t, *J =* 7.7 Hz, 1H), 7.16 (t, *J =* 4.4 Hz, 1H), 7.11 (t, *J =* 8.7 Hz, 2H), 6.91 (d, *J =* 6.3 Hz, 2H), 4.06 (t, *J* = 3.4 Hz, 2H), 3.69 (dd, *J =* 12.7, 2.7 Hz, 2H), 3.32 (dd, *J =* 26.5, 12.7 Hz, 2H), 2.74 (d, *J =* 5.0 Hz, 3H), 2.24 (s, 3H), 2.21 (dd, *J =* 8.9, 4.4 Hz, 2H), 1.98 (t, *J =* 6.7 Hz, 2H), 1.32 (s, 2H), 1.30 (s, 2H). ¹³C NMR (151 MHz, DMSO-*d*₆) δ 170.32, 147.80, 144.99, 144.08, 137.97, 133.86, 131.55, 129.50, 128.96, 126.12, 125.89, 124.06, 123.90, 123.17, 122.63, 116.02, 113.73, 62.39, 51.63, 38.65, 34.58, 23.97, 18.83, 18.80. MS (ESI, m/z):C28H31N3OS, [M+H]+458.224.

### Example 6:

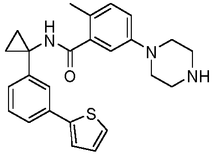

**C6:** ¹H NMR (600 MHz, DMSO-*d*₆) δ 9.41 (s, 1H), 9.03 (s, 1H), 7.57 (d, *J =* 5.1 Hz, 1H), 7.54 (d, *J =* 2.0 Hz, 1H), 7.52 - 7.48 (m, 2H), 7.36 (t, *J* = 7.7 Hz, 1H), 7.18 - 7.13 (m, 2H), 7.11 (d, *J =* 7.9 Hz, 1H), 7.00 (d, *J =* 7.5 Hz, 2H), 3.44 - 3.37 (m, 4H), 3.22 (p, *J =* 4.6 Hz, 4H), 2.26 (s, 3H), 1.36 - 1.30 (m, 4H). ¹³C NMR (151 MHz, DMSO-*d*₆) δ 170.20, 148.22, 144.98, 144.07, 137.94, 133.89, 131.71, 129.50, 128.96, 127.12, 126.12, 124.07, 123.94, 123.20, 122.59, 117.87, 115.37, 46.27, 42.97, 34.60, 18.95, 18.80. MS (ESI, m/z):C25H27N3OS, [M+H]+418.193.

### Example 7:

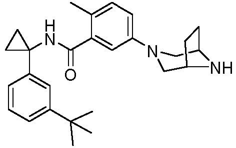

**C7:** ¹H NMR (600 MHz, DMSO-*d*₆) δ 9.34 (s, 1H), 8.92 (s, 1H), 7.34 (d, *J* = 2.3 Hz, 1H), 7.25 - 7.18 (m, 2H), 7.11 (d, *J =* 8.4 Hz, 1H), 6.96 (dd, *J* = 7.0, 2.2 Hz, 1H), 6.92 - 6.85 (m, 2H), 4.14 (s, 2H), 3.61 (dd, *J* = 12.7, 2.8 Hz, 2H), 3.10 (d, *J* = 12.0 Hz, 2H), 2.23 (s, 3H), 1.99 (dt, *J* = 12.5, 5.3 Hz, 2H), 1.97 - 1.90 (m, 2H), 1.28 (d, *J =* 1.9 Hz, 9H), 1.24 (dd, *J* = 5.8, 3.8 Hz, 4H). ¹³C NMR (151 MHz, DMSO-*d*₆) δ 170.16, 150.54, 148.08, 143.49, 138.03, 131.54, 128.30, 125.92, 122.88, 122.10, 121.67, 115.97, 113.71, 54.22, 51.13, 34.72, 31.67, 25.88, 18.78, 18.72. MS (ESI, m/z):C27H25N3O, [M+H]+418.284.

### Example 8:

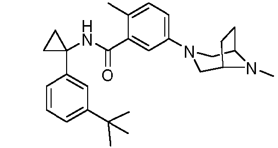

**C8:** ¹H NMR (600 MHz, DMSO-*d*₆) δ 8.94 - 8.90 (m, 1H), 7.34 (d, *J* = 1.8 Hz, 1H), 7.23 - 7.20 (m, 2H), 7.11 (d, *J* = 8.2 Hz, 1H), 6.96 (dt, *J* = 6.9, 1.8 Hz, 1H), 6.92 - 6.89 (m, 1H), 6.87 (d, *J =* 2.7 Hz, 1H), 4.07 (s, 2H), 3.68 (d, *J =* 12.2 Hz, 2H), 3.34 - 3.24 (m, 2H), 2.75 (dt, *J =* 8.1, 3.6 Hz, 3H), 2.26 - 2.18 (m, 5H), 1.97 (t, *J =* 6.9 Hz, 2H), 1.28 (d, *J =* 1.2 Hz, 9H), 1.22 - 1.29 (m, 4H). ¹³C NMR (151 MHz, DMSO-*d*₆) δ 170.14, 150.53, 147.71, 143.48, 138.08, 131.53, 128.30, 125.93, 122.89, 122.11, 121.68, 116.00, 113.78, 62.44, 51.74, 38.66, 34.73, 31.68, 23.91, 18.77, 18.72. MS (ESI, m/z):C28H37N3O, [M+H]+432.299.

### Example 9:

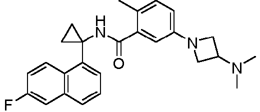

**C9:** ¹H NMR (600 MHz, DMSO-*d*₆) δ 9.07 (s, 1H), 8.74 (dd, *J =* 9.3, 5.7 Hz, 1H), 7.84 (d, *J =* 8.2 Hz, 1H), 7.79 (d, *J =* 7.1 Hz, 1H), 7.73 (dd, *J* = 10.2, 2.7 Hz, 1H), 7.54 - 7.46 (m, 2H), 6.98 (d, *J* = 8.2 Hz, 1H), 6.42 (dd, *J* = 8.2, 2.6 Hz, 1H), 6.19 (d, *J* = 2.6 Hz, 1H), 4.13 (s, 1H), 4.00 (t, *J =* 7.9 Hz, 2H), 3.83 (dd, *J =* 8.8, 5.4 Hz, 2H), 2.75 (s, 6H), 1.91 (s, 3H), 1.35 (q, *J =* 4.3 Hz, 2H), 1.19 (q, *J =* 4.6 Hz, 2H). ¹³C NMR (151 MHz, DMSO-*d*₆) δ 169.93, 160.15 (d, *J =* 243.5 Hz), 148.98, 138.35 (d, *J =* 11.8 Hz), 134.85, 131.23, 129.41, 128.67 (d, *J* = 8.6 Hz), 128.40, 127.65 (d, *J =* 5.1 Hz), 126.85, 124.40, 116.12 (d, *J* = 24.7 Hz), 113.16, 111.77 (d, *J* = 19.7 Hz), 110.79, 55.57, 54.46, 34.52, 18.41, 14.65. MS (ESI, m/z):C26H28FN3O, [M+H]+418.228.

### Example 10:

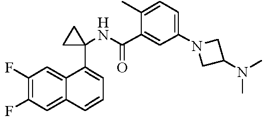

**C10:** ¹H NMR (600 MHz, DMSO-*d*₆) δ 9.06 (s, 1H), 8.64 (dd, *J =* 13.2, 8.2 Hz, 1H), 8.02 (dd, *J* = 11.7, 8.5 Hz, 1H), 7.86 (d, *J* = 8.3 Hz, 1H), 7.82 (d, *J* = 7.1 Hz, 1H), 7.51 (t, *J* = 7.7 Hz, 1H), 6.93 (d, *J* = 8.2 Hz, 1H), 6.35 (dd, *J* = 8.2, 2.5 Hz, 1H), 6.12 (d, *J =* 2.6 Hz, 1H), 3.82 (t, *J =* 7.0 Hz, 2H), 3.42 (d, *J =* 13.2 Hz, 2H), 3.13 (s, 1H), 2.08 (s, 6H), 1.91 (s, 3H), 1.36 - 1.30 (m, 2H), 1.18 (q, *J* = 4.8 Hz, 2H). ¹³C NMR (151 MHz, DMSO-*d*₆) δ 170.15, 150.03, 138.63 - 136.56 (m), 131.18, 129.44 (d, *J* = 26.9 Hz), 127.62 (d, *J* = 4.6 Hz), 126.27, 123.19, 114.92 (d, *J* = 15.9 Hz), 112.77, 112.21 (d, *J* = 17.4 Hz), 110.39, 56.29, 41.94, 34.50, 18.33. MS (ESI, m/z):C26H27F2N3O, [M+H]+436.219.

### Example 11:

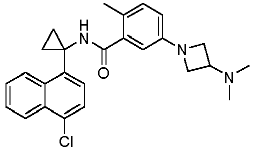

**C11:** ¹H NMR (600 MHz, DMSO-*d*₆) δ 9.08 (s, 1H), 8.77 - 8.72 (m, 1H), 8.26 - 8.20 (m, 1H), 7.80 (d, *J =* 7.6 Hz, 1H), 7.75 - 7.69 (m, 2H), 7.68 (d, *J* = 7.7 Hz, 1H), 6.99 (d, *J* = 8.2 Hz, 1H), 6.42 (dd,J= 8.2, 2.6 Hz, 1H), 6.19 (d, *J* = 2.6 Hz, 1H), 4.15 - 4.09 (m, 1H), 4.00 (t, *J* = 8.0 Hz, 2H), 3.79 (td, *J =* 11.7, 10.3, 5.7 Hz, 3H), 2.76 (s, 6H), 1.91 (s, 3H), 1.40 - 1.33 (m, 2H), 1.21 (d, *J* = 5.4 Hz, 2H). ¹³C NMR (151 MHz, DMSO-*d*₆) δ 170.01, 148.98, 138.22, 137.85, 133.46, 131.25, 130.50, 129.38, 127.57, 127.26, 126.34, 125.98, 124.72, 124.43, 113.22, 110.82, 55.73, 54.52, 34.27, 25.97, 18.43, 14.67. MS (ESI, m/z):C26H28ClN3O, [M+H]+434.199.

### Example 12:

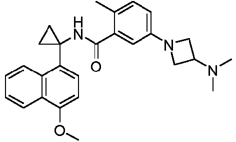

**C12:** ¹H NMR (600 MHz, DMSO-*d*₆) δ 8.94 (s, 1H), 8.63 (d, *J =* 8.5 Hz, 1H), 8.18 (d, *J* = 8.3 Hz, 1H), 7.73 (d, *J =* 7.9 Hz, 1H), 7.58 (ddd, *J =* 8.3, 6.7, 1.4 Hz, 1H), 7.50 (dd, *J =* 8.3, 6.8 Hz, 1H), 6.91 (d, *J* = 8.1 Hz, 2H), 6.33 (dd, *J* = 8.2, 2.5 Hz, 1H), 6.11 (d, *J* = 2.6 Hz, 1H), 3.97 (s, 3H), 3.80 (t, *J* = 7.0 Hz, 2H), 3.41 (t, *J* = 6.5 Hz, 2H), 3.18 - 3.10 (m, 1H), 2.08 (s, 6H), 1.91 (s, 3H), 1.35 - 1.26 (m, 2H), 1.12 (d, *J =* 5.4 Hz, 2H). ¹³C NMR (151 MHz, DMSO-*d*₆) δ 170.00, 154.62, 149.98, 138.22, 133.06, 131.08, 129.32, 125.53, 125.22, 123.26, 122.35, 112.63, 110.47, 103.71, 56.68, 56.27, 56.00, 46.02, 41.92, 34.12, 18.44, 14.62. MS (ESI, m/z):C27H31N3O2, [M+H]+430.249.

### Example 13:

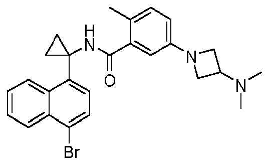

**C13:** ¹H NMR (600 MHz, DMSO-*d*₆) δ 9.06 (s, 1H), 8.77 - 8.71 (m, 1H), 8.22 - 8.16 (m, 1H), 7.86 (d, *J* = 7.7 Hz, 1H), 7.73 (d, *J* = 7.7 Hz, 1H), 7.72 - 7.67 (m, 2H), 6.92 (d, *J* = 8.2 Hz, 1H), 6.34 (dd, *J =* 8.2, 2.6 Hz, 1H), 6.11 (d, *J* = 2.6 Hz, 1H), 3.81 (t, *J* = 7.0 Hz, 2H), 3.42 (s, 2H), 3.15 (s, 1H), 2.25 - 2.02 (m, 6H), 1.89 (s, 3H), 1.38 - 1.34 (m, 2H), 1.19 (d, *J =* 5.7 Hz, 2H). ¹³C NMR (151 MHz, DMSO-*d*₆) δ 170.22, 149.98, 138.57, 137.94, 133.60, 131.72, 131.13, 129.80, 129.71, 127.80, 127.43, 127.21, 126.41, 123.24, 121.77, 112.75, 110.43, 56.65, 56.27, 41.91, 34.33, 18.41, 14.59. MS (ESI, m/z):C26H28BrN3O, [M+H]+478.149.

### Example 14:

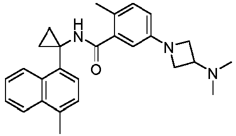

**C14:** 1H NMR (600 MHz, DMSO-d6) δ 8.98 (s, 1H), 8.72 - 8.66 (m, 1H), 8.06 - 8.00 (m, 1H), 7.69 (d, J = 7.2 Hz, 1H), 7.61 - 7.53 (m, 2H), 7.31 (dd, J = 7.2, 1.1 Hz, 1H), 6.91 (d, J = 8.2 Hz, 1H), 6.34 (dd, J = 8.2, 2.6 Hz, 1H), 6.11 (d, J = 2.6 Hz, 1H), 3.80 (t, J = 7.0 Hz, 2H), 3.42 (t, J = 6.6 Hz, 2H), 3.15 (s, 1H), 2.64 (s, 3H), 2.10 (s, 6H), 1.91 (s, 3H), 1.33 (q, J = 3.1 Hz, 2H), 1.14 (q, J = 4.5 Hz, 2H). 13C NMR (151 MHz, DMSO-d6) δ 170.03, 149.96, 138.14, 136.33, 133.91, 132.87, 132.33, 131.09, 128.56, 126.16, 125.79, 125.09, 123.31, 112.67, 110.51, 56.65, 56.26, 41.90, 34.48, 19.58, 18.47, 14.63. MS (ESI, m/z):C27H31N3O, [M+H]+414.254.

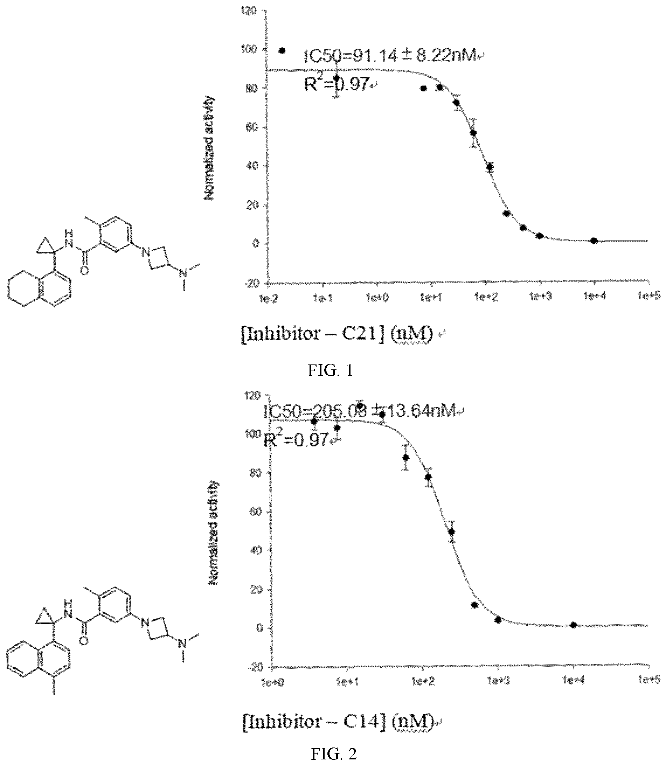

FIG. 2 shows the inhibition curve of compound C14.

### Example 15:

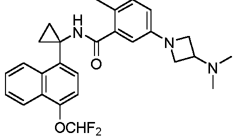

**C15:** ¹H NMR (600 MHz, DMSO-d6) δ 9.03 (s, 1H), 8.72 (d, J = 8.4 Hz, 1H), 8.12 (d, J = 8.4 Hz, 1H), 7.81 (d, J = 7.9 Hz, 1H), 7.71 - 7.62 (m, 2H), 7.55 - 7.29 (m, 1H), 7.27 (d, J = 7.9 Hz, 1H), 6.92 (d, J = 8.1 Hz, 1H), 6.34 (dd, J = 8.4, 2.5 Hz, 1H), 6.12 (d, J = 2.5 Hz, 1H), 3.80 (t, J *=* 7.0 Hz, 2H), 3.40 (t, J = 6.6 Hz, 2H), 3.11 (p, J = 6.3 Hz, 1H), 2.07 (s, 6H), 1.91 (s, 3H), 1.35 (s, 2H), 1.18 - 1.15 (m, 2H). 13C NMR (151 MHz, DMSO-d6) δ 170.18, 150.02, 146.55, 131.13, 128.76, 127.21, 126.73, 125.87, 121.98, 117.32 (t, J *=* 257.8 Hz), 112.65, 110.44, 56.75, 56.30, 41.98, 14.58. MS (ESI, m/z):C27H29F2N3O, [M+H]+466.230.

### Example 16:

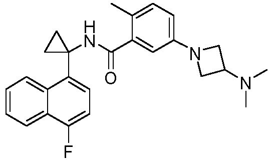

**C16:** ¹H NMR (400 MHz, DMSO-*d*₆) δ 9.02 (s, 1H), 8.72 (d, J = 6.5 Hz, 1H), 8.07 (d, J = 6.5 Hz, 1H), 7.78 (s, 1H), 7.67 (t, J = 8.7 Hz, 2H), 7.28 (t, J = 8.5 Hz,1H), 6.90 (d, J = 7.6 Hz, 1H), 6.34 (d, J = 7.2 Hz, 1H), 6.11 (s, 1H), 3.79 (s, 2H), 3.10 (s, 1H), 2.06 (s, 6H), 1.87 (s, 3H), 1.33 (s, 2H), 1.23 (s, 2H), 1.16 (s, 2H). ¹³C NMR (101 MHz, DMSO-*d*₆) δ 169.69, 153.27 (d, J_{H-F} = 245.2 Hz), 149.57, 137.55, 132.64 (d, J_{H-F} = 23.8 Hz), 130.65, 129.11, 128.66, 128.57, 125.41, 126.87, 126.21, 125.40 (d, J_{H-F} = 15.2 Hz), 122.73, 112.24, 109.94, 56.30, 55.84, 41.53, 33.64, 17.93, 14.08. MS (ESI, m/z): C₂₆H₂₈FN₃O, [M+H] +418.221.

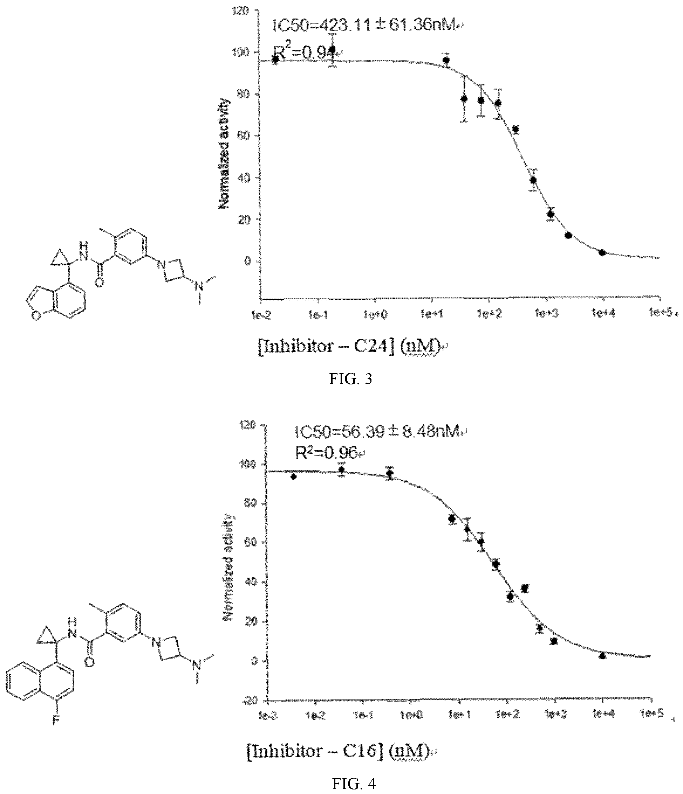

FIG. 4 shows the inhibition curve of compound C16.

### Example 17:

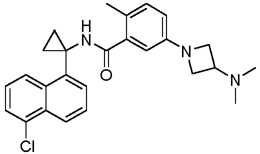

**C17:** ¹H NMR (400 MHz, Methanol-d4) δ 8.61 (d, J = 8.4 Hz, 1H), 8.26 (d, J = 8.6 Hz, 1H), 8.02 (d, J = 7.2 Hz, 1H), 7.84 - 7.37 (m, 3H), 7.01 (d, J = 7.9 Hz, 1H), 6.48 (d, J = 7.9 Hz, 1H), 6.26 (s, 1H), 4.07 (d, J = 8.4 Hz, 2H), 3.81 (s, 2H), 3.33 (s, 6H), 2.82 (s, 3H), 1.98 (s, 2H), 1.48 (s, 2H). ¹³C NMR (100 MHz, Methanol-d4) δ 168.39, 147.15, 134.48, 133.47, 133.09, 132.97, 132.49, 132.11, 130.49, 127.97, 126.87, 125.47, 125.09, 125.04, 123.29, 116.05, 112.55, 61.03, 49.74, 42.05, 35.70, 20.80, 18.80. MS(ESI, m/z): C26H28ClN3O, [M+H]+434.191.

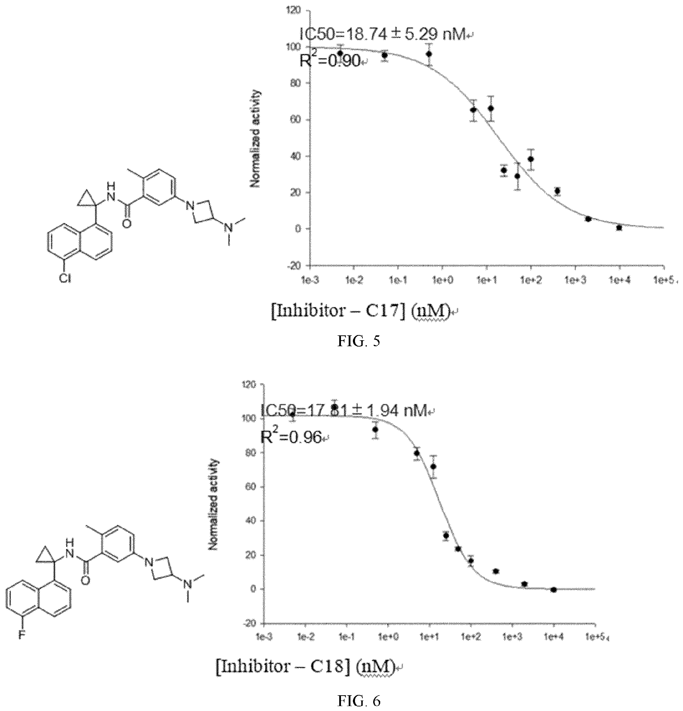

FIG. 5 shows the inhibition curve of compound C17.

### Example 18:

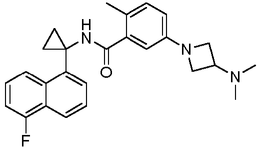

**C18:** ¹H NMR (400 MHz, Methanol-d4) δ 8.42 (d, J = 8.5 Hz, 1H), 8.02 (dd, J = 28.6, 7.7 Hz, 2H), 7.65 - 7.44 (m, 2H), 7.23 (t, J = 9.1 Hz, 1H), 6.97 (d, J = 8.1 Hz, 1H), 6.44 (d, J = 8.2 Hz, 1H), 6.24 (s, 1H), 3.96 (t, J = 7.3 Hz, 2H), 3.70 - 3.41 (m, 3H), 3.33 (s, 3H), 2.45 (s, 6H), 1.96 (s, 2H), 1.46 (s, 2H). ¹³C NMR (100 MHz, Methanol-d4) δ 168.39, 161.79, 159.27, 147.15, 134.48, 134.38, 134.35, 132.97, 132.11, 132.03, 130.49, 126.98, 126.90, 125.47, 125.44, 123.77, 123.57, 123.53, 123.45, 121.61, 121.58, 116.05, 112.69, 112.55, 112.49, 61.03, 49.74, 42.05, 35.98, 20.80, 18.80. MS(ESI, m/z): C26H28FN3O, [M+H]+418.223.

FIG. 6 shows the inhibition curve of compound C18.

### Example 19:

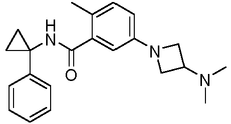

**C19:** ¹H NMR (600 MHz, DMSO-*d*₆) δ 8.88 (s, 1H), 7.30 (t, *J* = 7.6 Hz, 2H), 7.26 - 7.20 (m, 2H), 7.18 (t, *J* = 7.5 Hz, 1H), 7.06 (d, *J* = 8.3 Hz, 1H), 6.47 (d, *J* = 6.8 Hz, 2H), 4.03 (s, 2H), 3.79 (s, 2H), 3.47 (s, 1H), 2.20 (s, 3H), 1.24 (s, 4H). ¹³C NMR (151 MHz, DMSO-*d*₆) δ 170.20, 143.96, 137.91, 131.41, 128.85, 128.50, 126.83, 126.01, 125.11, 124.57, 113.17, 110.89, 55.97, 54.13, 52.84, 34.63, 19.58, 18.92, 18.67. MS (ESI, m/z):C22H27N3O, [M+H]+350.222.

### Example 20:

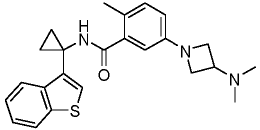

**C20:** ¹H NMR (600 MHz, DMSO-*d*₆) δ 9.11 (s, 1H), 8.32 (d, *J =* 8.0 Hz, 1H), 7.96 (d, *J =* 8.0 Hz, 1H), 7.67 (s, 1H), 7.43 (t, *J* = 7.5 Hz, 1H), 7.38 (d, *J* = 7.7 Hz, 1H), 7.00 (d, *J* = 8.2 Hz, 1H), 6.43 (dd, *J* = 8.1, 2.6 Hz, 1H), 6.27 (d, *J* = 2.6 Hz, 1H), 4.18 (d, *J* = 8.0 Hz, 1H), 4.01 (t, *J* = 7.8 Hz, 2H), 3.91 (dd, *J* = 8.6, 5.6 Hz, 2H), 2.71 (s, 6H), 2.51 (d, *J* = 4.5 Hz, 3H), 1.98 (s, 3H), 1.21 (d, *J* = 4.2 Hz, 2H), 1.18 (d, *J* = 4.3 Hz, 2H). ¹³C NMR (151 MHz, DMSO-*d*₆) δ 169.94, 148.98, 140.23, 138.79, 138.20, 137.75, 131.24, 125.73, 124.69, 124.48, 124.31, 123.50, 123.41, 113.14, 110.90, 55.19, 54.27, 40.52, 40.08, 30.79, 18.55, 14.13. MS (ESI, m/z):C24H27N3OS, [M+H]+406.194.

### Example 21:

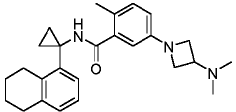

**C21:** ¹H NMR (600 MHz, DMSO-*d*₆) δ 8.68 (s, 1H), 7.40 (dd, *J =* 7.6, 1.4 Hz, 1H), 7.01 (t, *J =* 7.5 Hz, 1H), 6.95 (dd, *J =* 14.7, 7.9 Hz, 2H), 6.37 (dd, *J =* 8.2, 2.5 Hz, 1H), 6.20 (d, *J =* 2.5 Hz, 1H), 3.87 (t, *J =* 7.1 Hz, 2H), 3.49 (t, *J =* 6.5 Hz, 2H), 3.23 (s, 1H), 2.97 (t, *J* = 6.2 Hz, 2H), 2.74 (t, *J =* 6.2 Hz, 2H), 2.15 (s, 6H), 2.03 (s, 3H), 1.75 (ddt, *J =* 18.6, 11.3, 3.7 Hz, 4H), 1.12 (q, *J* = 4.7, 4.2 Hz, 2H), 1.01 (t, *J* = 3.5 Hz, 2H). ¹³C NMR (151 MHz, DMSO-*d*₆) δ 169.95, 149.94, 139.85, 138.19, 137.24, 136.73, 131.11, 129.08, 128.34, 124.73, 123.43, 112.65, 110.58, 56.56, 56.28, 41.85, 34.82, 29.84, 26.33, 23.28, 23.08, 18.50, 14.59. MS (ESI, m/z):C26H33N3O, [M+H]+404.269.

FIG. 1 shows the inhibition curve of compound C21.

### Example 22:

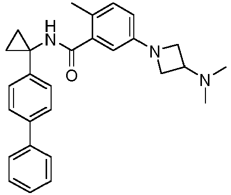

**C22:** ¹H NMR (600 MHz, DMSO-*d*₆) δ 8.92 (s, 1H), 7.70 - 7.64 (m, 2H), 7.63 - 7.59 (m, 2H), 7.50 - 7.40 (m, 2H), 7.35 (td, *J = 7.3, 1.3* Hz, 1H), 7.34 - 7.30 (m, 2H), 7.04 (d, *J =* 8.1 Hz, 1H), 6.47 (d, *J =* 2.5 Hz, 1H), 6.43 (dd, *J =* 8.1, 2.5 Hz, 1H), 3.93 (t, *J =* 7.0 Hz, 2H), 3.54 (t, *J =* 6.4 Hz, 2H), 3.20 (s, 1H), 2.21 (s, 3H), 2.13 (s, 6H), 1.31 - 1.26 (m, 4H). ¹³C NMR (151 MHz, DMSO-*d*₆) δ 170.41, 150.16, 143.40, 140.42, 137.88, 137.74, 131.37, 129.44, 129.38, 127.66, 126.93, 126.81, 125.70, 123.65, 112.91, 110.64, 56.79, 56.36, 41.99, 34.50, 18.95, 18.77. MS (ESI, m/z):C28H31N3O, [M+H]+426.254.

### Example 23:

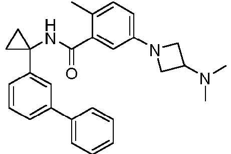

**C23:** ¹H NMR (400 MHz, Chloroform-d) δ 7.61 - 7.51 (m, 4H), 7.47 - 7.29 (m, 6H), 7.03 (d, *J =* 8.2 Hz, 1H), 6.55 - 6.40 (m, 3H), 3.96 (t, *J =* 6.9 Hz, 2H), 3.70 - 3.62 (m, 2H), 3.28 (q, *J =* 6.3 Hz, 1H), 3.10 (q, *J =* 7.4 Hz, 22H), 2.32 (s, 3H), 2.23 (s, 6H), 1.39 (s, 4H). MS (ESI, m/z):C28H31N3O, [M+H]+426.254.

### Example 24:

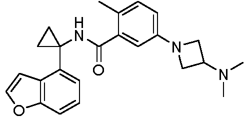

**C24:** ¹H NMR (600 MHz, DMSO-d6) δ 9.04 (s, 1H), 8.00 (d, J = 2.2 Hz, 1H), 7.46 (d, J = 8.1 Hz, 1H), 7.34 (d, J = 2.2 Hz, 1H), 7.30 (d, J = 7.5 Hz, 1H), 7.24 (t, J = 7.8 Hz, 1H), 6.97 (d, J = 8.2 Hz, 1H), 6.39 (dd, J = 8.2, 2.6 Hz, 1H), 6.26 (d, J = 2.6 Hz, 1H), 3.88 (t, J = 7.1 Hz, 2H), 3.52 (t, J = 6.3 Hz, 2H), 2.88 (s, 1H), 2.17 (s, 6H), 2.02 (s, 3H), 1.23 (q, J = 2.5 Hz, 4H). 13C NMR (151 MHz, DMSO-d6) δ 170.05, 154.97, 149.94, 145.67, 137.98, 136.48, 131.20, 126.61, 124.13, 123.48, 122.29, 112.78, 110.52, 110.26, 106.70, 56.27, 41.83, 34.48, 18.55, 16.96, 15.04. MS (ESI, m/z):C24H27N3O2, [M+H]+390.217.

FIG. 3 shows the inhibition curve of compound C24.

### Example 25:

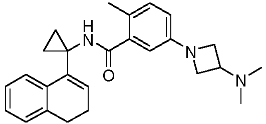

**C25:** ¹H NMR (600 MHz, DMSO-d6) δ 8.56 (s, 1H), 7.79 (d, J = 7.7 Hz, 1H), 7.19 (td, J = 7.2, 2.2 Hz, 1H), 7.15 - 7.09 (m, 2H), 6.91 (d, J = 8.2 Hz, 1H), 6.33 (dd, J = 8.2, 2.6 Hz, 1H), 6.26 (t, J = 4.6 Hz, 1H), 6.10 (d, J = 2.5 Hz, 1H), 3.81 (t, J = 7.0 Hz, 2H), 3.42 (t, J = 6.5 Hz, 2H), 3.17 (s, 1H), 2.65 (t, J = 8.0 Hz, 2H), 2.22 (td, J = 8.0, 4.6 Hz, 2H), 2.11 (s, 6H), 1.92 (s, 3H), 1.08 (q, J = 4.6 Hz, 2H), 0.95 (q, J = 4.7 Hz, 2H). ¹³C NMR (151 MHz, DMSO-d6) δ 169.96, 149.94, 138.36, 136.76, 136.45, 134.00, 131.02, 128.08, 127.76, 126.86, 126.37, 124.66, 123.36, 112.55, 110.51, 56.70, 56.29, 43.52, 41.92, 34.25, 27.87, 22.96, 19.04, 18.28, 13.49. MS (ESI, m/z):C26H31N3O, [M+H]+402.254..

### Example 26:

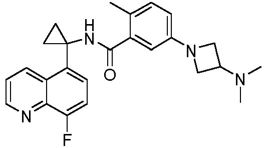

**C26:** ¹H NMR (400 MHz, DMSO) δ 9.13 (dt, J = 8.7, 1.7 Hz, 1H), 9.05 (s, 1H), 8.98 (dd, J = 4.2, 1.6 Hz, 1H), 7.85 (dd, J = 8.1, 5.1 Hz, 1H), 7.71 (dd, J = 8.7, 4.1 Hz, 1H), 7.54 (dd, J = 10.8, 8.0 Hz, 1H), 6.91 (d, J = 8.2 Hz, 1H), 6.34 (dd, J = 8.1, 2.6 Hz, 1H), 6.12 (d, J = 2.5 Hz, 1H), 3.81 (t, J = 7.0 Hz, 2H), 3.40 (dd, J = 7.4, 5.7 Hz, 2H), 3.11 (p, J = 6.1 Hz, 1H), 2.06 (s, 6H), 1.86 (s, 3H), 1.35 (q, J = 4.7 Hz, 2H), 1.27 - 1.16 (m, 2H). ¹⁹F NMR (376 MHz, DMSO) δ -126.10.

MS (ESI, m/z)C25H27F4O[M+H]+ 419.217

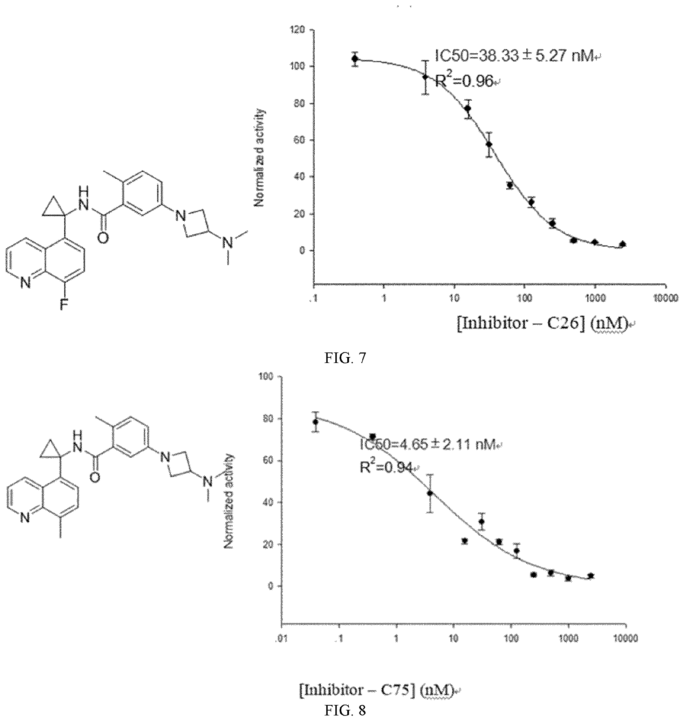

FIG. 7 shows the inhibition curve of compound C26.

### Example 27:

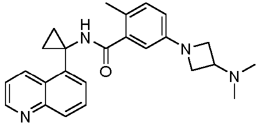

**C27:** ¹H NMR (600 MHz, DMSO) δ 9.20 (d, *J* = 5.1 Hz, 1H), 8.88 (d, *J* = 4.4 Hz, 1H), 8.67 (d, *J* = 8.4 Hz, 1H), 8.05 (d, *J =* 8.4 Hz, 1H), 7.77 (ddd, *J =* 8.4, 6.8, 1.5 Hz, 1H), 7.70 (d, *J* = 4.4 Hz, 1H), 7.69 - 7.64 (m, 1H), 6.95 (d, *J* = 8.2 Hz, 1H), 6.39 (dd, *J* = 8.2, 2.6 Hz, 1H), 6.18 (d, *J* = 2.6 Hz, 1H), 3.91 (d, *J* = 7.0 Hz, 2H), 3.66 (s, 2H), 3.07 (dp, *J* = 7.7, 4.0 Hz, 1H), 2.42 (s, 6H), 1.88 (s, 3H), 1.42 - 1.32 (m, 2H), 1.28 - 1.23 (m, 2H). ¹³C NMR (151 MHz, DMSO) δ 170.31, 150.66, 149.45, 148.73, 146.85, 137.83, 131.20, 130.11, 129.37, 127.44, 126.63, 125.90, 123.87, 123.01, 113.04, 110.63, 55.72, 55.39, 45.95, 40.96, 40.53, 33.94, 18.38, 14.07, 8.98. MS (ESI, m/z):C25H28N4O, [M+H]+401.234.

### Example 28:

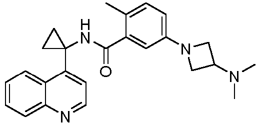

**C28:** ¹H NMR (600 MHz, DMSO) δ 9.20 (d, *J =* 7.8 Hz, 1H), 8.88 (d, *J* = 4.4 Hz, 1H), 8.66 (d, *J* = 8.4 Hz, 1H), 8.06 (d, *J =* 8.4 Hz, 1H), 7.80 - 7.74 (m, 1H), 7.74 - 7.64 (m, 2H), 6.98 (dd, *J =* 8.3, 2.1 Hz, 1H), 6.42 (dd, *J* = 7.9, 2.5 Hz, 1H), 6.20 (d, *J = 2.5 Hz,* 1H), 4.11 (s, 1H), 3.98 (t, *J* = 7.5 Hz, 2H), 3.89 - 3.78 (m, 2H), 2.69 (s, 6H), 1.90 (d, *J* = 2.5 Hz, 3H), 1.37 (q,*J* = 5.0 Hz, 2H), 1.26 (q, *J* = 5.1 Hz, 2H). ¹³C NMR (151 MHz, DMSO) δ 170.24, 150.67, 149.08, 148.72, 146.83, 137.94, 131.25, 130.11, 129.40, 127.44, 126.67, 125.88, 123.06, 113.24, 110.78, 54.54, 33.94, 18.39, 14.11. MS (ESI, m/z):C25H28N4O, [M+H]+401.233.

### Example 29:

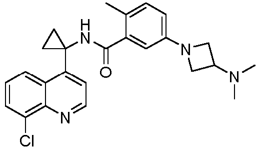

**C29:** ¹H NMR (600 MHz, DMSO) δ 9.16 (dd, *J =* 8.6, 1.6 Hz, 1H), 9.11 (d, *J =* 2.8 Hz, 1H), 9.05 (dd, *J* = 4.1, 1.6 Hz, 1H), 7.93 (d, *J* = 7.8 Hz, 1H), 7.85 (d, *J* = 7.7 Hz, 1H), 7.73 (dd, *J* = 8.6, 4.1 Hz, 1H), 6.96 (d, *J =* 8.2 Hz, 1H), 6.39 (dd, *J* = 8.2, 2.6 Hz, 1H), 6.16 (d, *J* = 2.5 Hz, 1H), 3.91 (t, *J*= 7.2 Hz, 2H), 3.64 (s, 2H), 2.51 - 2.38 (m, 6H), 1.88 (s, 3H), 1.39 - 1.33 (m, 2H), 1.22 (q, *J* = 4.7 Hz, 2H). ¹³C NMR (151 MHz, DMSO) δ 170.15, 151.15, 149.49, 144.22, 138.42, 138.01, 134.61, 132.39, 131.20, 129.45, 129.12, 128.76, 123.74, 122.43, 113.03, 110.52, 55.90, 55.48, 33.72, 18.36, 14.46, 9.09. MS (ESI, m/z):C25H27ClN4O, [M+H]+435.195.

### Example 30:

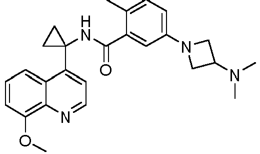

**C30:** ¹H NMR (600 MHz, DMSO) δ 9.11 (s, 1H), 8.80 (d, *J* = 4.3 Hz, 1H), 8.17 (d, *J* = 8.5 Hz, 1H), 7.71 (d, *J* = 4.3 Hz, 1H), 7.56 (t, *J =* 8.1 Hz, 1H), 7.18 (d, *J =* 7.7 Hz, 1H), 6.93 (d, *J =* 8.2 Hz, 1H), 6.36 (dd, *J =* 8.2, 2.5 Hz, 1H), 6.15 (d, *J =* 2.5 Hz, 1H), 3.96 (s, 3H), 3.84 (t, *J =* 7.2 Hz, 2H), 3.48 (s, 2H), 3.24 (s, 1H), 2.18 (s, 6H), 1.88 (s, 3H), 1.34 (t, *J =* 3.4 Hz, 2H), 1.22 (q, *J =* 5.0 Hz, 2H). ¹³C NMR (151 MHz, DMSO) δ 170.34, 156.19, 149.86, 148.87, 146.49, 140.70, 137.77, 131.15, 128.58, 123.53, 123.43, 117.33, 112.86, 110.49, 108.25, 56.18, 56.11, 41.49 , 34.24, 18.38, 14.11. MS (ESI, m/z):C26H30N4O2, [M+H]+431.244.

### Example 31:

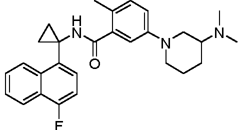

**C31:** ¹H NMR (600 MHz, DMSO) δ 9.07 (s, 1H), 8.74 (d, *J* = 8.4 Hz, 1H), 8.08 (d, *J* = 8.2 Hz, 1H), 7.80 (dd, *J* = 8.0, 5.5 Hz, 1H), 7.72 - 7.62 (m, 2H), 7.29 (dd, *J* = 10.6, 7.9 Hz, 1H), 7.00 (d, *J =* 8.5 Hz, 1H), 6.90 (dd, *J* = 8.3, 2.6 Hz, 1H), 6.66 (d, *J* = 2.7 Hz, 1H), 3.82 - 3.76 (m, 1H), 3.46 - 3.41 (m, 1H), 3.22 (s, 1H), 2.82 (d, *J =* 11.2 Hz, 1H), 2.73 (s, 6H), 2.60 (t, *J =* 10.5 Hz, 1H), 2.06 (s, 1H), 1.93 (s, 3H), 1.83 - 1.77 (m, 1H), 1.55 (h, *J* = 6.9 Hz, 2H), 1.37 (q, *J* = 3.1 Hz, 2H), 1.18 (d, *J* = 5.4 Hz, 2H). ¹³C NMR (151 MHz, DMSO) δ 170.01, 158.54, 156.88, 148.56, 138.21, 134.51 (d, *J* = 4.0 Hz), 133.52 (d, *J =* 4.6 Hz), 131.40, 129.12 (d, *J* = 8.3 Hz), 127.36, 126.69, 126.11, 125.85, 123.42 (d, *J* = 16.0 Hz), 120.85 (d, *J* = 5.3 Hz), 117.95, 115.71, 109.08, 108.96, 60.98, 49.72, 49.55, 34.14, 24.55, 23.25, 18.42, 14.59. MS (ESI, m/z):C28H32FN3O, [M+H]+446.260.

### Example 32:

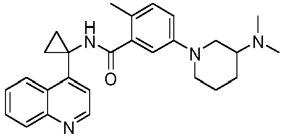

**C32:** ¹H NMR (600 MHz, DMSO) δ 9.19 (s, 1H), 8.88 (d, *J* = 4.4 Hz, 1H), 8.68 (d, *J* = 8.4 Hz, 1H), 8.06 (d, *J =* 8.4 Hz, 1H), 7.77 (ddd, *J* = 8.4, 6.7, 1.4 Hz, 1H), 7.71 (d, *J* = 4.4 Hz, 1H), 7.66 (ddd, *J* = 8.3, 6.7, 1.3 Hz, 1H), 7.01 (d, *J* = 8.4 Hz, 1H), 6.92 (dd, *J* = 8.4, 2.6 Hz, 1H), 6.67 (d, *J* = 2.8 Hz, 1H), 3.75 (d, *J =* 11.8 Hz, 1H), 3.44 (d, *J* = 12.3 Hz, 1H), 3.24 (s, 1H), 2.83 (t, *J* = 11.0 Hz, 2H), 2.76 (s, 6H), 2.63 (t, *J =* 11.3 Hz, 2H), 2.07 - 2.03 (m, 1H), 1.92 (s, 3H), 1.81 (dd, *J =* 10.3, 6.0 Hz, 1H), 1.64 - 1.49 (m, 3H), 1.39 (q, *J*= 4.0 Hz, 2H), 1.26 (q, *J* = 4.3 Hz, 2H). ¹³C NMR (151 MHz, DMSO) δ 170.26, 150.67, 148.74, 148.59, 146.84, 137.94, 131.44, 130.14, 129.37, 127.45, 126.64, 126.17, 125.87, 123.04, 118.09, 115.73, 61.08, 49.72, 49.55, 40.53, 33.99, 24.54, 23.17, 18.39, 14.09. MS (ESI, m/z):C27H32N4O, [M+H]+429.265.

### Example 33:

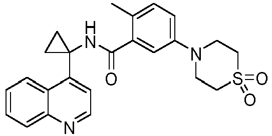

**C33:** ¹H NMR (600 MHz, DMSO) δ 9.17 (s, 1H), 8.88 (d, *J =* 4.3 Hz, 1H), 8.66 (dd, *J =* 8.5, 1.4 Hz, 1H), 8.06 (dd, *J =* 8.4, 1.2 Hz, 1H), 7.77 (ddd, *J* = 8.3, 6.7, 1.4 Hz, 1H), 7.70 (d, *J =* 4.3 Hz, 1H), 7.65 - 7.57 (m, 1H), 7.01 (d, *J =* 8.4 Hz, 1H), 6.95 (dd, *J* = 8.4, 2.7 Hz, 1H), 6.68 (d, *J* = 2.7 Hz, 1H), 3.70 - 3.65 (m, 4H), 3.10 (dd, *J* = 6.7, 3.7 Hz, 4H), 1.91 (s, 3H), 1.39 (t,*J* = 3.5 Hz, 2H), 1.27 (q, *J =* 4.4 Hz, 2H). ¹³C NMR (151 MHz, DMSO) δ 170.12, 150.65, 148.73, 146.85, 145.69, 138.01, 131.74, 130.15, 129.42, 127.42, 126.69, 126.11, 125.79, 122.93, 117.37, 114.93, 50.18, 47.36, 34.06, 18.31, 14.03. MS (ESI, m/z):C24H25N3O3S, [M+H]+436.169.

### Example 34:

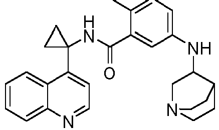

**C34:** ¹H NMR (600 MHz, DMSO) δ 9.16 (d, *J* = 5.1 Hz, 1H), 8.87 (d, *J* = 4.4 Hz, 1H), 8.66 (d, *J* = 8.6 Hz, 1H), 8.05 (d, *J =* 8.4 Hz, 1H), 7.77 (ddd, *J =* 8.3, 6.8, 1.4 Hz, 1H), 7.70 (d, *J =* 4.4 Hz, 1H), 7.67 (ddd, *J =* 8.3, 6.9, 1.3 Hz, 1H), 6.86 (d, *J =* 8.3 Hz, 1H), 6.50 (d, *J =* 8.4 Hz, 1H), 6.28 (d, *J =* 2.7 Hz, 1H), 3.61 (d, *J =* 7.6 Hz, 1H), 3.47 (d, *J =* 11.7 Hz, 1H), 3.08 (s, 1H), 3.01 (s, 1H), 2.69 (d, *J =* 12.8 Hz, 1H), 2.00 (d, *J =* 4.5 Hz, 1H), 1.92 (s, 1H), 1.87 (s, 3H), 1.79 (s, 1H), 1.72 (s, 1H), 1.53 (s, 1H), 1.35 (s, 2H), 1.25 (s, 2H), 1.23 (dd, *J* = 7.2, 4.6 Hz, 2H). ¹³C NMR (151 MHz, DMSO) δ 170.62, 150.65, 148.73, 146.87, 145.39, 138.20, 131.33, 130.10, 129.37, 127.46, 126.70, 125.88, 123.01, 122.20, 113.49, 111.69, 53.25, 47.69, 46.22, 45.71, 40.53, 33.85, 23.90, 22.62, 18.24, 17.81, 14.11. MS (ESI, m/z):C27H30N3O, [M+H]+427.249.

### Example 35:

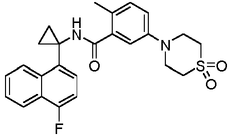

**C35:** ¹H NMR (600 MHz, DMSO) δ 9.04 (s, 1H), 8.71 (d, *J =* 8.5 Hz, 1H), 8.10 - 8.06 (m, 1H), 7.79 (dd, *J =* 8.0, 5.6 Hz, 1H), 7.71 (ddd, *J* = 8.4, 6.8, 1.4 Hz, 1H), 7.65 (ddd, *J* = 8.1, 6.8, 1.1 Hz, 1H), 7.29 (dd, *J* = 10.7, 7.9 Hz, 1H), 7.00 (d, *J =* 8.4 Hz, 1H), 6.94 (dd, *J =* 8.4, 2.7 Hz, 1H), 6.66 (d, *J =* 2.7 Hz, 1H), 3.69 - 3.64 (m, 4H), 3.09 (dd, *J =* 6.7, 3.7 Hz, 4H), 1.92 (s, 3H), 1.36 (t, *J* = 3.0 Hz, 2H), 1.19 (q, *J* = 4.8 Hz, 2H). ¹³C NMR (151 MHz, DMSO) δ 169.88, 158.54, 156.89, 145.70, 138.35, 134.48, 134.45, 133.50, 133.47, 131.69, 129.05 (d, *J =* 8.4 Hz), 127.40, 126.72, 126.10, 125.76, 123.44 (d, *J =* 16.3 Hz), 120.88 (d, *J* = 5.4 Hz), 117.26, 114.97, 109.01 (d, *J* = 19.3 Hz), 50.19, 47.39, 40.53, 34.20, 18.33, 14.51. MS (ESI, m/z):C25H25FN2O3S, [M+H]+453.164.

### Example 36:

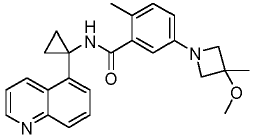

**C36:** ¹H NMR (600 MHz, DMSO) δ 9.16 (s, 1H), 8.87 (d, *J* = 4.3 Hz, 1H), 8.67 (d, *J* = 8.4 Hz, 1H), 8.05 (d, *J* = 8.4 Hz, 1H), 7.77 (dd, *J =* 8.5, 6.8 Hz, 1H), 7.71 - 7.63 (m, 2H), 6.93 (d, *J* = 8.1 Hz, 1H), 6.37 (dd, *J* = 8.2, 2.6 Hz, 1H), 6.16 (d,*J* = 2.5 Hz, 1H), 3.59 (q, *J* = 7.6 Hz, 4H), 3.16 (s, 3H), 2.69 (s, 1H), 1.88 (s, 3H), 1.44 (s, 3H), 1.37 (q, *J =* 5.1 Hz, 2H), 1.25 (q,*J* = 5.1 Hz, 2H). ¹³C NMR (151 MHz, DMSO) δ 170.35, 150.62, 149.73, 148.70, 146.92, 137.63, 131.18, 130.09, 129.38, 127.44, 126.61, 125.89, 123.44, 122.95, 113.05, 110.65, 73.24, 62.57, 62.53, 53.83, 50.68, 33.98, 22.52, 18.47, 18.39, 17.18, 14.04. MS (ESI, m/z):C25H27N3O2, [M+H]+402.218.

### Example 37:

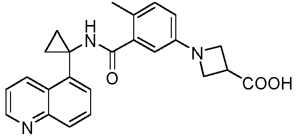

**C37:** ¹H NMR (600 MHz, DMSO) δ 9.15 (s, 1H), 8.87 (d, *J* = 4.4 Hz, 1H), 8.66 (dd, *J* = 8.5, 1.4 Hz, 1H), 8.05 (dd, *J* = 8.5, 1.3 Hz, 1H), 7.76 (ddd, *J* = 8.3, 6.7, 1.4 Hz, 1H), 7.70 (d, *J =* 4.4 Hz, 1H), 7.66 (ddd, *J* = 8.3, 6.7, 1.3 Hz, 1H), 6.85 (d, *J =* 8.3 Hz, 1H), 6.51 (dd, *J* = 8.2, 2.6 Hz, 1H), 6.32 (d, *J* = 2.6 Hz, 1H), 3.82 (qd, *J* = 10.9, 5.6 Hz, 2H), 3.31 - 3.27 (m, 1H), 3.16 (dd, *J* = 13.4, 7.5 Hz, 1H), 2.97 - 2.85 (m, 1H), 1.86 (s, 3H), 1.35 (t, *J* = 3.7 Hz, 2H), 1.24 (q, *J* = 5.4 Hz, 2H). ¹³C NMR (151 MHz, DMSO) δ 173.08, 170.65, 150.59, 148.65, 147.02, 146.28, 138.00, 131.30, 130.02, 129.40, 127.46, 126.70, 125.88, 122.99, 122.12, 113.07, 111.37, 47.03, 44.07, 42.54, 33.90, 18.27, 14.11. MS (ESI, m/z):C24H23N3O3, [M+H]+402.177.

### Example 38:

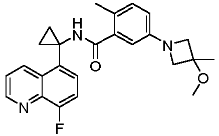

**C38:** ¹H NMR (600 MHz, DMSO) δ 9.14 (dt, *J* = 8.7, 1.5 Hz, 1H), 9.05 (s, 1H), 8.98 (dd, *J* = 4.1, 1.6 Hz, 1H), 7.85 (dd, *J =* 8.0, 5.0 Hz, 1H), 7.71 (dd, *J* = 8.6, 4.1 Hz, 1H), 7.54 (dd, *J =* 10.8, 8.0 Hz, 1H), 6.92 (d, *J* = 8.2 Hz, 1H), 6.36 (dd, *J* = 8.2, 2.6 Hz, 1H), 6.14 (d, *J =* 2.6 Hz, 1H), 3.58 (q, *J =* 7.7 Hz, 4H), 3.15 (s, 3H), 1.87 (s, 3H), 1.43 (s, 3H), 1.34 (t, *J =* 3.2 Hz, 2H), 1.20 (q, *J* = 4.8 Hz, 2H). ¹³C NMR (151 MHz, DMSO) δ 173.08, 170.65, 150.59, 148.65, 147.02, 146.28, 138.00, 131.30, 130.02, 129.40, 127.46, 126.70, 125.88, 122.99, 122.12, 113.07, 111.37, 47.03. ¹³C NMR (151 MHz, DMSO) δ 170.16, 157.95, 156.26, 150.67, 149.77, 138.35, 138.27, 137.88, 134.92, 134.89, 134.05, 131.17, 129.20, 129.15, 128.83, 123.30, 122.50, 112.98, 112.93, 112.81, 110.52, 73.24, 62.57, 50.68, 40.53, 38.72, 33.63, 22.52, 18.35, 14.39. MS (ESI, m/z):C25H26FN3O2, [M+H]+420.208.

### Example 39:

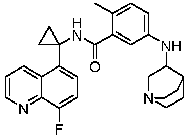

**C39:** ¹H NMR (699 MHz, dmso) δ 9.11 (d, *J =* 8.6 Hz, 1H), 9.00 (s, 1H), 8.95 (d, *J* = 4.2 Hz, 1H), 7.82 (dd, *J* = 8.0, 4.8 Hz, 1H), 7.68 (dd, *J =* 8.6, 4.1 Hz, 1H), 7.52 (dd, *J =* 10.6, 7.9 Hz, 1H), 6.79 (d, *J =* 8.2 Hz, 1H), 6.40 (dd, *J =* 8.3, 2.5 Hz, 1H), 6.21 (d, *J* = 2.5 Hz, 1H), 3.21 (s, 1H), 3.03 (t, *J =* 11.5 Hz, 1H), 2.70 (s, 1H), 2.58 (s, 1H), 2.31 (d, *J =* 14.0 Hz, 1H), 1.88 (d, *J =* 2.9 Hz, 1H), 1.82 (s, 3H), 1.76 - 1.72 (m, 1H), 1.66 (s, 1H), 1.52 (s, 1H), 1.41 (s, 1H), 1.29 (s, 2H), 1.17 (dd, *J =* 12.5, 7.6 Hz, 4H). MS (ESI, m/z):C27H29FN4O, [M+H]+445.240.

### Example 40:

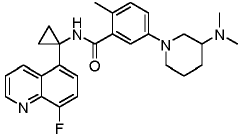

**C40:** ¹H NMR (600 MHz, DMSO) δ 9.17 (dt, *J =* 8.6, 1.6 Hz, 1H), 9.12 (s, 1H), 8.98 (dd, *J* = 4.1, 1.6 Hz, 1H), 7.85 (dd, *J =* 8.0, 5.0 Hz, 1H), 7.71 (dd, *J =* 8.7, 4.1 Hz, 1H), 7.54 (dd, *J =* 10.7, 8.0 Hz, 1H), 6.99 (d, *J =* 8.4 Hz, 1H), 6.89 (dd, *J =* 8.4, 2.7 Hz, 1H), 6.66 (d, *J* = 2.7 Hz, 1H), 3.79 (d, *J =* 11.8 Hz, 1H), 3.49 - 3.43 (m, 1H), 3.06 (s, 1H), 2.79 - 2.70 (m, 2H), 2.65 (s, 6H), 2.58 (ddd, *J* = 11.8, 8.6, 2.5 Hz, 2H), 2.06 - 2.02 (m, 1H), 1.91 (s, 3H), 1.79 (dt, *J* = 9.4, 3.3 Hz, 1H), 1.38 (q, *J =* 3.7 Hz, 2H), 1.20 (q, *J =* 4.3 Hz, 2H). ¹³C NMR (151 MHz, DMSO) δ 170.07, 157.96, 156.27, 150.66, 148.68, 138.36, 138.28, 138.03, 134.93, 134.89, 134.07, 131.42, 129.23, 129.18, 128.85, 125.83, 122.52, 117.86, 115.49, 112.93, 112.81, 60.84, 50.10, 49.53, 33.66, 24.88, 23.40, 18.38, 14.41. MS (ESI, m/z):C27H31FN4O, [M+H]+447.248.

### Example 41:

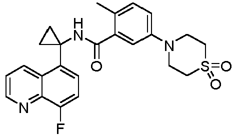

**C41:** ¹H NMR (600 MHz, DMSO) δ 9.12 (dt, *J* = 8.7, 1.6 Hz, 1H), 9.07 (s, 1H), 8.98 (dd, *J* = 4.1, 1.6 Hz, 1H), 7.85 (dd, *J =* 8.0, 5.0 Hz, 1H), 7.73 (dd, *J* = 8.6, 4.1 Hz, 1H), 7.54 (dd, *J =* 10.7, 8.0 Hz, 1H), 7.01 (d, *J* = 8.4 Hz, 1H), 6.94 (dd, *J =* 8.4, 2.8 Hz, 1H), 6.67 (d, *J* = 2.8 Hz, 1H), 3.69 - 3.64 (m, 4H), 3.11 - 3.06 (m, 4H), 1.90 (s, 3H), 1.42 - 1.35 (m, 2H), 1.22 (q,*J* = 4.9 Hz, 2H).¹³C NMR (151 MHz, DMSO) δ 169.93, 157.97, 156.28, 150.71, 145.76, 138.37, 138.30, 134.88, 134.85, 133.96, 131.70, 129.17, 129.12, 128.82, 125.96, 122.53, 117.28, 114.86, 112.94, 112.81, 50.20, 47.39, 40.53, 33.71, 18.27, 14.38. MS (ESI, m/z):C24H24FN3O3S, [M+H]+454.153.

### Example 42:

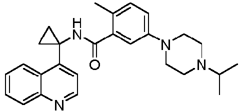

**C42:** ¹H NMR (699 MHz, dmso) δ 9.17 (s, 1H), 8.86 (d, *J =* 4.3 Hz, 1H), 8.64 (d, *J* = 8.5 Hz, 1H), 8.04 (d, *J =* 8.4 Hz, 1H), 7.75 (t, *J =* 7.5 Hz, 1H), 7.68 (d, *J =* 4.4 Hz, 1H), 7.64 (t, *J =* 7.7 Hz, 1H), 7.02 (d, *J =* 8.2 Hz, 1H), 6.91 (d, *J =* 8.5 Hz, 1H), 6.64 (s, 1H), 3.72 (d, *J=* 13.0 Hz, 3H), 3.46 (d, *J =* 10.9 Hz, 4H), 3.08 (d, *J =* 11.7 Hz, 1H), 2.86 (d, *J =* 12.7 Hz, 1H), 1.90 (s, 3H), 1.35 (s, 2H), 1.26 (d, *J =* 6.6 Hz, 6H), 1.25 - 1.24 (m, 2H). MS (ESI, m/z):C27H32N4O, [M+H]+429.265.

### Example 43:

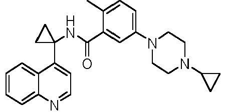

**C43:** ¹H NMR (699 MHz, dmso) δ 9.14 (s, 1H), 8.85 (d, *J =* 4.3 Hz, 1H), 8.64 (d, *J =* 8.4 Hz, 1H), 8.03 (d, *J =* 8.4 Hz, 1H), 7.75 (t, *J=* 7.7 Hz, 1H), 7.67 (d, *J =* 4.4 Hz, 1H), 7.63 (t, *J =* 7.6 Hz, 1H), 6.93 (d, *J =* 8.3 Hz, 1H), 6.82 - 6.80 (m, 1H), 6.57 (s, 1H), 2.94 (s, 3H), 2.60 (s, 3H), 1.87 (s, 3H), 1.61 (s, 1H), 1.35 (q, *J* = 5.0 Hz, 3H), 1.23 (s, 3H), 0.41 (s, 2H), 0.31 (s, 2H). MS (ESI, m/z):C27H30N4O, [M+H]+427.249.

### Example 44:

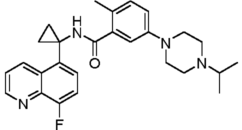

**C44:** ¹H NMR (699 MHz, dmso) δ 9.78 (s, 1H), 9.11 (d, *J* = 8.7 Hz, 1H), 9.08 (s, 1H), 8.96 (d, *J* = 4.1 Hz, 1H), 7.83 (dd,*J* = 7.9, 4.8 Hz, 1H), 7.69 (dd, *J =* 8.6, 4.1 Hz, 1H), 7.53 (dd, *J =* 10.6, 7.9 Hz, 1H), 7.01 (d, *J =* 8.4 Hz, 1H), 6.90 (dd, *J =* 8.3, 2.7 Hz, 1H), 6.63 (d, *J* = 2.8 Hz, 1H), 3.71 (d, J= 12.9 Hz, 2H), 3.49 (d, *J* = 9.8 Hz, 1H), 3.44 (d, *J* = 12.0 Hz, 2H), 3.05 (t, *J* = 10.9 Hz, 2H), 2.92 (t, *J =* 12.5 Hz, 2H), 1.88 (s, 3H), 1.33 (d, *J*= 5.0 Hz, 2H), 1.27 (d, *J =* 6.6 Hz, 6H), 1.19 (s, 2H). MS (ESI, m/z):C27H31FN4O, [M+H]+447.256.

### Example 45:

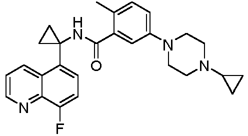

**C45:** ¹H NMR (699 MHz, dmso) δ 9.11 (d, *J =* 8.6 Hz, 1H), 9.03 (s, 1H), 8.95 (d, *J =* 4.0 Hz, 1H), 7.82 (dd, *J* = 7.8, 4.7 Hz, 1H), 7.68 (dd, *J* = 8.6, 4.1 Hz, 1H), 7.52 (dd, *J =* 10.8, 8.1 Hz, 1H), 6.93 (d, *J =* 8.3 Hz, 1H), 6.80 (dd, *J =* 8.3, 2.6 Hz, 1H), 6.56 (d, *J =* 2.8 Hz, 1H), 2.95 (d, *J =* 5.0 Hz, 4H), 2.60 (d, *J =* 5.0 Hz, 4H), 1.85 (s, 3H), 1.60 (s, 1H), 1.33 (s, 2H), 1.18 (d, *J* = 5.1 Hz, 2H), 0.41 (d, J= 6.5 Hz, 2H), 0.30 (s, 2H). MS (ESI, m/z):C27H29FN4O, [M+H]+445.239.

### Example 46:

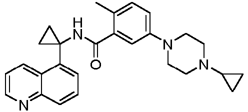

**C46:** ¹H NMR (699 MHz, dmso) δ 9.07 (d, *J =* 8.5 Hz, 1H), 9.05 (s, 1H), 8.90 (d, *J =* 4.0 Hz, 1H), 7.92 (d, *J =* 8.4 Hz, 1H), 7.85 (d, *J =* 7.1 Hz, 1H), 7.69 (t, *J =* 7.7 Hz, 1H), 7.57 (dd, *J =* 8.6, 4.1 Hz, 1H), 6.94 (d, *J =* 8.3 Hz, 1H), 6.81 (s, 1H), 6.56 (s, 1H), 2.94 (s, 3H), 2.60 (s, 2H), 1.87 (s, 4H), 1.60 (s, 1H), 1.34 (s, 3H), 1.18 (s, 3H), 0.41 (s, 2H), 0.30 (s, 2H). MS (ESI, m/z):C27H30N4O, [M+H]+427.249.

### Example 47:

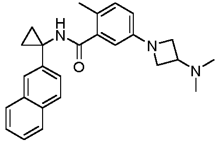

**C47:** ¹H NMR (699 MHz, dmso) δ 8.98 - 8.93 (m, 1H), 7.82 (h, *J =* 8.1 Hz, 2H), 7.75 - 7.70 (m, 1H), 7.44 (dq, *J =* 17.2, 8.1 Hz, 2H), 7.37 (q, *J =* 8.4 Hz, 1H), 7.01 (q, *J =* 8.1 Hz, 1H), 6.47 - 6.42 (m, 1H), 6.42 - 6.37 (m, 1H), 3.90 (t, *J=* 8.5 Hz, 2H), 3.54 (s, 1H), 2.46 (s, 4H), 2.21 - 1.97 (m, 9H), 1.36 - 1.31 (m, 2H), 1.30 - 1.25 (m, 2H). MS (ESI, m/z):C26H29N3O, [M+H]+400.238.

### Example 48:

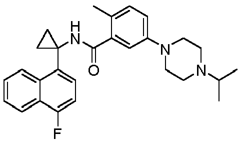

**C48:** ¹H NMR (600 MHz, DMSO) δ 9.08 (d, J = 4.9 Hz, 1H), 8.72 (d, J = 8.5 Hz, 1H), 8.09 (d, J = 8.2 Hz, 1H), 7.80 (dd, J = 7.9, 5.5 Hz, 1H), 7.72 - 7.62 (m, 2H), 7.30 (dd, J = 10.6, 7.9 Hz, 1H), 7.03 (d, J = 8.4 Hz, 1H), 6.92 (dd, J = 8.6, 2.9 Hz, 1H), 6.66 (d, J = 2.7 Hz, 1H), 3.72 (s, 2H), 3.48 (s, 2H), 3.46 (s, 1H), 3.09 (q, J = 10.6 Hz, 2H), 2.99 (s, 1H), 2.92 (t, J = 12.4 Hz, 1H), 1.93 (s, 3H), 1.36 (s, 2H), 1.29 (d, J *=* 6.6 Hz, 6H), 1.21 - 1.16 (m, 2H). MS (ESI, m/z):C28H32FN3O, [M+H]+446.260.

### Example 49:

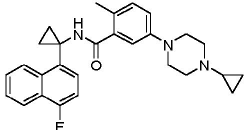

**C49:** ¹H NMR (699 MHz, dmso) δ 9.00 (d, *J =* 3.9 Hz, 1H), 8.71 - 8.67 (m, 1H), 8.04 (dd, *J =* 8.5, 3.7 Hz, 1H), 7.76 (d, *J =* 8.1 Hz, 1H), 7.65 (d, *J* = 8.5 Hz, 1H), 7.62 (d, *J* = 8.0 Hz, 1H), 7.26 (d, *J* = 10.1 Hz, 1H), 6.93 - 6.89 (m, 1H), 6.78 (d, *J* = 8.3 Hz, 1H), 6.54 (s, 1H), 2.93 (d, *J* = 6.3 Hz, 4H), 2.58 (d, *J =* 5.5 Hz, 4H), 1.87 (d, *J* = 3.8 Hz, 3H), 1.59 (s, 1H), 1.32 (s, 2H), 1.14 (s, 2H), 0.40 (t, *J =* 5.3 Hz, 2H), 0.29 (s, 2H). MS (ESI, m/z):C28H30FN3O, [M+H]+444.244

### Example 50:

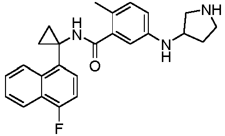

**C50:** ¹H NMR (600 MHz, DMSO) δ 9.04 (d, *J =* 6.0 Hz, 1H), 8.72 (d, *J =* 8.7 Hz, 1H), 8.08 (d, *J =* 8.2 Hz, 1H), 7.81 - 7.76 (m, 1H), 7.69 (q, *J =* 7.3 Hz, 2H), 7.67 - 7.61 (m, 2H), 7.30 - 7.26 (m, 1H), 6.89 (d, *J =* 8.2 Hz, 1H), 6.51 (s, 1H), 6.29 (d, *J* = 10.1 Hz, 1H), 1.89 (s, 3H), 1.33 (q, *J* = 6.0 Hz, 3H), 1.16 (d, *J* = 6.3 Hz, 3H). MS (ESI, m/z): C25H26FN3O, [M+H]+404.2138.

### Example 51:

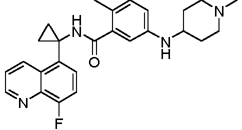

**C51:** ¹H NMR (600 MHz, DMSO) δ 9.13 (dt, *J* = 8.7, 1.6 Hz, 1H), 9.03 (s, 1H), 8.98 (dd, *J* = 4.1, 1.6 Hz, 1H), 7.85 (dd, *J =* 8.0, 5.0 Hz, 1H), 7.71 (dd, *J* = 8.6, 4.1 Hz, 1H), 7.54 (dd, *J =* 10.7, 8.0 Hz, 1H), 6.83 (d, *J =* 8.2 Hz, 1H), 6.48 (dd, *J* = 8.2, 2.5 Hz, 1H), 6.26 (d, *J* = 2.5 Hz, 1H), 5.45 (s, 1H), 3.24 (s, 1H), 3.06 (s, 2H), 2.58 (s, 1H), 2.49 (s, 3H), 1.89 (d, *J* = 12.4 Hz, 2H), 1.85 (s, 3H), 1.47 (s, 1H), 1.32 (q, *J* = 4.6 Hz, 2H), 1.19 (q, *J* = 4.8 Hz, 2H). MS (ESI, m/z): C26H29FN4O, [M+H]+433.232.

### Example 52:

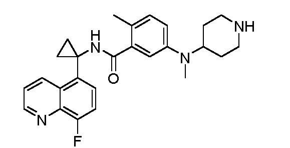

**C52:** ¹H NMR (600 MHz, DMSO) δ 9.16 (d, *J =* 8.6 Hz, 1H), 9.07 (s, 1H), 8.98 (t, *J* = 6.4 Hz, 1H), 7.84 (dd, *J* = 8.2, 4.7 Hz, 1H), 7.70 (td, *J* = 8.4, 4.2 Hz, 1H), 7.57 - 7.51 (m, 1H), 7.06 (d, *J* = 8.2 Hz, 1H), 6.94 (d, *J* = 8.4 Hz, 1H), 6.79 - 6.74 (m, 1H), 6.46 (d, *J =* 17.9 Hz, 1H), 3.78 (d, *J =* 12.4 Hz, 1H), 3.25 (d, *J =* 12.8 Hz, 2H), 2.87 (t, *J =* 12.5 Hz, 2H), 2.61 (s, 3H), 1.88 (s, 5H), 1.62 (d, *J =* 13.1 Hz, 2H), 1.36 (s, 2H), 1.20 (d, *J* = 6.1 Hz, 2H). MS (ESI, m/z): C26H29FN4O, [M+H]+433.232.

### Example 53:

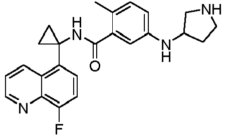

**C53:** ¹H NMR (600 MHz, DMSO) δ 9.16 (s, 1H), 9.08 (s, 1H), 8.99 (dt, *J =* 4.2, 2.3 Hz, 1H), 7.86 (ddd, *J =* 8.3, 4.8, 2.1 Hz, 1H), 7.74 (dq, *J =* 7.6, 4.1 Hz, 1H), 7.60 - 7.52 (m, 2H), 6.93 - 6.86 (m, 2H), 6.52 (s, 1H), 6.30 (s, 1H), 1.87 (s, 3H), 1.34 (t, *J =* 2.9 Hz, 3H), 1.20 (dd, *J =* 6.9, 4.8 Hz, 3H). MS (ESI, m/z):C24H25FN4O , [M+H]+405.208.

### Example 54:

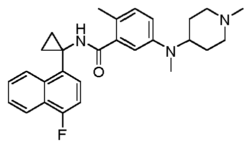

**C54:** ¹H NMR (600 MHz, DMSO) δ 9.03 (s, 1H), 8.76 - 8.70 (m, 1H), 8.07 (d, *J =* 8.2 Hz, 1H), 7.78 (t, *J* = 6.7 Hz, 1H), 7.69 - 7.61 (m, 1H), 7.33 - 7.22 (m, 1H), 7.06 (s, 1H), 6.92 (d, *J =* 8.5 Hz, 1H), 6.77 - 6.73 (m, 1H), 6.44 (d, *J =* 2.9 Hz, 1H), 3.02 (s, 3H), 2.73 - 2.65 (m, 3H), 2.14 (d, *J* = 9.4 Hz, 2H), 2.09 - 1.99 (m, 2H), 1.90 (s, 3H), 1.35 (s, 2H), 1.17 (s, 2H). MS (ESI, m/z): C28H32FN3O , [M+H]+446.767.

### Example 55:

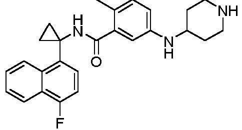

C55: ¹H NMR (600 MHz, DMSO) δ 8.98 (s, 1H), 8.72 (d, *J* = 8.3 Hz, 1H), 8.07 (d, *J* = 8.1 Hz, 1H), 7.79 (dd, *J* = 7.9, 5.6 Hz, 1H), 7.70 - 7.66 (m, 1H), 7.64 (t, *J* = 7.5 Hz, 1H), 7.28 (dd, *J =* 10.6, 7.9 Hz, 1H), 6.80 (d, *J =* 8.3 Hz, 1H), 6.45 (dd, *J =* 8.3, 2.5 Hz, 1H), 6.24 (d, *J* = 2.5 Hz, 1H), 5.33 (d, *J =* 8.2 Hz, 1H), 2.98 - 2.92 (m, 2H), 2.54 (d, *J =* 11.8 Hz, 2H), 1.86 (s, 3H), 1.78 (dd, *J* = 13.1, 3.5 Hz, 2H), 1.32 (s, 2H), 1.15 (d, *J* = 5.5 Hz, 2H), 0.89 - 0.80 (m, 2H). MS (ESI, m/z): C26H28FN3O , [M+H]+418.222.

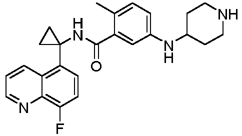

### Example 56:

**C56:** ¹H NMR (600 MHz, DMSO) δ 9.13 (dd, *J* = 8.7, 1.6 Hz, 1H), 9.01 (s, 1H), 8.98 (dd, *J* = 4.1, 1.7 Hz, 1H), 7.84 (dd,*J* = 8.1, 4.9 Hz, 1H), 7.70 (dd, *J =* 8.7, 4.1 Hz, 1H), 7.53 (dd, *J =* 10.7, 8.0 Hz, 1H), 6.80 (d, *J =* 8.2 Hz, 1H), 6.44 (dd, *J =* 8.3, 2.5 Hz, 1H), 6.23 (s, 1H), 5.28 (d, *J=* 8.2 Hz, 1H), 3.08 (d, *J =* 9.4 Hz, 1H), 2.88 (dt, *J =* 12.5, 3.7 Hz, 2H), 2.46 - 2.40 (m, 2H), 1.84 (s, 3H), 1.74 (dd, *J* = 13.0, 3.6 Hz, 2H), 1.32 (q, *J =* 4.5 Hz, 2H), 1.19 (d, *J =* 5.4 Hz, 2H), 1.11 (dd, *J* = 10.6, 3.2 Hz, 2H). MS (ESI, m/z): C25H27FN4O , [M+H]+419.217.

### Example 57:

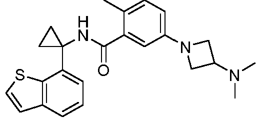

**C57:** ¹H NMR (400 MHz, Methanol-*d*₄) δ 7.78 (dd, *J =* 7.9, 1.2 Hz, 1H), 7.62 (dd, *J* = 7.3, 1.1 Hz, 1H), 7.60 (d, *J =* 5.5 Hz, 1H), 7.44 (d, *J =* 5.5 Hz, 1H), 7.36 (t, *J* = 7.6 Hz, 1H), 6.99 (d, *J =* 8.2 Hz, 1H), 6.46 (dd, *J =* 8.2, 2.6 Hz, 1H), 6.41 (d, *J =* 2.5 Hz, 1H), 3.95 (dd, *J* = 7.7, 6.7 Hz, 2H), 3.59 (dd, *J =* 7.6, 5.8 Hz, 2H), 3.29 (q, *J* = 6.2 Hz, 1H), 2.25 (s, 6H), 2.07 (s, 3H), 1.36 (q, *J* = 2.4 Hz, 4H). ¹³C NMR (101 MHz, Methanol-*d*₄) δ 172.81, 149.54, 140.40, 139.14, 136.76, 135.41, 130.73, 125.79, 124.48, 124.11, 123.89, 123.77, 122.42, 112.83, 110.33, 56.23, 55.94, 40.54, 35.28, 16.99, 13.67. MS (ESI, m/z):C24H27N3OS, [M+H]+406.1948.

### Example 58:

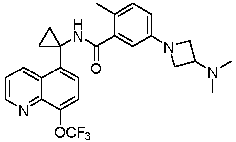

**C58:** ¹H NMR (400 MHz, DMSO-*d*₆) δ 9.17 (dd, *J* = 8.6, 1.6 Hz, 1H), 9.08 (s, 1H), 9.04 (dd, *J* = 4.2, 1.6 Hz, 1H), 7.93 (d,*J* = 8.0 Hz, 1H), 7.77 (dd, *J* = 7.9, 1.4 Hz, 1H), 7.74 (dd, *J* = 8.6, 4.2 Hz, 1H), 6.92 (d, *J* = 8.2 Hz, 1H), 6.35 (dd,*J* = 8.2, 2.5 Hz, 1H), 6.13 (d, *J =*2.5 Hz, 1H), 3.81 (t, *J* = 7.0 Hz, 2H), 3.41 (dd, *J* = 7.4, 5.6 Hz, 2H), 3.11 (p, *J* = 6.1 Hz, 1H), 2.07 (s, 6H), 1.86 (s, 3H), 1.37 (q, *J* = 4.9, 4.3 Hz, 2H), 1.25 (t, *J =* 3.4 Hz, 2H). ¹³C NMR (101 MHz, DMSO-*d*₆) δ 170.31, 151.39, 150.05, 143.87, 141.28, 138.62, 137.80, 134.24, 131.15, 128.85, 128.73, 123.08, 122.60, 120.88, 119.66, 112.77, 110.26, 56.73, 56.30, 41.97, 33.76, 18.29, 14.39. MS (ESI, m/z):C26H27F3N4O2, [M+H]+485.2159.

### Example 59:

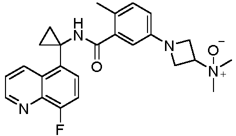

**C59:** ¹H NMR (400 MHz, DMSO-*d*₆) δ 9.14 (dt, *J* = 8.7, 1.6 Hz, 1H), 9.11 (s, 1H), 8.97 (dd, *J* = 4.2, 1.6 Hz, 1H), 7.85 (dd, *J* = 8.1, 5.1 Hz, 1H), 7.70 (dd, *J* = 8.7, 4.1 Hz, 1H), 7.53 (dd, *J* = 10.8, 8.0 Hz, 1H), 6.93 (d, *J* = 8.2 Hz, 1H), 6.37 (dd, *J* = 8.2, 2.5 Hz, 1H), 6.15 (d, *J* = 2.5 Hz, 1H), 4.39 (p, *J* = 6.3 Hz, 1H), 4.07 (dd, *J =* 7.5, 5.8 Hz, 2H), 3.80 (t, *J* = 7.3 Hz, 2H), 2.87 (s, 6H), 1.86 (s, 3H), 1.35 (q, *J* = 4.8, 4.4 Hz, 2H), 1.19 (q, *J* = 4.9 Hz, 2H). ¹³C NMR (101 MHz, DMSO-*d*₆) δ 170.22, 150.64, 150.02, 137.98, 134.95, 134.05, 131.02, 128.84, 123.31, 122.48, 112.93, 112.76, 110.47, 64.45, 56.78, 55.36, 52.36, 33.61, 18.24, 14.40. MS (ESI, m/z):C25H27FN4O2, [M+H]+435.2191.

### Example 60:

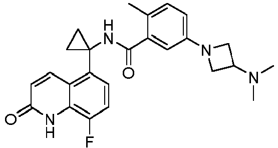

**C60:** ¹H NMR (400 MHz, Methanol-*d*₄) δ 8.78 (dd, *J* = 9.9, 1.7 Hz, 1H), 7.62 (dd, *J =* 8.4, 5.0 Hz, 1H), 7.34 (dd, *J =* 10.7, 8.4 Hz, 1H), 6.99 (d, *J* = 8.3 Hz, 1H), 6.75 (d, *J* = 9.9 Hz, 1H), 6.45 (dd, *J =* 8.3, 2.6 Hz, 1H), 6.26 (d, *J* = 2.5 Hz, 1H), 3.94 (t, *J =* 7.1 Hz, 2H), 3.56 (dd, *J =* 7.5, 5.7 Hz, 2H), 3.26 (p, *J =* 6.3 Hz, 1H), 2.22 (s, 6H), 2.03 (s, 3H), 1.45 - 1.39 (m, 2H), 1.29 (t, *J* = 3.5 Hz, 2H). MS (ESI, m/z):C25H27FN4O2, [M+H]+435.2191.

### Example 61:

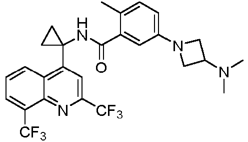

**61:** ¹H NMR (400 MHz, DMSO-*d*₆) δ 9.29 (s, 1H), 9.07 (d, *J* = 8.5 Hz, 1H), 8.37 (d, *J* = 7.1 Hz, 1H), 8.20 (s, 1H), 8.05 - 7.94 (m, 1H), 6.94 (d, *J =* 8.3 Hz, 1H), 6.37 (dd, *J =* 8.1, 2.5 Hz, 1H), 6.14 (d, *J =* 2.5 Hz, 1H), 3.82 (t, *J =* 7.1 Hz, 2H), 3.43 (t, *J* = 6.5 Hz, 2H), 3.18 (d, *J =* 5.2 Hz, 1H), 2.11 (s, 6H), 1.89 (s, 3H), 1.44 (q, *J* = 2.3 Hz, 2H), 1.24 (q, *J* = 3.7, 3.1 Hz, 2H). MS (ESI, m/z):C27H26F6N4O, [M+H]+537.2084.

### Example 62:

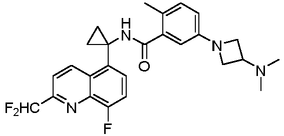

**C62:** ¹H NMR (400 MHz, DMSO-*d*₆) δ 9.37 (dd, *J* = 8.9, 1.6 Hz, 1H), 9.10 (s, 1H), 8.04 - 7.91 (m, 2H), 7.68 (dd, *J =* 10.7, 8.0 Hz, 1H), 7.18 (t, *J =* 54.6 Hz, 1H), 6.96 (d, *J =* 8.2 Hz, 1H), 6.39 (dd, *J* = 8.2, 2.5 Hz, 1H), 6.16 (d, *J* = 2.5 Hz, 1H), 3.92 (t, *J* = 7.3 Hz, 2H), 3.66 (d, *J* = 7.0 Hz, 2H), 3.44 (dt, *J* = 9.3, 4.6 Hz, 1H), 2.49 (s, 6H), 1.88 (s, 3H), 1.37 (q, *J* = 4.9, 4.3 Hz, 2H), 1.25 (t, *J =* 3.3 Hz, 2H). ¹³C NMR (101 MHz, DMSO-*d*₆) δ 170.14, 155.74, 149.39, 137.99, 137.24, 136.61, 135.13, 131.22, 130.99, 129.27, 123.83, 118.17, 114.49, 114.20, 114.02, 113.05, 110.53, 56.49, 55.96, 55.37, 49.06, 41.02, 33.60, 19.02, 18.31, 14.43. MS (ESI, m/z):C26H27F3N4O, [M+H]+469.2210.

### Example 63:

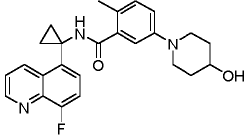

**C63:** ¹H NMR (400 MHz, DMSO-*d*₆) δ 9.15 (dt, *J* = 8.7, 1.6 Hz, 1H), 9.04 (s, 1H), 8.98 (dd, *J* = 4.2, 1.5 Hz, 1H), 7.85 (dd, *J =* 8.1, 5.1 Hz, 1H), 7.70 (dd, *J* = 8.7, 4.1 Hz, 1H), 7.54 (dd, *J =* 10.8, 8.0 Hz, 1H), 6.94 (d, *J =* 8.4 Hz, 1H), 6.82 (dd, *J =* 8.4, 2.7 Hz, 1H), 6.58 (d, *J* = 2.6 Hz, 1H), 4.64 (d, *J* = 4.2 Hz, 1H), 3.58 (dq, *J* = 9.0, 4.5 Hz, 1H), 3.48 - 3.34 (m, 2H), 2.72 (ddd, *J* = 12.9, 10.1, 3.0 Hz, 2H), 1.88 (s, 3H), 1.76 (dd, *J* = 13.1, 4.1 Hz, 2H), 1.46 - 1.38 (m, 2H), 1.36 (q, *J* = 4.7 Hz, 2H), 1.20 (q, *J* = 4.9 Hz, 2H). ¹³C NMR (101 MHz, DMSO-*d*₆) δ 170.23, 150.63, 149.14, 137.91, 134.96, 134.04, 131.29, 129.18, 129.10, 128.83, 124.74, 122.43, 117.19, 114.79, 112.94, 112.76, 66.38, 47.08, 34.20, 33.67, 18.30, 14.36. MS (ESI, m/z):C25H26FN3O2, [M+H]+420.2082.

### Example 64:

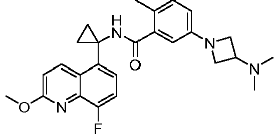

**C64:** ¹H NMR (400 MHz, DMSO-*d*₆) δ 9.15 - 8.84 (m, 2H), 7.66 (dd, *J* = 8.1, 5.0 Hz, 1H), 7.46 (dd, *J =* 10.9, 8.1 Hz, 1H), 7.18 (d, *J=* 9.1 Hz, 1H), 6.92 (d, *J =* 8.2 Hz, 1H), 6.34 (dd, *J* = 8.1, 2.6 Hz, 1H), 6.12 (d, *J =* 2.6 Hz, 1H), 4.01 (s, 3H), 3.81 (t, *J =* 7.0 Hz, 2H), 3.41 (dd, *J* = 7.4, 5.6 Hz, 2H), 3.11 (p, *J =* 6.2 Hz, 1H), 2.06 (s, 6H), 1.88 (s, 3H), 1.32 (q, *J =* 4.8, 4.4 Hz, 2H), 1.22 - 1.07 (m, 2H). ¹³C NMR (101 MHz, DMSO-*d*₆) δ 170.11, 162.14, 157.09, 154.60, 150.06, 137.98, 137.52, 136.14, 136.02, 134.93, 134.88, 131.13, 126.82, 126.74, 125.86, 125.83, 123.08, 113.89, 113.67, 113.49, 112.71, 110.25, 56.77, 56.30, 53.80, 41.98, 33.73, 18.33, 14.45. MS (ESI, m/z):C26H29FN4O2, [M+H]+449.2347.

### Example 65:

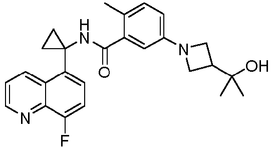

**C65:** ¹H NMR (400 MHz, DMSO-*d*₆) δ 9.14 (dt, *J* = 8.7, 1.7 Hz, 1H), 9.06 (s, 1H), 8.97 (dd, *J =* 4.1, 1.5 Hz, 1H), 7.85 (dd, *J =* 8.0, 5.1 Hz, 1H), 7.71 (dd, *J =* 8.7, 4.1 Hz, 1H), 7.54 (dd, *J =* 10.8, 8.0 Hz, 1H), 6.89 (d, *J =* 8.2 Hz, 1H), 6.31 (dd, *J =* 8.2, 2.5 Hz, 1H), 6.08 (d, *J* = 2.5 Hz, 1H), 4.34 (s, 1H), 3.66 (t, *J* = 7.6 Hz, 2H), 3.55 (t, *J* = 6.9 Hz, 2H), 2.72 - 2.60 (m, 1H), 1.85 (s, 3H), 1.35 (q, *J* = 4.7, 4.3 Hz, 2H), 1.20 (t, *J =* 3.2 Hz, 2H), 1.03 (s, 6H). ¹³C NMR (101 MHz, DMSO-*d*₆) δ 170.31, 158.36, 155.83, 150.64, 150.35, 138.37, 138.26, 137.88, 134.96, 134.91, 134.07, 131.01, 129.20, 129.13, 128.84, 128.82, 122.47, 112.95, 112.77, 112.44, 109.96, 68.17, 53.03, 33.61, 27.11, 26.81, 18.31, 14.39. MS (ESI, m/z):C26H28FN3O2, [M+H]+434.2238.

### Example 66:

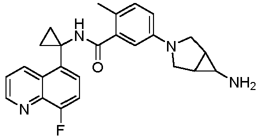

**C66:** ¹H NMR (400 MHz, DMSO-*d*₆) δ 9.19 (d, *J* = 8.7 Hz, 1H), 9.06 (s, 1H), 9.00 (dd, J= 4.2, 1.5 Hz, 1H), 8.45 (d, *J* = 4.7 Hz, 3H), 7.86 (dd, *J =* 8.0, 5.0 Hz, 1H), 7.74 (dd, *J =* 8.6, 4.2 Hz, 1H), 7.56 (dd, *J* = 10.8, 8.0 Hz, 1H), 6.91 (d, *J =* 8.3 Hz, 1H), 6.49 (dd, *J =* 8.4, 2.6 Hz, 1H), 6.26 (d, *J* = 2.6 Hz, 1H), 3.48 (d, *J =* 9.5 Hz, 2H), 3.08 (d, *J =* 9.1 Hz, 2H), 2.39 - 2.31 (m, 1H), 2.09 (d, *J =* 2.8 Hz, 2H), 1.87 (s, 3H), 1.40 (s, 3H), 1.36 (q, *J* = 5.0 Hz, 2H), 1.20 (t, *J =* 3.4 Hz, 2H). ¹³C NMR (101 MHz, DMSO-*d*₆) δ 170.26, 150.45, 145.85, 137.99, 131.31, 122.74, 122.51, 113.97, 111.49, 66.82, 49.57, 33.63, 31.48, 26.81, 21.65, 18.27, 14.41. MS (ESI, m/z):C25H25FN4O, [M+H]+417.2085.

### Example 67:

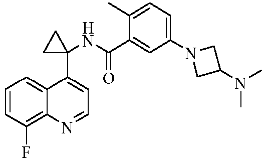

**C67:** ¹H NMR (400 MHz, DMSO) δ 9.16 (s, 1H), 8.93 (d, J = 4.4 Hz, 1H), 8.47 (d, J = 8.3 Hz, 1H), 7.79 (d, J = 4.4 Hz, 1H), 7.70 - 7.52 (m, 2H), 6.92 (d, J = 8.2 Hz, 1H), 6.35 (dd, J = 8.2, 2.5 Hz, 1H), 6.14 (d, J = 2.5 Hz, 1H), 3.81 (t, J = 7.0 Hz, 2H), 3.42 (t, J = 6.4 Hz, 2H), 3.13 (t, J = 6.4 Hz, 1H), 2.08 (s, 6H), 1.86 (s, 3H), 1.42 - 1.33 (m, 2H), 1.27 (q, J = 5.7, 5.1 Hz, 2H). 13C NMR (101 MHz, DMSO) δ 170.45, 159.56, 157.03, 150.87, 150.01, 147.02 (d, J = 2.7 Hz), 138.76 (d, J = 11.3 Hz), 137.61, 131.16, 129.27, 126.53 (d, J = 8.4 Hz), 123.61 (d, J = 81.9 Hz), 121.89 (d, J = 4.8 Hz), 113.50 (d, J = 18.4 Hz), 112.82, 110.37, 56.68, 56.29, 41.94, 34.16, 18.31, 14.09. MS (ESI, m/z):C25H27FN4O, [M+H]+419.217.

### Example 68:

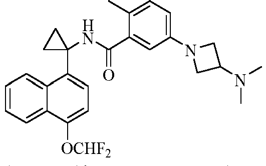

**C68:** ¹H NMR (400 MHz, DMSO) δ 9.03 (s, 1H), 8.72 (d, *J* = 8.2 Hz, 1H), 8.16 - 8.06 (m, 1H), 7.81 (d, *J* = 7.9 Hz, 1H), 7.72 - 7.60 (m, 2H), 7.50 (d, *J* = 74.0 Hz, 1H), 7.26 (d, *J* = 7.8 Hz, 1H), 6.91 (d, *J* = 8.2 Hz, 1H), 6.34 (dd, *J =* 8.1, 2.5 Hz, 1H), 6.11 (d, *J* = 2.5 Hz, 1H), 3.80 (t, *J* = 7.0 Hz, 2H), 3.40 (t, *J* = 6.5 Hz, 2H), 3.11 (p, *J =* 6.2 Hz, 1H), 2.06 (s, 6H), 1.90 (s, 3H), 1.35 (t, *J =* 3.6 Hz, 2H), 1.17 (d, *J = 5.1* Hz, 2H). 13C NMR (101 MHz, DMSO) δ 170.17, 150.01, 146.57 (d, *J* = 3.0 Hz), 137.99, 135.39, 133.33, 131.12, 128.75, 127.19, 126.71, 126.18, 125.88, 123.22, 121.98, 119.88, 117.32, 112.69, 110.44, 56.72, 56.30, 41.96, 34.18, 18.42, 14.58. MS (ESI, m/z):C27H29F2N3O2, [M+H]+466.223.

### Example 69:

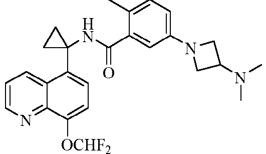

**C69:** ¹H NMR (400 MHz, DMSO) δ 9.15 (dd, J = 8.6, 1.7 Hz, 1H), 9.06 (s, 1H), 8.99 (dd, J = 4.2, 1.6 Hz, 1H), 7.87 (d, J = 8.0 Hz, 1H), 7.71 (dd, J = 8.6, 4.1 Hz, 1H), 7.59 (d, J = 42.5 Hz, 1H), 7.52 - 7.25 (m, 1H), 6.92 (d, J = 8.2 Hz, 1H), 6.35 (dd, J = 8.2, 2.6 Hz, 1H), 6.12 (d, J = 2.5 Hz, 1H), 3.81 (t, J = 7.0 Hz, 2H), 3.41 (dd, J = 7.4, 5.6 Hz, 2H), 3.11 (p, J = 6.1 Hz, 1H), 2.07 (s, 6H), 1.88 (s, 3H), 1.36 (q, J = 4.7, 4.2 Hz, 2H), 1.20 (q, J = 4.9 Hz, 2H). 13C NMR (101 MHz, DMSO) δ 170.41, 151.48, 150.13, 146.72, 138.29, 138.12, 136.74, 134.41, 131.16, 129.40, 128.82, 123.26, 122.33, 118.90, 117.33, 113.82, 111.35, 60.08, 55.79, 42.69, 33.69, 17.61, 13.10. MS (ESI, m/z):C26H28F2N4O2, [M+H]+467.218.

### Example 70:

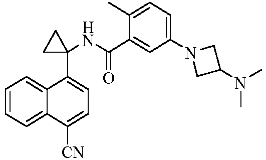

**C70:** ¹H NMR (400 MHz, DMSO) δ 9.15 (s, 1H), 8.86 - 8.76 (m, 1H), 8.20 - 8.09 (m, 2H), 7.94 (d, J = 7.5 Hz, 1H), 7.86 - 7.72 (m, 2H), 6.91 (d, J = 8.2 Hz, 1H), 6.34 (dd, J = 8.2, 2.5 Hz, 1H), 6.12 (d, J = 2.6 Hz, 1H), 3.80 (t, J = 7.0 Hz, 2H), 3.40 (dd, J = 7.4, 5.7 Hz, 2H), 3.11 (p, J = 6.2 Hz, 1H), 2.07 (s, 6H), 1.86 (s, 3H), 1.40 (q, J = 4.7, 4.2 Hz, 2H), 1.25 (q, J = 4.8 Hz, 2H). 13C NMR (101 MHz, DMSO) δ 170.39, 150.01, 144.24, 137.72, 133.05, 132.46, 131.92, 131.15, 128.95, 128.43, 127.86, 126.78, 125.38, 123.19, 118.18, 112.79, 110.38, 108.84, 56.70, 56.29, 41.95, 34.66, 18.34, 14.56. MS (ESI, m/z):C27H28N4O, [M+H]+425.226

### Example 71

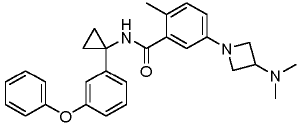

**C71:** ¹H NMR (400 MHz, DMSO-*d*₆) δ 8.86 (s, 1H), 8.20 (s, 1H), 7.38 (t, *J* = 7.9 Hz, 2H), 7.32 (t, *J* = 8.2 Hz, 1H), 7.12 (t, *J* = 7.4 Hz, 1H), 7.01 (t, *J =* 7.3 Hz, 3H), 6.97 - 6.91 (m, 2H), 6.85 - 6.79 (m, 1H), 6.42 (dd, *J =* 8.2, 2.3 Hz, 1H), 6.35 (d, *J =* 2.2 Hz, 1H), 3.96 - 3.89 (m, 2H), 3.63 - 3.57 (m, 2H), 3.18 - 3.10 (m, 1H), 2.32 (s, 6H), 2.12 (s, 3H). MS (ESI, m/z):C28H31N3O2, [M+H]+442.242.

### Example 72

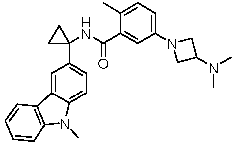

**C72:** ¹H NMR (400 MHz, DMSO-*d*₆) δ 8.96 (s, 1H), 8.13 - 8.06 (m, 2H), 7.57 (d, *J =* 8.2 Hz, 1H), 7.55 - 7.41 (m, 3H), 7.22 - 7.16 (m, 1H), 7.05 - 6.99 (m, 1H), 6.43 (s, 2H), 3.94 - 3.89 (m, 2H), 3.55 - 3.50 (m, 2H), 3.21 - 3.16 (m, 1H), 2.19 (s, 3H), 2.11 (s, 6H), 1.32 - 1.29 (m, 2H), 1.25 - 1.22 (m, 2H). MS (ESI, m/z):C29H32N4O, [M+H]+453.258.

### Example 73

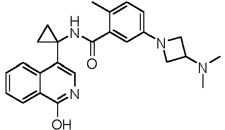

**C73:** ¹H NMR (400 MHz, DMSO-*d*₆) δ 11.25 (s, 1H), 8.88 (s, 1H), 8.38 - 8.17 (m, 2H), 7.84 - 7.71 (m, 1H), 7.58 - 7.46 (m, 1H), 7.39 - 7.29 (m, 1H), 6.91 (d, *J =* 7.2 Hz, 1H), 6.41 - 6.28 (m, 1H), 6.20 - 6.09 (m, 1H), 3.86 - 3.74 (m, 2H), 3.16 - 3.04 (m, 1H), 2.06 (s, 6H), 1.92 (s, 3H), 1.26 - 1.13 (m, 2H), 1.11 - 0.98 (m, 2H). MS (ESI, m/z):C25H28N4O2, [M+H]+417.221.

### Example 74

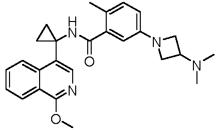

**C74:** ¹H NMR (400 MHz, DMSO-*d*₆) δ 9.03 (s, 1H), 8.59 (d, *J =* 8.3 Hz, 1H), 8.26 (s, 1H), 8.20 (d, *J =* 8.2 Hz, 1H), 7.83 (t, *J* = 7.5 Hz, 1H), 7.63 (t, *J* = 7.4 Hz, 1H), 6.91 (d, *J* = 8.1 Hz, 1H), 6.34 (d, *J =* 7.8 Hz, 1H), 6.13 (s, 1H), 4.05 (s, 3H), 3.81 (t, *J =* 6.7 Hz, 2H), 3.20 (s, 1H), 3.03 (dd, *J =* 14.3, 7.1 Hz, 2H), 2.11 (s, 6H), 1.90 (s, 3H), 1.30 (s, 2H), 1.14 (s, 2H). MS (ESI, m/z):C26H30N4O2, [M+H]+431.237.

### Example 75

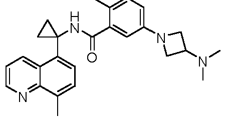

**C75:** ¹H NMR (400 MHz, DMSO-*d*₆) δ 9.09 (d, *J =* 8.2 Hz, 1H), 9.06 - 8.99 (m, 1H), 8.98 - 8.92 (m, 1H), 7.75 (d, *J* = 7.1 Hz, 1H), 7.67 - 7.54 (m, 2H), 6.92 (d, *J =* 8.0 Hz, 1H), 6.34 (d, *J* = 7.5 Hz, 1H), 6.11 (s, 1H), 3.80 (t, *J =* 6.6 Hz, 2H), 3.44 - 3.37 (m, 2H), 3.11 (s, 1H), 2.70 (s, 3H), 2.06 (s, 5H), 1.89 (s, 3H), 1.38 - 1.29 (m, 2H), 1.20 - 1.12 (m, 2H). MS (ESI, m/z):C26H30N4O, [M+H]+415.242.

FIG. 8 shows the inhibition curve of compound C75.

### Example 76

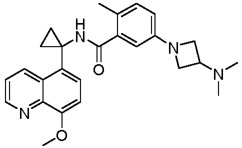

**C76:** ¹H NMR (400 MHz, DMSO-*d*₆) δ 9.05 (dd, *J* = 8.6, 1.5 Hz, 1H), 8.96 (s, 1H), 8.85 (dd, *J* = 4.0, 1.5 Hz, 1H), 7.78 (d, J = 8.0 Hz, 1H), 7.60 (dd, *J* = 8.6, 4.1 Hz, 1H), 7.13 (d, *J* = 8.1 Hz, 1H), 6.91 (d, *J* = 8.3 Hz, 1H), 6.34 (dd, *J* = 8.2, 2.4 Hz, 1H), 6.11 (d, *J* = 2.4 Hz, 1H), 3.96 (s, 3H), 3.85 - 3.76 (m, 2H), 3.48 - 3.40 (m, 2H), 3.21 - 3.13 (m, 1H), 2.10 (s, 6H), 1.88 (s, 3H), 1.35 - 1.29 (m, 2H), 1.16 - 1.11 (m, 2H). MS (ESI, m/z):C26H30N4O2, [M+H]+431.237.

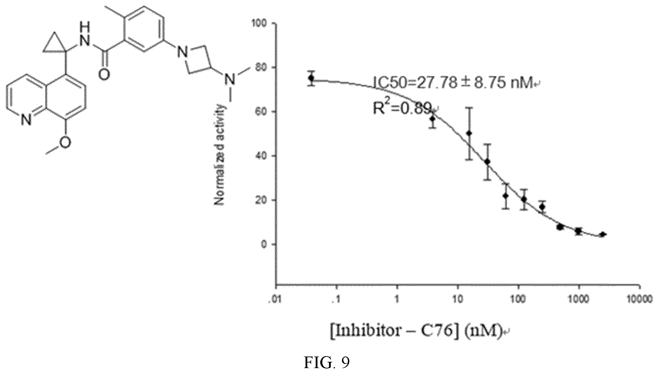

FIG. 9 shows the inhibition curve of compound C76.

### Example 77

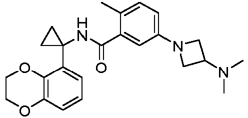

**C77:** ¹H NMR (400 MHz, DMSO-*d*₆) δ 8.48 (s, 1H), 7.02 (dd, *J =* 6.0, 3.2 Hz, 1H), 6.96 (d, *J =* 8.2 Hz, 1H), 6.76 - 6.70 (m, 2H), 6.37 (dd, *J* = 8.2, 2.4 Hz, 1H), 6.28 (d, *J* = 2.4 Hz, 1H), 4.32 - 4.27 (m, 2H), 4.26 - 4.20 (m, 2H), 3.90 - 3.85 (m, 2H), 3.52 - 3.46 (m, 2H), 3.21 - 3.13 (m, 1H), 2.11 (s, 6H), 2.08 (s, 3H), 1.08 (d, *J =* 3.2 Hz, 4H). MS (ESI, m/z):C24H29N3O3, [M+H]+408.221.

### Example 78

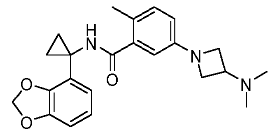

**C78:** ¹H NMR (400 MHz, DMSO-*d*₆) δ 8.82 (s, 1H), 7.01 (d, *J =* 8.6 Hz, 1H), 6.88 (dd, *J* = 7.4, 1.9 Hz, 1H), 6.82 - 6.75 (m, 2H), 6.44 - 6.39 (m, 2H), 5.96 (s, 2H), 3.94 - 3.87 (m, 2H), 3.55 - 3.48 (m, 2H), 3.21 - 3.13 (m, 1H), 2.16 (s, 3H), 2.11 (s, 6H), 1.43 - 1.38 (m, 2H), 1.17 - 1.12 (m, 2H). MS (ESI, m/z):C23H27N3O3, [M+H]+394.205.

### Example 79

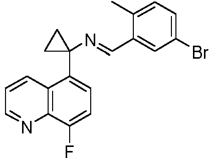

**C79:** ¹H NMR (400 MHz, CDCl₃) δ 9.02 (d, *J* = 4.0 Hz, 1H), 8.45 (d, *J =* 8.6 Hz, 1H), 7.93 (s, 1H), 7.72 (s, 1H), 7.61 - 7.55 (m, 1H), 7.53 - 7.48 (m, 1H), 7.48 - 7.42 (m, 1H), 7.36 - 7.31 (m, 1H), 6.92 (d, *J* = 8.1 Hz, 1H), 1.93 (s, 3H), 1.83 - 1.75 (m, 2H), 1.49 - 1.41 (m, 2H). MS (ESI, m/z):C20H16BrFN2, [M+H]+383.048.

### Example 80

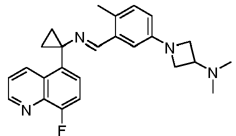

**C80:** ¹H NMR (400 MHz, DMSO-*d*₆) δ 9.00 (d, *J =* 4.0 Hz, 1H), 8.44 (d, *J =* 8.5 Hz, 1H), 7.71 (s, 1H), 7.70 - 7.60 (m, 3H), 6.90 (d, *J =* 8.2 Hz, 1H), 6.70 (d, *J =* 2.4 Hz, 1H), 6.40 - 6.35 (m, 1H), 3.89 - 3.82 (m, 2H), 3.47 - 3.42 (m, 2H), 3.16 - 3.10 (m, 1H), 2.08 (s, 6H), 1.83 (s, 3H), 1.66 - 1.62 (m, 2H), 1.45 - 1.40 (m, 2H). MS (ESI, m/z):C25H27FN4, [M+H]+402.222.

### Example 81

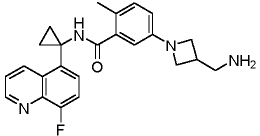

**C81:** ¹H NMR (400 MHz, CDCl₃) δ 9.10 - 9.04 (m, 1H), 9.02 (s, 1H), 7.96 - 7.89 (m, 1H), 7.63 - 7.56 (m, 1H), 7.46 - 7.36 (m, 1H), 7.30 (s, 1H), 7.00 - 6.92 (m, 1H), 6.41 - 6.33 (m, 2H), 6.24 - 6.19 (m, 1H), 3.92 - 3.84 (m, 2H), 3.52 - 3.44 (m, 2H), 2.98 - 2.91 (m, 2H), 2.77 - 2.66 (m, 1H), 2.07 (d, *J* = 4.7 Hz, 3H), 1.65 - 1.58 (m, 2H), 1.42 - 1.35 (m, 2H). MS (ESI, m/z):C24H25FN4O, [M+H]+405.201.

### Example 82

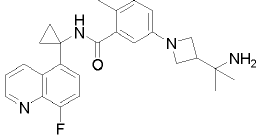

**C82:** ¹H NMR (400 MHz, DMSO-*d*₆) δ 9.23 (d, *J* = 8.6 Hz, 1H), 8.93 (d, *J* = 3.8 Hz, 1H), 7.87 - 7.77 (m, 1H), 7.64 (dd, *J =* 8.6, 4.1 Hz, 1H), 7.54 - 7.42 (m, 1H), 6.77 (d, *J* = 8.2 Hz, 1H), 6.45 - 6.37 (m, 1H), 6.31 - 6.27 (m, 1H), 4.06 - 4.00 (m, 1H), 3.85 - 3.76 (m, 1H), 3.09 - 3.01 (m, 1H), 2.70 - 2.55 (m, 2H), 1.88 (s, 3H), 1.75 - 1.67 (m, 1H), 1.26 - 1.20 (m, 2H), 1.12 - 1.07 (m, 2H), 1.06 (s, 3H), 0.93 (s, 3H). MS (ESI, m/z):C26H29FN4O, [M+H]+432.233.

### Example 83

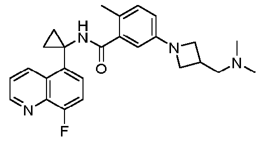

**C83:** ¹H NMR (400 MHz, DMSO-*d*₆) δ 9.14 (d, *J* = 8.6 Hz, 1H), 9.07 (s, 1H), 8.97 (d, *J =* 3.3 Hz, 1H), 7.90 - 7.82 (m, 1H), 7.75 - 7.69 (m, 1H), 7.59 - 7.50 (m, 1H), 6.92 (d, *J =* 8.2 Hz, 1H), 6.36 - 6.28 (m, 1H), 6.11 (d, *J =* 2.1 Hz, 1H), 3.89 - 3.82 (m, 2H), 3.43 - 3.37 (m, 3H), 3.00 - 2.92 (m, 1H), 2.85 (s, 2H), 2.40 (s, 7H), 1.86 (s, 3H), 1.38 - 1.32 (m, 2H), 1.22 - 1.17 (m, 2H). MS (ESI, m/z):C26H29FN4O, [M+H]+432.233.

### Example 84

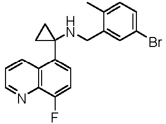

**C84:** ¹H NMR (400 MHz, DMSO-*d*₆) δ 9.09 - 9.03 (m, 1H), 8.99 - 8.95 (m, 1H), 7.68 (dd, *J* = 8.6, 4.1 Hz, 1H), 7.56 - 7.43 (m, 2H), 7.26 (d, *J* = 1.9 Hz, 1H), 7.22 - 7.17 (m, 1H), 6.95 (d, *J* = 8.1 Hz, 1H), 3.47 (s, 2H), 1.91 (s, 3H), 1.20 - 1.13 (m, 2H), 0.95 - 0.89 (m, 2H). MS (ESI, m/z):C20H18BrFN2, [M+H]+385.064.

### Example 85

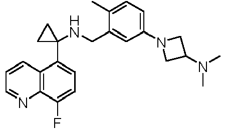

**C85:** ¹H NMR (400 MHz, DMSO-*d*₆) δ 9.06 (d, *J =* 8.6 Hz, 1H), 9.00 - 8.94 (m, 1H), 7.72 - 7.65 (m, 1H), 7.57 - 7.52 (m, 1H), 7.52 - 7.46 (m, 1H), 6.78 (d, *J =* 8.0 Hz, 1H), 6.18 - 6.10 (m, 2H), 3.76 - 3.69 (m, 2H), 3.41 (s, 2H), 3.11 - 3.04 (m, 1H), 2.06 (s, 6H), 1.81 (s, 3H), 1.19 - 1.13 (m, 2H), 0.94 - 0.88 (m, 2H). MS (ESI, m/z):C25H29FN4, [M+H]+405.238.

### Example 86

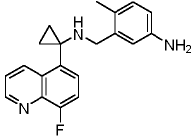

**C86:** ¹H NMR (400 MHz, CDCl₃) δ 9.08 - 9.02 (m, 1H), 8.92 - 8.85 (m, 1H), 7.60 - 7.51 (m, 2H), 7.41 - 7.33 (m, 1H), 6.86 - 6.80 (m, 1H), 6.49 - 6.41 (m, 2H), 3.49 (s, 2H), 1.89 (s, 3H), 1.28 - 1.23 (m, 2H), 1.06 - 0.99 (m, 2H). MS (ESI, m/z):C20H20FN3, [M+H]+322.164.

### Example 87

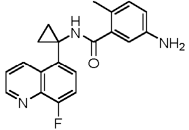

**C87:** ¹H NMR (400 MHz, DMSO-*d*₆) δ 9.25 - 9.12 (m, 1H), 9.10 - 8.95 (m, 2H), 7.94 - 7.84 (m, 1H), 7.78 - 7.67 (m, 1H), 7.61 - 7.49 (m, 1H), 7.24 (s, 1H), 6.76 (d, *J =* 7.9 Hz, 1H), 6.55 - 6.41 (m, 1H), 6.28 (s, 1H), 4.90 (s, 2H), 1.84 (s, 3H), 1.37 - 1.29 (m, 2H), 1.22 - 1.14 (m, 2H). MS (ESI, m/z):C20H18FN3O, [M+H]+336.143.

### Example 88:

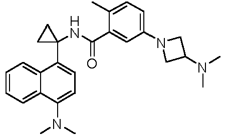

**C88:** ¹H NMR (400 MHz, DMSO) δ 8.93 (s, 1H), 8.71 - 8.61 (m, 1H), 8.24 - 8.14 (m, 1H), 7.72 (d, J = 7.7 Hz, 1H), 7.59 - 7.47 (m, 2H), 7.06 (d, J = 7.8 Hz, 1H), 6.91 (d, J = 8.2 Hz, 1H), 6.33 (dd, J = 8.2, 2.4 Hz, 1H), 6.12 (d, J = 2.4 Hz, 1H), 3.79 (t, J = 7.0 Hz, 2H), 3.48 - 3.37 (m, 2H), 3.17 - 3.05 (m, 1H), 2.81 (s, 6H), 2.06 (s, 6H), 1.94 (s, 3H), 1.32 (s, 2H), 1.12 (s, 2H). ¹³C NMR (151 MHz, DMSO) δ 170.00, 150.41, 149.98, 138.13, 133.45, 132.62, 131.09, 129.06, 128.77, 126.11, 125.84, 125.01, 124.72, 123.26, 113.53, 112.61, 110.51, 56.72, 56.30, 45.37, 41.96, 34.33, 18.49, 14.70. MS (ESI, m/z):C28H34N4O, [M+H]+443.273.

### Example 89:

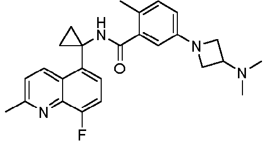

**C89:** ¹H NMR (400 MHz, DMSO-*d*₆) δ 9.03 - 8.96 (m, 2H), 7.75 (dd, J = 8.1, 5.0 Hz, 1H), 7.58 (d, J = 8.8 Hz, 1H), 7.46 (dd, J = 10.9, 8.0 Hz, 1H), 6.93 (d, J = 8.2 Hz, 1H), 6.36 (dd, J = 8.1, 2.5 Hz, 1H), 6.13 (d, J = 2.5 Hz, 1H), 3.86 (d, J = 7.5 Hz, 2H), 3.52 (d, J = 14.5 Hz, 2H), 2.69 (s, 4H), 2.29 (s, 6H), 1.87 (s, 3H), 1.32 (q, J = 4.9 Hz, 2H), 1.16 (td, J = 5.0, 2.5 Hz, 2H). ¹³C NMR (101 MHz, MeOD) δ 173.88, 160.82, 150.73, 138.44, 135.60, 132.18, 129.83, 125.25, 124.33, 114.27, 113.88, 113.69, 111.13, 57.66, 56.86, 41.78, 34.62, 24.57, 18.22, 14.96. MS (ESI, m/z):C26H29FN4O, [M+H]+433.240.

### Example 90:

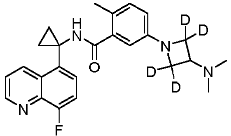

**C90:** ¹H NMR (400 MHz, Methanol-d₄) δ 9.20 (dt, J = 8.9, 1.6 Hz, 1H), 8.93 (dd, J = 4.3, 1.6 Hz, 1H), 8.00 (dd, J = 8.1, 5.0 Hz, 1H), 7.78 - 7.67 (m, 1H), 7.49 (dd, J = 10.6, 8.1 Hz, 1H), 6.96 (d, J = 8.3 Hz, 1H), 6.44 (dd, J = 8.2, 2.6 Hz, 1H), 6.23 (d, J = 2.5 Hz, 1H), 3.22 (s, 1H), 2.21 (s, 6H), 1.94 (s, 3H), 1.52 - 1.45 (m, 2H), 1.38 - 1.31 (m, 2H). ¹³C NMR (101 MHz, MeOD) δ 173.98, 159.65, 157.11, 151.12, 151.07, 146.30, 139.25, 139.14, 138.26, 135.70, 135.44, 133.94, 132.12, 130.82, 130.74, 130.27, 126.34, 124.90, 123.39, 120.09, 114.24, 113.92, 113.73, 111.10, 57.19, 42.02, 34.58, 18.22, 14.94. MS (ESI, m/z):C25H23D4FN4O, [M+H]+423.249.

### Example 91:

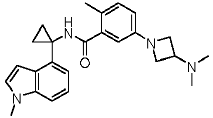

**C91:** ¹H NMR (400 MHz, Methanol-d₄) δ 7.30 (t, J = 7.0 Hz, 2H), 7.20 (d, J = 3.2 Hz, 1H), 7.14 (t, J = 7.7 Hz, 1H), 7.04 (d, J = 8.2 Hz, 1H), 6.85 (d, J = 3.2 Hz, 1H), 6.48 (dd, J = 8.2, 2.6 Hz, 1H), 6.36 (d, J = 2.6 Hz, 1H), 4.14 - 4.01 (m, 2H), 3.83 (s, 5H), 3.37 (s, 1H), 2.85 (s, 6H), 2.09 (s, 3H), 1.36 - 1.26 (m, 4H).¹³C NMR (101 MHz, MeOD) 13C NMR (101 MHz, DMSO-d₆) δ 169.19, 148.54, 137.97, 136.63, 134.07, 130.77, 128.83, 127.08, 124.15, 120.43, 118.59, 112.59, 110.57, 108.48, 99.98, 55.33, 54.92, 54.20, 34.39, 32.56, 18.21, 14.30. MS (ESI, m/z):C25H30N4O, [M+H]+403.249.

### Preparation of compounds:

(1) The synthetic route for preparation of substituted naphthyl-cyclopropylamine intermediate (1-(substituted naphth-1-yl)cyclopropylamine) was as follows:

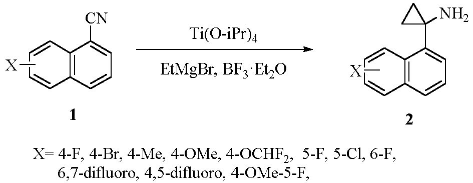

Substituted naphthonitrile (1, 10 mmol, 1.0 e.q.) was placed in a round bottom flask, 10 mL of dry tetrahydrofuran was added as a solvent, and tetraisopropyl titanate (10 mmol, 1.1 e.q.) was then added. The reaction system was cooled to -78°C, and ethyl Grignard reagent (20 mmol, 2.0 e.q.) was then slowly added dropwise to the reaction system. After the dropwise addition was completed, the reaction system was warmed to room temperature and reacted for 1.5 h. Boron trifluoride diethyl etherate (20 mmol, 2.0 e.q.) was then added dropwise to the reaction system. After the dropwise addition was completed, the mixture was reacted with stirring at room temperature for 3 h. After the reaction was completed, 20 mL of 2 N hydrochloric acid was added dropwise to the reaction system, the reaction was quenched with stirring for 20 min, and an excess saturated sodium hydroxide solution was then added. The mixture was extracted with ethyl acetate, and the organic phase was collected, concentrated to dryness by rotary evaporation, and separated by silica gel column chromatography to give substituted naphthyl-cyclopropylamine intermediate **2.**

(2) The synthetic route for preparation of the compounds of Examples 1-3 was as follows:

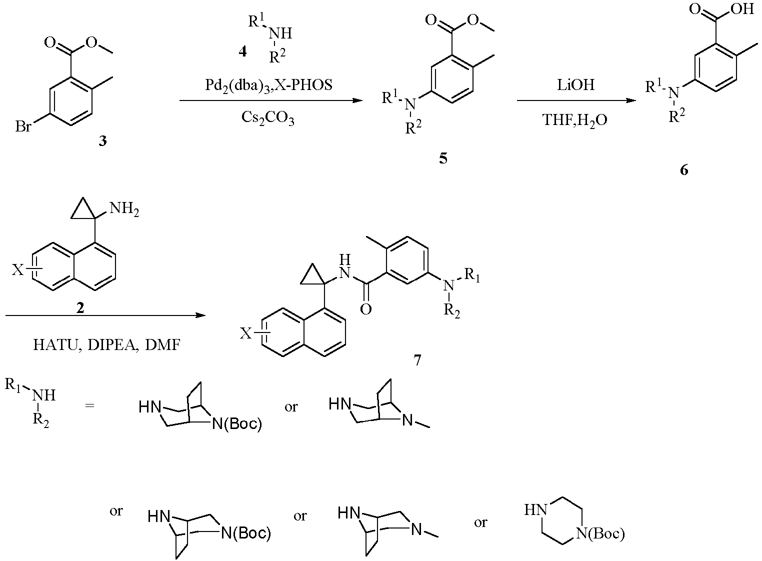

Methyl 2-methyl-5-bromobenzoate **(3,** 2 mmol, 1.0 e.q.) was placed in a 50 mL sealed tube. Amine compound **4** containing nitrogen and hydrogen (3 mmol, 1.5 e.q.), tris(dibenzylideneacetone)dipalladium (0.04 mmol, 0.02 e.q.), X-PHOS ligand (0.08 mmol, 0.04 e.q.), and cesium carbonate (4 mmol, 2.0 e.q.) were added. Toluene solvent (10 mL) was then added, and the reaction system was heated to 110°C in the sealed tube, reacted with stirring overnight under argon atmosphere. After the conversion of the substrate was completed as detected by thin-layer chromatography plate, the organic solvent was removed by rotary evaporation, and the mixture was separated and purified by column chromatography to give intermediate **5.**

The intermediate 5 (1.0 e.q.) was placed in a round bottom flask, tetrahydrofuran:water = 2:1 was added as a solvent, and lithium hydroxide (4.0 e.q.) was then added to the reaction system. The mixture was reacted with stirring at 60°C for 6 h, and the reaction system was then acidified with 2 N hydrochloric acid. After ethyl acetate was added, a white solid was precipitated, filtered out under vacuum, and dried to give intermediate **6.**

**6** and the previously obtained three-membered ring amine intermediate **2** were added to a DMF solvent according to an equivalent ratio of 1:1. HATU (1.5 e.q.) and DIPEA (2.0 e.q.) were added, and the mixture was reacted at 70°C for 12 h. The reaction solution was extracted with ethyl acetate, and washed with a saturated ammonium chloride solution for three times. The organic phase was concentrated to dryness by rotary evaporation and purified by silica gel column chromatography to give product **7.**

When Boc (tert-butoxycarbonyl) protection was present in the amine compound **6,** finally, the final product was obtained after the removal of the tert-butoxycarbonyl from compound **7** by hydrochloric acid.

(3) The synthetic route for preparation of the compounds of Examples 4-6 was as follows:

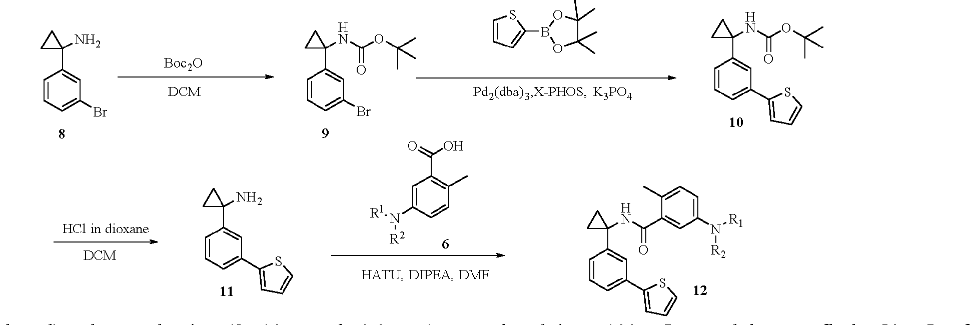

1-(3-bromophenyl)cyclopropylamine **(8,** 10 mmol, 1.0 e.q.) was placed in a 100 mL round bottom flask, 50 mL of dichloromethane as a solvent was added, and di-*tert*-butyl dicarbonate (40 mmol, 4.0 e.q.) was then added. The mixture was reacted with stirring at room temperature for 4 h. The organic solvent was removed by rotary evaporation, and the mixture was separated and purified by column chromatography to give intermediate **9.**

The intermediate **9** (4 mmol, 1.0 e.q.) was placed in a 50 mL sealed tube. Thiophene-2-boronic acid pinacol ester (6 mmol, 1.5 e.q.), tris(dibenzylideneacetone)dipalladium (0.08 mmol, 0.02 e.q.), X-PHOS ligand (0.16 mmol, 0.04 e.q.), and potassium phosphate (10 mmol, 2.5 e.q.) were added. DMF:ethanol:water = (10 mL: 10 mL:5 mL) was then added as a solvent, and the reaction system was heated to 95°C in the sealed tube, stirred and reacted overnight under argon atmosphere. After the conversion of the substrate was completed as detected by thin-layer chromatography plate, the organic solvent was removed by rotary evaporation, the mixture was extracted with ethyl acetate to give organic phase, and the organic phase was separated and purified by column chromatography to give intermediate **10.**

The intermediate **10** (4 mmol, 1.0 e.q.) was dissolved in 20 mL of dichloromethane as a solvent, 2 mL of 4 N hydrochloric acid-dioxane solution was added. The mixture was reacted with stirring at room temperature for 2 h. A white solid was precipitated, filtered out under vacuum, and dried to give intermediate **11.**

**11** and the previously obtained carboxylic acid intermediate **6** were added to DMF as a solvent according to an equivalent ratio of 1:1. HATU (1.5 e.q.) and DIPEA (2.0 e.q.) were added. The mixture was reacted at 70°C for 12 h. The reaction solution was extracted with ethyl acetate, and washed with a saturated ammonium chloride solution for three times. The organic phase was concentrated to dryness by rotary evaporation, and purified by silica gel column chromatography to give product **12.** When Boc (*tert*-butoxycarbonyl) protection was present in the amine compound 6, finally, the final product was obtained after the removal of the tert-butoxycarbonyl from compound **7** by hydrochloric acid.

(4) The synthetic route for preparation of the compounds of Examples 7 and 8 was as follows:

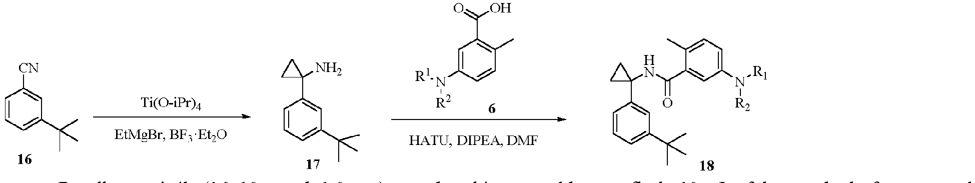

*m*-*tert*-Butylbenzomtrile (16, 10 mmol, 1.0 e.q.) was placed in a round bottom flask, 10 mL of dry tetrahydrofuran was added as a solvent, and tetraisopropyl titanate (10 mmol, 1.1 e.q.) was then added. The reaction system was cooled to -78°C, and ethyl Grignard reagent (20 mmol, 2.0 e.q.) was then slowly added dropwise to the reaction system. After the dropwise addition was completed, the reaction system was warmed to room temperature and reacted for 1.5 h. Boron trifluoride diethyl etherate (20 mmol, 2.0 e.q.) was then added dropwise to the reaction system. After the dropwise addition was completed, the mixture was reacted with stirring at room temperature for 3 h. After the reaction was completed, 20 mL of 2 N hydrochloric acid was added dropwise to the reaction system, the reaction was quenched with stirring for 20 min, and an excess saturated sodium hydroxide solution was then added. The mixture was extracted with ethyl acetate, and the organic phase was collected, concentrated to dryness by rotary evaporation, and separated by silica gel column chromatography to give *m*-*tert*-butylphenyl-cyclopropylamine intermediate **17.**

The *m*-*tert*-butylphenyl-cyclopropylamine intermediate **17** and the previously obtained carboxylic acid intermediate 6 were added to DMF as a solvent according to an equivalent ratio of 1:1. HATU (1.5 e.q.) and DIPEA (2.0 e.q.) were added, and the mixture was reacted at 70°C for 12 h. The reaction solution was extracted with ethyl acetate, and washed with a saturated ammonium chloride solution for three times. The organic phase was concentrated to dryness by rotary evaporation, and purified by silica gel column chromatography to give product **18.**

In Example 7, Boc (*tert*-butoxycarbonyl) protection was present in the amine compound **6,** and finally, the final product was obtained after the removal of the tert-butoxycarbonyl from compound **18** by hydrochloric acid.

(5) The synthetic route for preparation of the compounds of Examples 9-18 was as follows:

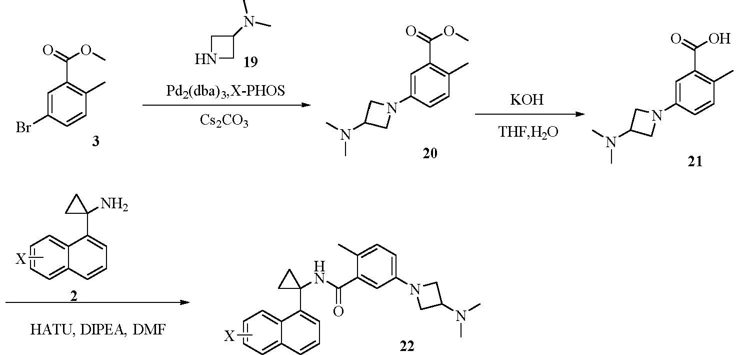

Methyl 2-methyl-5-bromobenzoate **(3**, 10 mmol, 1.0 e.q.) was placed in a 350 mL sealed tube. 3-dimethylaminoazetidine **(19,** 15 mmol, 1.5 e.q.), tris(dibenzylideneacetone)dipalladium (0.2 mmol, 0.02 e.q.), X-PHOS ligand (0.4 mmol, 0.04 e.q.), and cesium carbonate (50 mmol, 5.0 e.q.) were added, and toluene as a solvent (60 mL) was then added. The reaction system was heated to 110°C in the sealed tube, stirred and reacted overnight under argon atmosphere. After the conversion of the substrate was completed as detected by thin-layer chromatography plate, the organic solvent was removed by rotary evaporation, and the mixture was separated and purified by column chromatography to give intermediate **20.**

The intermediate **20** (1.0 e.q.) was placed in a round bottom flask, tetrahydrofuran: water = 2:1 was added as a solvent, and potassium hydroxide (4.0 e.q.) was then added to the reaction system. After the mixture was reacted with stirring at 60°C for 6 h, the organic solvent was removed by rotary evaporation, and the remaining aqueous solution was then acidified with 2 N hydrochloric acid and adjusted to pH = 1. The aqueous solution was then concentrated to dryness by rotary evaporation, and extracted with a methanol solution to give organic substance. The organic substance was filtered under vacuum, the filter residue was removed, and the filtrate was collected and concentrated to dryness by rotary evaporation to give a white solid, which was intermediate **21.**

**21** and the previously obtained three-membered ring amine intermediate **2** were added to DMF as a solvent according to an equivalent ratio of 1:1. HATU (1.5 e.q.) and DIPEA (2.0 e.q.) were added. The mixture was reacted at 70°C for 12 h. The reaction solution was extracted with ethyl acetate, and washed with a saturated ammonium chloride solution for three times. The organic phase was concentrated to dryness by rotary evaporation, and purified by silica gel column chromatography to give product **22.** The final product **22** was the compounds in the related examples.

(6) The synthetic route for preparation of the compound of Example 19 was as follows:

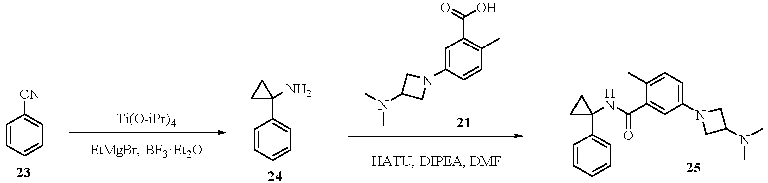

Benzonitrile **(23**, 10 mmol, 1.0 e.q.) was placed in a round bottom flask, 10 mL of dry tetrahydrofuran was added as a solvent, and tetraisopropyl titanate (10 mmol, 1.1 e.q.) was then added. The reaction system was cooled to -78°C, and ethyl Grignard reagent (20 mmol, 2.0 e.q.) was then slowly added dropwise to the reaction system. After the dropwise addition was completed, the reaction system was warmed to room temperature, and reacted for 1.5 h. Boron trifluoride diethyl etherate (20 mmol, 2.0 e.q.) was then added dropwise to the reaction system. After the dropwise addition was completed, the mixture was reacted with stirring at room temperature for 3 h. After the reaction was completed, 20 mL of 2 N hydrochloric acid was added dropwise to the reaction system, the reaction was quenched with stirring for 20 min, and an excess saturated sodium hydroxide solution was then added. The mixture was extracted with ethyl acetate, and the organic phase was collected, concentrated to dryness by rotary evaporation, and separated by silica gel column chromatography to give *m*-*tert*-butylphenyl-cyclopropylamine intermediate **24.**

The cyclopropylamine intermediate **24** and the previously obtained carboxylic acid intermediate **21** were added to DMF as a solvent according to an equivalent ratio of 1:1. HATU (1.5 e.q.) and DIPEA (2.0 e.q.) were added. The mixture was reacted at 70°C for 12 h. The reaction solution was extracted with ethyl acetate, and washed with a saturated ammonium chloride solution for three times. The organic phase was concentrated to dryness by rotary evaporation, and purified by silica gel column chromatography to give product **25.** The final product **25** was **C19.**

(7) The synthetic route for preparation of the compound of Example 20 was as follows:

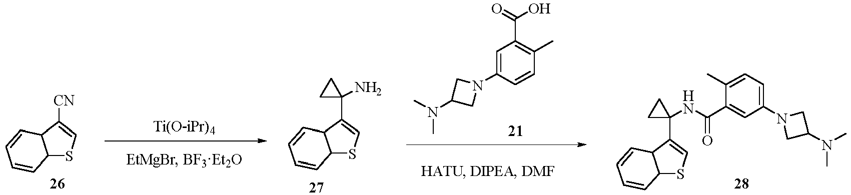

Benzo[B]thiophene-3-carbonitrile **(26,** 10 mmol, 1.0 e.q.) was placed in a round bottom flask, 10 mL of dry tetrahydrofuran was added as a solvent, and tetraisopropyl titanate (10 mmol, 1.1 e.q.) was then added. The reaction system was cooled to - 78°C, and ethyl Grignard reagent (20 mmol, 2.0 e.q.) was then slowly added dropwise to the reaction system. After the dropwise addition was completed, the reaction system was warmed to room temperature, and reacted for 1.5 h. Boron trifluoride diethyl etherate (20 mmol, 2.0 e.q.) was then added dropwise to the reaction system. After the dropwise addition was completed, the mixture was reacted with stirring at room temperature for 3 h. After the reaction was completed, 20 mL of 2 N hydrochloric acid was added dropwise to the reaction system, the reaction was quenched with stirring for 20 min, and an excess saturated sodium hydroxide solution was then added. The mixture was extracted with ethyl acetate, and the organic phase was collected, concentrated to dryness by rotary evaporation, and separated by silica gel column chromatography to give m-tert-butylphenyl-cyclopropylamine intermediate **27.**

The cyclopropylamine intermediate **27** and the previously obtained carboxylic acid intermediate **21** were added to DMF as a solvent according to an equivalent ratio of 1:1. HATU (1.5 e.q.) and DIPEA (2.0 e.q.) were added. The mixture was reacted at 70°C for 12 h. The reaction solution was extracted with ethyl acetate, and washed with a saturated ammonium chloride solution for three times. The organic phase was concentrated to dryness by rotary evaporation, and purified by silica gel column chromatography to give product **28.** The final product **28** was **C20.**

(8) The synthetic route for preparation of the compound of Example 21 was as follows:

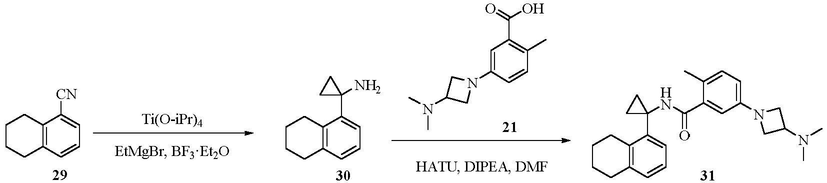

5,6,8,9-tetrahydro-1-cyanonaphthalene **(29,** 10 mmol, 1.0 e.q.) was placed in a round bottom flask, 10 mL of dry tetrahydrofuran was added as a solvent, and tetraisopropyl titanate (10 mmol, 1.1 e.q.) was then added. The reaction system was cooled to -78°C, and ethyl Grignard reagent (20 mmol, 2.0 e.q.) was then slowly added dropwise to the reaction system. After the dropwise addition was completed, the reaction system was warmed to room temperature, and reacted for 1.5 h. Boron trifluoride diethyl etherate (20 mmol, 2.0 e.q.) was then added dropwise to the reaction system. After the dropwise addition was completed, the mixture was reacted with stirring at room temperature for 3 h. After the reaction was completed, 20 mL of 2 N hydrochloric acid was added dropwise to the reaction system, the reaction was quenched with stirring for 20 min, and an excess saturated sodium hydroxide solution was then added. The mixture was extracted with ethyl acetate, and the organic phase was collected, concentrated to dryness by rotary evaporation, and separated by silica gel column chromatography to give *m*-*tert*-butylphenyl-cyclopropylamine intermediate **30.**

The cyclopropylamine intermediate **30** and the previously obtained carboxylic acid intermediate **21** were added to DMF as a solvent according to an equivalent ratio of 1:1. HATU (1.5 e.q.) and DIPEA (2.0 e.q.) were added. The mixture was reacted at 70°C for 12 h. The reaction solution was extracted with ethyl acetate, and washed with a saturated ammonium chloride solution for three times. The organic phase was concentrated to dryness by rotary evaporation, and purified by silica gel column chromatography to give product **31.** The final product **31** was **C21.**

(9) The synthetic route for preparation of the compound of Example 22 was as follows:

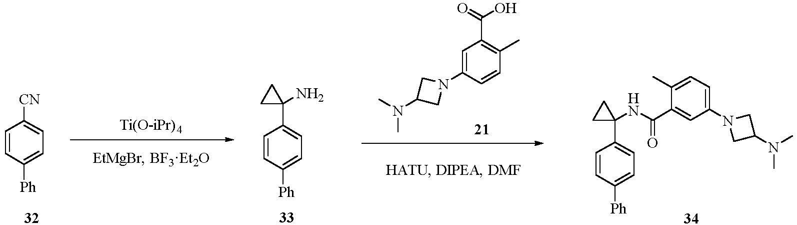

4-cyanobiphenyl **(32,** 10 mmol, 1.0 e.q.) was placed in a round bottom flask, 10 mL of dry tetrahydrofuran was added as a solvent, and tetraisopropyl titanate (10 mmol, 1.1 e.q.) was then added. The reaction system was cooled to -78°C, and ethyl Grignard reagent (20 mmol, 2.0 e.q.) was then slowly added dropwise to the reaction system. After the dropwise addition was completed, the reaction system was warmed to room temperature, and reacted for 1.5 h. Boron trifluoride diethyl etherate (20 mmol, 2.0 e.q.) was then added dropwise to the reaction system. After the dropwise addition was completed, the mixture was reacted with stirring at room temperature for 3 h. After the reaction was completed, 20 mL of 2 N hydrochloric acid was added dropwise to the reaction system, the reaction was quenched with stirring for 20 min, and an excess saturated sodium hydroxide solution was then added. The mixture was extracted with ethyl acetate, and the organic phase was collected, concentrated to dryness by rotary evaporation, and separated by silica gel column chromatography to give *m*-*tert*-butylphenyl-cyclopropylamine intermediate **33.**

The cyclopropylamine intermediate **33** and the previously obtained carboxylic acid intermediate **21** were added to DMF as a solvent according to an equivalent ratio of 1:1. HATU (1.5 e.q.) and DIPEA (2.0 e.q.) were added. The mixture was reacted at 70°C for 12 h. The reaction solution was extracted with ethyl acetate, and washed with a saturated ammonium chloride solution for three times. The organic phase was concentrated to dryness by rotary evaporation, and purified by silica gel column chromatography to give product **34.** The final product **34** was Example **C22.**

(10) The synthetic route for preparation of the compound of Example 23 was as follows:

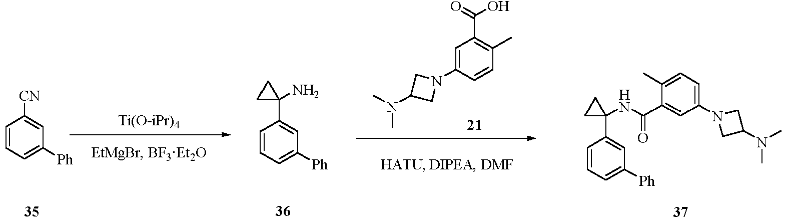

3-cyanobiphenyl **(35,** 10 mmol, 1.0 e.q.) was placed in a round bottom flask, 10 mL of dry tetrahydrofuran was added as a solvent, and tetraisopropyl titanate (10 mmol, 1.1 e.q.) was then added. The reaction system was cooled to -78°C, and ethyl Grignard reagent (20 mmol, 2.0 e.q.) was then slowly added dropwise to the reaction system. After the dropwise addition was completed, the reaction system was warmed to room temperature and reacted for 1.5 h. Boron trifluoride diethyl etherate (20 mmol, 2.0 e.q.) was then added dropwise to the reaction system. After the dropwise addition was completed, the mixture was reacted with stirring at room temperature for 3 h. After the reaction was completed, 20 mL of 2 N hydrochloric acid was added dropwise to the reaction system, the reaction was quenched with stirring for 20 min, and an excess saturated sodium hydroxide solution was then added. The mixture was extracted with ethyl acetate, and the organic phase was collected, concentrated to dryness by rotary evaporation, and separated by silica gel column chromatography to give *m-tert-*butylphenyl-cyclopropylamine intermediate **36.**

The cyclopropylamine intermediate **36** and the previously obtained carboxylic acid intermediate **21** were added to DMF as a solvent according to an equivalent ratio of 1:1. HATU (1.5 e.q.) and DIPEA (2.0 e.q.) were added. The mixture was reacted at 70°C for 12 h. The reaction solution was extracted with ethyl acetate, and washed with a saturated ammonium chloride solution for three times. The organic phase was concentrated to dryness by rotary evaporation, and purified by silica gel column chromatography to give product **37.** The final product **37** was **C23.**

(11) The synthetic route for preparation of the compound of Example 24 was as follows:

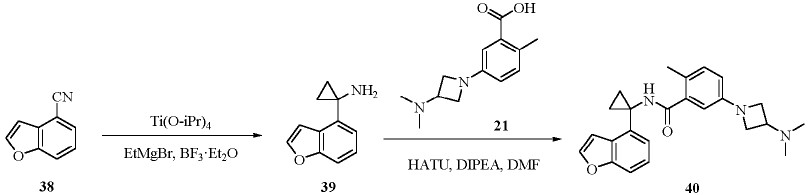

Benzofuran-4-carbonitrile **(38,** 10 mmol, 1.0 e.q.) was placed in a round bottom flask, 10 mL of dry tetrahydrofuran was added as a solvent, and tetraisopropyl titanate (10 mmol, 1.1 e.q.) was then added. The reaction system was cooled to -78°C, and ethyl Grignard reagent (20 mmol, 2.0 e.q.) was then slowly added dropwise to the reaction system. After the dropwise addition was completed, the reaction system was warmed to room temperature and reacted for 1.5 h. Boron trifluoride diethyl etherate (20 mmol, 2.0 e.q.) was then added dropwise to the reaction system. After the dropwise addition was completed, the mixture was reacted with stirring at room temperature for 3 h. After the reaction was completed, 20 mL of 2 N hydrochloric acid was added dropwise to the reaction system, the reaction was quenched with stirring for 20 min, and an excess saturated sodium hydroxide solution was then added. The mixture was extracted with ethyl acetate, and the organic phase was collected, concentrated to dryness by rotary evaporation, and separated by silica gel column chromatography to give *m-tert-*butylphenyl-cyclopropylamine intermediate **39.**

The cyclopropylamine intermediate **39** and the previously obtained carboxylic acid intermediate **21** were added to DMF as a solvent according to an equivalent ratio of 1:1. HATU (1.5 e.q.) and DIPEA (2.0 e.q.) were added, and the mixture was reacted at 70°C for 12 h. The reaction solution was extracted with ethyl acetate, and washed with a saturated ammonium chloride solution for three times. The organic phase was concentrated to dryness by rotary evaporation, and purified by silica gel column chromatography to give product **40.** The final product **40** was **C24.**

(12) The synthetic route for preparation of the compound of Example 25 was as follows:

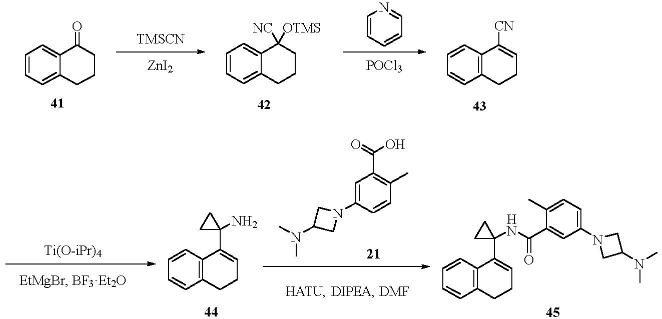

1-tetralone **(41**, 20 mmol, 1.0 e.q.), trimethylsilyl cyanide (24 mmol, 1.2 e.q.), and zinc iodide (0.5 mmol, 0.025 e.q.) were placed in a 250 mL round bottom flask, and 100 mL of toluene was added as a solvent. The mixture was reacted with stirring at room temperature for 8 h. After the reaction was completed as detected by thin-layer chromatography plate, the organic solvent was removed by rotary evaporation and then separated and purified by silica gel column chromatography to give intermediate **42.** The intermediate **42** (15 mmol, 1.0 e.q.) was placed in a 250 mL round bottom flask, 50 mL of pyridine was added, and phosphorus oxychloride (45 mmol, 3.0 e.q.) was added dropwise at room temperature. After the addition was completed, the mixture was warmed to 80°C, and reacted for 8 h. After the reaction was completed, the organic solvent was removed by rotary evaporation. The reaction system was adjusted to neutrality, and extracted with ethyl acetate to give organic phase. The organic phase was concentrated to dryness by rotary evaporation, and separated and purified by silica gel column chromatography to give intermediate **43.**

The intermediate **43** (10 mmol, 1.0 e.q.) was placed in a round bottom flask, 10 mL of dry tetrahydrofuran was added as a solvent, and tetraisopropyl titanate (10 mmol, 1.1 e.q.) was then added. The reaction system was cooled to -78°C, and ethyl Grignard reagent (20 mmol, 2.0 e.q.) was then slowly added dropwise to the reaction system. After the dropwise addition was completed, the reaction system was warmed to room temperature and reacted for 1.5 h. Boron trifluoride diethyl etherate (20 mmol, 2.0 e.q.) was then added dropwise to the reaction system. After the dropwise addition was completed, the mixture was reacted with stirring at room temperature for 3 h. After the reaction was completed, 20 mL of 2 N hydrochloric acid was added dropwise to the reaction system, the reaction was quenched with stirring for 20 min, and an excess saturated sodium hydroxide solution was then added. The mixture was extracted with ethyl acetate, and the organic phase was collected, concentrated to dryness by rotary evaporation, and separated by silica gel column chromatography to give *m-tert-*butylphenyl-cyclopropylamine intermediate **44.**

The cyclopropylamine intermediate **44** and the previously obtained carboxylic acid intermediate **21** were added to DMF as a solvent according to an equivalent ratio of 1:1. HATU (1.5 e.q.) and DIPEA (2.0 e.q.) were added, and the mixture was reacted at 70°C for 12 h. The reaction solution was extracted with ethyl acetate, and washed with a saturated ammonium chloride solution for three times. The organic phase was concentrated to dryness by rotary evaporation, and purified by silica gel column chromatography to give product **45.** The final product **45** was **C25.**

(13) The synthetic route for preparation of the compound of Example 26 was as follows:

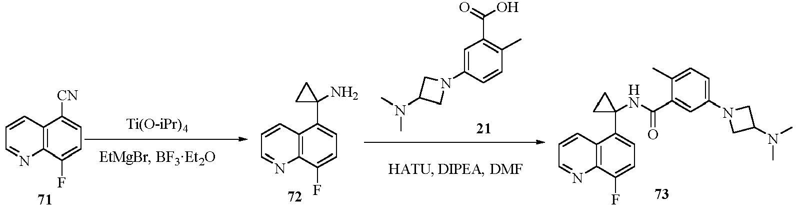

8-fluoroquinoline-4-carbonitrile **(71,** 10 mmol, 1.0 e.q.) was placed in a round bottom flask, 10 mL of dry tetrahydrofuran was added as a solvent, and tetraisopropyl titanate (10 mmol, 1.1 e.q.) was then added. The reaction system was cooled to - 78°C, and ethyl Grignard reagent (20 mmol, 2.0 e.q.) was then slowly added dropwise to the reaction system. After the dropwise addition was completed, the reaction system was warmed to room temperature and reacted for 1.5 h. Boron trifluoride diethyl etherate (20 mmol, 2.0 e.q.) was then added dropwise to the reaction system. After the dropwise addition was completed, the mixture was reacted with stirring at room temperature for 3 h. After the reaction was completed, 20 mL of 2 N hydrochloric acid was added dropwise to the reaction system, the reaction was quenched with stirring for 20 min, and an excess saturated sodium hydroxide solution was then added. The mixture was extracted with ethyl acetate, and the organic phase was collected, concentrated to dryness by rotary evaporation, and separated by silica gel column chromatography to give 8-fluoroquinolyl-cyclopropylamine intermediate **72.**

The cyclopropylamine intermediate **72** and the previously obtained carboxylic acid intermediate **21** were added to DMF as a solvent according to an equivalent ratio of 1:1. HATU (1.5 e.q.) and DIPEA (2.0 e.q.) were added. The mixture was reacted at 70°C for 12 h. The reaction solution was extracted with ethyl acetate, and washed with a saturated ammonium chloride solution for three times. The organic phase was concentrated to dryness by rotary evaporation, and purified by silica gel column chromatography to give product **73.** The final product **73** was **C26.**

(14) The general synthetic route for preparation of the compound of Example 27 was as follows:

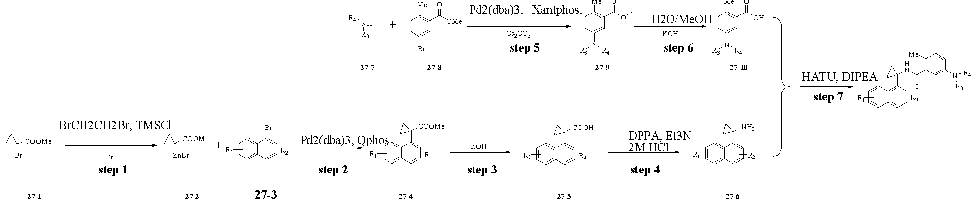

The preparation of the branched route step 1 was as follows:

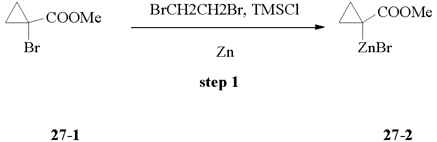

1.64 g (25 mmol, 2.5 eq.) of zinc powder was weighed, washed with 2 M hydrochloric acid for 3 times or washed with 0.5 M hydrochloric acid with stirring for 5 min, separately washed with absolute ethanol and anhydrous ether for 3 times, and dried with stirring for 2 h with a vacuum pump at 140°C in an oil bath. The activated zinc powder was placed in a three-necked flask, extra dry tetrahydrofuran (5 mL) was added under Ar atmosphere, and 173 µL (2 mmol, 0.2 eq.) of 1,2-dibromoethane was added. The mixture was heated at reflux to 75°C. The reaction was continued for 30 min after generation of large amount of bubbles was observed. The mixture was naturally cooled to room temperature, 255 µL (2 mmol, 0.2 eq) of trimethylchlorosilane was slowly added, and the reaction was continued for 15 min after generation of large amount of bubbles was observed. The mixture was heated to 65°C. 1.04 mL of methyl 1-bromocyclopropanecarboxylate (10 mmol, 1.0 eq) was dissolved in 15 mL of extra dry tetrahydrofuran and slowly added dropwise to the reaction system. The mixture was reacted at 65°C for 4 h (when a large amount was added, the reaction may be carried out overnight to ensure complete reaction) to give intermediate **27-2,** which was directly added in step 2 without post-treatment.

The preparation of the branched route step 2 was as follows:

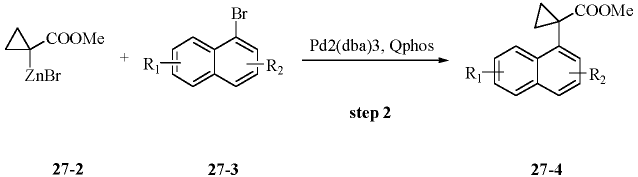

The synthetic route for preparation of intermediate 27-4a was as follows:

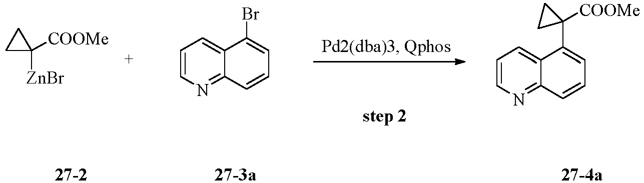

2080 mg (10 mmol, 1.0 eq.) of 5-bromoquinoline 27-3a was weighed, 91.5 mg (0.1 mmol, 0.01 eq) of Pd₂(dba)₃ and 71 mg (0.1 mmol, 0.01 eq) of Qphos were added. The mixture was dissolved in 20 mL of anhydrous tetrahydrofuran. Intermediate 27-2 Reformatsky reagent (20 mmol, 1 N, 2.0 eq) dissolved in 20 mL of tetrahydrofuran was added under Ar atmosphere. The mixture was stirred at room temperature for 30 min. After the reaction was completely converted as detected, the solvent was removed by rotary evaporation, and the mixture was dissolved in ethyl acetate, washed three times with water and once with saturated brine. The organic phase was retained and dried over anhydrous sodium sulfate. The organic phase was purified by column chromatography to give product intermediate **27-4a.**

The synthetic route for preparation of intermediate **27-4b** was as follows:

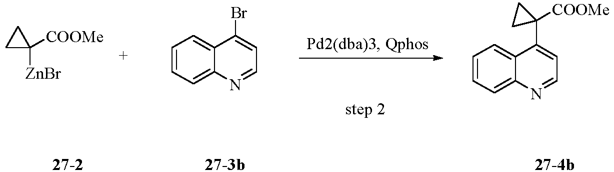

2080 mg (10 mmol, 1.0 eq.) of 4-bromoquinoline **27-3b** was weighed, 91.5 mg (0.1 mmol, 0.01 eq) of Pd₂(dba)₃ and 71 mg (0.1 mmol, 0.01 eq) of Qphos were added. The mixture was dissolved in 20 mL of anhydrous tetrahydrofuran. Intermediate 27-2 Reformatsky reagent (20 mmol, 1 N, 2.0 eq) dissolved in 20 mL of tetrahydrofuran was added under Ar atmosphere. The mixture was stirred at room temperature for 30 min. After the reaction was completely converted as detected, the solvent was removed by rotary evaporation, and the mixture was dissolved in ethyl acetate, washed three times with water and once with saturated brine. The organic phase was retained and dried over anhydrous sodium sulfate. The organic phase was purified by column chromatography to give product intermediate **27-4b.**

The synthetic route for preparation of intermediate **27-4c** was as follows:

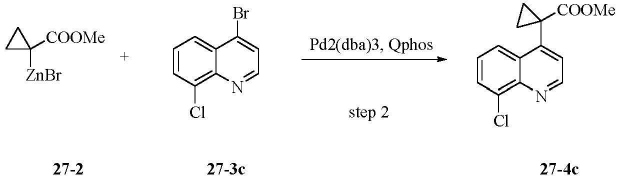

2410 mg (10 mmol, 1.0 eq.) of 4-bromo-8-chloroquinoline **27-3c** was weighed, 91.5 mg (0.1 mmol, 0.01 eq) of Pd₂(dba)₃ and 71 mg (0.1 mmol, 0.01 eq) of Qphos were added. The mixture was dissolved in 20 mL of anhydrous tetrahydrofuran. Intermediate **27-2** Reformatsky reagent (20 mmol, 1 N, 2.0 eq) dissolved in 20 mL of tetrahydrofuran was added under Ar atmosphere. The mixture was stirred at room temperature for 30 min. After the reaction was completely converted as detected, the solvent was removed by rotary evaporation, and the mixture was dissolved in ethyl acetate, washed three times with water and once with saturated brine. The organic phase was retained and dried over anhydrous sodium sulfate. The organic phase was purified by column chromatography to give product intermediate **27-4c.**

The synthetic route for preparation of intermediate **27-4d** was as follows:

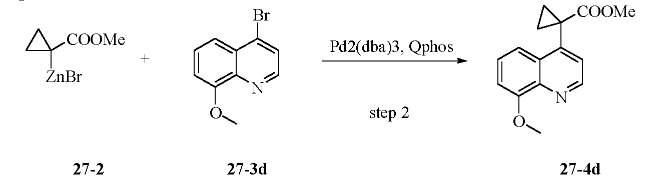

2370 mg (10 mmol, 1.0 eq.) of 4-bromo-8-methoxyquinoline 27-**3d** was weighed, 91.5 mg (0.1 mmol, 0.01 eq) of Pd₂(dba)₃ and 71 mg (0.1 mmol, 0.01 eq) of Qphos were added. The mixture was dissolved in 20 mL of anhydrous tetrahydrofuran. Intermediate 27-2 Reformatsky reagent (20 mmol, 1 N, 2.0 eq) dissolved in 20 mL of tetrahydrofuran was added under Ar atmosphere. The mixture was stirred at room temperature for 30 min. After the reaction was completely converted as detected, the solvent was removed by rotary evaporation, and the mixture was dissolved in ethyl acetate, washed three times with water and once with saturated brine. The organic phase was retained and dried over anhydrous sodium sulfate. The organic phase was purified by column chromatography to give product intermediate **27-4d.**

The synthetic route for preparation of intermediate **27-4e** was as follows:

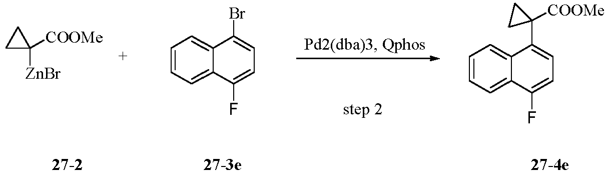

2239 mg (10 mmol, 1.0 eq.) of 1-bromo-4-fluoronaphthalene **27-3e** was weighed, 91.5 mg (0.1 mmol, 0.01 eq) of Pd₂(dba)₃ and 71 mg (0.1 mmol, 0.01 eq) of Qphos were added. The mixture was dissolved in 20 mL of anhydrous tetrahydrofuran. Intermediate 27-2 Reformatsky reagent (20 mmol, 1 N, 2.0 eq) dissolved in 20 mL of tetrahydrofuran was added under Ar atmosphere. The mixture was stirred at room temperature for 30 min. After the reaction was completely converted as detected, the solvent was removed by rotary evaporation, and the mixture was dissolved in ethyl acetate, washed three times with water and once with saturated brine. The organic phase was retained and dried over anhydrous sodium sulfate. The organic phase was purified by column chromatography to give product intermediate **27-4e.**

The synthetic route for preparation of intermediate 27-4f was as follows:

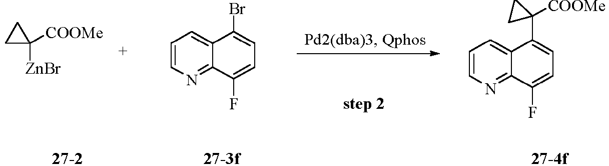

2249 mg (10 mmol, 1.0 eq.) of 5-bromo-8-fluoroquinoline **27-3f** was weighed, 91.5 mg (0.1 mmol, 0.01 eq) of Pd₂(dba)₃ and 71 mg (0.1 mmol, 0.01 eq) of Qphos were added. The mixture was dissolved in 20 mL of anhydrous tetrahydrofuran. Intermediate **27-2** Reformatsky reagent (20 mmol, 1 N, 2.0 eq) dissolved in 20 mL of tetrahydrofuran was added under Ar atmosphere. The mixture was stirred at room temperature for 30 min. After the reaction was completely converted as detected, the solvent was removed by rotary evaporation, and the mixture was dissolved in ethyl acetate, washed three times with water and once with saturated brine. The organic phase was retained and dried over anhydrous sodium sulfate. The organic phase was purified by column chromatography to give product intermediate **27-4f.**

The synthetic route for preparation of intermediate **27-4g** was as follows:

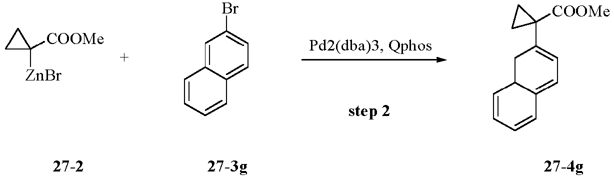

2059 mg (10 mmol, 1.0 eq.) of 2-bromonaphthalene **27-3g** was weighed, 91.5 mg (0.1 mmol, 0.01 eq) of Pd₂(dba)₃ and 71 mg (0.1 mmol, 0.01 eq) of Qphos were added. The mixture was dissolved in 20 mL of anhydrous tetrahydrofuran. Intermediate **27-2** Reformatsky reagent (20 mmol, 1 N, 2.0 eq) dissolved in 20 mL of tetrahydrofuran was added under Ar atmosphere. The mixture was stirred at room temperature for 30 min. After the reaction was completely converted as detected, the solvent was removed by rotary evaporation, and the mixture was dissolved in ethyl acetate, washed three times with water and once with saturated brine. The organic phase was retained and dried over anhydrous sodium sulfate. The organic phase was purified by column chromatography to give product intermediate **27-4g.**

The preparation of the branched route step 3 was as follows:

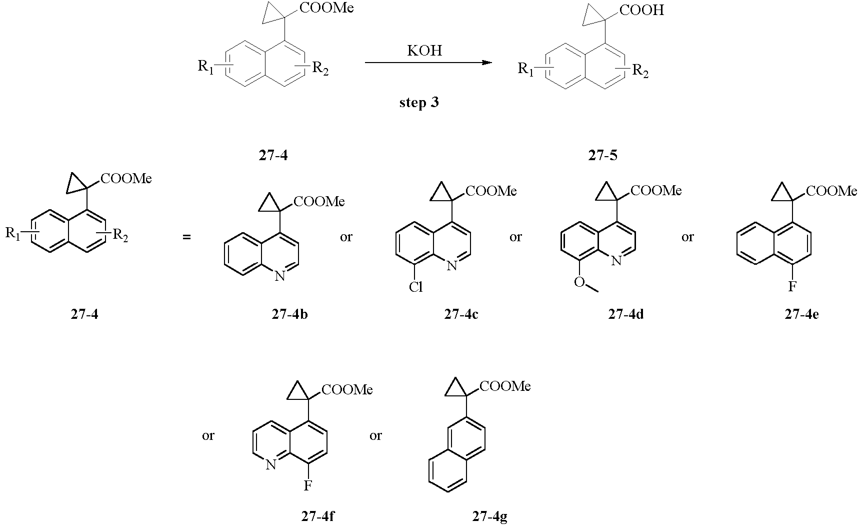

The synthetic route for preparation of intermediate 27-5a was as follows:

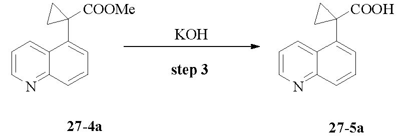

2518 mg (11.09 mmol, 1.0 eq) of intermediate 27-4a was weighed and dissolved in a mixed solvent of tetrahydrofuran/methanol/water (3:1:1, 65 mL), and 2484 mg of potassium hydroxide (44.37 mmol, 4.0 eq) was added. The mixture was reacted at 50°C for 8 h. After the reactant was completely converted as detected, 2 N HCl solution was added to adjust pH to 3. After all solvents were removed by rotary evaporation, 25 mL of methanol was added. The mixture was filtered, the filtrate was concentrated to dryness by rotary evaporation to give a gray solid, and finally the gray solid was washed repeatedly with DCM/PE to give a white solid product 27-5a.

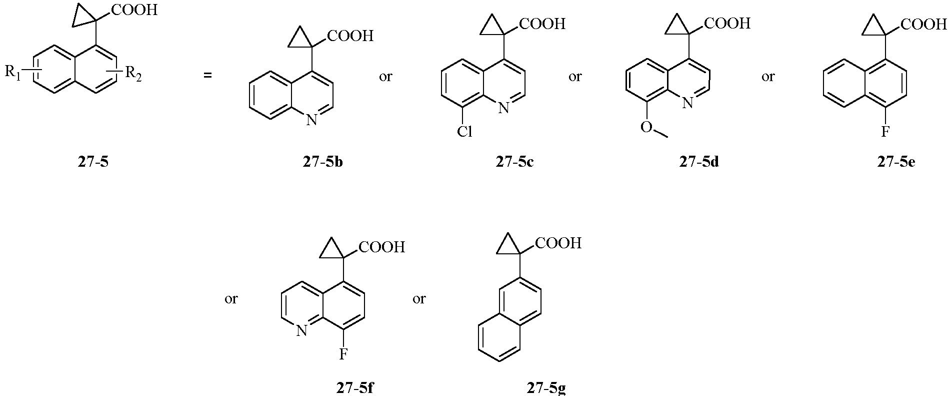

The routes for intermediates 27-5b to 27-5g were the same as the preparation method of intermediate 27-5a.

The preparation route of the branched route step 4 was as follows:

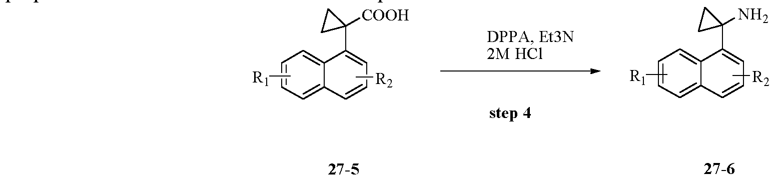

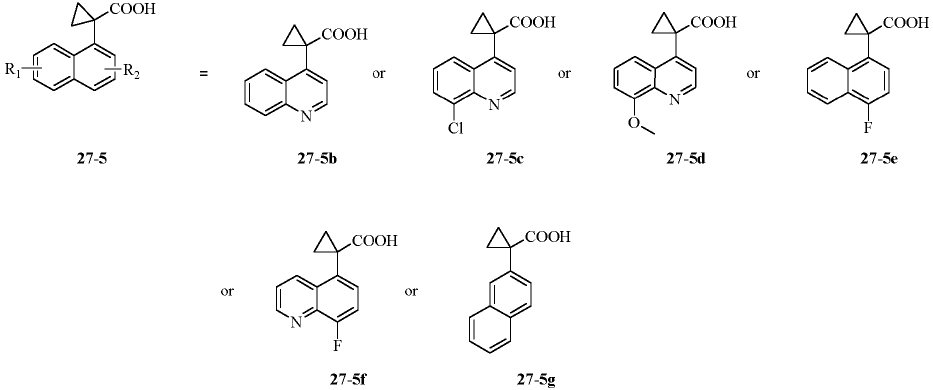

The synthetic route for preparation of intermediate 27-6a was as follows:

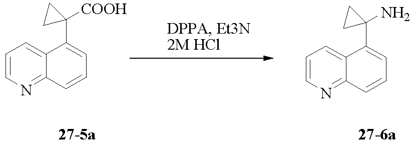

426 mg (2 mmol, 1.0 eq) of intermediate **27-5a** was weighed and dissolved in 25 mL of extra dry toluene, 0.611 mL of triethylamine (4.4 mmol, 2.2 eq) was added, and 0.516 mL of DPPA (2.4 mmol, 1.2 eq) was added under Ar atmosphere. The mixture was stirred at room temperature for 30 min until all carboxylic acid materials were converted into acyl azide, and heated to 75°C and reacted for 4 h until most acyl azide was converted into isocyanate. Excess hydrochloric acid (2 M aqueous solution, > 4.0 eq) was added, and the mixture was cooled to 60°C and reacted overnight. A sodium bicarbonate solution was added to adjust the pH to alkalinity, and the mixture was extracted with ethyl acetate. The organic phase was added to 2.5 mL of HCl (4 M HCl in dioxane,10 mmol, 2.0 eq) solution, and the mixture was filtered by using a sand-core funnel and washed with petroleum ether and ethyl acetate for multiple times to give a white powder product **27-6a.**

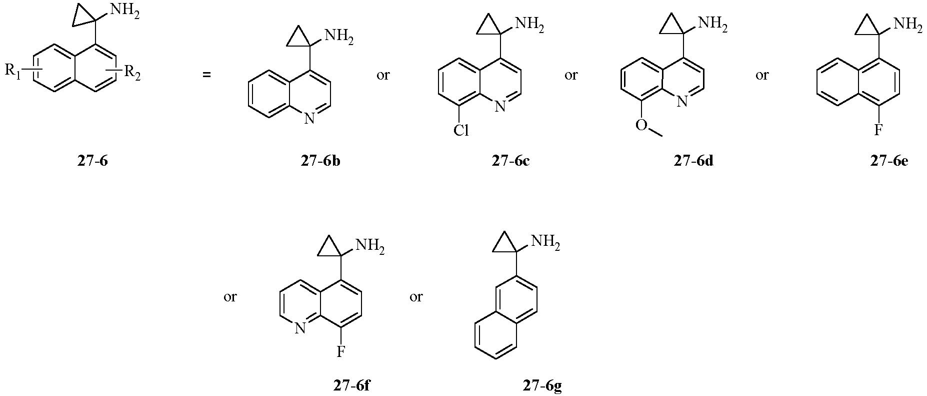

The routes for intermediates **27-6b to 27-6g** were the same as the preparation method of intermediate **27-6a.**

The preparation route of the branched route step 5 was as follows:

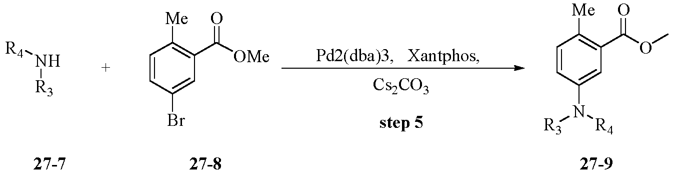

The synthetic route for preparation of intermediate **27-9a** was as follows:

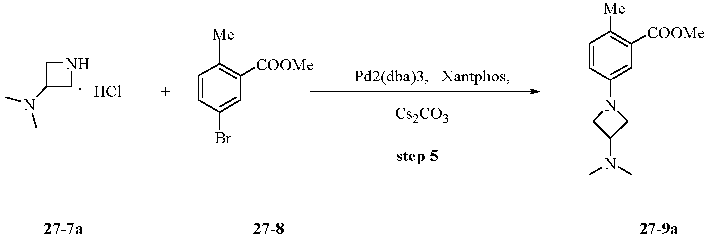

4580 mg of methyl 2-methyl-5-bromobenzoate **27-8** (20 mmol, 1.0 eq), 3740 mg of 3-(dimethylamino)azetidine dihydrochloride **27-7a** (22 mmol, 1.1 eq), 370 mg (0.4 mmol, 0.02 eq) of Pd₂(dba)₃, 760 mg of XPhos (1.6 mmol, 0.08 eq), and 26080 mg of cesium carbonate (80 mmol, 4.0 eq) were weighed, dissolved in 100 mL of toluene, placed in a sealed tube, and heated to 110°C overnight under Ar atmosphere. The mixture was washed with water and ethyl acetate, and the organic phase was retained. The organic phase was subjected to column chromatography to give product intermediate **27-9a.**

The synthetic route for preparation of intermediate **27-9b** was as follows:

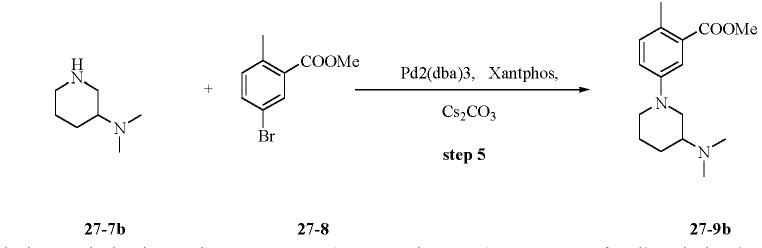

1700 mg of methyl 2-methyl-5-bromobenzoate **27-8** (7.4 mmol, 1.0 eq), 1000 mg of 3-dimethylaminopiperidine **27-7b** (7.8 mmol, 1.05 eq), 204 mg (0.22 mmol, 0.03 eq) of Pd₂(dba)₃, 425 mg of XPhos (0.89 mmol, 0.12 eq), and 9600 mg of cesium carbonate (29.72 mmol, 4.0 eq) were weighed, dissolved in 45 mL of toluene, placed in a sealed tube, and heated to 110°C and reacted overnight underAr atmosphere. The mixture was washed with water and ethyl acetate, and the organic phase was retained. The organic phase was subjected to column chromatography to give product intermediate **27-9b.**

The synthetic route for preparation of intermediate **27-9c** was as follows:

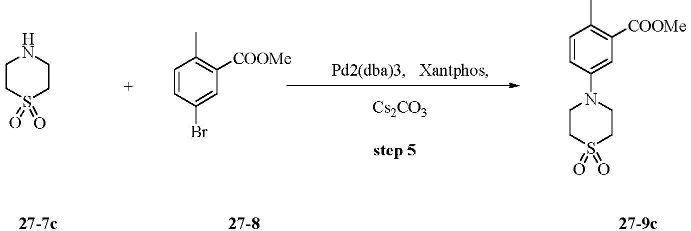

2290 mg of methyl 2-methyl-5-bromobenzoate **27-8** (10 mmol, 1.0 eq), 1480 mg of thiomorpholine-1,1-dioxide **27-7c** (11 mmol, 1.1 eq), 274.5 mg (0.3 mmol, 0.03 eq) of Pd₂(dba)₃, 572 mg of XPhos (1.2 mmol, 0.12 eq), and 1304 mg of cesium carbonate (40 mmol, 4.0 eq) were weighed, dissolved in 60 mL of toluene, placed in a sealed tube, and heated to 110°C and reacted overnight under Ar atmosphere. The mixture was washed with water and ethyl acetate, and the organic phase was retained. The organic phase was subjected to column chromatography to give product intermediate **27-9c.**

The synthetic route for preparation of intermediate **27-9d** was as follows:

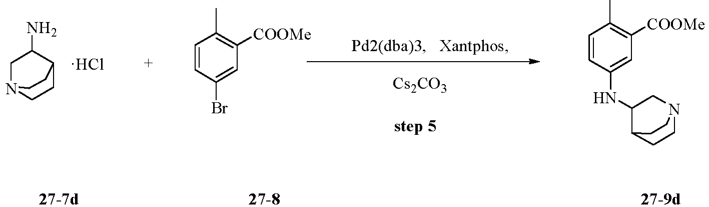

2290 mg of methyl 2-methyl-5-bromobenzoate **27-8** (10 mmol, 1.0 eq), 2190 mg of 3-aminoquinuclidine hydrochloride 27-7d (11 mmol, 1.1 eq), 92 mg (0.1 mmol, 0.01 eq) of Pd₂(dba)₃, 1431 mg of XPhos (0.3 mmol, 0.03 eq), and 1304 mg of cesium carbonate (40 mmol, 4.0 eq) were weighed, dissolved in 60 mL of toluene, placed in a sealed tube, and heated to 110°C and reacted overnight under Ar atmosphere. The mixture was washed with water and ethyl acetate, and the organic phase was retained. The organic phase was subjected to column chromatography to give product intermediate **27-9d.**

The synthetic route for preparation of intermediate **27-9e** was as follows:

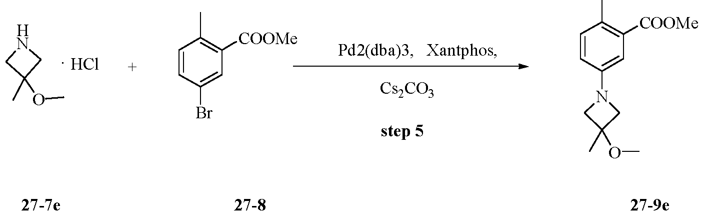

1420 mg of methyl 2-methyl-5-bromobenzoate **27-8** (6.2 mmol, 1.0 eq), 936 mg of **27-7e** (11 mmol, 1.1 eq), 56 mg (0.1 mmol, 0.01 eq) of Pd₂(dba)₃, 118 mg of XPhos (0.4 mmol, 0.04 eq), and 8300 mg of cesium carbonate (40 mmol, 4.0 eq) were weighed, dissolved in 50 mL of toluene, placed in a sealed tube, and heated to 110°C and reacted overnight under Ar atmosphere. The mixture was washed with water and ethyl acetate, and the organic phase was retained. The organic phase was subjected to column chromatography to give product intermediate **27-9e.**

The synthetic route for preparation of intermediate **27-9f** was as follows:

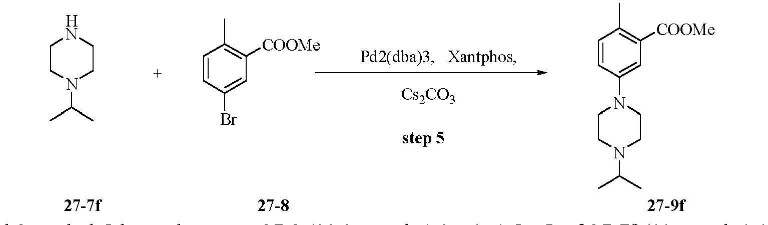

2290 mg of methyl 2-methyl-5-bromobenzoate **27-8** (10.0 mmol, 1.0 eq), 1.5 mL of **27-7f** (11 mmol, 1.1 eq), 91.5 mg (0.1 mmol, 0.01 eq) of Pd₂(dba)₃, 190 mg of XPhos (0.4 mmol, 0.04 eq), and 16300 mg of cesium carbonate (50 mmol, 5.0 eq) were weighed, dissolved in 50 mL of toluene, placed in a sealed tube, and heated to 110°C and reacted overnight under Ar atmosphere. The mixture was washed with water and ethyl acetate, and the organic phase was retained. The organic phase was subjected to column chromatography to give product intermediate **27-9f.**

The synthetic route for preparation of intermediate **27-9g** was as follows:

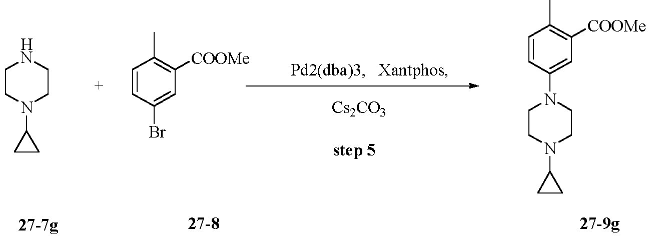

2290 mg of methyl 2-methyl-5-bromobenzoate **27-8** (10.0 mmol, 1.0 eq), 1388 mg of **27-7g** (11 mmol, 1.1 eq), 91.5 mg (0.1 mmol, 0.01 eq) of Pd₂(dba)₃, 190 mg of XPhos (0.4 mmol, 0.04 eq), and 16300 mg of cesium carbonate (50 mmol, 5.0 eq) were weighed, dissolved in 50 mL of toluene, placed in a sealed tube, and heated to 110°C and reacted overnight under Ar atmosphere. The mixture was washed with water and ethyl acetate, and the organic phase was retained. The organic phase was subjected to column chromatography to give product intermediate **27-9g.**

The synthetic route for preparation of intermediate **27-9h** was as follows:

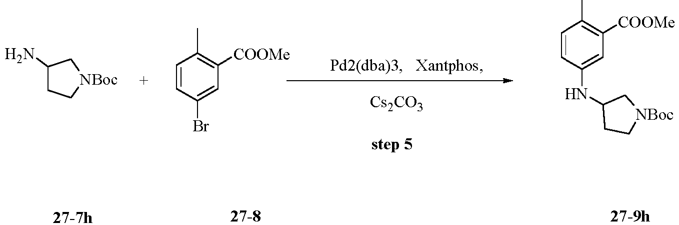

2290 mg of methyl 2-methyl-5-bromobenzoate **27-8** (10.0 mmol, 1.0 eq), 2 mL of **27-7h** (11 mmol, 1.1 eq), 274 mg (0.3 mmol, 0.03 eq) of Pd₂(dba)₃, 571 mg of XPhos (1.2 mmol, 0.12 eq), and 13040 mg of cesium carbonate (50 mmol, 5.0 eq) were weighed, dissolved in 50 mL of toluene, placed in a sealed tube, and heated to 110°C and reacted overnight under Ar atmosphere. The mixture was washed with water and ethyl acetate, and the organic phase was retained. The organic phase was subjected to column chromatography to give product intermediate **27-9h.**

The synthetic route for preparation of intermediate **27-9i** was as follows:

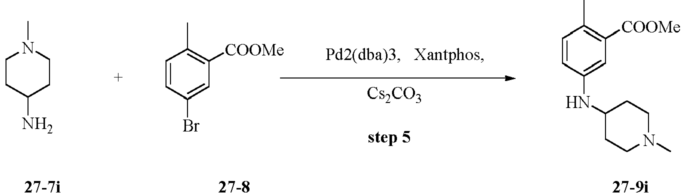

2290 mg of methyl 2-methyl-5-bromobenzoate **27-8** (10.0 mmol, 1.0 eq), 2 mL of **27-7i** (11 mmol, 1.1 eq), 92 mg (0.1 mmol, 0.01 eq) of Pd₂(dba)₃, 190 mg of XPhos (0.4 mmol, 0.04 eq), and 13040 mg of cesium carbonate (50 mmol, 5.0 eq) were weighed, dissolved in 50 mL of toluene, placed in a sealed tube, and heated to 110°C and reacted overnight under Ar atmosphere. The mixture was washed with water and ethyl acetate, and the organic phase was retained. The organic phase was subjected to column chromatography to give product intermediate **27-9i.**

The synthetic route for preparation of intermediate **27-9j** was as follows:

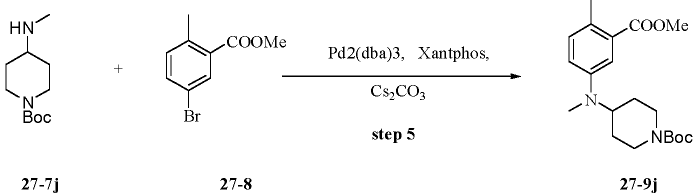

2290 mg of methyl 2-methyl-5-bromobenzoate **27-8** (10.0 mmol, 1.0 eq), 2350 mg of **27-7j** (11 mmol, 1.1 eq), 92 mg (0.1 mmol, 0.01 eq) of Pd₂(dba)₃, 190 mg of XPhos (0.4 mmol, 0.04 eq), and 13040 mg of cesium carbonate (50 mmol, 5.0 eq) were weighed, dissolved in 50 mL of toluene, placed in a sealed tube, and heated to 110°C and reacted overnight under Ar atmosphere. The mixture was washed with water and ethyl acetate, and the organic phase was retained. The organic phase was subjected to column chromatography to give product intermediate **27-9j.**

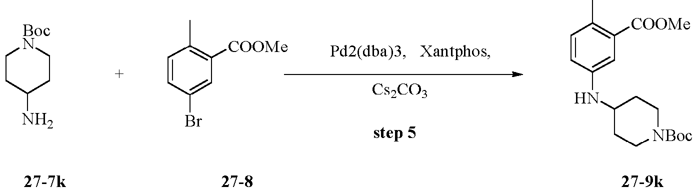

2290 mg of methyl 2-methyl-5-bromobenzoate **27-8** (10.0 mmol, 1.0 eq), 2200 mg of **27-7k** (11 mmol, 1.1 eq), 92 mg (0.1 mmol, 0.01 eq) of Pd₂(dba)₃, 190 mg of XPhos (0.4 mmol, 0.04 eq), and 13040 mg of cesium carbonate (50 mmol, 5.0 eq) were weighed, dissolved in 50 mL of toluene, placed in a sealed tube, and heated to 110°C and reacted overnight under Ar atmosphere. The mixture was washed with water and ethyl acetate, and the organic phase was retained. The organic phase was subjected to column chromatography to give product intermediate **27-9k.**

The preparation of the branched route step 6 was as follows:

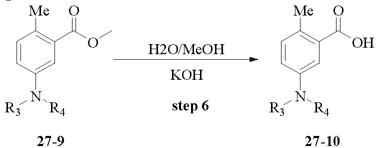

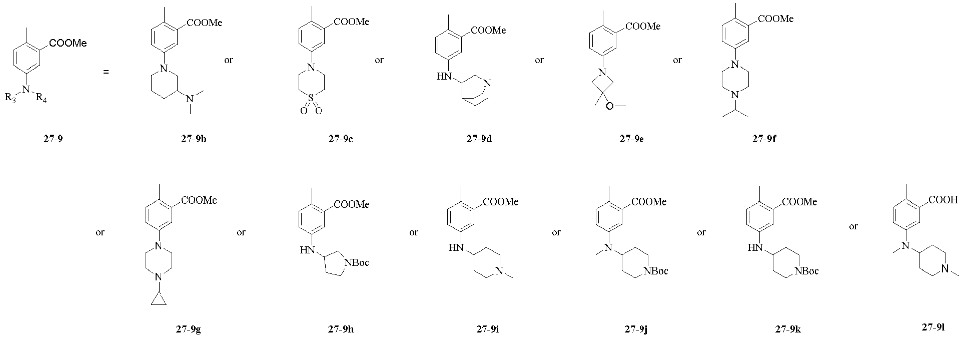

The synthetic route for preparation of intermediate **27-10a** was as follows:

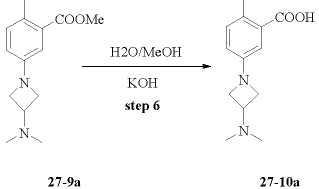

Intermediate **27-9a** (10 mmol, 1 eq.) was weighed and dissolved in a mixed solution of 25 mL methanol and 25 mL water, potassium hydroxide (40 mmol, 4 eq.) was added, and the mixture was heated to 60°C and stirred overnight. After the reaction was completed, excess hydrochloric acid was added to adjust the pH of the reaction solution to acidity (do not over-acidified, as the product has the risk of ring opening). The solvent was completely removed by rotary evaporation (after the first rotary evaporation, a small amount of methanol may be added for multiple times for rotary evaporation to remove water as much as possible), methanol was added, and the mixture was stirred and filtered under vacuum. If the filtered solid still contained many products, the solid was dissolved with methanol for multiple times and filtered under vacuum until the solid was completely insoluble (no fluorescence was detected by thin-layer chromatography plate and ultraviolet light). The filtrate was collected, concentrated, and recrystallized with dichloromethane to give product intermediate **27-10a.**

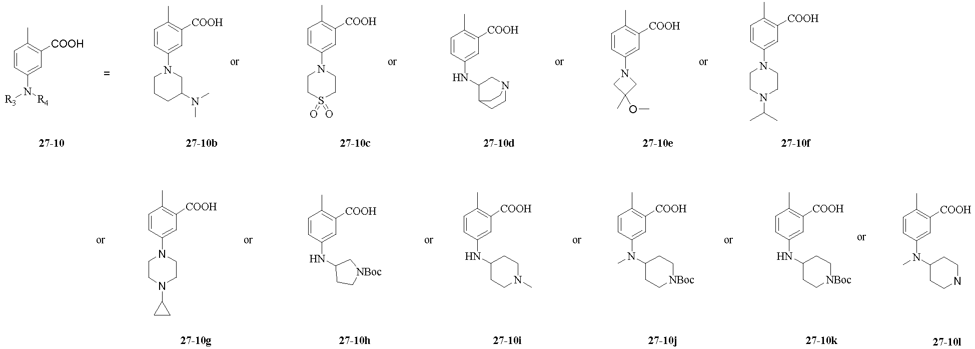

The routes for intermediates **27-10b to 27-10l** were the same as the preparation method of intermediate **27-10a.**

The preparation of the final product branched route step 7 was as follows:

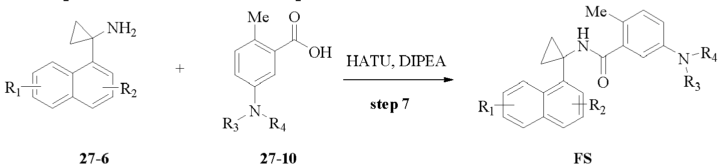

The synthetic route for preparation of the compound of Example 27 was as follows:

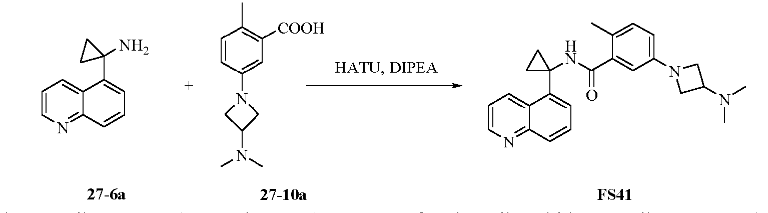

184 mg of amine intermediate **27-6a** (1 mmol, 1.0 eq), 234 mg of carboxylic acid intermediate **27-10a** (1 mmol, 1.0 eq), and 869 µL of DIPEA (5 mmol, 5.0 eq) were weighed, dissolved in 5 mL of DMF, and stirred until the mixture was completely dissolved. 570 mg of HATU (1.5 mmol, 1.5 eq) was added. The mixture was reacted at room temperature for 3 h. After the reaction starting materials were completely converted, the reaction solution was washed with water and ethyl acetate to remove DMF. The organic phase was retained and concentrated to dryness by rotary evaporation to give a crude product, which was purified by column chromatography to give the final product FS41, i.e., the **compound of Example 27.**

(15) The synthetic route for preparation of the compound of Example **28** was as follows:

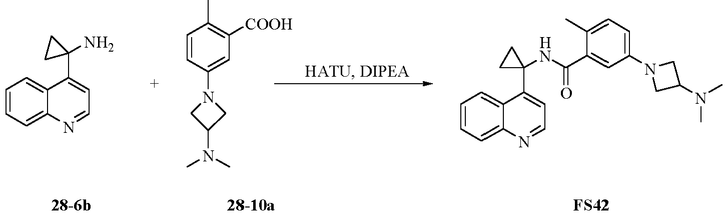

30 mg of amine intermediate **28-6b** (0.154 mmol, 1.0 eq), 36 mg of carboxylic acid intermediate **28-10a** (0.154 mmol, 1.0 eq), and 133 µL of DIPEA (0.77 mmol, 5.0 eq) were weighed, dissolved in 3 mL of THF, and stirred until the mixture was completely dissolved. 87 mg of HATU (0.231 mmol, 1.5 eq) was added. The mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product, which was purified by column chromatography to give the final product FS42, i.e., the **compound of Example 28.**

(16) The synthetic route for preparation of the compound of Example **29** was as follows:

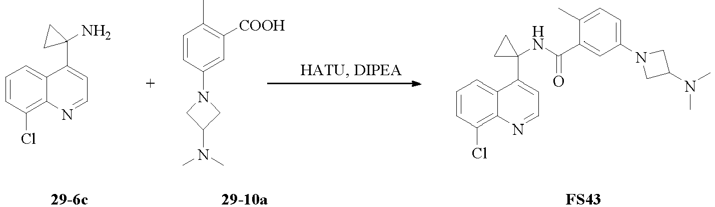

132 mg of amine intermediate **29-6c** (0.58 mmol, 1.0 eq), 135 mg of carboxylic acid intermediate **29-10a** (0.58 mmol, 1.0 eq), and 504 µL of DIPEA (2.9 mmol, 5.0 eq) were weighed, dissolved in 5 mL of THF, and stirred until the mixture was completely dissolved. 330 mg of HATU (0.87 mmol, 1.5 eq) was added. The mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product, which was purified by column chromatography to give the final product FS43, i.e., the **compound of Example 29.**

(17) The synthetic route for preparation of the compound of Example **30** was as follows:

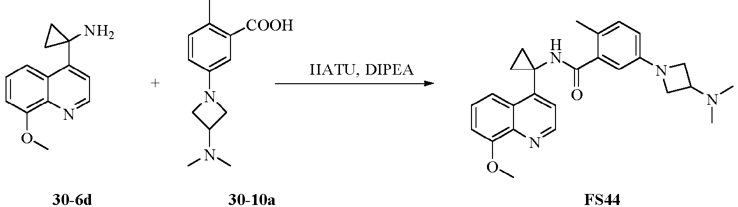

85 mg of amine intermediate **30-6d** (0.38 mmol, 1.0 eq), 106 mg of carboxylic acid intermediate **30-10a** (0.45 mmol, 1.0 eq), and 330 µL of DIPEA (1.9 mmol, 5.0 eq) were weighed, dissolved in 3 mL of THF, and stirred until the mixture was completely dissolved. 216 mg of HATU (0.57 mmol, 1.5 eq) was added. The mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product, which was purified by column chromatography to give the final product FS44, i.e., the **compound of Example 30.**

(18) The synthetic route for preparation of the compound of Example **31** was as follows:

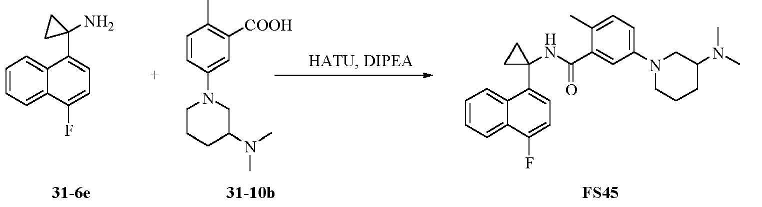

118 mg of amine intermediate **31-6e** (0.5 mmol, 1.0 eq), 158 mg of carboxylic acid intermediate **31-10b** (0.6 mmol, 1.2 eq), and 434 µL of DIPEA (2.5 mmol, 5.0 eq) were weighed, dissolved in 5 mL of DMF, and stirred until the mixture was completely dissolved. 285 mg of HATU (0.75 mmol, 1.5 eq) was added. The mixture was reacted at room temperature for 4 h. After the reaction starting materials were completely converted, the reaction solution was washed with water and ethyl acetate to remove DMF. The organic phase was retained and concentrated to dryness by rotary evaporation to give a crude product, which was purified by column chromatography to give the final product FS45, i.e., the **compound of Example 31.**

(19) The synthetic route for preparation of the compound of Example **32** was as follows:

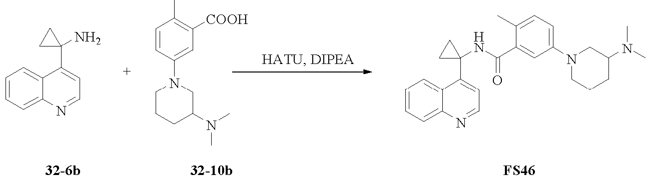

115 mg of amine intermediate **32-6b** (0.5 mmol, 1.0 eq), 157 mg of carboxylic acid intermediate **32-10b** (0.6 mmol, 1.2 eq), and 434 µL of DIPEA (2.5 mmol, 5.0 eq) were weighed, dissolved in 5 mL of THF, and stirred until the mixture was completely dissolved. 285 mg of HATU (0.75 mmol, 1.5 eq) was added. The mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product, which was purified by column chromatography to give the final product FS46, i.e., the **compound of Example 32.**

(20) The synthetic route for preparation of the compound of Example **33** was as follows:

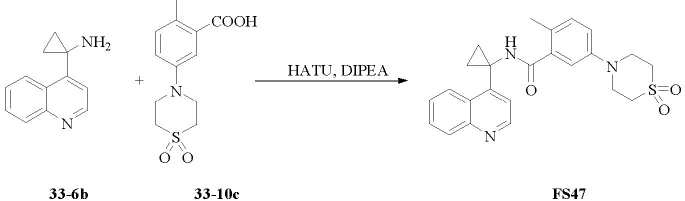

92 mg of amine intermediate **33-6b** (0.5 mmol, 1.0 eq), 161 mg of carboxylic acid intermediate **33-10c** (0.6 mmol, 1.2 eq), and 434 µL of DIPEA (2.5 mmol, 5.0 eq) were weighed, dissolved in 5 mL of THF, and stirred until the mixture was completely dissolved. 285 mg of HATU (0.75 mmol, 1.5 eq) was added. The mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product, which was purified by column chromatography to give the final product FS47, i.e., the **compound of Example 33.**

(21) The synthetic route for preparation of the compound of Example **34** was as follows:

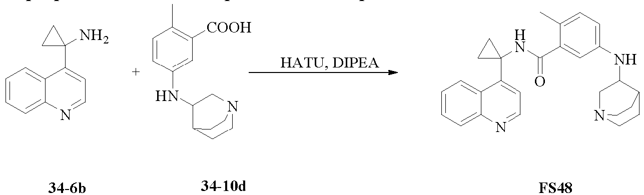

92 mg of amine intermediate 34-6b (0.5 mmol, 1.0 eq), 143 mg of carboxylic acid intermediate **34-10d** (0.55 mmol, 1.1 eq), and 434 µL of DIPEA (2.5 mmol, 5.0 eq) were weighed, dissolved in 5 mL of THF, and stirred until the mixture was completely dissolved. 285 mg of HATU (0.75 mmol, 1.5 eq) was added. The mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product, which was purified by column chromatography to give the final product FS48, i.e., the **compound of Example 34.**

(22) The synthetic route for preparation of the compound of Example **35** was as follows:

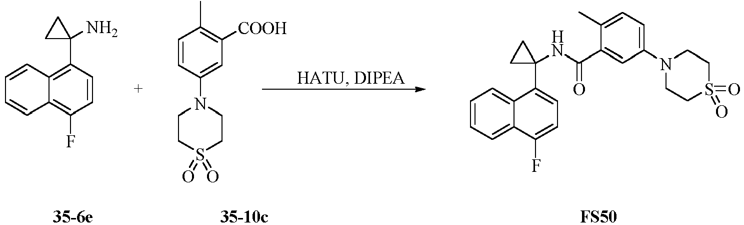

100.5 mg of amine intermediate **35-6e** (0.5 mmol, 1.0 eq), 134 mg of carboxylic acid intermediate **35-10c** (0.5 mmol, 1.0 eq), and 434 µL of DIPEA (2.5 mmol, 5.0 eq) were weighed, dissolved in 5 mL of DMF, and stirred until the mixture was completely dissolved. 285 mg of HATU (0.75 mmol, 1.5 eq) was added. The mixture was reacted at room temperature for 4 h. After the reaction starting materials were completely converted, the reaction solution was washed with water and ethyl acetate to remove DMF. The organic phase was retained and concentrated to dryness by rotary evaporation to give a crude product, which was purified by column chromatography to give the final product FS50, i.e., the **compound of Example 35.**

(23) The synthetic route for preparation of the compound of Example 36 was as follows:

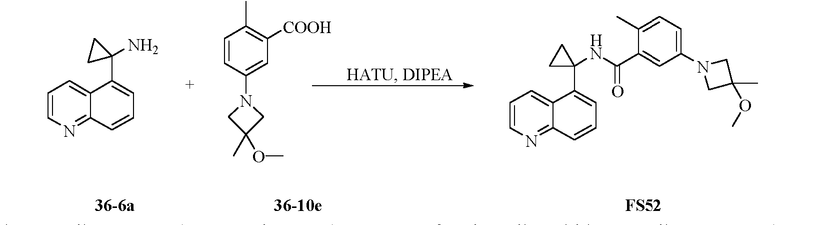

92 mg of amine intermediate 36-6a (0.5 mmol, 1.0 eq), 129 mg of carboxylic acid intermediate 36-10e (0.55 mmol, 1.1 eq), and 434 µL of DIPEA (2.5 mmol, 5.0 eq) were weighed, dissolved in 5 mL of THF, and stirred until the mixture was completely dissolved. 285 mg of HATU (0.75 mmol, 1.5 eq) was added. The mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product, which was purified by column chromatography to give the final product FS52, i.e., the **compound of Example 36.**

(24) The synthetic route for preparation of the compound of Example **37** was as follows:

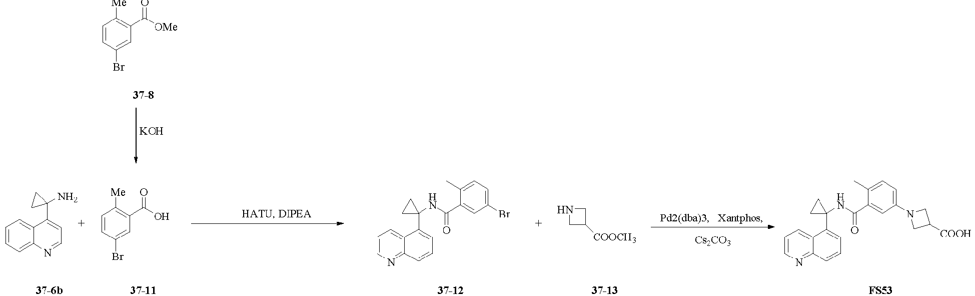

4240 mg of intermediate **37-8** (20 mmol, 1.0 eq) and 1920 mg of lithium hydroxide (80 mmol,4.0 eq.) were weighed, dissolved in H2O/MeOH/THF = 1/2/2 (75 mL), and reacted at 50°C overnight. After the reaction was completed, methanol and tetrahydrofuran were removed by rotary evaporation, the reaction solution was washed with ethyl acetate and water, and the aqueous phase was retained. 2 N HCl solution was then added to adjust the pH of the aqueous solution to acidity, and the reaction solution was washed with EA and water. The organic phase was retained and concentrated to dryness by rotary evaporation to give intermediate **37-11.**

276 mg of amine intermediate **37-6a** (1.5 mmol, 1.0 eq), 322 mg of carboxylic acid intermediate **37-11** (1.5 mmol, 1.0 eq), and 1304 µL of DIPEA (7.5 mmol, 5.0 eq) were weighed, dissolved in 5 mL of THF, and stirred until the mixture was completely dissolved. 855 mg of HATU (2.25 mmol, 1.5 eq) was added, and the mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product, which was purified by column chromatography to give intermediate **37-12.**

220 mg of intermediate **37-12** (0.58 mmol, 1.0 eq.) was weighed, and 5.3 mg of Pd₂(dba)₃ (0.058 mmol, 0.01 eq), 11 mg of Xphos (0.023 mmol, 0.01 eq), and 756 mg of cesium carbonate (2.32 mmol, 4.0 eq) were added, dissolved in 5 mL of toluene, placed in a sealed tube, and heated to 110°C overnight under Ar atmosphere. After the reaction solution was cooled to room temperature, toluene was removed by rotary evaporation. The reaction solution was then washed with 2 N HCl solution and ethyl acetate. The aqueous phase was retained, and the pH of the aqueous solution was then adjusted to alkalinity with a saturated aqueous sodium bicarbonate solution. Water and ethyl acetate were then added for washing, and the organic phase was retained. The organic phase was concentrated to dryness by rotary evaporation and subjected to column chromatography to give product FS53, which was the **compound of Example 37.**

(25) The synthetic route for preparation of the compound of Example **38** was as follows:

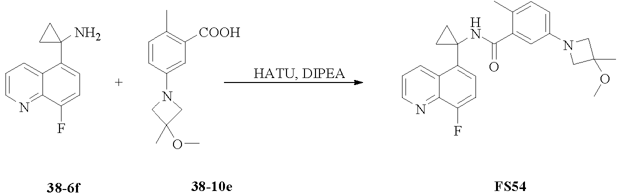

113 mg of amine intermediate **38-6f** (0.56 mmol, 1.4 eq.), 94 mg of carboxylic acid intermediate **38-10e** (0.4 mmol, 1.0 eq.), and 139 µL of DIPEA (0.8 mmol, 2.0 eq.) were weighed, dissolved in 3 mL of THF, and stirred until the mixture was completely dissolved. 182 mg of HATU (0.48 mmol, 1.2 eq.) was added. The mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product, which was purified by column chromatography to give the final product FS54, i.e., the **compound of Example 38.**

(26) The synthetic route for preparation of the compound of Example 39 was as follows:

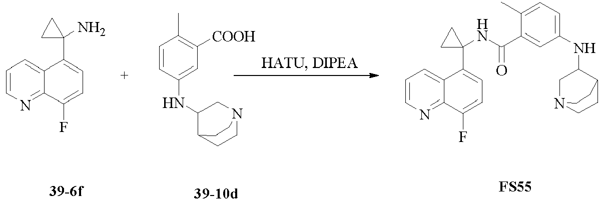

101 mg of amine intermediate 39-6f (0.5 mmol, 1.0 eq.), 143 mg of carboxylic acid intermediate **39-10d** (0.55 mmol, 1.0 eq.), and 434 µL of DIPEA (2.5 mmol, 5.0 eq.) were weighed, dissolved in 3 mL of THF, and stirred until the mixture was completely dissolved. 285 mg of HATU (0.75 mmol, 1.5 eq.) was added. The mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product, which was purified by column chromatography to give the final product FS55, i.e., the **compound of Example 39.**

(27) The synthetic route for preparation of the compound of Example **40** was as follows:

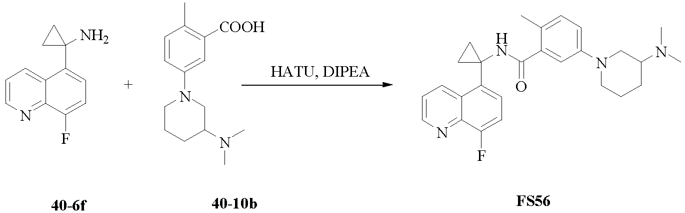

101 mg of amine intermediate **40-6f** (0.5 mmol, 1.0 eq.), 157 mg of carboxylic acid intermediate **40-10b** (0.6 mmol, 1.2 eq.), and 434 µL of DIPEA (2.5 mmol, 5.0 eq.) were weighed, dissolved in 3 mL of THF, and stirred until the mixture was completely dissolved. 285 mg of HATU (0.75 mmol, 1.5 eq.) was added. The mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product, which was purified by column chromatography to give the final product FS56, i.e., the **compound of Example 40.**

(28) The synthetic route for preparation of the compound of Example **41** was as follows:

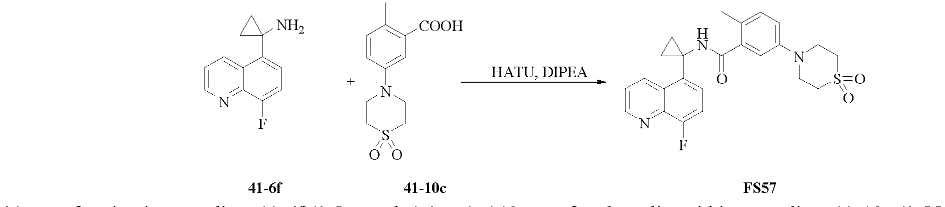

101 mg of amine intermediate **41-61** (0.5 mmol, 1.0 eq., 148 mg of carboxylic acid intermediate **41-10c** (0.55 mmol, 1.1 eq.), and 434 µL of DIPEA (2.5 mmol, 5.0 eq.) were weighed, dissolved in 3 mL of THF, and stirred until the mixture was completely dissolved. 285 mg of HATU (0.75 mmol, 1.5 eq.) was added. The mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product, which was purified by column chromatography to give the final product FS57, i.e., the compound **of Example 41.**

(29) The synthetic route for preparation of the compound of Example **42** was as follows:

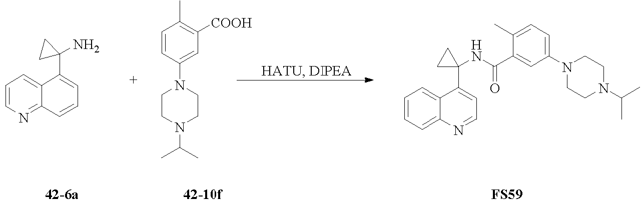

92 mg of amine intermediate 42-6a (0.5 mmol, 1.0 eq.), 144 mg of carboxylic acid intermediate **42-10f** (0.55 mmol, 1.1 eq.), and 434 µL of DIPEA (2.5 mmol, 5.0 eq.) were weighed, dissolved in 3 mL of THF, and stirred until the mixture was completely dissolved. 285 mg of HATU (0.75 mmol, 1.5 eq.) was added. The mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product, which was purified by column chromatography to give the final product FS59, i.e., the **compound of Example 42.**

(30) The synthetic route for preparation of the compound of Example **43** was as follows:

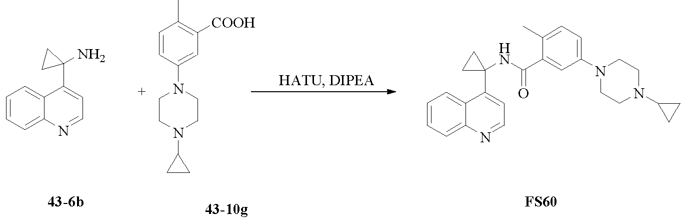

92 mg of amine intermediate **43-6b** (0.5 mmol, 1.0 eq.), 143 mg of carboxylic acid intermediate **43-10g** (0.55 mmol, 1.1 eq.), and 434 µL of DIPEA (2.5 mmol, 5.0 eq.) were weighed, dissolved in 3 mL of THF, and stirred until the mixture was completely dissolved. 285 mg of HATU (0.75 mmol, 1.5 eq.) was added. The mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product, which was purified by column chromatography to give the final product FS60, i.e., the **compound of Example 43.**

(31) The synthetic route for preparation of the compound of Example **44** was as follows:

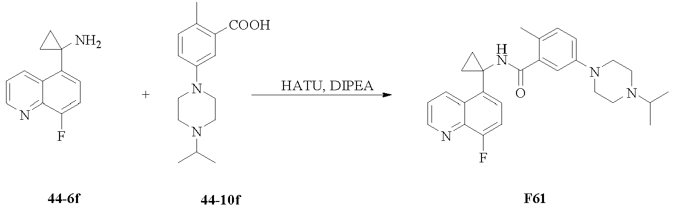

101 mg of amine intermediate **44-6f** (0.5 mmol, 1.0 eq.), 144 mg of carboxylic acid intermediate **44-10f** (0.55 mmol, 1.1 eq.), and 434 µL of DIPEA (2.5 mmol, 5.0 eq.) were weighed, dissolved in 3 mL of THF, and stirred until the mixture was completely dissolved. 285 mg of HATU (0.75 mmol, 1.5 eq.) was added. The mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product, which was purified by column chromatography to give the final product F61, i.e., the **compound of Example 44.**

(32) The synthetic route for preparation of the compound of Example **45** was as follows:

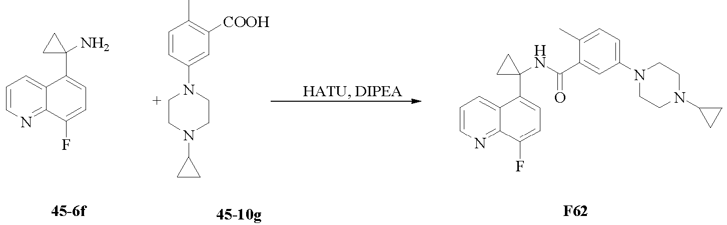

101 mg of amine intermediate **45-6f** (0.5 mmol, 1.0 eq.), 143 mg of carboxylic acid intermediate **45-10g** (0.55 mmol, 1.1 eq.), and 434 µL of DIPEA (2.5 mmol, 5.0 eq.) were weighed, dissolved in 3 mL of THF, and stirred until the mixture was completely dissolved. 285 mg of HATU (0.75 mmol, 1.5 eq.) was added. The mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product, which was purified by column chromatography to give the final product F62, i.e., the **compound of Example 45.**

(33) The synthetic route for preparation of the compound of Example **46** was as follows:

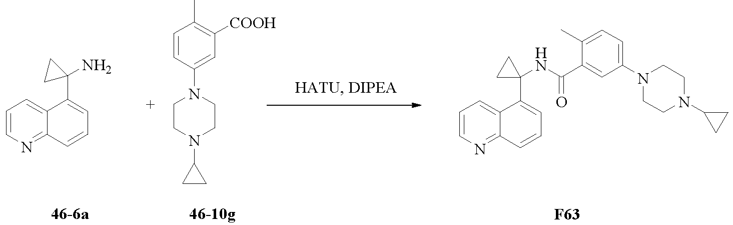

184 mg of amine intermediate **46-6a** (1.0 mmol, 1.0 eq.), 572 mg of carboxylic acid intermediate **46-10g** (1.1 mmol, 1.1 eq.), and 870 µL of DIPEA (5.0 mmol, 5.0 eq.) were weighed, dissolved in 3 mL of THF, and stirred until the mixture was completely dissolved. 570 mg of HATU (1.5 mmol, 1.5 eq.) was added. The mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product, which was purified by column chromatography to give the final product F63, i.e., the **compound of Example 46.**

(34) The synthetic route for preparation of the compound of Example **47** was as follows:

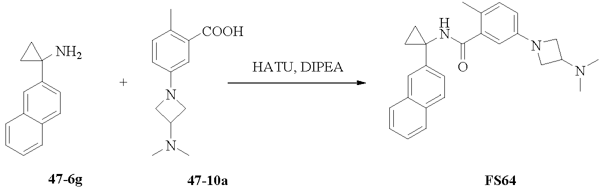

92 mg of amine intermediate **47-6g** (0.5 mmol, 1.0 eq.), 129 mg of carboxylic acid intermediate **47-10a** (0.55 mmol, 1.1 eq.), and 434 µL of DIPEA (2.5 mmol, 5.0 eq.) were weighed, dissolved in 3 mL of THF, and stirred until the mixture was completely dissolved. 285 mg of HATU (0.75 mmol, 1.5 eq.) was added. The mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product, which was purified by column chromatography to give the final product FS64, i.e., the **compound of Example 47.**

(35) The synthetic route for preparation of the compound of Example **48** was as follows:

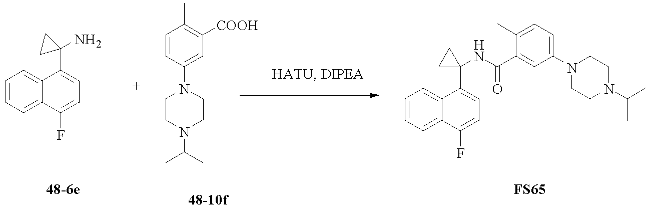

201 mg of amine intermediate **48-6e** (1.0 mmol, 1.0 eq.), 288 mg of carboxylic acid intermediate **48-10f** (1.1 mmol, 1.1 eq.), and 870 µL of DIPEA (5.0 mmol, 5.0 eq.) were weighed, dissolved in 3 mL of THF, and stirred until the mixture was completely dissolved. 570 mg of HATU (1.5 mmol, 1.5 eq.) was added. The mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product, which was purified by column chromatography to give the final product FS65, i.e., the **compound of Example 48.**

(36) The synthetic route for preparation of the compound of Example **49** was as follows:

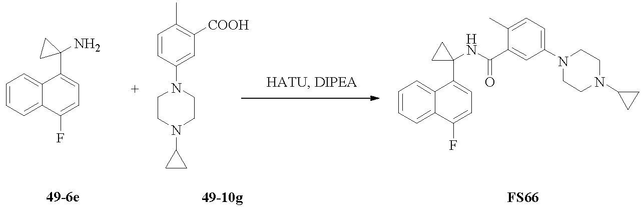

201 mg of amine intermediate **49-6e** (1.0 mmol, 1.0 eq.), 286 mg of carboxylic acid intermediate **49-10g** (1.1 mmol, 1.1 eq.), and 870 µL of DIPEA (5.0 mmol, 5.0 eq.) were weighed, dissolved in 3 mL of THF, and stirred until the mixture was completely dissolved. 570 mg of HATU (1.5 mmol, 1.5 eq.) was added. The mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product, which was purified by column chromatography to give the final product FS66, i.e., the **compound of Example 49.**

(37) The synthetic route for preparation of the compound of Example **50** was as follows:

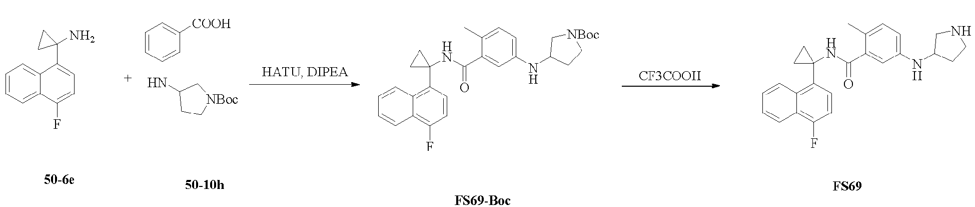

201 mg of amine intermediate **50-6e** (1.0 mmol, 1.0 eq.), 321 mg of carboxylic acid intermediate **50-10h** (1.0 mmol, 1.0 eq.), and 870 µL of DIPEA (5.0 mmol, 5.0 eq.) were weighed, dissolved in 3 mL of THF, and stirred until the mixture was completely dissolved. 570 mg of HATU (1.5 mmol, 1.5 eq.) was added. The mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product **FS69-Boc.** It was then dissolved in 2 mL of DCM and stirred until it was completely dissolved. CF3COOH (10 mmol, 10 eq.) was added and stirred at room temperature for 2 h. After the reaction was completed, the reaction solution was washed with DCM/H2O, and the aqueous phase was retained. The pH of the aqueous phase was then adjusted to alkalinity, the aqueous phase was extracted with ethyl acetate, and the organic phase was retained and purified by column chromatography to give the final product FS69, i.e., the **compound of Example 50.**

(38) The synthetic route for preparation of the compound of Example **51** was as follows:

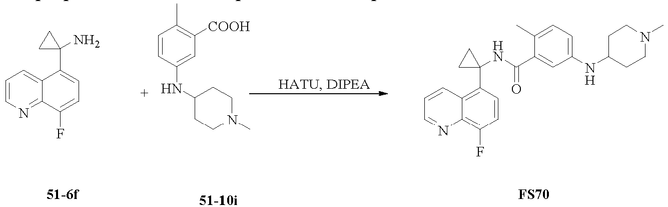

202 mg of amine intermediate **51-6f** (1.0 mmol, 1.0 eq.), 372 mg of carboxylic acid intermediate **51-10i** (1.5 mmol, 1.5 eq.), and 870 µL of DIPEA (5.0 mmol, 5.0 eq.) were weighed, dissolved in 3 mL of THF, and stirred until the mixture was completely dissolved. 570 mg of HATU (1.5 mmol, 1.5 eq.) was added. The mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product, which was purified by column chromatography to give the final product FS70, i.e., the **compound of Example 51.**

(39) The synthetic route for preparation of the compound of Example **52** was as follows:

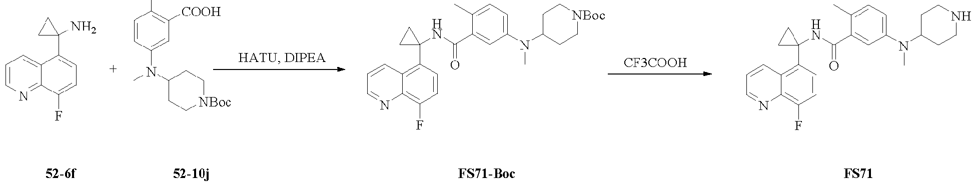

202 mg of amine intermediate **52-6f** (1.0 mmol, 1.0 eq.), 522 mg of carboxylic acid intermediate **52-10j** (1.5 mmol, 1.5 eq.), and 870 µL of DIPEA (5.0 mmol, 5.0 eq.) were weighed, dissolved in 3 mL of THF, and stirred until the mixture was completely dissolved. 570 mg of HATU (1.5 mmol, 1.5 eq.) was added. The mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product **FS71-Boc.** It was then dissolved in 2 mL of DCM and stirred until it was completely dissolved. CF3COOH (10 mmol, 10 eq.) was added and stirred at room temperature for 2 h. After the reaction was completed, the reaction solution was washed with DCM/H2O, and the aqueous phase was retained. The pH of the aqueous phase was then adjusted to alkalinity, the aqueous phase was extracted with ethyl acetate, and the organic phase was retained and purified by column chromatography to give the final product FS71, i.e., the **compound of Example 52.**

(40) The synthetic route for preparation of the compound of Example **53** was as follows:

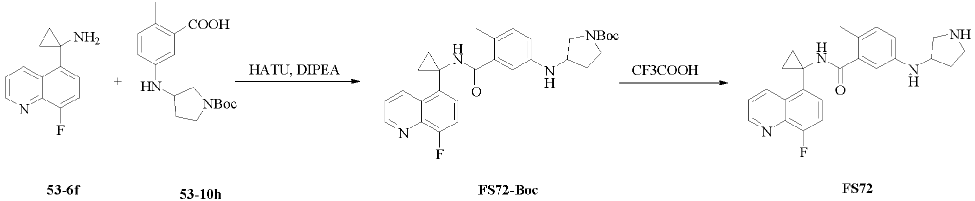

303 mg of amine intermediate **53-6f** (1.5 mmol, 1.0 eq.), 481 mg of carboxylic acid intermediate **53-10h** (1.5 mmol, 1.0 eq.), and 1303 µL of DIPEA (7.5 mmol, 5.0 eq.) were weighed, dissolved in 3 mL of THF, and stirred until the mixture was completely dissolved. 855 mg of HATU (2.25 mmol, 1.5 eq.) was added. The mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product **FS72-Boc.** It was then dissolved in 2 mL of DCM and stirred until it was completely dissolved. CF3COOH (10 mmol, 10 eq.) was added and stirred at room temperature for 2 h. After the reaction was completed, the reaction solution was washed with DCM/H2O, and the aqueous phase was retained. The pH of the aqueous phase was then adjusted to alkalinity, the aqueous phase was extracted with ethyl acetate, and the organic phase was retained and purified by column chromatography to give the final product FS72, i.e., the **compound of Example 53.**

(41) The synthetic route for preparation of the compound of Example **54** was as follows:

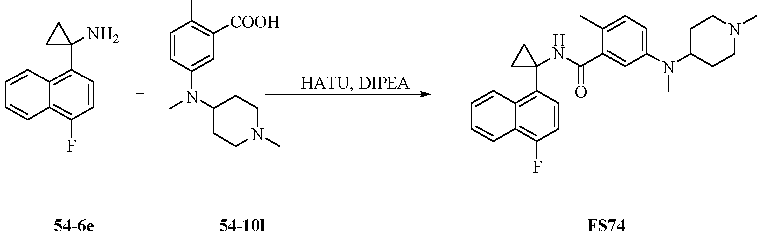

202 mg of amine intermediate **54-6e** (1.0 mmol, 1.0 eq.), 370 mg of carboxylic acid intermediate **54-10l** (1.5 mmol, 1.5 eq.), and 870 µL of DIPEA (5.0 mmol, 5.0 eq.) were weighed, dissolved in 3 mL of THF, and stirred until the mixture was completely dissolved. 570 mg of HATU (1.5 mmol, 1.5 eq.) was added. The mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product, which was purified by column chromatography to give the final product FS74, i.e., the **compound of Example 54.**

(42) The synthetic route for preparation of the compound of Example **55** was as follows:

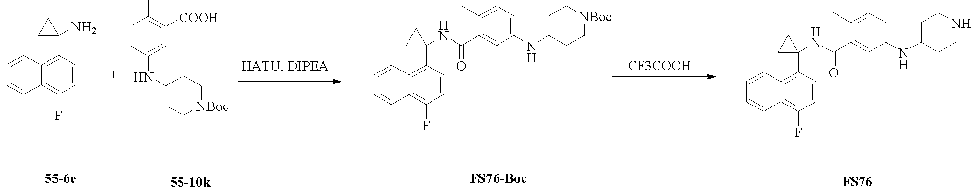

201 mg of amine intermediate **55-6e** (1.0 mmol, 1.0 eq.), 334 mg of carboxylic acid intermediate **55-10k** (1.0 mmol, 1.0 eq.), and 870 µL of DIPEA (5.0 mmol, 5.0 eq.) were weighed, dissolved in 3 mL of THF, and stirred until the mixture was completely dissolved. 570 mg of HATU (1.5 mmol, 1.5 eq.) was added. The mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product **FS76-Boc.** It was then dissolved in 2 mL of DCM and stirred until it was completely dissolved. CF3COOH (10 mmol, 10 eq.) was added and stirred at room temperature for 2 h. After the reaction was completed, the reaction solution was washed with DCM/H2O, and the aqueous phase was retained. The pH of the aqueous phase was then adjusted to alkalinity, the aqueous phase was extracted with ethyl acetate, and the organic phase was retained and purified by column chromatography to give the final product FS76, i.e., the **compound of Example 55.**

(43) The synthetic route for preparation of the compound of Example **56** was as follows:

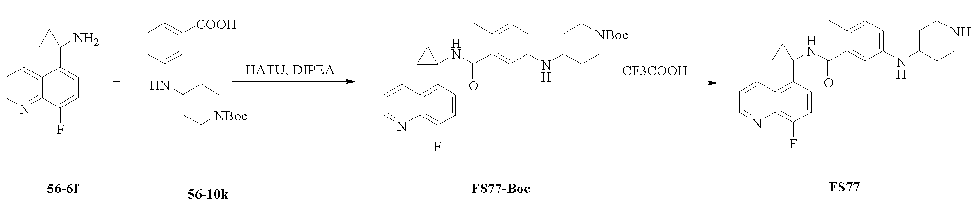

101 mg of amine intermediate **56-6f** (0.5 mmol, 1.0 eq.), 167 mg of carboxylic acid intermediate **56-10k** (0.5 mmol, 1.0 eq.), and 434 µL of DIPEA (2.5 mmol, 5.0 eq.) were weighed, dissolved in 3 mL of THF, and stirred until the mixture was completely dissolved. 285 mg of HATU (0.75 mmol, 1.5 eq.) was added. The mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product **FS77-Boc.** It was then dissolved in 2 mL of DCM and stirred until it was completely dissolved. CF3COOH (10 mmol, 10 eq.) was added and stirred at room temperature for 2 h. After the reaction was completed, the reaction solution was washed with DCM/H2O, and the aqueous phase was retained. The pH of the aqueous phase was then adjusted to alkalinity, the aqueous phase was extracted with ethyl acetate, and the organic phase was retained and purified by column chromatography to give the final product FS77, i.e., the **compound of Example 56.**

(44) The synthetic route for preparation of the compound of Example **57** was as follows:

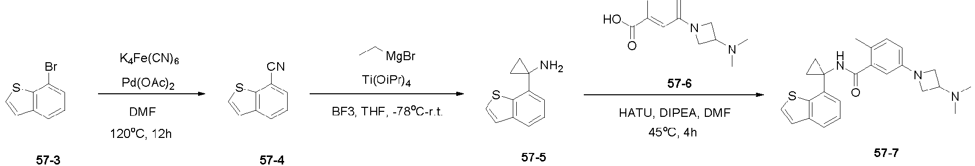

7-bromobenzo[b]thiophene **(57-3,** 10 mmol, 1.0 e.q.) was placed in a round bottom flask and dissolved in 50 mL of dry DMF. Potassium ferrocyanide (5 mmol, 0.5 e.q.), Pd₂(dba)₃ (0.5 mmol, 0.05 e.q.), and cesium carbonate (15 mmol, 1.5 e.q.) were then added. The mixture was warmed to 120°C and stirred for 12 h under argon atmosphere. After the reaction was completed, 50 mL of water was added to the reaction system. The mixture was extracted with ethyl acetate, and the organic phase was collected, concentrated to dryness by rotary evaporation, and separated by silica gel column chromatography to give benzo[*b*]thiophene-7-carbonitrile intermediate **57-4.**

Benzo[*b*]thiophene-7-carbonitrile **(2,** 10 mmol, 1.0 e.q.) was placed in a round bottom flask, 10 mL of dry tetrahydrofuran was added as a solvent, and tetraisopropyl titanate (10 mmol, 1.1 e.q.) was then added. The reaction system was cooled to - 78°C, and ethyl Grignard reagent (20 mmol, 2.0 e.q.) was then slowly added dropwise to the reaction system. After the dropwise addition was completed, the reaction system was warmed to room temperature and reacted for 1.5 h. Boron trifluoride diethyl etherate (20 mmol, 2.0 e.q.) was then added dropwise to the reaction system. After the dropwise addition was completed, the mixture was stirred and reacted at room temperature for 3 h. After the reaction was completed, 20 mL of 2 N hydrochloric acid was added dropwise to the reaction system, the reaction was quenched with stirring for 20 min, and an excess saturated sodium hydroxide solution was then added. The mixture was extracted with ethyl acetate, and the organic phase was collected, concentrated to dryness by rotary evaporation, and separated by silica gel column chromatography to give intermediate **57-5.**

The intermediate **57-5** and the previously obtained carboxylic acid intermediate **57-6** were added to DMF as a solvent according to an equivalent ratio of 1:1. HATU (1.5 e.q.) and DIPEA (2.0 e.q.) were added, and the mixture was reacted at 50°C for 12 h. The reaction solution was extracted with ethyl acetate and washed with a saturated ammonium chloride solution for three times. The organic phase was concentrated to dryness by rotary evaporation and purified by silica gel column chromatography to give product **57-7.** The final product **57-7** was the compound of Example **57.**

(45) The synthetic route for preparation of the compound of Example **58** was as follows:

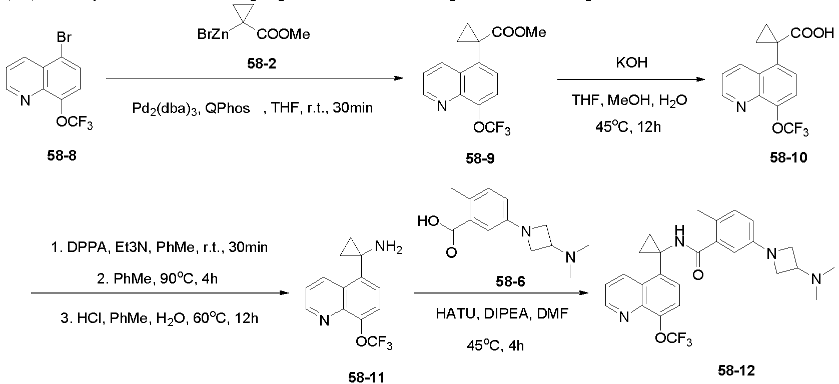

5-bromo-8-trifluoromethoxyquinoline **(58-8,** 10 mmol, 1.0 e.q.), Pd₂(dba)₃ (0.1 mmol, 0.01 e.q.), and 71 mg of QPhos (0.1 mmol, 0.01 e.q.) were placed in a round bottom flask and dissolved in 20 mL of anhydrous tetrahydrofuran. Intermediate 58-2 Reformatsky Reagent (20 mmol, 1 N, 2.0 e.q.) dissolved in 20 mL of tetrahydrofuran was added under argon atmosphere. The mixture was stirred at room temperature for 30 min. After the reaction was completely converted as detected, the solvent was removed by rotary evaporation, and the mixture was dissolved in ethyl acetate, washed three times with water and once with saturated brine. The organic phase was retained and dried over anhydrous sodium sulfate. The organic phase was purified by column chromatography to give product intermediate **58-9.**

The intermediate 58-9 (10 mmol, 1.0 eq) was dissolved in a mixed solvent of tetrahydrofuran/methanol/water (3:1:1, 50 mL), and potassium hydroxide (40 mmol, 4.0 eq) was added. The mixture was reacted at 50°C for 8 h. After the reactant was completely converted as detected, 2 N HCl solution was added to adjust the pH to 3. After all solvents were removed by rotary evaporation, 25 mL of methanol was added. The mixture was filtered, the filtrate was concentrated to dryness by rotary evaporation to give a light yellow solid, and finally the light-yellow solid was washed repeatedly with DCM/PE to give a white solid product **58-10.**

The intermediate **58-10** (2 mmol, 1.0 eq) was weighed and dissolved in 25 mL of extra dry toluene, triethylamine (4.4 mmol, 2.2 eq) was added, and DPPA (2.4 mmol, 1.2 eq) was added under argon atmosphere. The mixture was stirred at room temperature for 30 min until all carboxylic acid materials were converted into acyl azide, and heated to 75°C and reacted for 4 h until most acyl azide was converted into isocyanate. Excess hydrochloric acid (2 M aqueous solution, > 4.0 eq) was added, and the mixture was cooled to 60°C and reacted overnight. After the pH was adjusted to alkalinity with a sodium bicarbonate solution, the mixture was extracted with ethyl acetate, and the organic phase was collected, concentrated to dryness by rotary evaporation, and separated by silica gel column chromatography to give intermediate **58-11.**

The intermediate **58-11** and the previously obtained carboxylic acid intermediate **58-6** were added to a DMF solvent according to an equivalent ratio of 1:1. HATU (1.5 e.q.) and DIPEA (2.0 e.q.) were added, and the mixture was reacted at 50°C for 12 h. The reaction solution was extracted with ethyl acetate and washed with a saturated ammonium chloride solution for three times. The organic phase was concentrated to dryness by rotary evaporation and purified by silica gel column chromatography to give product **58-12.** The final product **58-12** was the compound of Example **58.**

(46) The synthetic route for preparation of the compound of Example **59** was as follows:

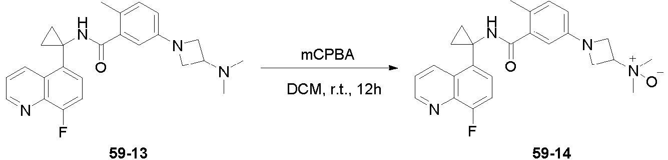

The product **59-13** (10 mmol, 1.0 e.q.) obtained previously and mCPBA (12 mmol, 1.2 e.q.) were placed in a round bottom flask and dissolved with 30 mL of DCM. The mixture was stirred at room temperature for 12 h. After the reaction was completely converted as detected, triphenylphosphine (5 mmol, 0.5 e.q.) was added, and the mixture was then stirred at room temperature for 4 h. The solvent was removed by rotary evaporation. The mixture was purified by column chromatography to give product **59-14.** The final product **59-14** was the compound of Example **59.**

(47) The synthetic route for preparation of the compound of Example **60** was as follows:

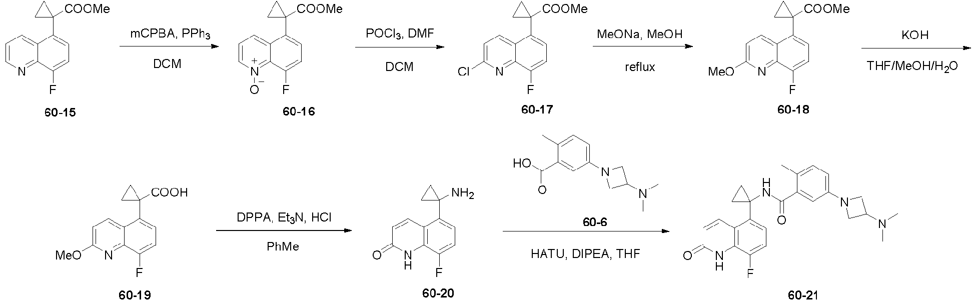

The product **60-15** (10 mmol, 1.0 e.q.) obtained previously and mCPBA (12 mmol, 1.2 e.q.) were placed in a round bottom flask and dissolved with 30 mL of DCM. The mixture was stirred at room temperature for 12 h. After the reaction was completely converted as detected, triphenylphosphine (5 mmol, 0.5 e.q.) was added, and the mixture was then stirred at room temperature for 4 h. The solvent was removed by rotary evaporation. The mixture was purified by column chromatography to give intermediate **60-16.**

The intermediate **60-16** (3 mmol, 1.0 e.q.) was placed in a round bottom flask and dissolved with 30 mL of DCM. Phosphorus oxychloride (3.6 mmol, 1.2 e.q.) was added dropwise with stirring under an ice bath, and DMF (1.5 mmol, 0.5 e.q.) was then added dropwise. The mixture was stirred at room temperature for 12 h. After the reaction was completely converted as detected, a saturated sodium bicarbonate solution was added dropwise under an ice bath to adjust the pH of the solution to 8, and the mixture was extracted. The organic phase was washed with water for 2 times, washed with saturated brine for 1 time, collected, and concentrated to dryness by rotary evaporation to give intermediate **60-17** without purification.

The intermediate **60-17** (3 mmol, 1.0 e.q.) was placed in a round bottom flask and dissolved with 30 mL of anhydrous methanol. Sodium methoxide (5 M, 30 mmol, 10 e.q.) was added, and the mixture was stirred at reflux at 70°C for 12 h. After the reaction was completely converted as detected, the solvent was removed by rotary evaporation, and the mixture was extracted with a proper amount of ethyl acetate and a saturated ammonium chloride solution. The organic phase was extracted and washed for 1 time with saturated brine, collected, and concentrated to dryness to give intermediate **60-18** without purification.

The intermediate **60-18** (3 mmol, 1.0 e.q.) was dissolved in a mixed solvent of tetrahydrofuran/methanol/water (3:1:1, 50 mL), and potassium hydroxide (12 mmol, 4.0 eq) was added. The mixture was reacted at 50°C for 8 h. After the reactant was completely converted as detected, 2 N HCl solution was added to adjust the pH to 3. After all solvents were removed by rotary evaporation, 25 mL of methanol was added. The mixture was filtered, the filtrate was concentrated to dryness by rotary evaporation to give a light yellow solid, and finally the light-yellow solid was washed repeatedly with DCM/PE to give a white solid product **60-19.**

The intermediate **60-19** (2 mmol, 1.0 eq) was weighed and dissolved in 25 mL of extra dry toluene, triethylamine (4.4 mmol, 2.2 eq) was added, and DPPA (2.4 mmol, 1.2 eq) was added under argon atmosphere. The mixture was stirred at room temperature for 30 min until all carboxylic acid materials were converted into acyl azide, and heated to 75°C and reacted for 4 h until most acyl azide was converted into isocyanate. Excess hydrochloric acid (2 M aqueous solution, > 4.0 eq) was added, and the mixture was cooled to 60°C and reacted overnight. After the pH was adjusted to alkalinity with a sodium bicarbonate solution, the mixture was extracted with ethyl acetate, and the organic phase was collected, concentrated to dryness by rotary evaporation, and separated by silica gel column chromatography to give intermediate **60-20.**

The intermediate **60-20** and the previously obtained carboxylic acid intermediate **60-6** were added to DMF as a solvent according to an equivalent ratio of 1:1. HATU (1.5 e.q.) and DIPEA (2.0 e.q.) were added, and the mixture was reacted at 50°C for 12 h. The reaction solution was extracted with ethyl acetate and washed with a saturated ammonium chloride solution for three times. The organic phase was concentrated to dryness by rotary evaporation and purified by silica gel column chromatography to give product **60-21.** The final product **60-21** was the compound of Example **60.**

(48) The synthetic route for preparation of the compound of Example **61** was as follows:

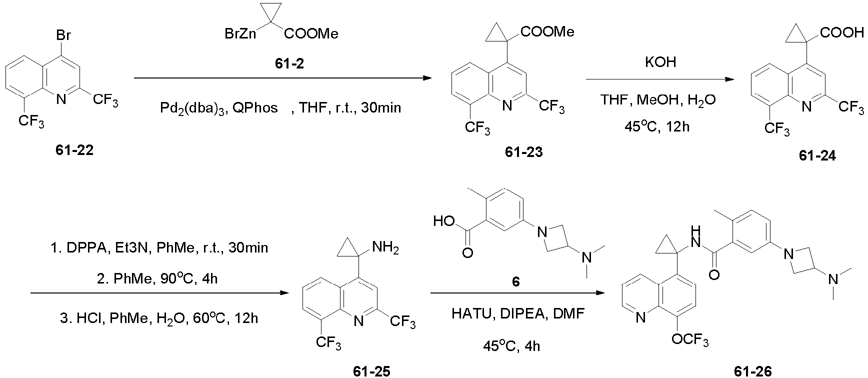

4-bromo-2,8-bis(trifluoromethyl)quinoline **(61-22,** 10 mmol, 1.0 e.q.), Pd₂(dba)₃ (0.1 mmol, 0.01 e.q.), and 71 mg of QPhos (0.1 mmol, 0.01 e.q.) were placed in a round bottom flask and dissolved in 20 mL of anhydrous tetrahydrofuran. Intermediate 61-2 Reformatsky Reagent (20 mmol, 1 N, 2.0 e.q.) dissolved in 20 mL of tetrahydrofuran was added under argon atmosphere. The mixture was stirred at room temperature for 30 min. After the reaction was completely converted as detected, the solvent was removed by rotary evaporation, and the mixture was dissolved in ethyl acetate, washed three times with water and once with saturated brine. The organic phase was retained and dried over anhydrous sodium sulfate. The organic phase was purified by column chromatography to give product intermediate **61-23.**

The intermediate **61-23** (10 mmol, 1.0 eq) was dissolved in a mixed solvent of tetrahydrofuran/methanol/water (3:1:1, 50 mL), and potassium hydroxide (40 mmol, 4.0 eq) was added. The mixture was reacted at 50°C for 8 h. After the reactant was completely converted as detected, 2 N HCl solution was added to adjust the pH to 3. After all solvents were removed by rotary evaporation, 25 mL of methanol was added. The mixture was filtered, the filtrate was concentrated to dryness by rotary evaporation to give a light yellow solid, and finally the light-yellow solid was washed repeatedly with DCM/PE to give a white solid product **61-24.**

The intermediate **61-24** (2 mmol, 1.0 eq) was weighed and dissolved in 25 mL of extra dry toluene, triethylamine (4.4 mmol, 2.2 eq) was added, and DPPA (2.4 mmol, 1.2 eq) was added under argon atmosphere. The mixture was stirred at room temperature for 30 min until all carboxylic acid materials were converted into acyl azide, and heated to 75°C and reacted for 4 h until most acyl azide was converted into isocyanate. Excess hydrochloric acid (2 M aqueous solution, > 4.0 eq) was added, and the mixture was cooled to 60°C and reacted overnight. After the pH was adjusted to alkalinity with a sodium bicarbonate solution, the mixture was extracted with ethyl acetate, and the organic phase was collected, concentrated to dryness by rotary evaporation, and separated by silica gel column chromatography to give intermediate **61-25.**

The intermediate **61-25** and the previously obtained carboxylic acid intermediate 61-6 were added to a DMF solvent according to an equivalent ratio of 1:1. HATU (1.5 e.q.) and DIPEA (2.0 e.q.) were added, and the mixture was reacted at 50°C for 12 h. The reaction solution was extracted with ethyl acetate and washed with a saturated ammonium chloride solution for three times. The organic phase was concentrated to dryness by rotary evaporation and purified by silica gel column chromatography to give product **61-26.** The final product **61-26** was the compound of Example **61.**

(49) The synthetic route for preparation of the compound of Example **62** was as follows:

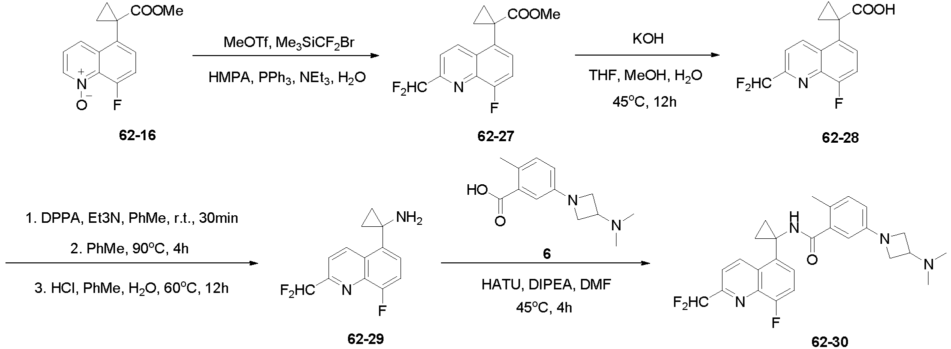

The intermediate **62-16** (4.4 mmol, 1.0 e.q.) was placed in a round bottom flask and dissolved with 40 mL of DCM. Methyl trifluoromethanesulfonate (4.4 mmol, 1.0 e.q.) was added dropwise with stirring at room temperature. The mixture was stirred at room temperature for 1 h. The solvent was removed by rotary evaporation, and the mixture was dissolved in 20 mL of anhydrous acetonitrile and stirred under a dry ice-acetone bath. Difluorobromomethyltrimethylsilane (19.8 mmol, 4.5 e.q.) and triphenylphosphine (13.2 mmol, 3.0 e.q.) were added sequentially, and HMPA was added dropwise. The mixture was stirred for 3 h, the ice bath was removed, and the mixture was stirred at room temperature for 15 min. The mixture was stirred under a dry ice-acetone bath again, triethylamine (22.0 mmol, 5.0 e.q.) and 20 mL of water were added sequentially, the ice bath was removed, and the mixture was stirred at room temperature for 12 h. After the reaction was completely converted as detected, the mixture was extracted with a proper amount of water and MTBE and washed once with saturated brine. The organic phase was retained and dried over anhydrous sodium sulfate. The organic phase was purified by column chromatography to give product intermediate **62-27.**

The intermediate **62-27** (10 mmol, 1.0 eq) was dissolved in a mixed solvent of tetrahydrofuran/methanol/water (3:1:1, 50 mL), and potassium hydroxide (40 mmol, 4.0 eq) was added. The mixture was reacted at 50°C for 8 h. After the reactant was completely converted as detected, 2 N HCl solution was added to adjust the pH to 3. After all solvents were removed by rotary evaporation, 25 mL of methanol was added. The mixture was filtered, the filtrate was concentrated to dryness by rotary evaporation to give a light yellow solid, and finally the light-yellow solid was washed repeatedly with DCM/PE to give a white solid product **62-28.**

The intermediate **62-28** (2 mmol, 1.0 eq) was weighed and dissolved in 25 mL of extra dry toluene, triethylamine (4.4 mmol, 2.2 eq) was added, and DPPA (2.4 mmol, 1.2 eq) was added under argon atmosphere. The mixture was stirred at room temperature for 30 min until all carboxylic acid materials were converted into acyl azide, and heated to 75°C and reacted for 4 h until most acyl azide was converted into isocyanate. Excess hydrochloric acid (2 M aqueous solution, > 4.0 eq) was added, and the mixture was cooled to 60°C and reacted overnight. After the pH was adjusted to alkalinity with a sodium bicarbonate solution, the mixture was extracted with ethyl acetate, and the organic phase was collected, concentrated to dryness by rotary evaporation, and separated by silica gel column chromatography to give intermediate **62-29.**

The intermediate **62-29** and the previously obtained carboxylic acid intermediate **6** were added to DMF as a solvent according to an equivalent ratio of 1:1. HATU (1.5 e.q.) and DIPEA (2.0 e.q.) were added, and the mixture was reacted at 50°C for 12 h. The reaction solution was extracted with ethyl acetate and washed with a saturated ammonium chloride solution for three times. The organic phase was concentrated to dryness by rotary evaporation and purified by silica gel column chromatography to give product **62-30.** The final product **62-30** was the compound of Example **62.**

(50) The synthetic route for preparation of the compound of Example **63** was as follows:

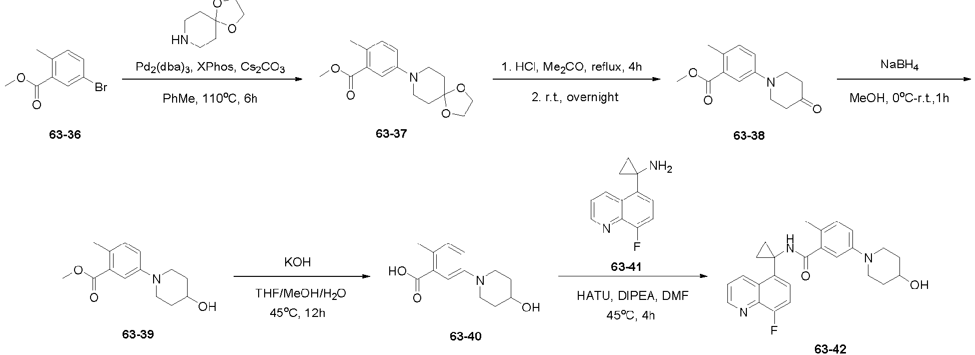

Methyl 2-methyl-5-bromobenzoate **63-36** (10 mmol, 1.0 e.q.), 4-piperidone ethylene ketal (10 mmol, 1.0 e.q.), Pd₂(dba)₃ (0.2 mmol, 0.02 eq), XPhos (0.8 mmol, 0.08 e.q.), and cesium carbonate (40 mmol, 4.0 eq) were dissolved in 50 mL of toluene, placed in a sealed tube, and heated to 110°C and reacted overnight under Ar atmosphere. The mixture was washed with water and ethyl acetate, and the organic phase was retained. The organic phase was subjected to column chromatography to give product intermediate **63-37.**

The intermediate **63-37** (10 mmol, 1.0 e.q.) was placed in a round bottom flask and dissolved in 30 mL of acetone. 10 mL of 5 N hydrochloric acid was added. The mixture was heated at reflux at 60°C and stirred for 4 h, and then stirred at room temperature overnight. After the reaction was completed, the solvent was removed by rotary evaporation. The mixture was extracted with water and ethyl acetate, and the organic phase was retained. The organic phase was subjected to column chromatography to give product intermediate **63-38.**

The intermediate **63-38** (3.3 mmol, 1.0 e.q.) was placed in around bottom flask and dissolved in 15 mL of anhydrous methanol. Sodium borohydride (3.7 mmol, 1.1 e.q.) was added with stirring under an ice bath, the ice bath was then removed, and the mixture was stirred at room temperature for 6 h. After the reaction was completed, the solvent was removed by rotary evaporation. The mixture was extracted with water and ethyl acetate, and the organic phase was retained. The organic phase was subjected to column chromatography to give product intermediate **63-39.**

The intermediate **63-39** (2.0 mmol, 1.0 eq) was dissolved in a mixed solvent of tetrahydrofuran/methanol/water (3:1:1, 50 mL), and potassium hydroxide (8 mmol, 4.0 eq) was added. The mixture was reacted at 50°C for 8 h. After the reactant was completely converted as detected, the solvent was removed by rotary evaporation, the pH was adjusted to 1 with 2 N HCl solution, and the mixture was extracted with ethyl acetate. The organic phase was extracted and washed for 1 time with saturated brine, collected, and concentrated to dryness to give a white solid product **63-40.**

The intermediate **63-40** and the previously obtained amine intermediate **63-41** were added to DMF as a solvent according to an equivalent ratio of 1:1. HATU (1.5 e.q.) and DIPEA (2.0 e.q.) were added, and the mixture was reacted at 50°C for 12 h. The reaction solution was extracted with ethyl acetate and washed with a saturated ammonium chloride solution for three times. The organic phase was concentrated to dryness by rotary evaporation and purified by silica gel column chromatography to give product **63-42.** The final product **63-42** was the compound of Example **63.**

(51) The synthetic route for preparation of the compound of Example **64** was as follows:

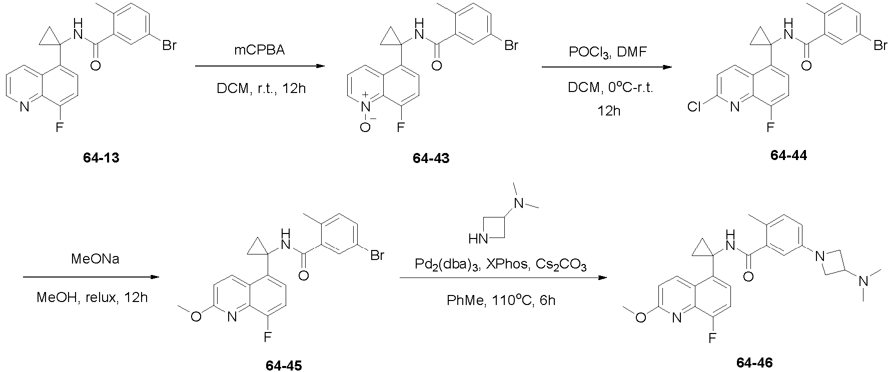

The product **64-13** (10 mmol, 1.0 e.q.) obtained previously and mCPBA (12 mmol, 1.2 e.q.) were placed in a round bottom flask and dissolved with 30 mL of DCM. The mixture was stirred at room temperature for 12 h. After the reaction was completely converted as detected, triphenylphosphine (5 mmol, 0.5 e.q.) was added, and the mixture was then stirred at room temperature for 4 h. The solvent was removed by rotary evaporation. The mixture was purified by column chromatography to give intermediate **64-43.**

The intermediate **64-43** (10 mmol, 1.0 e.q.) was placed in around bottom flask and dissolved with 30 mL of DCM. Phosphorus oxychloride (12 mmol, 1.2 e.q.) was added dropwise with stirring under an ice bath, and DMF (5 mmol, 0.5 e.q.) was then added dropwise. The mixture was stirred at room temperature for 12 h. After the reaction was completely converted as detected, a saturated sodium bicarbonate solution was added dropwise under an ice bath to adjust the pH of the solution to 8, and the mixture was extracted. The organic phase was washed with water for 2 times, washed with saturated brine for 1 time, collected, and concentrated to dryness by rotary evaporation to give intermediate **64-44** without purification.

The intermediate **64-44** (10 mmol, 1.0 e.q.) was placed in a round bottom flask and dissolved with 30 mL of anhydrous methanol. Sodium methoxide (5 M, 100 mmol, 10 e.q.) was added, and the mixture was stirred at reflux at 70°C for 12 h. After the reaction was completely converted as detected, the solvent was removed by rotary evaporation, and the mixture was extracted with a proper amount of ethyl acetate and a saturated ammonium chloride solution. The organic phase was extracted and washed for **1** time with saturated brine, collected, and concentrated to dryness to give intermediate **64-45** without purification.

The intermediate **64-45** (10 mmol, 1.0 e.q.), 3-(dimethylamino)azetidine (10 mmol, 1.0 e.q.), Pd₂(dba)₃ (0.2 mmol, 0.02 eq), XPhos (0.8 mmol, 0.08 e.q.), and cesium carbonate (40 mmol, 4.0 eq) were dissolved in 50 mL toluene, placed in a sealed tube, and heated to 110°C and reacted overnight under Ar atmosphere. The mixture was washed with water and ethyl acetate, and the organic phase was retained. The organic phase was subjected to column chromatography to give product **64-46.** The final product **64-46** was Example **64.**

(52) The synthetic route for preparation of the compound of Example 65 was as follows:

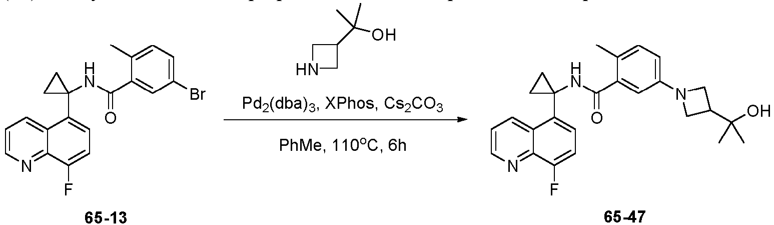

Intermediate **65-13** (10 mmol, 1.0 e.q.), 2-(azetidin-3-yl)propan-2-ol (10 mmol, 1.0 e.q.), Pd₂(dba)₃ (0.2 mmol, 0.02 eq), XPhos (0.8 mmol, 0.08 e.q.), and cesium carbonate (40 mmol, 4.0 eq) were dissolved in 50 mL toluene, placed in a sealed tube, and heated to 110°C and reacted for 6 h under Ar atmosphere. The mixture was washed with water and ethyl acetate, and the organic phase was retained. The organic phase was subjected to column chromatography to give product **65-47.** The final product **65-47** was the compound of Example **65.**

(53) The synthetic route for preparation of the compound of Example **66** was as follows:

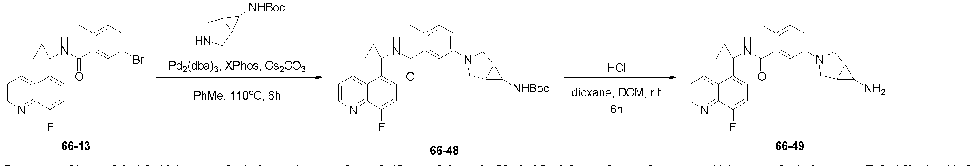

Intermediate **66-13** (10 mmol, 1.0 e.q.), *tert-butyl* (3-azabicyclo[3.1.0]-6-hexyl)-carbamate (10 mmol, 1.0 e.q.), Pd₂(dba)₃ (0.2 mmol, 0.02 eq), XPhos (0.8 mmol, 0.08 e.q.), and cesium carbonate (40 mmol, 4.0 eq) were dissolved in 50 mL toluene, placed in a sealed tube, and heated to 110°C and reacted for 6 h under Ar atmosphere. The mixture was washed with water and ethyl acetate, and the organic phase was retained. The organic phase was subjected to column chromatography to give intermediate **66-48.**

The intermediate **66-48** (10 mmol, 1.0 e.q.) was placed in a round bottom flask and dissolved in 50 mL of DCM. A solution of hydrochloric acid in dioxane (4.0 M, 40 mmol, 4.0 e.q.) was added, and the mixture was stirred at room temperature overnight. After the reaction was completely converted, the solvent was removed by rotary evaporation, a saturated sodium carbonate solution was added, and the mixture was extracted with DCM. The organic phase was dried over anhydrous magnesium sulfate, filtered under vacuum, and slurried with DCM/cyclohexane after the solvent was removed by rotary evaporation to give a yellow solid, which was product **66-49.** The final product **66-49** was the compound of Example **66.**

The preparation of the branched route step 2 was as follows:

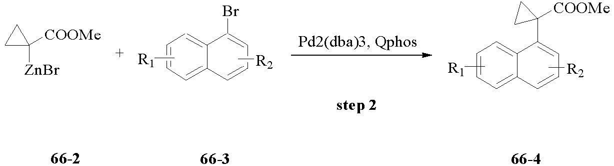

The synthetic route for preparation of intermediate 66-4i was as follows:

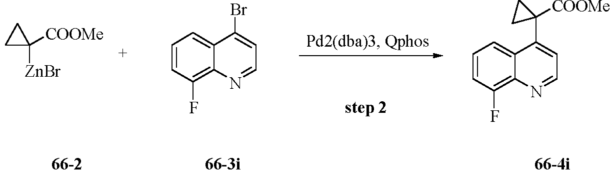

2260 mg (10 mmol, 1.0 eq.) of 4-bromo-8-fluoroquinoline 66-3i was weighed, 91.5 mg (0.1 mmol, 0.01 eq) of Pd2(dba)3 and 71 mg (0.1 mmol, 0.01 eq) of Qphos were added, and the mixture was dissolved in 20 mL of anhydrous tetrahydrofuran. Intermediate 66-2 Reformatsky reagent (20 mmol, 1 N, 2.0 eq) dissolved in 20 mL of tetrahydrofuran was added under Ar atmosphere. The mixture was stirred at room temperature for 30 min. After the reaction was completely converted as detected, the solvent was removed by rotary evaporation, and the mixture was dissolved in ethyl acetate, washed three times with water and once with saturated brine. The organic phase was retained and dried over anhydrous sodium sulfate. The organic phase was purified by column chromatography to give product intermediate 66-4i.

The synthetic route for preparation of intermediate 66-4j was as follows:

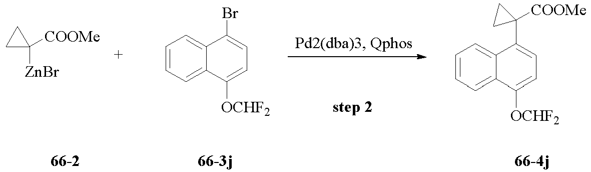

2730 mg (10 mmol, 1.0 eq.) of 1-bromo-4-(difluoromethoxy)naphthalene 66-3j was weighed, 91.5 mg (0.1 mmol, 0.01 eq) of Pd2(dba)3 and 71 mg (0.1 mmol, 0.01 eq) of Qphos were added, and the mixture was dissolved in 20 mL of anhydrous tetrahydrofuran. Intermediate 66-2 Reformatsky reagent (20 mmol, 1 N, 2.0 eq) dissolved in 20 mL of tetrahydrofuran was added under Ar atmosphere. The mixture was stirred at room temperature for 30 min. After the reaction was completely converted as detected, the solvent was removed by rotary evaporation, and the mixture was dissolved in ethyl acetate, washed three times with water and once with saturated brine. The organic phase was retained and dried over anhydrous sodium sulfate. The organic phase was purified by column chromatography to give product intermediate 66-4j.

The synthetic route for preparation of intermediate 66-4k was as follows:

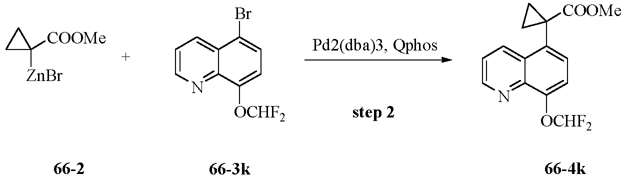

2740 mg (10 mmol, 1.0 eq.) of 5-bromo-8-(difluoromethoxy)quinoline 66-3k was weighed, 91.5 mg (0.1 mmol, 0.01 eq) of Pd2(dba)3 and 71 mg (0.1 mmol, 0.01 eq) of Qphos were added, and the mixture was dissolved in 20 mL of anhydrous tetrahydrofuran. Intermediate 66-2 Reformatsky reagent (20 mmol, 1 N, 2.0 eq) dissolved in 20 mL of tetrahydrofuran was added under Ar atmosphere. The mixture was stirred at room temperature for 30 min. After the reaction was completely converted as detected, the solvent was removed by rotary evaporation, and the mixture was dissolved in ethyl acetate, washed three times with water and once with saturated brine. The organic phase was retained and dried over anhydrous sodium sulfate. The organic phase was purified by column chromatography to give product intermediate 66-4k.

The synthetic route for preparation of intermediate 66-4l was as follows:

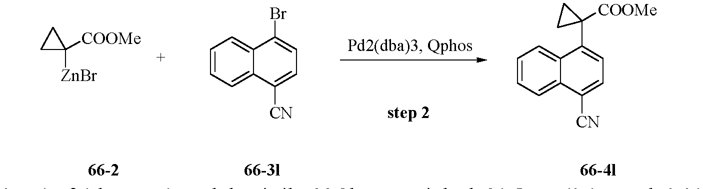

2320 mg (10 mmol, 1.0 eq.) of 4-bromo-1-naphthonitrile 66-31 was weighed, 91.5 mg (0.1 mmol, 0.01 eq) of Pd2(dba)3 and 71 mg (0.1 mmol, 0.01 eq) of Qphos were added, and the mixture was dissolved in 20 mL of anhydrous tetrahydrofuran. Intermediate 66-2 Reformatsky reagent (20 mmol, 1 N, 2.0 eq) dissolved in 20 mL of tetrahydrofuran was added under Ar atmosphere. The mixture was stirred at room temperature for 30 min. After the reaction was completely converted as detected, the solvent was removed by rotary evaporation, and the mixture was dissolved in ethyl acetate, washed three times with water and once with saturated brine. The organic phase was retained and dried over anhydrous sodium sulfate. The organic phase was purified by column chromatography to give product intermediate 66-4l.

The preparation of the branched route step 3 was as follows:

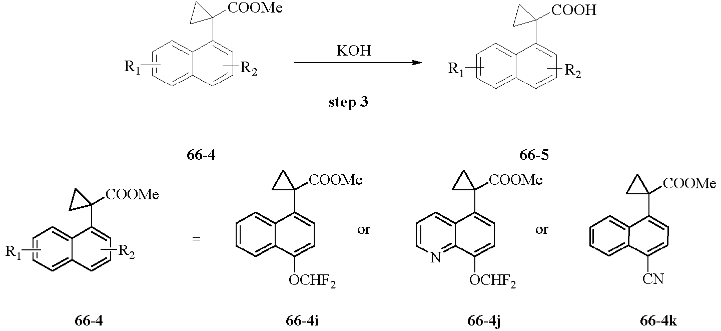

The synthetic route for preparation of intermediate 66-5i was as follows:

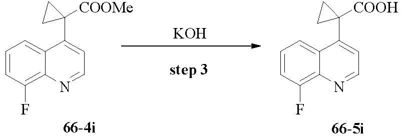

2450 mg (10 mmol, 1.0 eq) of intermediate 66-4i was weighed and dissolved in a mixed solvent of tetrahydrofuran/methanol/water (3:1:1, 65 mL), and 2240 mg of potassium hydroxide (40 mmol, 4.0 eq) was added. The mixture was reacted at 50°C for 8 h. After the reactant was completely converted as detected, 2 N HCl solution was added to adjust the pH to 3. After all solvents were removed by rotary evaporation, 25 mL of methanol was added. The mixture was filtered, the filtrate was concentrated to dryness by rotary evaporation to give a gray solid, and finally the gray solid was washed repeatedly with DCM/PE to give a white solid product **66-5i.**

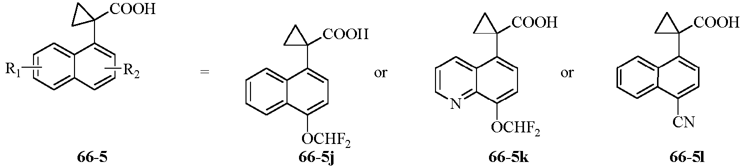

The routes for intermediates **66-5j** to **66-5l** were the same as the preparation method of intermediate 66-5i.

The preparation route of the branched route step 4 was as follows:

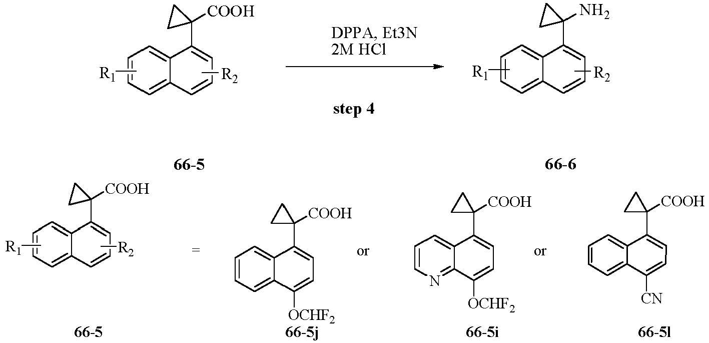

The synthetic route for preparation of intermediate **66-6i** was as follows:

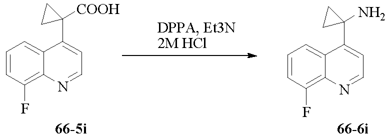

462 mg (2 mmol, 1.0 eq) of intermediate **66-5i** was weighed and dissolved in 25 mL of extra dry toluene, 0.611 mL of triethylamine (4.4 mmol, 2.2 eq) was added, and 0.516 mL of DPPA (2.4 mmol, 1.2 eq) was added under Ar atmosphere. The mixture was stirred at room temperature for 30 min until all carboxylic acid materials were converted into acyl azide, and heated to 75°C and reacted for 4 h until most acyl azide was converted into isocyanate. Excess hydrochloric acid (2 M aqueous solution, > 4.0 eq) was added, and the mixture was cooled to 60°C and reacted overnight. A sodium bicarbonate solution was added to adjust the pH to alkalinity, and the mixture was extracted with ethyl acetate. The organic phase was added to 2.5 mL of HCl (4 M HCl in dioxane,10 mmol, 2.0 eq) solution, and the mixture was filtered by using a sand-core funnel and washed with petroleum ether and ethyl acetate for multiple times to give a white powder product **66-6i.**

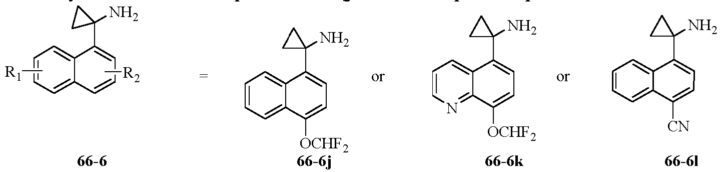

The routes of intermediates 66-6j to 66-6l were the same as the preparation method of intermediate 66-6a.

The preparation of the final product branched route step 7 was as follows:

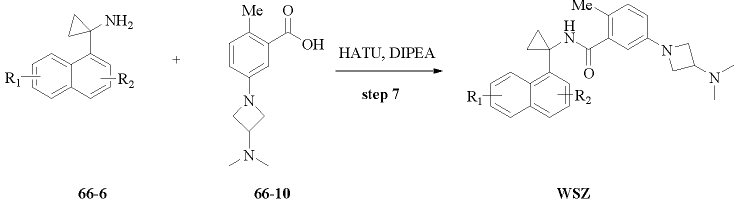

(54) The synthetic route for preparation of the compound of Example **67** was as follows:

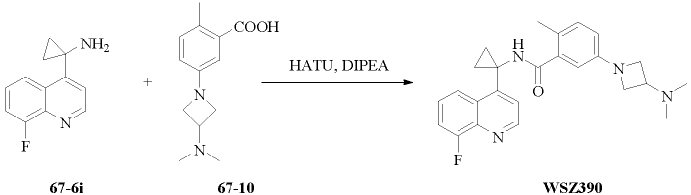

202 mg of amine intermediate **67-6i** (1 mmol, 1.0 eq), 234 mg of carboxylic acid intermediate **67-10** (1 mmol, 1.0 eq), and 890 µL of DIPEA (5 mmol, 5.0 eq) were weighed, dissolved in 5 mL of DMF, and stirred until the mixture was completely dissolved. 570 mg of HATU (1.5 mmol, 1.5 eq) was added, and the mixture was reacted at room temperature for 3 h. After the reaction starting materials were completely converted, the reaction solution was washed with water and ethyl acetate to remove DMF. The organic phase was retained and concentrated to dryness by rotary evaporation to give a crude product, which was purified by column chromatography to give the final product WSZ390, i.e., the **compound of Example 67.**

(55) The synthetic route for preparation of the compound of Example **68** was as follows:

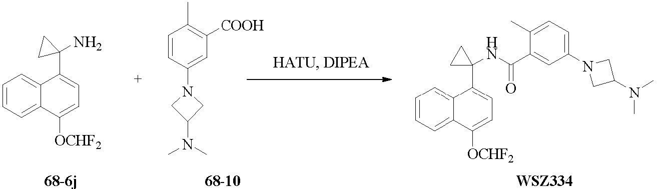

249 mg of amine intermediate **68-6j** (1 mmol, 1.0 eq), 234 mg of carboxylic acid intermediate **68-10** (1 mmol, 1.0 eq), and 890 µL of DIPEA (5 mmol, 5.0 eq) were weighed, dissolved in 5 mL of DMF, and stirred until the mixture was completely dissolved. 570 mg of HATU (1.5 mmol, 1.5 eq) was added, and the mixture was reacted at room temperature for 3 h. After the reaction starting materials were completely converted, the reaction solution was washed with water and ethyl acetate to remove DMF. The organic phase was retained and concentrated to dryness by rotary evaporation to give a crude product, which was purified by column chromatography to give the final product WSZ334, i.e., the **compound of Example 68.**

(56) The synthetic route for preparation of the compound of Example **69** was as follows:

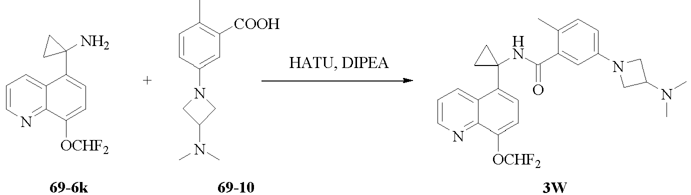

250 mg of amine intermediate **69-6k** (1 mmol, 1.0 eq), 234 mg of carboxylic acid intermediate **69-10** (1 mmol, 1.0 eq), and 890 µL of DIPEA (5 mmol, 5.0 eq) were weighed, dissolved in 5 mL of DMF, and stirred until the mixture was completely dissolved. 570 mg of HATU (1.5 mmol, 1.5 eq) was added, and the mixture was reacted at room temperature for 3 h. After the reaction starting materials were completely converted, the reaction solution was washed with water and ethyl acetate to remove DMF. The organic phase was retained and concentrated to dryness by rotary evaporation to give a crude product, which was purified by column chromatography to give the final product 3W, i.e., the **compound of Example 69.**

(57) The synthetic route for preparation of the compound of Example **70** was as follows:

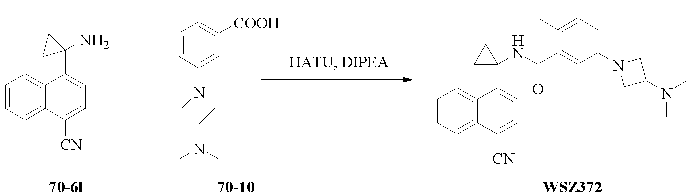

208 mg of amine intermediate **70-6l** (1 mmol, 1.0 eq), 234 mg of carboxylic acid intermediate **70-10** (1 mmol, 1.0 eq), and 890 µL of DIPEA (5 mmol, 5.0 eq) were weighed, dissolved in 5 mL of DMF, and stirred until the mixture was completely dissolved. 570 mg of HATU (1.5 mmol, 1.5 eq) was added, and the mixture was reacted at room temperature for 3 h. After the reaction starting materials were completely converted, the reaction solution was washed with water and ethyl acetate to remove DMF. The organic phase was retained and concentrated to dryness by rotary evaporation to give a crude product, which was purified by column chromatography to give the final product WSZ372, i.e., the **compound of Example 70.**

(58) The synthetic routes for preparation of the compounds of Examples **73, 74, 75, 76, 77, 78, 81, 82, and 83** were as follows: 1. The synthetic route for preparation of intermediate A-5a was as follows:

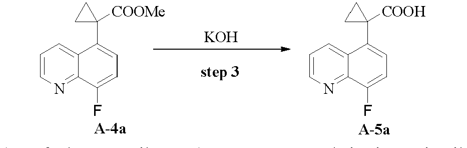

2518 mg (11.09 mmol, 1.0 eq) of intermediate **A-4a** was weighed and dissolved in a mixed solvent of tetrahydrofuran/methanol/water (3:1:1, 65 mL), and 2484 mg of potassium hydroxide (44.37 mmol, 4.0 eq) was added. The mixture was reacted at 50°C for 8 h. After the reactant was completely converted as detected, a 2 N HCl solution was added to adjust the pH to 3. After all solvents were removed by rotary evaporation, 25 mL of methanol was added. The mixture was filtered, the filtrate was concentrated to dryness by rotary evaporation to give a gray solid, and finally the gray solid was washed repeatedly with DCM/PE to give a white solid product **A-5a.**

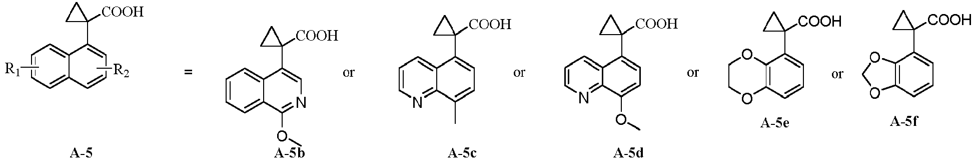

The routes of intermediates **A-5b to A-5f** were the same as the preparation method of intermediate A-5a.

2. The preparation route of the branched route step 4 was as follows:

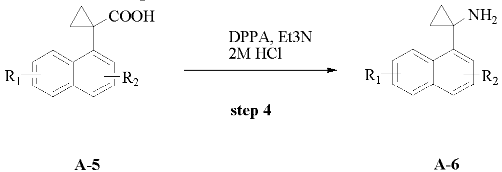

3. The synthetic route for preparation of intermediate **A-6a** was as follows:

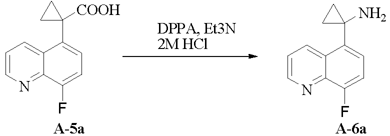

426 mg (2 mmol, 1.0 eq) of intermediate **A-5a** was weighed and dissolved in 25 mL of extra dry toluene, 0.611 mL of triethylamine (4.4 mmol, 2.2 eq) was added, and 0.516 mL of DPPA (2.4 mmol, 1.2 eq) was added under Ar atmosphere. The mixture was stirred at room temperature for 30 min until all carboxylic acid materials were converted into acyl azide, and heated to 75°C and reacted for 4 h until most acyl azide was converted into isocyanate. Excess hydrochloric acid (2 M aqueous solution, > 4.0 eq) was added, and the mixture was cooled to 60°C and reacted overnight. After the pH was adjusted to alkalinity with a sodium bicarbonate solution, the mixture was extracted with ethyl acetate and subjected to column chromatography to give a white powder product **A-6a.**

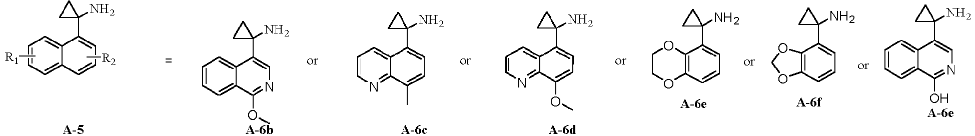

4. The synthetic route for preparation of intermediate A-6b was as follows:

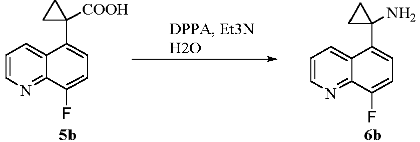

426 mg (2 mmol, 1.0 eq) of intermediate **A-5a** was weighed and dissolved in 25 mL of extra dry toluene, 0.611 mL of triethylamine (4.4 mmol, 2.2 eq) was added, and 0.516 mL of DPPA (2.4 mmol, 1.2 eq) was added under Ar atmosphere. The mixture was stirred at room temperature for 30 min until all carboxylic acid materials were converted into acyl azide, and heated to 75°C and reacted for 4 h until most acyl azide was converted into isocyanate. 4 M water was added, and the mixture was cooled to 60°C and reacted overnight. After the pH was adjusted to alkalinity with a sodium bicarbonate solution, the mixture was extracted with ethyl acetate and subjected to column chromatography to give a gray powder product **A-6b.**

The routes of intermediates **A-6c to A-6g** were the same as the preparation method of intermediate **A-6a.**

5. The preparation route for branched route step 5 was as follows:

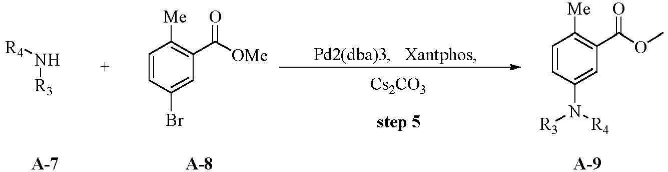

6. The synthetic route for preparation of intermediate **A-9a** was as follows:

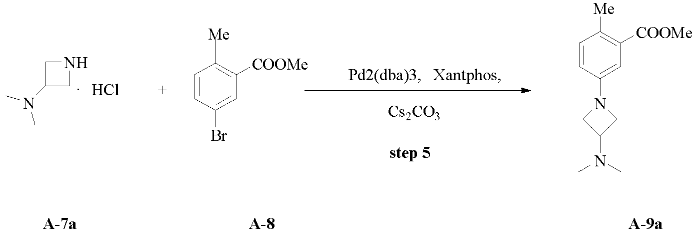

4580 mg of methyl 2-methyl-5-bromobenzoate **A-8** (20 mmol, 1.0 eq), 3740 mg of 3-(dimethylamino)azetidine dihydrochloride **A-7a** (22 mmol, 1.1 eq), 370 mg (0.4 mmol, 0.02 eq) of Pd₂(dba)₃, 760 mg of XPhos (1.6 mmol, 0.08 eq), and 26080 mg of cesium carbonate (80 mmol, 4.0 eq) were weighed, dissolved in 100 mL of toluene, placed in a sealed tube, and heated to 110°C overnight under Ar atmosphere. The mixture was washed with water and ethyl acetate, and the organic phase was retained. The organic phase was subjected to column chromatography to give product intermediate **A-9a.**

7. The preparation of the branched route step 6 was as follows:

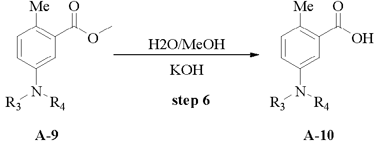

8. The synthetic route for preparation of intermediate **A-10a** was as follows:

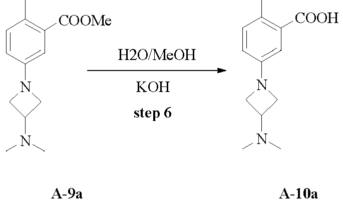

Intermediate **A-9a** (10 mmol, 1 eq.) was weighed and dissolved in a mixed solution of 25 mL methanol and 25 mL water, potassium hydroxide (40 mmol, 4 eq.) was added, and the mixture was heated to 60°C and stirred overnight. After the reaction was completed, excess hydrochloric acid was added to adjust the pH of the reaction solution to acidity (do not over-acidified, as the product has the risk of ring opening). The solvent was completely removed by rotary evaporation (after the first rotary evaporation, a small amount of methanol may be added for multiple times for rotary evaporation to remove water as much as possible), methanol was added, and the mixture was stirred and filtered under vacuum. If the filtered solid still contained many products, the solid was dissolved with methanol for multiple times and filtered under vacuum until the solid was completely insoluble (no fluorescence was detected by thin-layer chromatography plate and ultraviolet light). The filtrate was collected, concentrated, and recrystallized with dichloromethane to give product intermediate **A-10a.**

9. The preparation of the final product branched route step 7 was as follows:

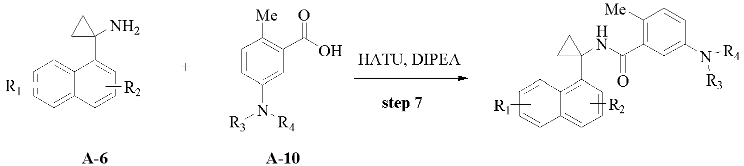

10. The synthetic route for preparation of the compound of Example **74** was as follows:

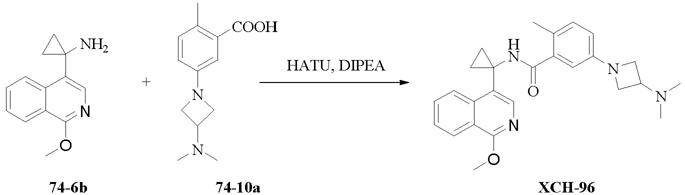

214 mg of amine intermediate **74-6b** (1 mmol, 1.0 eq), 270 mg of carboxylic acid intermediate **74-10a** (1 mmol, 1.0 eq), and 1008 µL of DIPEA (5 mmol, 5.0 eq) were weighed, dissolved in 10 mL of DMF, and stirred until the mixture was completely dissolved. 668 mg of HATU (1.5 mmol, 1.5 eq) was added, and the mixture was reacted at room temperature for 3 h. After the reaction starting materials were completely converted, the reaction solution was washed with water and ethyl acetate to remove DMF. The organic phase was retained and concentrated to dryness by rotary evaporation to give a crude product, which was purified by column chromatography to give the final product XCH-96, i.e., the **compound of Example 74.**

11. The synthetic route for preparation of the compound of Example **73** was as follows:

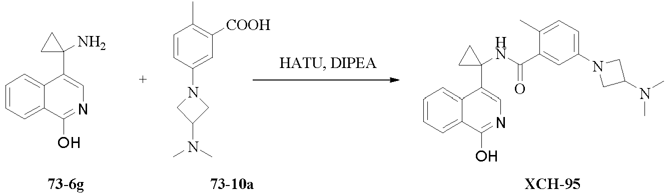

200 mg of amine intermediate **73-6g** (1 mmol, 1.0 eq), 270 mg of carboxylic acid intermediate **73-10a** (1 mmol, 1.0 eq), and 1008 µL of DIPEA (5 mmol, 5.0 eq) were weighed, dissolved in 10 mL of DMF, and stirred until the mixture was completely dissolved. 668 mg of HATU (1.5 mmol, 1.5 eq) was added, and the mixture was reacted at room temperature for 3 h. After the reaction starting materials were completely converted, the reaction solution was washed with water and ethyl acetate to remove DMF. The organic phase was retained and concentrated to dryness by rotary evaporation to give a crude product, which was purified by column chromatography to give the final product XCH-95, i.e., the **compound of Example** 73.

12. The synthetic route for preparation of the compound of Example **75** was as follows:

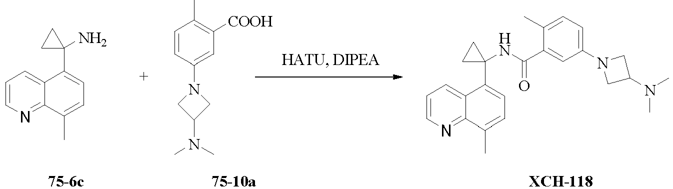

198 mg of amine intermediate **75-6c** (1 mmol, 1.0 eq), 270 mg of carboxylic acid intermediate **75-10a** (1 mmol, 1.0 eq), and 1008 µL of DIPEA (5 mmol, 5.0 eq) were weighed, dissolved in 10 mL of DMF, and stirred until the mixture was completely dissolved. 668 mg of HATU (1.5 mmol, 1.5 eq) was added, and the mixture was reacted at room temperature for 3 h. After the reaction starting materials were completely converted, the reaction solution was washed with water and ethyl acetate to remove DMF. The organic phase was retained and concentrated to dryness by rotary evaporation to give a crude product, which was purified by column chromatography to give the final product XCH-118, i.e., the **compound of Example 75.**

13. The synthetic route for preparation of the compound of Example 76 was as follows:

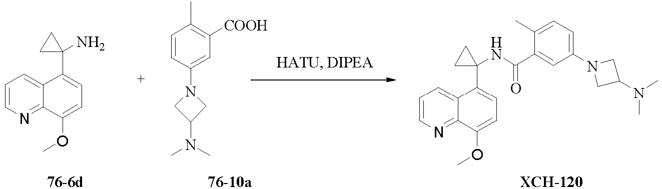

214 mg of amine intermediate **76-6d** (1 mmol, 1.0 eq), 270 mg of carboxylic acid intermediate **76-10a** (1 mmol, 1.0 eq), and 1008 µL of DIPEA (5 mmol, 5.0 eq) were weighed, dissolved in 10 mL of DMF, and stirred until the mixture was completely dissolved. 668 mg of HATU (1.5 mmol, 1.5 eq) was added, and the mixture was reacted at room temperature for 3 h. After the reaction starting materials were completely converted, the reaction solution was washed with water and ethyl acetate to remove DMF. The organic phase was retained and concentrated to dryness by rotary evaporation to give a crude product, which was purified by column chromatography to give the final product XCH-120, i.e., the **compound of Example 76.**

14. The synthetic route for preparation of the compound of Example **77** was as follows:

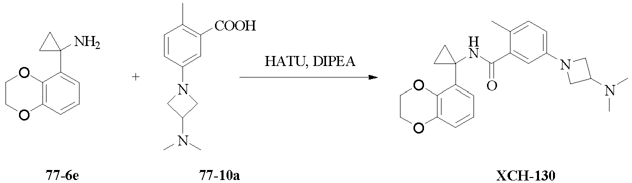

191 mg of amine intermediate **77-6e** (1 mmol, 1.0 eq), 270 mg of carboxylic acid intermediate **77-10a** (1 mmol, 1.0 eq), and 1008 µL of DIPEA (5 mmol, 5.0 eq) were weighed, dissolved in 10 mL of DMF, and stirred until the mixture was completely dissolved. 668 mg of HATU (1.5 mmol, 1.5 eq) was added, and the mixture was reacted at room temperature for 3 h. After the reaction starting materials were completely converted, the reaction solution was washed with water and ethyl acetate to remove DMF. The organic phase was retained and concentrated to dryness by rotary evaporation to give a crude product, which was purified by column chromatography to give the final product XCH-130, i.e., **Example 77.**

15. The synthetic route for preparation of the compound of Example **78** was as follows:

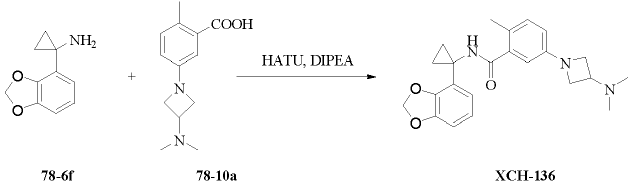

177 mg of amine intermediate **78-6f** (1 mmol, 1.0 eq), 270 mg of carboxylic acid intermediate **78-10a** (1 mmol, 1.0 eq), and 1008 µL of DIPEA (5 mmol, 5.0 eq) were weighed, dissolved in 10 mL of DMF, and stirred until the mixture was completely dissolved. 668 mg of HATU (1.5 mmol, 1.5 eq) was added, and the mixture was reacted at room temperature for 3 h. After the reaction starting materials were completely converted, the reaction solution was washed with water and ethyl acetate to remove DMF. The organic phase was retained and concentrated to dryness by rotary evaporation to give a crude product, which was purified by column chromatography to give the final product XCH-136, i.e., the **compound of Example 78.**

The preparation of the branched route step 8 was as follows:

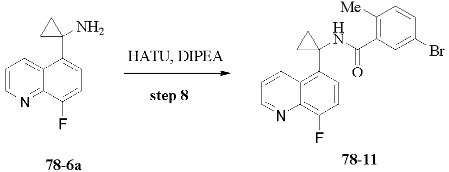

2-methyl-5-bromobenzoic acid and the previously obtained three-membered ring amine intermediate **78-6a** were added to DMF as a solvent according to an equivalent ratio of 1:1. HATU (1.5 e.q.) and DIPEA (2.0 e.q.) were added, and the mixture was reacted at 25°C for 3 h. The reaction solution was extracted with ethyl acetate and washed with a saturated ammonium chloride solution for three times. The organic phase was concentrated to dryness by rotary evaporation and purified by silica gel column chromatography to give intermediate **78-11.**

The preparation of the final product branched route step 9 was as follows:

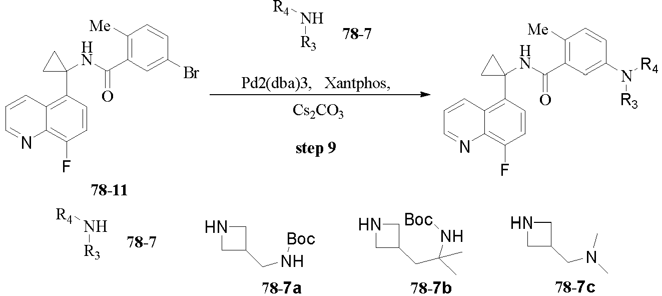

16. The synthetic route for preparation of the **intermediate compound XCH-200-Boc compound of Example 81** was as follows:

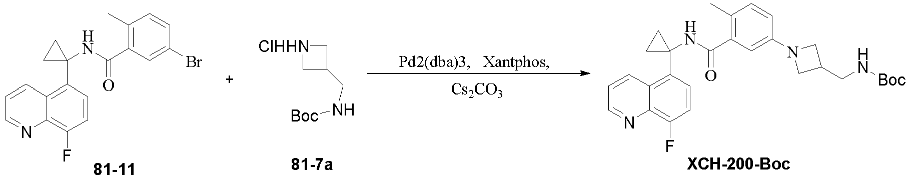

399 mg of **81-11** (1 mmol, 1.0 eq), 222 mg of 3-(dimethylamino)azetidine dihydrochloride **81-7a** (1 mmol, 1.1 eq), 23 mg (0.025 mmol, 0.02 eq) of Pd₂(dba)₃, 24 mg of XPhos (0.05 mmol, 0.04 eq), and 1220 mg of cesium carbonate (80 mmol, 3.0 eq) were weighed, dissolved in 10 mL of toluene, placed in a sealed tube, and heated to 110°C overnight under Ar atmosphere. The mixture was washed with water and ethyl acetate, and the organic phase was retained. The organic phase was subjected to column chromatography to give product intermediate **XCH-200-Boc.**

17. The synthetic route for preparation of the compound of Example **81** was as follows:

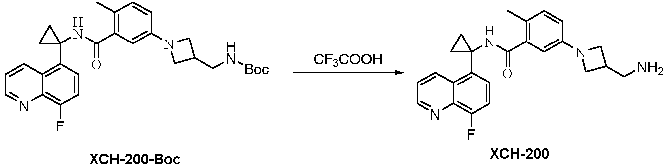

252 mg of **XCH-200-Boc** (0.5 mmol, 1.0 eq) was weighed and dissolved in 3 mL of toluene, and 1 mL of trifluoroacetic acid was added. The mixture was placed in a flask and stirred at room temperature overnight. The mixture was extracted with a saturated aqueous sodium bicarbonate solution and ethyl acetate, and the organic phase was retained. The organic phase was subjected to column chromatography to give the final product **XCH-200, which was the compound of Example 81.**

18. The synthetic route for preparation of the **intermediate compound XCH-205-Boc of Example 82** was as follows:

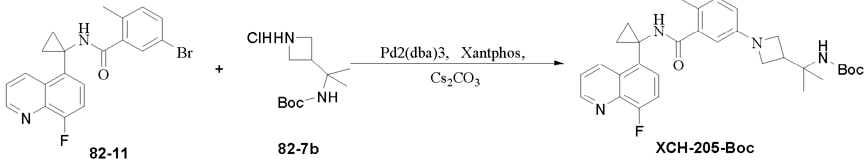

399 mg of 82-11 (1 mmol, 1.0 eq), 244 mg of 3-(dimethylamino)azetidine dihydrochloride **82-7b** (1 mmol, 1.1 eq), 23 mg (0.025 mmol, 0.02 eq) of Pd₂(dba)₃, 24 mg of XPhos (0.05 mmol, 0.04 eq), and 1220 mg of cesium carbonate (80 mmol, 3.0 eq) were weighed, dissolved in 10 mL of toluene, placed in a sealed tube, and heated to 110°C overnight under Ar atmosphere. The mixture was washed with water and ethyl acetate, and the organic phase was retained. The organic phase was subjected to column chromatography to give product intermediate **XCH-205-Boc.**

19. The synthetic route for preparation of the compound of Example **82** was as follows:

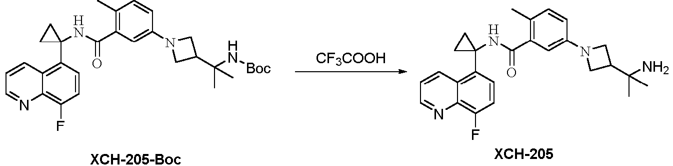

252 mg of **XCH-205-Boc** (0.5 mmol, 1.0 eq) was weighed and dissolved in 3 mL of toluene, and 1 mL of trifluoroacetic acid was added. The mixture was placed in a flask and stirred at room temperature overnight. The mixture was extracted with a saturated aqueous sodium bicarbonate solution and ethyl acetate, and the organic phase was retained. The organic phase was subjected to column chromatography to give the final product XCH-205, which was the **compound of Example 82.**

20. The synthetic route for preparation of the compound of Example **83** was as follows:

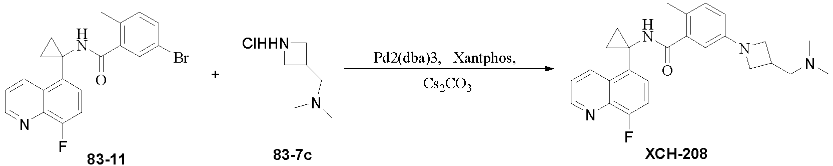

399 mg of **83-11** (1 mmol, 1.0 eq), 244 mg of 3-(dimethylamino)azetidine dihydrochloride **83-7c** (1 mmol, 1.1 eq), 23 mg (0.025 mmol, 0.02 eq) of Pd₂(dba)₃, 24 mg of XPhos (0.05 mmol, 0.04 eq), and 1220 mg of cesium carbonate (80 mmol, 3.0 eq) were weighed, dissolved in 10 mL of toluene, placed in a sealed tube, and heated to 110°C overnight under Ar atmosphere.

The mixture was washed with water and ethyl acetate, and the organic phase was retained. The organic phase was subjected to column chromatography to give the final product XCH-208, which was **the compound of Example 83.** (59) The synthetic routes for preparation of the compounds of Examples **79** and **80** were as follows:

The synthetic route for preparation of the compound of Example **79** was as follows:

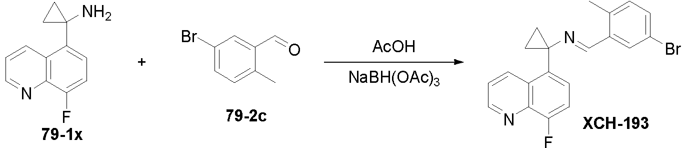

183 mg of **79-1x** (1 mmol, 1.0 eq), 122 mg of **79-2c** (1 mmol, 1.0 eq), 33 µL (1 mmol, 1 eq) of AcOH, 635 mg of NaBH(OAc)₃ (3 mmol, 3.0 eq) were weighed, dissolved in 10 mL of THF, placed in a flask, and stirred overnight under Ar atmosphere. The mixture was washed with a saturated aqueous sodium bicarbonate solution and ethyl acetate, and the organic phase was retained. The organic phase was subjected to column chromatography to give the final product XCH-193, which was the **compound of Example 79.** Nuclear magnetic resonance mass spectrum confirmed that a Schiff base product was obtained. The synthetic route for preparation of the compound of Example **80** was as follows:

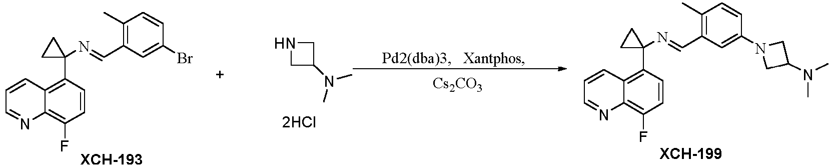

385 mg of the **compound of Example 79, i.e., XCH-193 (1** mmol, 1.0 eq), 244 mg of 3-(dimethylamino)azetidine dihydrochloride (1 mmol, 1.1 eq), 23 mg (0.025 mmol, 0.02 eq) of Pd₂(dba)₃, 24 mg of XPhos (0.05 mmol, 0.04 eq), and 1220 mg of cesium carbonate (80 mmol, 3.0 eq) were weighed, dissolved in 10 mL of toluene, placed in a sealed tube, and heated to 110°C overnight under Ar atmosphere. The mixture was washed with water and ethyl acetate, and the organic phase was retained. The organic phase was subjected to column chromatography to give the final product XCH-199, which was the **compound of Example 80.**

(60) The synthetic routes for preparation of the compounds of Examples **84, 85, and 86** were as follows:

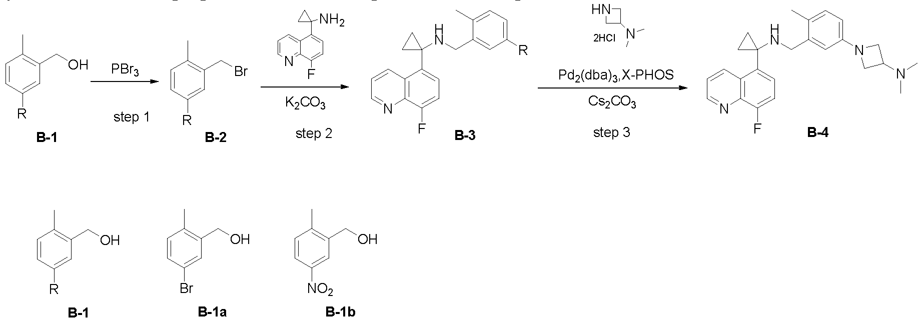

The preparation route of the branched route step 1 was as follows:

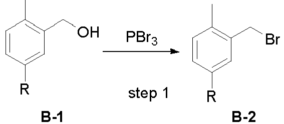

The synthetic route for preparation of intermediate **B-2a** was as follows:

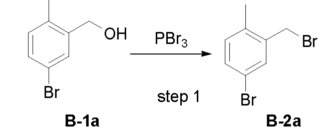

1000 mg (5 mmol, 1.0 eq) of intermediate b-1a was weighed and dissolved in 25 mL of dichloromethane, and 0.313 mL of PBr₃ (3.3 mmol, 0.6 eq) was added under an ice bath. The mixture was stirred at room temperature for 30 min. After the pH was adjusted to alkalinity with a saturated aqueous sodium bicarbonate solution, the mixture was extracted with ethyl acetate and subjected to column chromatography to give product **B-2a.**

The preparation of intermediate **B-2b** was the same as the preparation of intermediate **B-2a.**

The preparation route of the branched route step 2 was as follows:

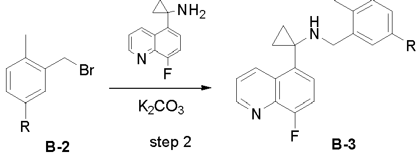

The synthetic route for preparation of the compound of Example **84** was as follows:

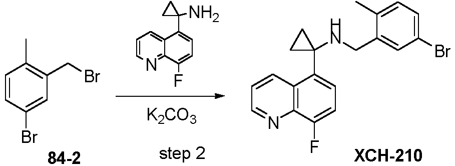

271 mg of **84-2** (1 mmol, 1.0 eq), 190 mg of three-membered ring amine intermediate (1 mmol, 1.0 eq), and 195 mg of K₂CO₃ (1.5 mmol, 1.5 eq) were weighed, dissolved in 10 mL of THF, placed in a flask, and stirred at room temperature overnight under Ar atmosphere. The mixture was washed with a saturated aqueous sodium chloride solution and ethyl acetate, and the organic phase was retained. The organic phase was subjected to column chromatography to give the final product XCH-210, which was the **compound of Example 84.**

The synthetic route for preparation of intermediate **84-3b** was as follows:

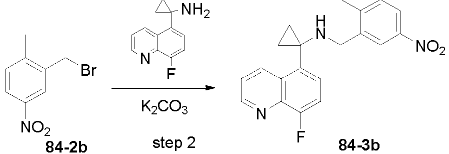

271 mg of 84-2b (1 mmol, 1.0 eq), 190 mg of three-membered ring amine intermediate (1 mmol, 1.0 eq), and 195 mg of K₂CO₃ (1.5 mmol, 1.5 eq) were weighed, dissolved in 10 mL of THF, placed in a flask, and stirred at room temperature overnight under Ar atmosphere. The mixture was washed with a saturated aqueous sodium chloride solution and ethyl acetate, and the organic phase was retained. The organic phase was subjected to column chromatography to give intermediate **84-3b.** The synthetic route of the preparation of the compound of Example **86** was as follows:

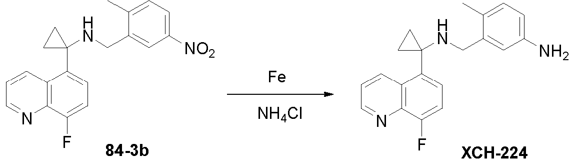

351 mg of **84-3b** (1 mmol, 1.0 eq), 109 mg of ammonium chloride (2 mmol, 2.0 eq), and 558 mg of Fe (10 mmol, 10.0 eq) were weighed, dissolved in a mixed solvent of 5 mL THF:5 mL EtOH:2.5 mL H₂O, placed in a flask, and stirred at 80°C for 5 h under Ar atmosphere. The mixture was filtered through celite and extracted with ethyl acetate, and the organic phase was retained. The organic phase was subjected to column chromatography to give the final product XCH-224, which was the **compound of Example 86.**

The synthetic route for preparation of the compound of Example **85** was as follows:

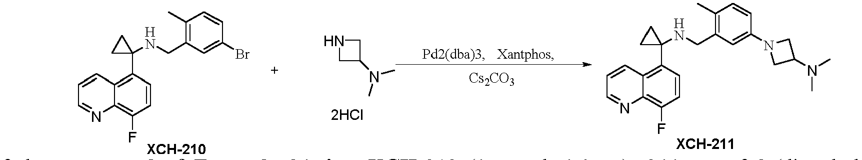

385 mg of the **compound of Example 84, i.e., XCH-210** (1 mmol, 1.0 eq), 244 mg of 3-(dimethylamino)azetidine dihydrochloride (1 mmol, 1.1 eq), 23 mg (0.025 mmol, 0.02 eq) of Pd₂(dba)₃, 24 mg of XPhos (0.05 mmol, 0.04 eq), and 1220 mg of cesium carbonate (80 mmol, 3.0 eq) were weighed, dissolved in 10 mL of toluene, placed in a sealed tube, and heated to 110°C overnight under Ar atmosphere. The mixture was washed with water and ethyl acetate, and the organic phase was retained. The organic phase was subjected to column chromatography to give the final product XCH-211, which was the **compound of Example 85.**

(61) The synthetic route for preparation of the compound of Example C87 was as follows:

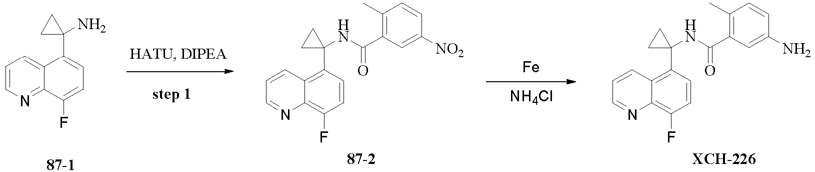

2-Methyl-5-nitrobenzoic acid and three-membered ring amine intermediate 87-1 were added to DMF as a solvent according to an equivalent ratio of 1:1. HATU (1.5 e.q.) and DIPEA (2.0 e.q.) were added, and the mixture was reacted at 25°C for 3 h. The reaction solution was extracted with ethyl acetate and washed with a saturated ammonium chloride solution for three times. The organic phase was concentrated to dryness by rotary evaporation and purified by silica gel column chromatography to give intermediate **87-2.**

365 mg of **87-2** (1 mmol, 1.0 eq), 109 mg of ammonium chloride (2 mmol, 2.0 eq), and 558 mg of Fe (10 mmol, 10.0 eq) were weighed, dissolved in a mixed solvent of 5 mL THF:5 mL EtOH:2.5 mL H₂O, placed in a flask, and stirred at 80°C for 5 h under Ar atmosphere. The mixture was filtered through celite and extracted with ethyl acetate, and the organic phase was retained. The organic phase was subjected to column chromatography to give the final product XCH-226, which was the **compound of Example 87.**

(62) The synthetic route for preparation of the compound of Example **88** was as follows:

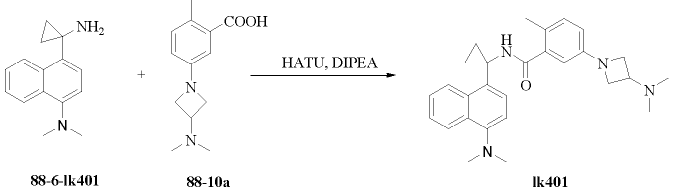

86 mg of amine intermediate **88-6-lk401** (0.38 mmol, 1.0 eq), 106 mg of carboxylic acid intermediate **88-10a** (0.45 mmol, 1.0 eq), and 467 µL of DIPEA (1.9 mmol, 5.0 eq) were weighed, dissolved in 3 mL of THF, and stirred until the mixture was completely dissolved. 216 mg of HATU (0.57 mmol, 1.5 eq) was added, and the mixture was reacted at room temperature for 4 h. After the reaction starting materials were completely converted, THF was removed by rotary evaporation, and the reaction solution was then washed with water and ethyl acetate. The organic phase was retained and concentrated to dryness by rotary evaporation to give a crude product, which was purified by column chromatography to give the final product **lk401,** i.e., the **compound of Example 88.**

(63) The synthetic route for preparation of the compound of Example 89 was as follows:

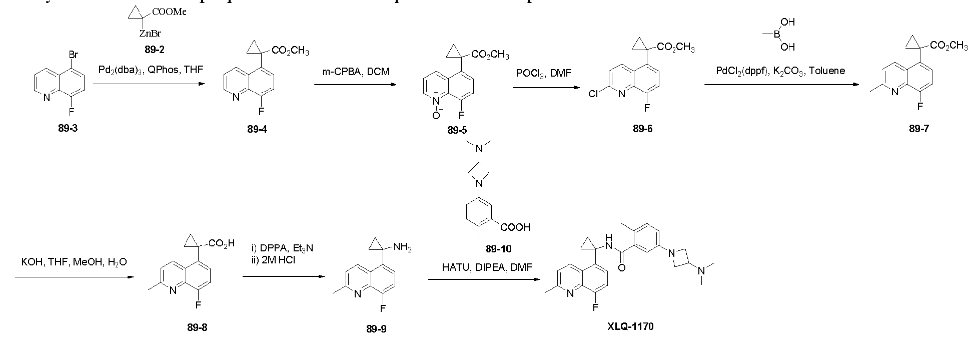

5-bromo-8-fluoroquinoline **89-3** (40 mmol, 1.0 e.q.), Pd₂(dba)₃ (0.4 mmol, 0.01 e.q.), and 366.3 mg of QPhos (0.4 mmol, 0.01 e.q.) were placed in a round bottom flask and dissolved in 80 mL of anhydrous tetrahydrofuran. Intermediate **2** Reformatsky Reagent (80 mmol, 1 N, 2.0 e.q.) dissolved in 20 mL of tetrahydrofuran was added under argon atmosphere. The mixture was stirred at room temperature for 30 min. After the reaction was completely converted as detected, the solvent was removed by rotary evaporation, and the mixture was dissolved in ethyl acetate, washed three times with water and once with saturated brine. The organic phase was retained and dried over anhydrous sodium sulfate. The organic phase was purified by column chromatography to give product intermediate **89-4.**

The intermediate **89-4** (10 mmol, 1.0 e.q.) obtained previously and m-CPBA (12 mmol, 1.2 e.q.) were placed in a round bottom flask and dissolved with 30 mL of DCM. The mixture was stirred at room temperature for 12 h. After the reaction was completely converted as detected, triphenylphosphine (5 mmol, 0.5 e.q.) was added, and the mixture was then stirred at room temperature for 4 h. The solvent was removed by rotary evaporation. The mixture was purified by column chromatography to give intermediate **89-5.**

The intermediate **89-5** (3 mmol, 1.0 e.q.) was placed in a round bottom flask and dissolved with 30 mL of DCM. Phosphorus oxychloride (3.6 mmol, 1.2 e.q.) was added dropwise with stirring under an ice bath, and DMF (1.5 mmol, 0.5 e.q.) was then added dropwise. The mixture was stirred at room temperature for 12 h. After the reaction was completely converted as detected, a saturated sodium bicarbonate solution was added dropwise under an ice bath to adjust the pH of the solution to 8, and the mixture was extracted with ethyl acetate. The organic phase was washed with water for 2 times, washed with saturated brine for 1 time, collected, and concentrated to dryness by rotary evaporation to give intermediate **89-6** without purification. The intermediate **89-6** (3 mmol, 1.0 e.q.), methylboronic acid (6.6 mmol, 2.2 e.q.), PdCl₂(dppf) (0.3 mmol, 0.1 e.q.), and K₂CO₃ (9 mmol, 3.0 e.q.) were placed in a round bottom flask and dissolved in 10 mL of toluene. The mixture was heated at 85°C and stirred for 12 h. After the reaction was completely converted as detected, the solvent was removed by rotary evaporation, and the mixture was dissolved in ethyl acetate, washed three times with water and once with saturated brine. The organic phase was retained and dried over anhydrous sodium sulfate. The organic phase was purified by column chromatography to give product intermediate **89-7.**

The intermediate **89-7** (3 mmol, 1.0 e.q.) was dissolved in a mixed solvent of tetrahydrofuran/methanol/water (3:1:1, 50 mL), and potassium hydroxide (12 mmol, 4.0 e.q.) was added. The mixture was reacted at 50°C for 8 h. After the reactant was completely converted as detected, a 2 N HCl solution was added to adjust the pH to 3. After all solvents were removed by rotary evaporation, 25 mL of methanol was added. The mixture was filtered, the filtrate was concentrated to dryness by rotary evaporation to give a light yellow solid, and finally the light-yellow solid was washed repeatedly with DCM/PE to give a white solid product **89-8.**

The intermediate **89-8** (2 mmol, 1.0 e.q.) was weighed and dissolved in 25 mL of extra dry toluene, triethylamine (4.4 mmol, 2.2 e.q.) was added, and DPPA (2.4 mmol, 1.2 e.q.) was added under argon atmosphere. The mixture was stirred at room temperature for 30 min until all carboxylic acid materials were converted into acyl azide, and heated to 75°C and reacted for 4 h until most acyl azide was converted into isocyanate. Excess hydrochloric acid (2 M aqueous solution, > 4.0 e.q.) was added, and the mixture was cooled to 60°C and reacted overnight. After the pH was adjusted to alkalinity with a sodium bicarbonate solution, the mixture was extracted with ethyl acetate, and the organic phase was collected, concentrated to dryness by rotary evaporation, and separated by silica gel column chromatography to give intermediate **89-9.**

The intermediate **89-9** and the previously obtained carboxylic acid intermediate **89-10** were added to a DMF solvent according to an equivalent ratio of 1:1. HATU (1.5 e.q.) and DIPEA (2.0 e.q.) were added, and the mixture was reacted at 50°C for 12 h. The reaction solution was extracted with ethyl acetate and washed with a saturated ammonium chloride solution for three times. The organic phase was concentrated to dryness by rotary evaporation and purified by silica gel column chromatography to give the final product **XLQ-1170, which was the compound of Example 89.**

(64) The synthetic route for preparation of the compound of Example **90** was as follows:

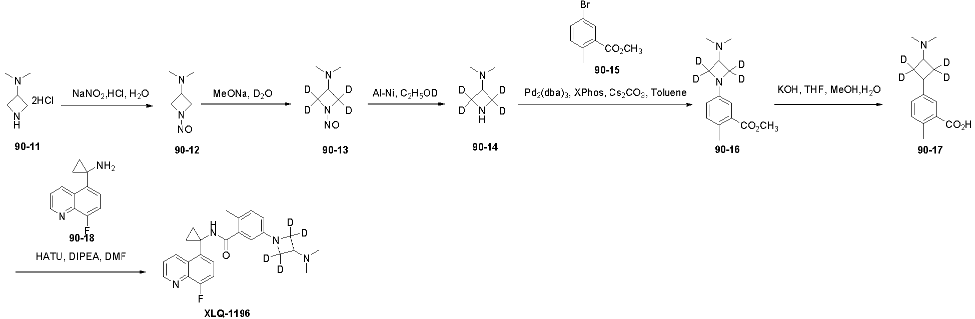

3-(dimethylamino)azetidine dihydrochloride **90-11** (6 mmol, 1.0 e.q.) was placed in a round bottom flask, dissolved in 2 mL of 2 M hydrochloric acid, and stirred at room temperature. Sodium nitrite (7.2 mmol, 1.2 e.q.) was dissolved in 1 mL of water and added dropwise to the reaction solution. The mixture was stirred at room temperature for 1.5 h. After the reaction was completely converted as detected, the mixture was extracted with ethyl acetate for 3 times and washed once with saturated brine. The organic phase was retained and dried over anhydrous sodium sulfate. The organic phase was purified by column chromatography to give product intermediate **90-12.**

The intermediate **90-12** (3 mmol, 1.0 e.q.) and sodium methoxide (3 mmol, 3.0 e.q.) were placed in a round bottom flask, and 1.5 mL of heavy water was slowly added dropwise under argon atmosphere. The mixture was heated at 80°C and stirred for 10 h. After the reaction was completely converted as detected, the mixture was extracted with ethyl acetate for 3 times and washed once with saturated brine. The organic phase was retained and dried over anhydrous sodium sulfate. The organic phase was purified by column chromatography to give product intermediate **90-13.**

The intermediate **90-13** (2.59 mmol, 1.0 e.q.) and sodium methoxide (7.77 mmol, 3.0 e.q.) were placed in a round bottom flask, and 2 mL of heavy water and 2 mL of deuterated ethanol (C₂H₅OD) were slowly added dropwise under argon atmosphere. The mixture was then heated to 70°C. After 24 h of reaction, the heating was stopped. After the mixture was cooled to room temperature, Al-Ni alloy (810 mg) was added in portions, and the mixture was stirred at room temperature overnight. The mixture was filtered under vacuum to remove solid metal. The filtrate was collected, extracted with ethyl acetate, and washed once with saturated brine. The organic phase was retained, dried over anhydrous sodium sulfate, filtered to remove the drying agent, and acidified with a solution of 4 M HCl in dioxane. The organic solvent was removed by rotary evaporation to give a solid product **90-14.**

The intermediate **90-14** (3.84 mmol, 1.1 e.q.), methyl 2-methyl-5-bromobenzoate **90-15** (3.49 mmol, 1.0 e.q.), Pd₂(dba)₃ (0.035 mmol, 0.01 e.q.), XPhos (0.14 mmol, 0.04 e.q.), and cesium carbonate (13.96 mmol, 4.0 e.q.) were dissolved in 20 mL toluene, placed in a sealed tube, and heated to 110°C and reacted overnight under Ar atmosphere. The mixture was washed with water and ethyl acetate, and the organic phase was retained. The organic phase was subjected to column chromatography to give product intermediate **90-16.**

Intermediate **90-16** (0.44 mmol, 1 e.q.) was weighed and dissolved in a mixed solution of 1.1 mL methanol and 1.1 mL water, potassium hydroxide (1.76 mmol, 4 e.q.) was added, and the mixture was heated to 60°C and stirred overnight. After the reaction was completed, excess hydrochloric acid was added to adjust the pH of the reaction solution to acidity (do not over-acidify, as the product has the risk of ring opening). The solvent was completely removed by rotary evaporation, and the mixture was dissolved with ethyl acetate. The organic phase was dried over anhydrous sodium sulfate and purified by column chromatography to give the product intermediate **90-17.**

The intermediate **90-17** and the previously obtained cyclopropylamine intermediate **90-18** were added to a DMF solvent according to an equivalent ratio of 1:1. HATU (1.5 e.q.) and DIPEA (2.0 e.q.) were added, and the mixture was reacted at 50°C for 12 h. The reaction solution was extracted with ethyl acetate and washed with a saturated ammonium chloride solution for three times. The organic phase was concentrated to dryness by rotary evaporation and purified by silica gel column chromatography to give the final product XLQ-1196, which was the **compound of Example 90.**

(65) The synthetic route for preparation of the compound of Example **91** was as follows:

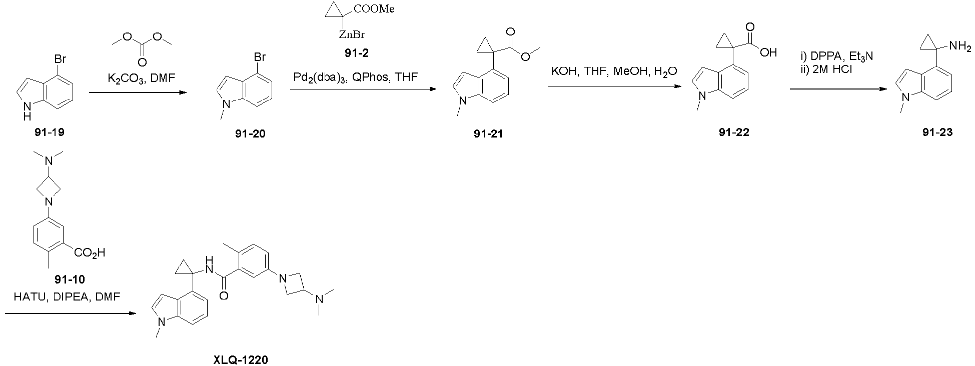

4-bromoindole **91-19** (10 mmol, 1.0 e.q.), dimethyl carbonate (29 mmol, 2.9 e.q.), and potassium carbonate (7 mmol, 0.7 e.q.) were placed in a round bottom flask and dissolved with 13 mL of DMF. The mixture was heated at 140°C and stirred for 4 h. After the reaction was completely converted as detected, the solvent was removed by rotary evaporation, and the mixture was dissolved in ethyl acetate, washed three times with water and once with saturated brine. The organic phase was retained and dried over anhydrous sodium sulfate. The organic phase was purified by column chromatography to give product intermediate **91-20.**

The intermediate **91-20** (2 mmol, 1.0 e.q.), Pd₂(dba)₃ (0.02 mmol, 0.01 e.q.), and 14.1 mg of QPhos (0.02 mmol, 0.01 e.q.) were placed in a round bottom flask and dissolved in 2 mL of anhydrous tetrahydrofuran. Intermediate **91-2** Reformatsky Reagent (4 mmol, 1 N, 2.0 e.q.) dissolved in 6 mL of tetrahydrofuran was added under argon atmosphere. The mixture was stirred at room temperature for 30 min. After the reaction was completely converted as detected, the solvent was removed by rotary evaporation, and the mixture was dissolved in ethyl acetate, washed three times with water and once with saturated brine. The organic phase was retained and dried over anhydrous sodium sulfate. The organic phase was purified by column chromatography to give product intermediate **91-21.**

The intermediate **91-21** (1.88 mmol, 1.0 e.q.) was dissolved in a mixed solvent of tetrahydrofuran/methanol/water (3:1:1, 2.16 mL), and potassium hydroxide (7.52 mmol, 4.0 e.q.) was added. The mixture was reacted at 50°C for 8 h. After the reactant was completely converted as detected, a 2 N HCl solution was added to adjust the pH to 3. After all solvents were removed by rotary evaporation, 15 mL of methanol was added. The mixture was filtered, the filtrate was concentrated to dryness by rotary evaporation to give a light yellow solid, and finally the light-yellow solid was washed repeatedly with DCM/PE to give a white solid product **91-22.**

The intermediate **91-22** (1.49 mmol, 1.0 e.q.) was weighed and dissolved in 6.4 mL of extra dry toluene, triethylamine (3.27 mmol, 2.2 e.q.) was added, and DPPA (1.64 mmol, 1.2 e.q.) was added under argon atmosphere. The mixture was stirred at room temperature for 30 min until all carboxylic acid materials were converted into acyl azide, and heated to 75°C and reacted for 8 h until most acyl azide was converted into isocyanate. Excess hydrochloric acid (2 M aqueous solution, > 4.0 e.q.) was added, and the mixture was cooled to 60°C and reacted overnight. After the pH was adjusted to alkalinity with a sodium bicarbonate solution, the mixture was extracted with ethyl acetate, and the organic phase was collected, concentrated to dryness by rotary evaporation, and separated by silica gel column chromatography to give intermediate **91-23.**

The intermediate **91-23** and the previously obtained carboxylic acid intermediate **10** were added to DMF as a solvent according to an equivalent ratio of 1:1. HATU (1.5 e.q.) and DIPEA (2.0 e.q.) were added, and the mixture was reacted at 50°C for 12 h. The reaction solution was extracted with ethyl acetate and washed with a saturated ammonium chloride solution for three times. The organic phase was concentrated to dryness by rotary evaporation and purified by silica gel column chromatography to give the final product XLQ-1220, which was the **compound of Example 91.**

Experimental Example 1: Assay of the Compounds Prepared in the above Examples for PLpro Inhibitory Activity Bioassay condition:
1. Reaction buffer: 20 mM HEPEs, pH 7.5, 100 mM NaCl, 1 mM TCEP
2. Preparation of mother liquor:
   (1) 20 µM Ub-AMC (Ub-AMC dry powder was directly dissolved with the reaction buffer and used after centrifugation for removing precipitates);
   (2) 400 nM PLpro (after molecular sieve purification, frozen at -80°C, thawed on ice before use, diluted with the reaction buffer);
   (3) 40 µM test compound (the test compound dry powder was dissolved with DMSO to 40 mM, diluted with 50% DMSO to 400 µM, and diluted with the reaction buffer to 40 µM);
3. For the reaction system of a single point inhibition test: 10 µM Ub-AMC, 100 nM PLpro, 1 µM test compound, total volume 20 µL, reacted in a 384-well plate;
   5 µL PLpro mother liquor + 5 µL test compound mother liquor were added to a 384-well plate and incubated at 4°C for 30 min;
   10 µL Ub-AMC mother liquor was added to the 384-well plate, and after the mixture was reacted at 37°C for 30 min, the fluorescence intensity of AMC was measured (excitation: 360 nm; emission: 460 nm);
4. Control group (+Control): DMSO at corresponding dilution factors replaced the test compound;
   blank group (Blank): Reaction buffer replaced PLpro;
5. Data processing: The Blank value was subtracted from the measured value, and normalization was performed on the basis of the DMSO value;
6. IC₅₀ determination:
   Test compound concentration gradient (nM): 10000, 5000, 1000, 500, 250, 125, 62.5, 31.25, 15.625, 10, 5, 2, 1, 0.5, 0.1, and 0.01
   The fluorescence value was measured after 15 min of reaction (the enzyme reaction rate was in a linear interval around 15 min and was in a non-linear interval at 30 min);
7. Data fitting: The data was normalized and processed using Sigmaplot (fitting equation: Logistic, 3 Parameter).

The results are shown in the table below.

**Table 1. Experimental results**

| **No.** | **Structure** | **inhibition of PLpro** (1 **µM)** | **IC50 for PLpro (nM)** |
|---|---|---|---|
| **C1** | 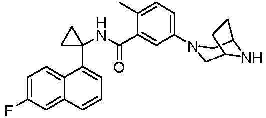 | 97.7% | 82.97±6.63 |
| **C2** | 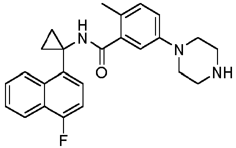 | 96.4% | 98.01±12.13 |
| **C3** | 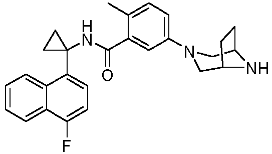 | 96.2% | 40.14±3.01 |
| **C4** | 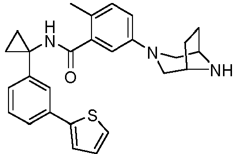 | 73.4% | - |
| **C5** | 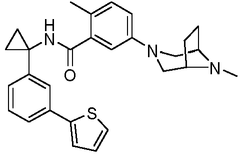 | 79.5% | 103.59±6.02 |
| **C6** | 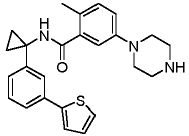 | 56.5% | |
| **C7** | 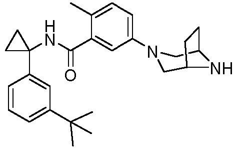 | 15% | |
| **C8** | 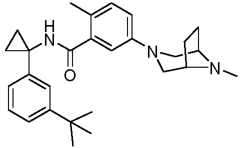 | 37.2% | |
| **C9** | 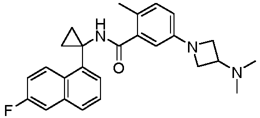 | 98.0% | 52.11±4.18 |
| **C10** | 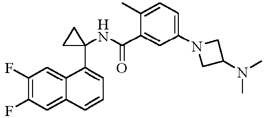 | 98.2% | 12.98±2.76 |
| **C11** | 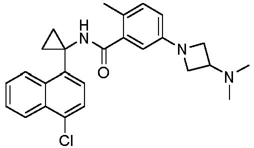 | 96.8% | 83.14±12.67 |
| **C12** | 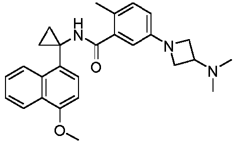 | 97.8% | 18.65±2.30 |
| **C13** | 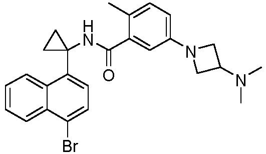 | 98.5% | 79.77±6.04 |
| **C14** | 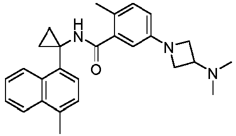 | 95.1% | 205.03±13.64 |
| **C15** | 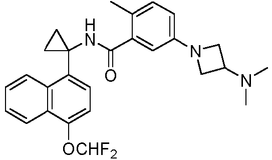 | 98.0% | |
| **C16** | 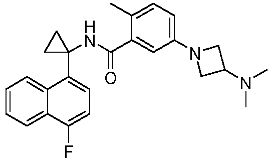 | 89.4% | 56.39±8.48 |
| **C17** | 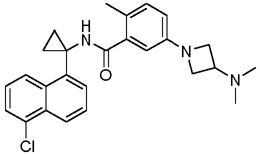 | 98.1% | 18.74±5.29 |
| **C18** | 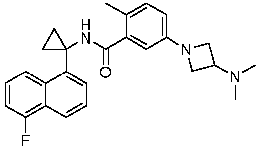 | 97.6% | 17.81±1.94 |
| **C19** | 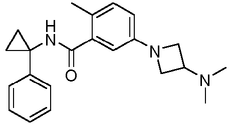 | 4.9% | |
| **C20** | 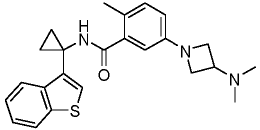 | 91.9% | 76.47±9.48 |
| **C21** | 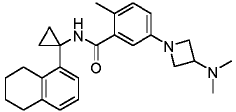 | 90.4% | 91.14±8.22 |
| **C22** | 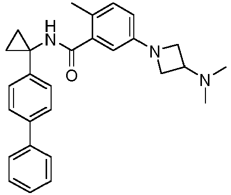 | 0% | |
| **C23** | 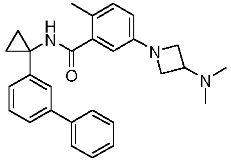 | 5.8% | |
| **C24** | 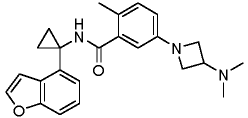 | 82.3% | 423.11±61.36 |
| **C25** | 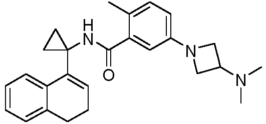 | 55% | |
| **C26** | 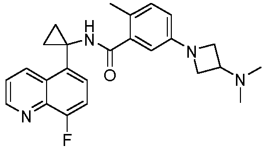 | 95.9% | 38.33±5.27 |
| **C27** | 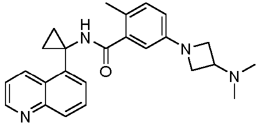 | 88.3% | 97.63 |
| **C28** | 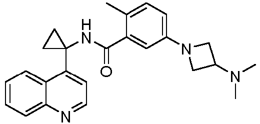 | 89.7% | 129.39 |
| **C29** | 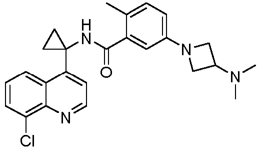 | 92.9% | 44.85 |
| **C30** | 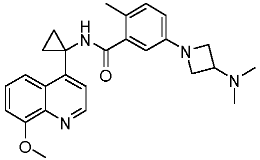 | 91.5% | 67.38 |
| **C31** | 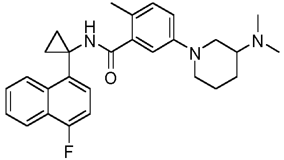 | 49.2% | |
| **C32** | 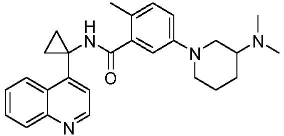 | 43.9% | |
| **C33** | 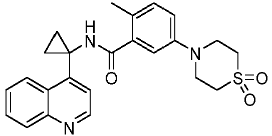 | 41.0% | |
| **C34** | 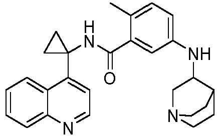 | 31.1% | |
| **C35** | 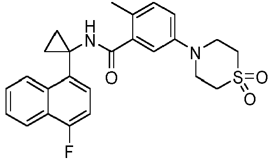 | 2.6% | |
| **C36** | 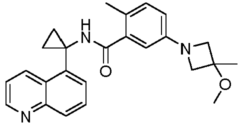 | 3.5% | |
| **C37** | 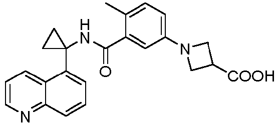 | 12.6% | |
| **C38** | 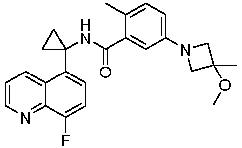 | 11.7% | |
| **C39** | 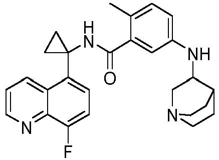 | 40.8% | |
| **C40** | 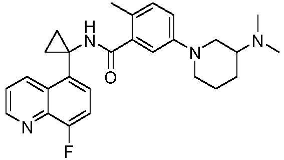 | 55.2% | |
| **C41** | 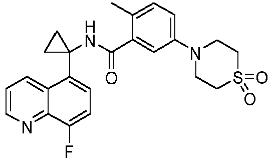 | 32.8% | |
| **C42** | 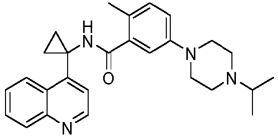 | 85.2% | |
| **C43** | 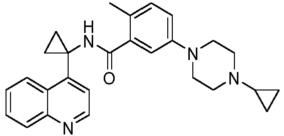 | 71.9% | |
| **C44** | 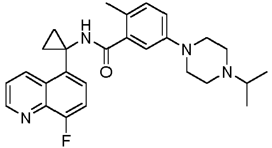 | 84.9% | |
| **C45** | 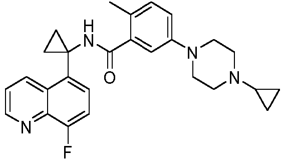 | 81.4% | |
| **C46** | 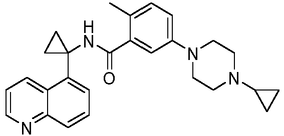 | 77.6% | |
| **C47** | 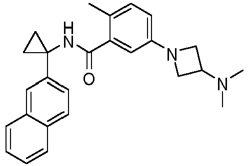 | 58.0% | |
| **C48** | 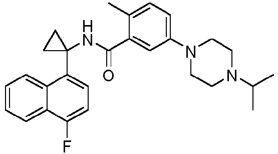 | 84.4% | |
| **C49** | 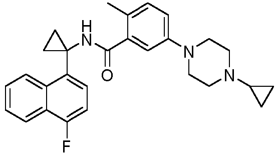 | 58.4% | |
| **C50** | 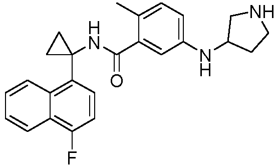 | 27.8% | |
| **C51** | 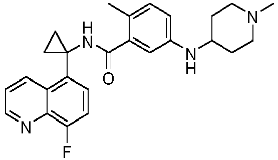 | 38.4% | |
| **C52** | 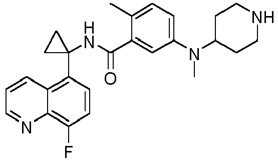 | - | |
| **C53** | 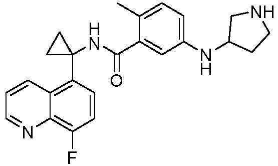 | 44.9% | |
| **C54** | 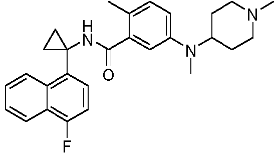 | - | |
| **C55** | 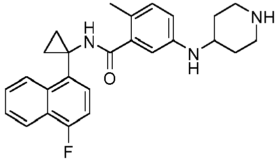 | - | |
| **C56** | 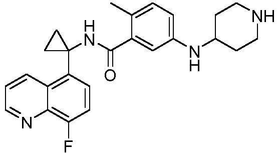 | 24.5% | |
| **C57** | 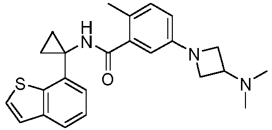 | **92%** | |
| **C58** | 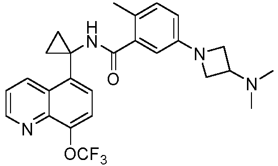 | **88%** | **220.13** |
| **C59** | 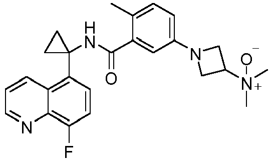 | **0%** | |
| **C60** | 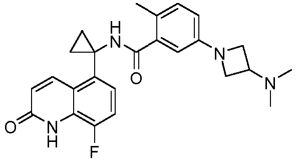 | **82%** | |
| **C61** | 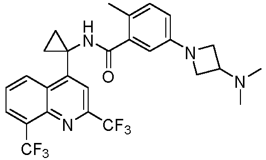 | **85%** | |
| **C62** | 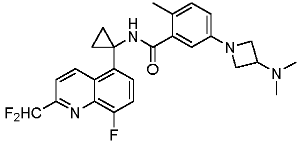 | **92%** | |
| **C63** | 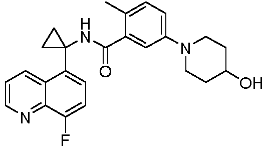 | **30%** | |
| **C64** | 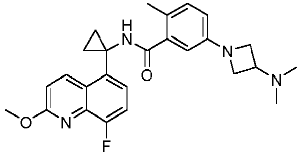 | **78%** | **50.56** |
| **C65** | 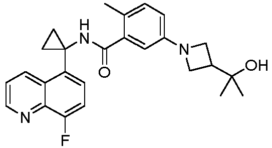 | **22%** | |
| **C66** | 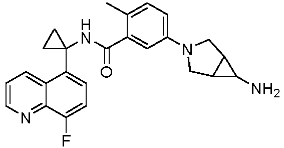 | **68%** | |
| **C67** | 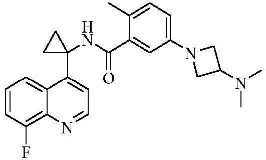 | 97.7% | 36.65 |
| **C68** | 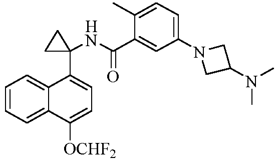 | 98.5% | 181.5 |
| **C69** | 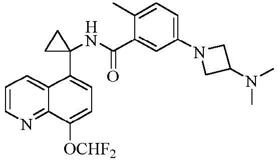 | 98.8% | 29.29 |
| **C70** | 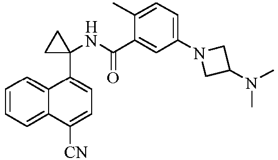 | 99.2% | 40.62 |
| **C71** | 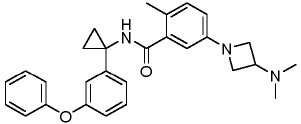 | 25.0% | |
| C72 | 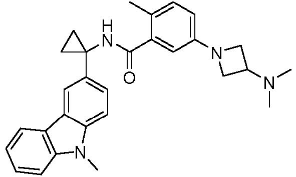 | 91.0% | |
| C73 | 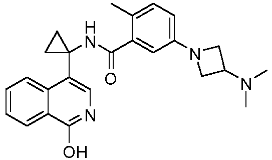 | 89.3% | 172.46±18.11 |
| C74 | 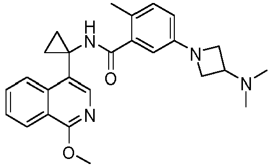 | 88.6% | 216.56±22.20 |
| C75 | 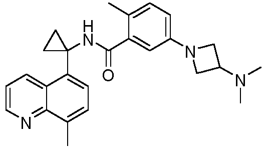 | 99.4% | 4.71±1.38 |
| C76 | 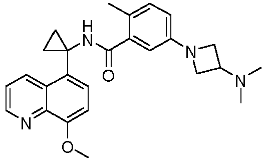 | 99.6% | 15.89±3.25 |
| C77 | 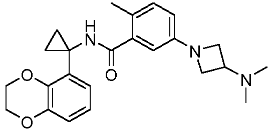 | 63.0% | |
| **C78** | 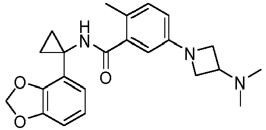 | 25.4% | |
| **C79** | 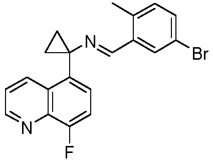 | 16.5% | |
| **C80** | 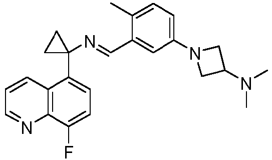 | 11.9% | |
| **C81** | 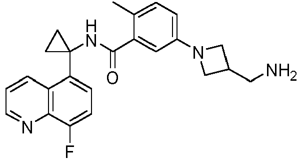 | 37.1% | |
| **C82** | 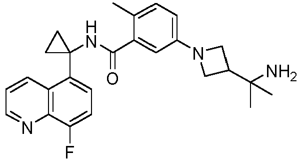 | 8.2% | |
| **C83** | 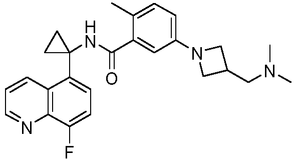 | 24.2% | |
| **C84** | 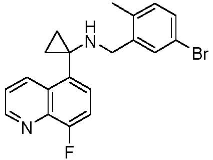 | - | |
| **C85** | 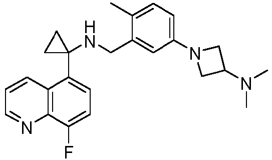 | 45.7% | |
| **C86** | 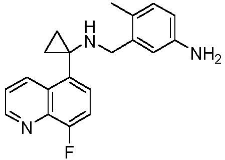 | 33.5% | |
| **C87** | 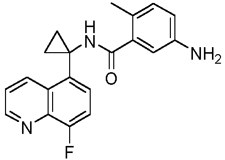 | 23.1% | |
| **C88** | 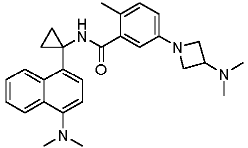 | 96.2% | 174.92 |
| **C89** | 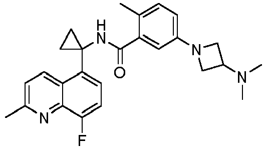 | 82% | 118.80 |
| **C90** | 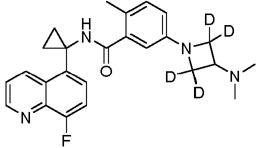 | **85%** | **69.83** |
| **C91** | 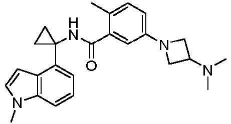 | 71% | - |
| **GRL0617** | 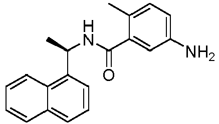 | 42.32% | 1210±280 |

| | | | |
|---|---|---|---|
| GRL0617 is a positive reference (Ghosh et al., 2009; Ghosh et al., 2010; Ratia et al., 2008). "-" represents undetermined. | | | |

Live virus assay for inhibition of SARS-CoV-2 infection on cells by the compound of Example 26:
To verify the anti-SARS-CoV-2 activity of the compound of Example 26, the anti-SARS-CoV-2 activity of the molecule of Example 26 was detected using a Calu-3 cell infection model of SARS-CoV-2 live virus.

Serially diluted Example 26 was mixed with an equal volume of 100TCID50 SARS-CoV-2, and added to a culture plate containing 1 × 10⁴/well Calu-3 cells. The cell culture plate was supplemented with 100 µL of DMEM + 2% FBS medium per well, placed in a cell culture incubator, and cultured for 48 h. The cell supernatant was collected.

For viral RNA extraction and quantitative real-time PCR (qRT-PCR), viral RNA in the cell supernatant was extracted using TRIzol LS reagent (Invitrogen) according to the manufacturer's instructions. Detection was performed using One-Step PrimeScrip RT-PCR Kit (Takara, Japan, Cat.#RR064A) according to the manufacturer's instructions. The RT-PCR program was: Reverse transcription: 95°C 10 s, 42°C 5 min; PCR reaction: (95°C 5 s, 56°C 30 s, 72°C 30 s)*40 cycles. Detection was performed with a BioRad quantitative fluorescence PCR instrument. The primer sequences were: SARS-CoV-2-N-F (SEQ ID NO: 1): GGGGAACTTCTCCTGCTAGAAT, SARS-CoV-2-N-R (SEQ ID NO: 2): CAGACATTTTG CTCTCAAGCTG, and SARS-CoV-2-N-probe (5'-FAM-SEQ ID NO: 3-TAMRA-3'): 5'-FAM- TTGCTGCTGCTTGACAGATT-TAMRA-3'. 48 h after infection, the cell supernatant was collected and evaluated by RT-qPCR for the quantitative viral RNA copy number to calculate the *in vitro* inhibitory potency of the test drug against SARS-CoV-2 (EC₅₀ and EC₉₀ values were determined). The inhibition rate of the compound of Example 26 against SARS-CoV-2 live virus (Delta strain) infection is as follows:

**Table 2**

| Concentration (nM) | Inhibition rate/% | | |
|---|---|---|---|
| 400 | 99.9639 | 99.9327 | 99.8544 |
| 200 | 99.8676 | 99.5887 | 99.6848 |
| 100 | 92.1557 | 93.9064 | 96.0939 |
| 50 | 92.4972 | 64.3219 | 67.0889 |
| 25 | -64.656 | 17.2863 | 31.5856 |
| 12.5 | -4.0599 | 50.6792 | 17.0436 |
| 6.25 | 37.9405 | 58.6715 | 67.2927 |
| 3.125 | 2.07603 | 45.3104 | -0.0016 |
| EC50 | 36.27± 4.11 nM | | |
| EC90 | 73.13 ± 7.23 nM | | |

The experiment proves that the compound of Example 26 can effectively inhibit SARS-CoV-2 live virus from infecting human cells in the *in vitro* experiment.

## Claims

1. A compound, or a pharmaceutically acceptable salt, stereoisomer, ester, prodrug, solvate and deuterated compound thereof, the compound having the following structure: 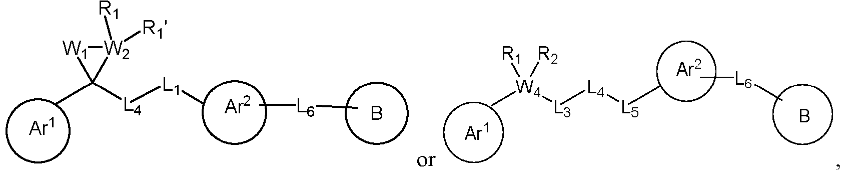 wherein
Ar¹ is substituted naphthyl, or substituted or unsubstituted non-naphthyl aryl;
Ar² is aryl or heteroaryl;
B is selected from: heterocyclyl, -S(O)ₜNR₁₅, halogen, and -NH₂;
W₁ is selected from: 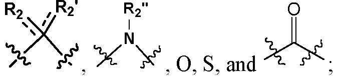
W₂ is selected from: C, N, and O; when W₂ is N, R₁^{'} is absent; when W₂ is O, R₁ and R₁' are absent;
W₄ is absent or selected from: C or S; when W₄ is absent, R₁ and R₂ are absent;
R₁, R₁^{'}, R₂, and R₂^{'} are independently selected from: H, D, (=O), -C₁-C₆ alkyl, -X, -CH₂X, -CHX₂, -CX₃, -OH, -NH₂, -COOH, and -O(C₁-C₆ alkyl);
R₂^{"} is selected from: H, C₁-C₆ alkyl, -OH, -(C₁-C₆ alkylene)-COOR₂₁, -(C₁-C₆ alkylene)-OR₂₁, and -(C₁-C₆ alkylene)-CONR₂₁R₂₂;
R₃ is selected from: H or C₁-C₆ alkyl;
L₁ is absent or selected from: C₁-C₆ alkylene, -CO-, -SO₂-, 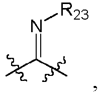 or -N(R₃)-;
L₃ and L₅ are absent or independently selected from: alkylene, heteroalkylene, cycloalkylene, heterocyclylene, and carbonyl, which can be optionally substituted;
L₄ is selected from: C₁-C₆ alkylene, -SO₂-, -NR₁₅C(O)-, -NR₁₅S(O)ₜ-, -C(O)-, -C(O)O-, -NR₁₅-, -C(O)NR₁₅-, -S(O)ₜNR₁₅-, 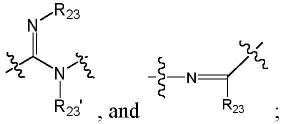
L₆ is absent or selected from: C₁-C₆ alkylene, -SO₂-, -NR₁₅C(O)-, -NR₁₅S(O)ₜ-, -C(O)-, -C(O)O-, -NR₁₅-, -C(O)NR₁₅-, - S(O)ₜNR₁₅-, and 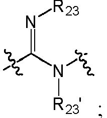
R₁₅ is selected from: H, D, alkyl, cycloalkyl, cycloalkylalkyl, aryl, aralkyl, heterocyclyl, heterocyclylalkyl, hydroxy, and alkoxy; alternatively, R₁₅, together with the nitrogen atom linked thereto and L₃ or L₅, forms heterocyclyl, which can be optionally substituted;
t is 1 or 2;
R₂₁ is H or C₁-C₆ alkyl;
R₂₂ is H or C₁-C₆ alkyl;
R₂₃ or R_{23'} is selected from H or C₁-C₆ alkyl; and
X is selected from F, Cl, Br, and I.

2. The compound according to claim 1, wherein the substituted naphthyl is selected from: 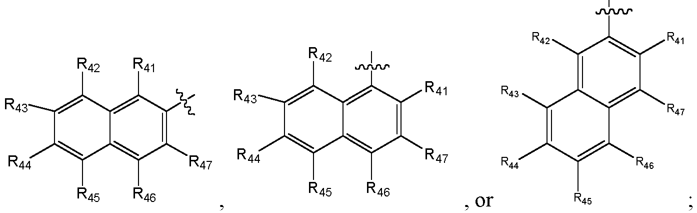
R₄₁, R₄₂, R₄₃, R₄₄, R₄₅, R₄₆, and R₄₇ represent substituents on the ring and are independently selected from: H, D, C₁-C₆ alkyl, C₁-C₆ haloalkyl, C₃-C₆ cycloalkyl, aryl, aralkyl, heterocyclyl, heterocyclylalkyl, halogen, -Si(C₁-C₆ alkyl)₃, -NO₂, -R_{L}-COR', -R_{L}-C(O)OR', -R_{L}-C(O)NR'R", -R_{L}-CH=NR', -R_{L}-CN, -R_{L}-OR', -R_{L}-OC(O)R', -R_{L}-S(O)ₜ-NR'R", -R_{L}-S(O)ₜ-R', -R_{L}-NR'R", - R_{L}-NR'C(O)R", -R_{L}-NR'S(O)ₜR", -NR'-R_{L}-NR'R", -R_{L}-NO₂, and -R_{L}-N=CR'R", which can be optionally substituted, and R₄₁, R₄₂, R₄₃, R₄₄, R₄₅, R₄₆, and R₄₇ are not simultaneously H;
t is 1 or 2;
R_{L} is absent or selected from: C₁-C₆ alkylene, C₃-C₆ heteroalkylene, C₃-C₆ cycloalkylene, C₃-C₆ heterocyclylene, -NR₄C(O)-, -NR₄S(O)ₜ-, -C(O)-, -C(O)O-, -NR₄-, -C(O)NR₄-, and -S(O)ₜNR₄-, which can be optionally substituted;
R' and R" are independently selected from: H, D, C₁-C₆ alkyl, C₁-C₆ haloalkyl, C₃-C₆ cycloalkyl, cycloalkylalkyl, aryl, aralkyl, heterocyclyl, heterocyclylalkyl, and halogen, which can be optionally substituted;
R₄ is selected from: H, D, alkyl, cycloalkyl, cycloalkylalkyl, aryl, aralkyl, heterocyclyl, heterocyclylalkyl, hydroxy, and alkoxy;
preferably, R₄₁, R₄₂, R₄₃, R₄₄, R₄₅, R₄₆, and R₄₇ are independently selected from: H, -D, -CH₃, -X, -CH₂F, -CHF₂, -CF₃, -OH, -CN, -OCH₃, -OCH₂X, -OCHX₂, -OCX₃, -NH₂, -NH(C₁-C₆ alkyl), -N(C₁-C₆ alkyl)(C₁-C₆ alkyl), -NO₂, -COO(C₁-C₆ alkyl), -COOH, -CN, -Si(CH₃)₃, -NHSO₂(C₁-C₆ alkyl), -SO₂NH₂, -SO₂(C₁-C₆ alkyl), -N(C₁-C₆ alkyl)SO₂(C₁-C₆ alkyl), -SO₂NH(C₁-C₆ alkyl), and -SO₂N(C₁-C₆ alkyl)(C₁-C₆ alkyl), and R₄₁, R₄₂, R₄₃, R₄₄, R₄₅, R₄₆, and R₄₇ are not simultaneously H;
more preferably, the substituted naphthyl is selected from:

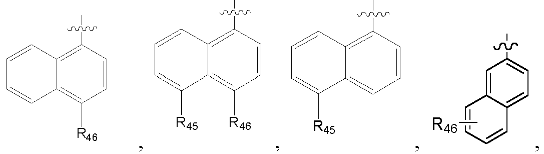

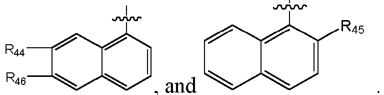

3. The compound according to claim 1, wherein the non-naphthyl aryl is selected from: phenyl, substituted phenyl, 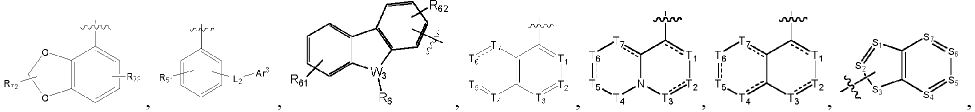 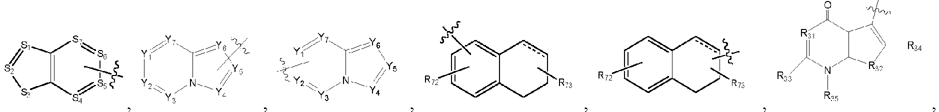 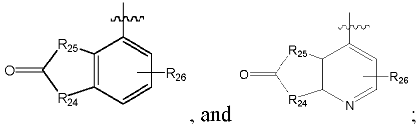
L₂ is absent or selected from: -O-, C₁-C₆ alkylene, -CO-, -CONR₅₃-, -NR₅₃-, -NR₅₃CO-, -(C₁-C₆ alkylene)-O-, -(C₁-C₆ alkylene)-CO-, -(C₁-C₆ alkylene)-CONR₅₃-, -(C₁-C₆ alkylene)-NR₅₃-, and -(C₁-C₆ alkylene)-NR₅₃CO-;
R₅₃ is selected from H, D, or C₁-C₆ alkyl;
R₅₁ is selected from: H, D, C₁-C₆ alkyl, C₁-C₆ haloalkyl, C₃-C₆ cycloalkyl, aryl, aralkyl, heterocyclyl, heterocyclylalkyl, halogen, -Si(C₁-C₆ alkyl), -NO₂-R_{L}-COR', -R_{L}-C(O)OR', -R_{L}-C(O)NR'R", -R_{L}-CH=NR', -R_{L}-CN, -R_{L}-OR', -R_{L}-OC(O)R', - R_{L}-S(O)ₜ-NR'R", -R_{L}-S(O)ₜ-R', -R_{L}-NR'R", -R_{L}-NR'C(O)R", -R_{L}-NR'S(O)ₜR", -NR'-R_{L}-NR'R", -R_{L}-NO₂, and -R_{L}-N=CR'R", which can be optionally substituted;
t is 1 or 2;
preferably, R₅₁ is selected from: H, -D, -CH₃, -X, -CF₃, -OH, -OCH₃, -OCH₂X, -OCHX₂, -OCX₃, -NH₂, -NH(C₁-C₆ alkyl), - N(C₁-C₆ alkyl)(C₁-C₆ alkyl), -NO₂, -COO(C₁-C₆ alkyl), -COOH, -CN, -Si(CH₃)₃, -NHSO₂(C₁-C₆ alkyl), -SO₂NH₂, -SO₂(C₁-C₆ alkyl), -N(C₁-C₆ alkyl)SO₂(C₁-C₆ alkyl), -SO₂NH(C₁-C₆ alkyl), and -SO₂N(C₁-C₆ alkyl)(C₁-C₆ alkyl);
Ar³ is selected from substituted or unsubstituted phenyl, substituted or unsubstituted oxygen-containing five-membered or six-membered heterocyclyl, substituted or unsubstituted nitrogen-containing five-membered or six-membered heterocyclyl, and substituted or unsubstituted sulfur-containing five-membered or six-membered heterocyclyl;
preferably, Ar³ is selected from phenyl, C₁-C₆ alkyl-substituted phenyl, furyl, pyrrolyl, thienyl, pyridinyl, pyrimidinyl, thiazolyl, imidazolyl, and oxazolyl, which can be optionally substituted; more preferably, Ar³ is selected from phenyl, *tert-*butylphenyl, thienyl, and pyridinyl, which can be optionally substituted;
W₃ is selected from N or CH;
R₆ is selected from H, D, C₁-C₆ alkyl, -OH, -(C₁-C₆ alkylene)-COOR₆₁, -(C₁-C₆ alkylene)-OR₆₁, and -(C₁-C₆ alkylene)-CONR₆₁;
R₆₁ is H, D, or C₁-C₆ alkyl;
preferably, W₃ is N; R₆ is H, D, CH₃, -CH₂COOH, and -CH₂COOCH₃;
R₆₂ is selected from: H, -D, C₁-C₆ alkyl, C₁-C₆ haloalkyl, C₃-C₆ cycloalkyl, aryl, aralkyl, heterocyclyl, heterocyclylalkyl, halogen, -Si(C₁-C₆ alkyl), -N₃, -B(OH)₂, -NO₂-R_{L}-COR', -R_{L}-C(O)OR', -R_{L}-C(O)NR'R", -R_{L}-CH=NR', -R_{L}-CN, -R_{L}-OR', - R_{L}-OC(O)R', -R_{L}-S(O)_{t'}-NR'R", -R_{L}-S(O)_{t'}-R', -R_{L}-NR'R", -R_{L}-NR'C(O)R", -R_{L}-NR'S(O)_{t'}R", -NR'-R_{L}-NR'R", -R_{L}-NO₂, and -R_{L}-N=CR'R", which can be optionally substituted;
T₁, T₂, T₃, T₄, T₅, T₆, and T₇ are independently selected from O, C-R₇, 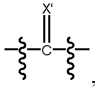 or N;
X' is N, O, and S;
when T₁-T₇ are selected from C-R₇, each R₇ can be independently selected from: H, -D, -CH₃, -X, -CF₃, -OH, -OCH₃, - OCH₂X, -OCHX₂, -OCX₃, -NH₂, -NH(C₁-C₆ alkyl), -N(C₁-C₆ alkyl)(C₁-C₆ alkyl), -NO₂, -COO(C₁-C₆ alkyl), -COOH, -CN, -Si(CH₃)₃, -NHSO₂(C₁-C₆ alkyl), -SO₂NH₂, -SO₂(C₁-C₆ alkyl), -N(C₁-C₆ alkyl)SO₂(C₁-C₆ alkyl), -SO₂NH(C₁-C₆ alkyl), - SO₂N(C₁-C₆ alkyl)(C₁-C₆ alkyl), -O(C₁-C₆ alkyl)NH₂, -O(C₁-C₆ alkyl)N(C₁-C₆ alkyl)(C₁-C₆ alkyl), -O(C₁-C₆ alkyl)NH(C₁-C₆ alkyl), and 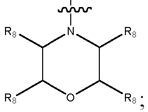
R₈ is selected from: H, -D, C₁-C₆ alkyl, -(C₁-C₆ alkylene)-COOR₆₁, -(C₁-C₆ alkylene)-OR₆₁, and -(C₁-C₆ alkylene)-CONR₆₁;
S₃ is selected from: O, S, NR₉₁, and CR₉₂R₉₃;
S₁, S₂, S₄, S₅, S₆, and S₇ are independently selected from: N and CR₉₄;
R₉₂, R₉₃, and R₉₄ are independently selected from: a connecting bond, H, -D, C₁-C₆ alkyl, C₁-C₆ haloalkyl, C₃-C₆ cycloalkyl, aryl, aralkyl, heterocyclyl, heterocyclylalkyl, halogen, -Si(C₁-C₆ alkyl), -NO₂-R_{L}-COR', -R_{L}-C(O)OR', -R_{L}-C(O)NR'R", -R_{L}-CH=NR', -R_{L}-CN, -R_{L}-OR', -R_{L}-OC(O)R', -R_{L}-S(O)ₜ-NR'R", -R_{L}-S(O)ₜ-R', -R_{L}-NR'R", -R_{L}-NR'C(O)R", -R_{L}-NR'S(O)ₜR", - NR'-R_{L}-NR'R", -R_{L}-NO₂, -R_{L}-N=CR'R", and 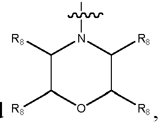 which can be optionally substituted;
preferably, R₉₂, R₉₃, and R₉₄ are independently selected from a connecting bond, H, -D, -CH₃, -F, -CF₃, -OH, -OCH₃, -NH₂, - NH(C₁-C₆ alkyl), -N(C₁-C₆ alkyl)(C₁-C₆ alkyl), -NO₂, -COO(C₁-C₆ alkyl), -COOH, -CN, -Si(CH₃)₃, -NHSO₂(C₁-C₆ alkyl), - SO₂NH₂, -SO₂(C₁-C₆ alkyl), -N(C₁-C₆ alkyl)SO₂(C₁-C₆ alkyl), -SO₂NH(C₁-C₆ alkyl), -SO₂N(C₁-C₆ alkyl)(C₁-C₆ alkyl), and 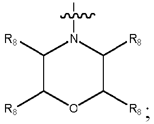
R₉₁ is selected from a connecting bond, H, -D, C₁-C₆ alkyl, -OH, -(C₁-C₆ alkylene)-COOR₆₁, -(C₁-C₆ alkylene)-OR₆₁, -(C₁-C₆ alkylene)-CONR₆₁, and 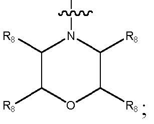
Y₁, Y₂, Y₃, Y₄, Y₅, Y₆, and Y₇ are independently selected from N or CR₁₁;
R₁₁ is selected from a connecting bond, H, D, C₁-C₆ alkyl, C₁-C₆ haloalkyl, C₃-C₆ cycloalkyl, aryl, aralkyl, heterocyclyl, heterocyclylalkyl, halogen, -Si(C₁-C₆ alkyl), -NO₂-R_{L}-COR', -R_{L}-C(O)OR', -R_{L}-C(O)NR'R", -R_{L}-CH=NR', -R_{L}-CN, -R_{L}-OR', -R_{L}-OC(O)R', -R_{L}-S(O)_{t'}-NR'R", -R_{L}-S(O)_{t'}-R', -R_{L}-NR'R", -R_{L}-NR'C(O)R", -R_{L}-NR'S(O)_{t'}R", -NR'-R_{L}-NR'R", -R_{L}-NO₂, and -R_{L}-N=CR'R", which can be optionally substituted;
preferably, R₁₁ is independently selected from a connecting bond, H, -D, -CH₃, -F, -CF₃, -OH, -OCH₃, -NH₂, -NH(C₁-C₆ alkyl), -N(C₁-C₆ alkyl)(C₁-C₆ alkyl), -NO₂, -COO(C₁-C₆ alkyl), -COOH, -CN, -Si(CH₃)₃, -NHSO₂(C₁-C₆ alkyl), -SO₂NH₂, - SO₂(C₁-C₆ alkyl), -N(C₁-C₆ alkyl)SO₂(C₁-C₆ alkyl), -SO₂NH(C₁-C₆ alkyl), and -SO₂N(C₁-C₆ alkyl)(C₁-C₆ alkyl);
R₇₂ and R₇₃ are independently selected from: H, -D, -CH₃, -X, -CF₃, -OH, -OCH₃, -NH₂, -NH(C₁-C₆ alkyl), -N(C₁-C₆ alkyl)(C₁-C₆ alkyl), -N₃, -B(OH)₂, -NO₂, -COO(C₁-C₆ alkyl), -COOH, -CN, -Si(CH₃)₃, -NHSO₂(C₁-C₆ alkyl), -SO₂NH₂, - SO₂(C₁-C₆ alkyl), -N(C₁-C₆ alkyl)SO₂(C₁-C₆ alkyl), -SO₂NH(C₁-C₆ alkyl), and -SO₂N(C₁-C₆ alkyl)(C₁-C₆ alkyl);
R₃₁ is N or CR₃₆; R₃₂ is NR₃₇ or -N=CR₃₈-;
R₃₅ or R₃₇ is independently selected from H, -D, C₁-C₆ alkyl, -OH, -(C₁-C₆ alkylene)-COOR₆₁, -(C₁-C₆ alkylene)-OR₆₁, and - (C₁-C₆ alkylene)-CONR₆₁;
R₃₃, R₃₄, R₃₆, and R₃₈ are independently selected from: H, -D, C₁-C₆ alkyl, C₁-C₆ haloalkyl, C₃-C₆ cycloalkyl, aryl, aralkyl, heterocyclyl, heterocyclylalkyl, halogen, -Si(C₁-C₆ alkyl)₃, -N₃, -B(OH)₂, -NO₂-R_{L}-COR', -R_{L}-C(O)OR', -R_{L}-C(O)NR'R", - R_{L}-CH=NR', -R_{L}-CN, -R_{L}-OR', -R_{L}-OC(O)R', -R_{L}-S(O)_{t'}-NR'R", -R_{L}-S(O)_{t'}-R', -R_{L}-NR'R", -R_{L}-NR'C(O)R", -R_{L}-NR'S(O)_{t'}R", -NR'-R_{L}-NR'R", -R_{L}-NO₂, and -R_{L}-N=CR'R", which can be optionally substituted;
preferably, R₃₃, R₃₄, R₃₆, and R₃₈ are independently selected from: H, -CH₃, -F, -CF₃, -OH, -OCH₃, -NH₂, -NH(C₁-C₆ alkyl), -N(C₁-C₆ alkyl)(C₁-C₆ alkyl), -NO₂, -COO(C₁-C₆ alkyl), -COOH, -CN, -Si(CH₃)₃, -NHSO₂(C₁-C₆ alkyl), -SO₂NH₂, -SO₂(C₁-C₆ alkyl), -N(C₁-C₆ alkyl)SO₂(C₁-C₆ alkyl), -SO₂NH(C₁-C₆ alkyl), and -SO₂N(C₁-C₆ alkyl)(C₁-C₆ alkyl);
R₂₄ is absent or selected from CR₂₃ and NR₂₇;
R₂₅ is selected from CR₂₈ and NR₂₉;
R₂₃, R₂₆, and R₂₈ are independently selected from H, D, C₁-C₆ alkyl, C₁-C₆ haloalkyl, C₃-C₆ cycloalkyl, aryl, aralkyl, heterocyclyl, heterocyclylalkyl, halogen, -Si(C₁-C₆ alkyl)₃, -N₃, -B(OH)₂, -NO₂-R_{L}-COR', -R_{L}-C(O)OR', -R_{L}-C(O)NR'R", - R_{L}-CH=NR', -R_{L}-CN, -R_{L}-OR', -R_{L}-OC(O)R', -R_{L}-S(O)_{t'}-NR'R", -R_{L}-S(O)_{t'}-R', -R_{L}-NR'R", -R_{L}-NR'C(O)R", -R_{L}-NR'S(O)_{t'}R", -NR'-R_{L}-NR'R", -R_{L}-NO₂, and -R_{L}-N=CR'R", which can be optionally substituted;
preferably, R₂₃, R₂₆, and R₂₈ are independently selected from H, D, -CH₃, -F, -CF₃, -OH, -OCH₃, -NH₂, -NH(C₁-C₆ alkyl), - N(C₁-C₆ alkyl)(C₁-C₆ alkyl), -NO₂, -COO(C₁-C₆ alkyl), -COOH, -CN, -Si(CH₃)₃, -NHSO₂(C₁-C₆ alkyl), -SO₂NH₂, -SO₂(C₁-C₆ alkyl), -N(C₁-C₆ alkyl)SO₂(C₁-C₆ alkyl), -SO₂NH(C₁-C₆ alkyl), and -SO₂N(C₁-C₆ alkyl)(C₁-C₆ alkyl);
R₂₇ and R₂₉ are independently selected from H, D, C₁-C₆ alkyl, -OH, -(C₁-C₆ alkylene)-COOR₆₁, -(C₁-C₆ alkylene)-OR₆₁, and -(C₁-C₆ alkylene)-CONR₆₁.

4. The compound according to claim 1, wherein W₁ is C, and W₂ is C; alternatively, W₁ is C, and W₂ is N; alternatively, W₁ is C, and W₂ is O.

5. The compound according to claim 1, wherein R₁ and R₂ are independently selected from: H, -D, O, C₁-C₃ alkyl, -COOH, - CF₃, and hydroxy; preferably, both R₁ and R₂ are H, and/or both R₁ and R₂ are -D.

6. The compound according to claim 1, wherein 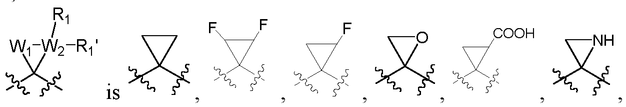 or 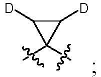 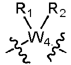 is

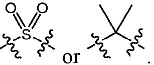

7. The compound according to claim 1, wherein Ar² has the following structure: 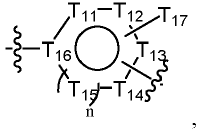 wherein
n is 0 or 1;
T₁₁-T₁₆ are independently selected from: C, N, O, and S;
T₁₇ represents one or more independent substituents on the ring selected from: H, alkyl, cycloalkyl, cycloalkylalkyl, aryl, aralkyl, heterocyclyl, heterocyclylalkyl, halogen, -R_{L}-COR', -R_{L}-C(O)OR', -R_{L}-C(O)NR'R", -R_{L}-CH=NR', -R_{L}-CN, -R_{L}-OR', -R_{L}-OC(O)R', -R_{L}-S(O)ₜ-NR'R", -R_{L}-S(O)ₜ-R', -R_{L}-NR'R", -R_{L}-NR'C(O)R", -R_{L}-NR'S(O)ₜR", -NR'-R_{L}-NR'R", -R_{L}-NO₂, and -R_{L}-N=CR'R", which can be optionally substituted.

8. The compound according to claim 7, wherein the compound has the following structure: 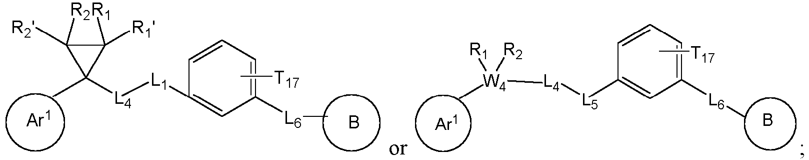
L₆ is absent or selected from: -NH-, -NR₁₅-, -NR₁₅C(O)-, -C(O)NR₁₅-, and C₁-C₆ alkylene;
R₁₅ is selected from: H and C₁-C₆ alkylene;
preferably, L₆ is absent or selected from: -NH-, -N(CH₃)-, -N(CH₃)C(O)-, and -NHC(O)-.

9. The compound according to claim 2, wherein B has the following structure: -F, -Cl, -Br, -I, -NH₂, -S(O)ₜNR₁₅, 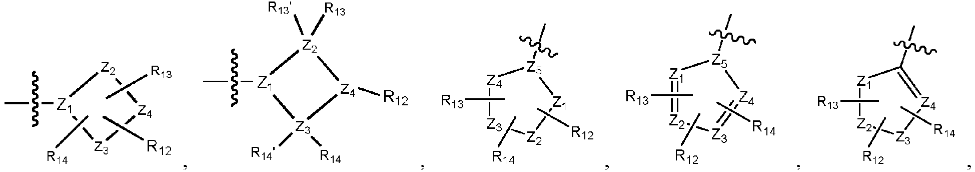 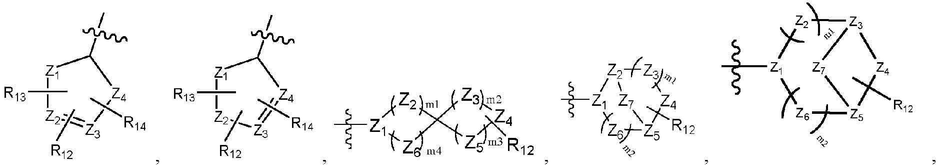 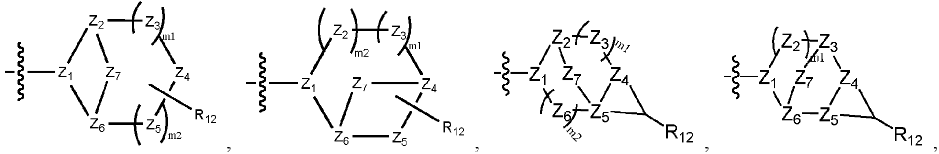 and 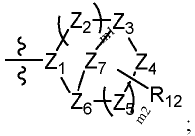
Z₂-Z₆ are independently selected from: C, N, O, and S;
Z₁ is selected from: C and N;
Z₇ is absent or selected from: a connecting bond, C, N, O, S, and C₁-C₆ alkylene;
m1-m4 are independently selected from an integer of 0-5;
R₁₂ represents one or more independent substituents on the ring selected from: H, D, (=O), alkyl, cycloalkyl, cycloalkylalkyl, aryl, aralkyl, heterocyclyl, heterocyclylalkyl, halogen, -R_{L}-COR', -R_{L}-C(O)OR', -R_{L}-C(O)NR'R", 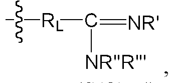 -R_{L}-CN, -R_{L}-OR', -R_{L}-OC(O)R', -R_{L}-S(O)ₜ-NR'R", -R_{L}-S(O)ₜ-R', -R_{L}-NR", -R_{L}-R'R", -R_{L}-NR'C(O)R", -R_{L}-NR'S(O)ₜR", -NR'-R_{L}-NR'R", -R_{L}-NO₂, and -R_{L}-N=CR'R", which can be optionally substituted;
R‴ is selected from: H, alkyl, cycloalkyl, cycloalkylalkyl, aryl, aralkyl, heterocyclyl, heterocyclylalkyl, halogen, and 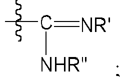
R₁₃ and R₁₃' are each independently selected from: H, D, (=O), alkyl, cycloalkyl, cycloalkylalkyl, aryl, aralkyl, heterocyclyl
heterocyclylalkyl, halogen, -N₃, -B(OH)₂, -R_{L}-COR', -R_{L}-C(O)OR', -R_{L}-C(O)NR'R", 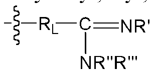
, -R_{L}-CN, -R_{L}-OR', - R_{L}-OC(O)R', -R_{L}-S(O)ₜ-NR'R", -R_{L}-S(O)ₜR', -R_{L}-NR'R", -R_{L}-NR'C(O)R", -R_{L}-NR'S(O)_{t'}R", -R_{L}-N(S(O)ₜR')(S(O)ₜR"), -NR'-R_{L}-NR"R‴, -R_{L}-NO₂, -R_{L}-N=CR'R", and -R_{L}-R'R", which can be optionally substituted;
R₁₄ and R₁₄' separately represent one or more independent substituents on the ring selected from: H, D, (=O), alkyl, cycloalkyl, cycloalkylalkyl, aryl, aralkyl, heterocyclyl, heterocyclylalkyl, halogen, -R_{L}-COR', -R_{L}-C(O)OR', -R_{L}-C(O)NR'R", 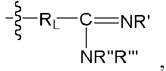 -R_{L}-CN, -R_{L}-OR', -R_{L}-OC(O)R', -R_{L}-S(O)ₜ-NR'R", -R_{L}-S(O)ₜ-R', -R_{L}-NR'R", -R_{L}-NR'C(O)R", -R_{L}-NR'S(O)ₜR", -NR'-R_{L}-NR'R", -R_{L}-NO₂, and -R_{L}-N=CR'R", which can be optionally substituted.

10. The compound according to claim 2, wherein B has the following structure: -F, -Cl, -Br, -I, -NH₂, -S(O)ₜNR₁₅, 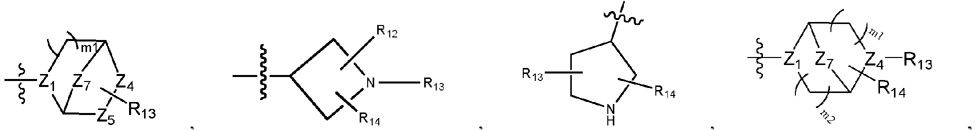 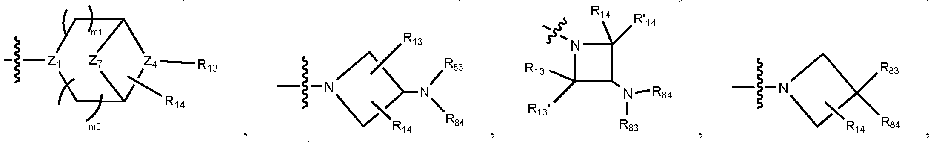 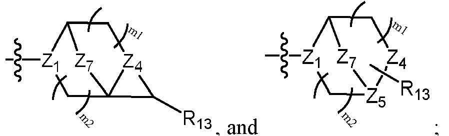
Z₁ and Z₄ are independently selected from: C, N, O, and S, and when Z₄ is O, R₁₃ is absent;
Z₇ is absent or selected from: a connecting bond, C, N, O, S, and C₁-C₃ alkylene;
m1 and m2 are independently selected from an integer of 0-5;
when Z₄ is S, R₁₃ is absent, or R₁₃ is carbonyl and R₁₄ is carbonyl;
R‴ is selected from: H, D, alkyl, cycloalkyl, cycloalkylalkyl, aryl, aralkyl, heterocyclyl, heterocyclylalkyl, halogen, and 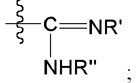
R₈₃ and R₈₄ are selected from: H, D, (=O), alkyl, cycloalkyl, cycloalkylalkyl, aryl, aralkyl, heterocyclyl, heterocyclylalkyl, halogen, -R_{L}-CR'R", -R_{L}-COR', -R_{L}-C(O)OR', -R_{L}-C(O)NR'R", 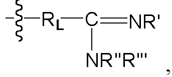 -R_{L}-CN, -R_{L}-OR', -R_{L}-OC(O)R', -R_{L}-S(O)t-NR'R", -R_{L}-S(O)ₜ-R', -R_{L}-NR'R", -R_{L}-NR'C(O)R", -R_{L}-NR'S(O)ₜR", -R_{L}-N(S(O)ₜR')(S(O)ₜR"), -NR'-R_{L}-NR"R‴, -R_{L}-NO₂, and -R_{L}-N=CR'R", which can be optionally substituted;
alternatively, R₈₃ and R₈₄, together with the N atom therebetween, form 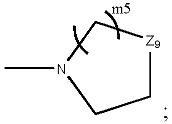
Z₉ is selected from S, NR₈₅, and O;
m₅ is selected from 1, 2, or 3;
R₈₅ is selected from H, D, (=O), alkyl, cycloalkyl, cycloalkylalkyl, aryl, aralkyl, heterocyclyl, heterocyclylalkyl, halogen, - R_{L}-COR', -R_{L}-C(O)OR', -R_{L}-C(O)NR'R", 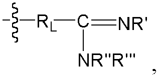 -R_{L}-CN, -R_{L}-OR', -R_{L}-OC(O)R', -R_{L}-S(O)ₜ-NR'R", -R_{L}-S(O)ₜ-R', -R_{L}-NR'R", -R_{L}-NR'C(O)R", -R_{L}-NR'S(O)_{t'}R", -R_{L}-N(S(O)ₜR')(S(O)ₜR"), -NR'-R_{L}-NR"R‴, -R_{L}-NO₂, and -R_{L}-N=CR'R", which can be optionally substituted.

11. The compound according to claim 10, wherein B has the following structure: -F, -Cl, -Br, -I, -NH₂, -S(O)ₜNR₁₅,

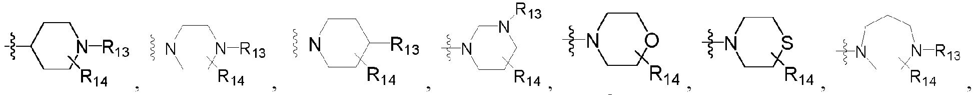

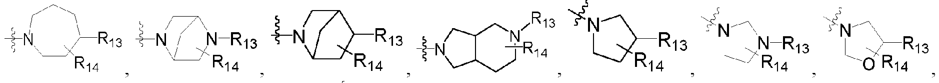

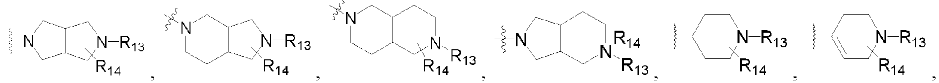

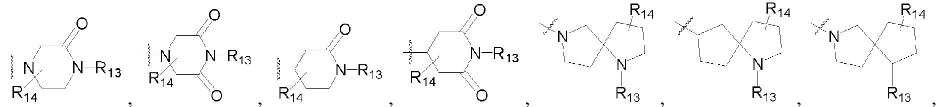

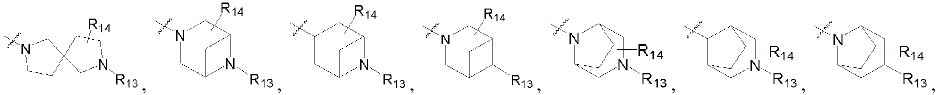

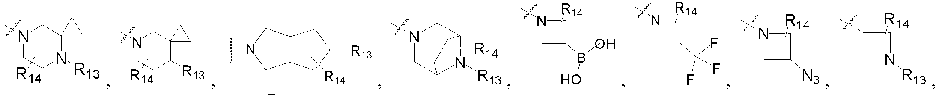

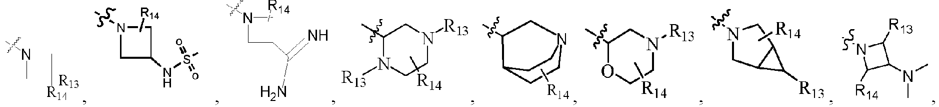

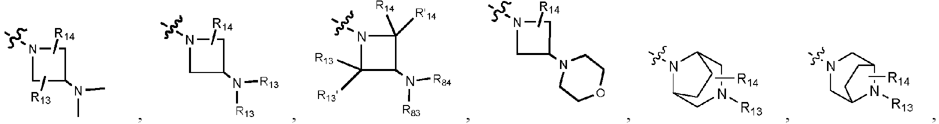

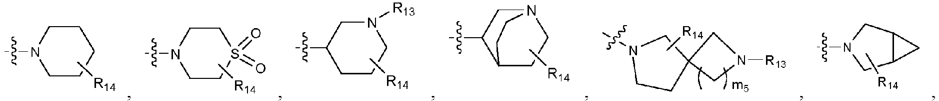

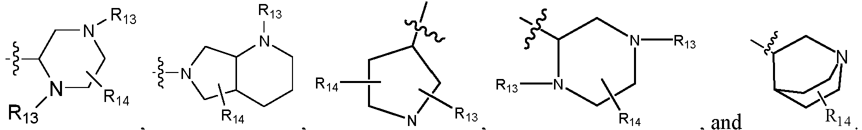

12. The compound according to claim 10 or 11, wherein R₁₄ and R₁₄' are each independently H, D, C₁-C₆ alkyl, amino, 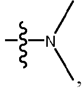 and -OCH₃.

13. The compound according to claim 10 or 11, wherein R₁₃ and R₁₃' are each independently selected from the following structures: -H, D, (=O), F, Cl, Br, I, methyl, ethyl, n-propyl, isopropyl, n-butyl, *tert*-butyl, -CF₃, -CH₂D, -OH, -N₃, -B(OH)₂, 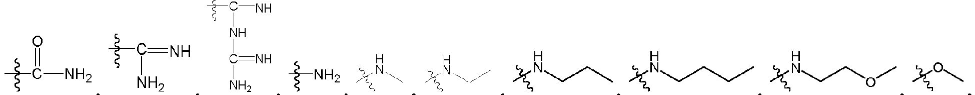 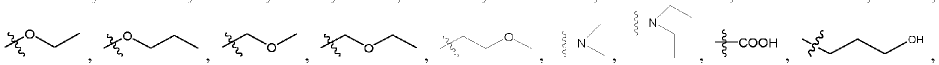 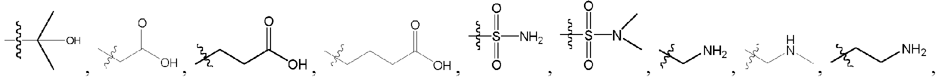 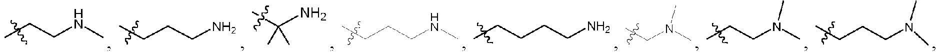 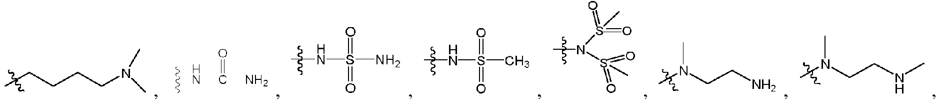 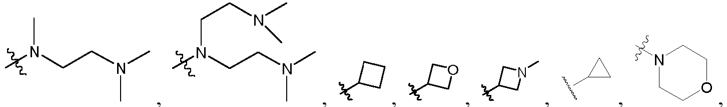 -CN

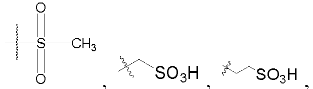

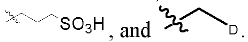

14. A pharmaceutical composition, comprising the compound or the pharmaceutically acceptable salt, the stereoisomer, the ester, the prodrug, the solvate, and the deuterated compound thereof according to any one of claims 1-13, and one or more pharmaceutically acceptable auxiliary materials.

15. Use of the compound or the pharmaceutically acceptable salt, the stereoisomer, the ester, the prodrug, the solvate, and the deuterated compound thereof according to any one of claims 1-13, or the pharmaceutical composition according to claim 14 in preparing a medicament for preventing and/or treating a disease or condition caused by or associated with viral infection.

16. The use according to claim 15, wherein the virus is a coronavirus, such as HCoV-229E, HCoV-OC43, HCoV-NL63, HCoV-HKU, SARS-CoV, MERS-CoV, and SARS-CoV-2.

17. The use according to claim 16, wherein the disease or condition is selected from: COVID-19, SARS, and MERS.

18. Use of the compound or the pharmaceutically acceptable salt, the stereoisomer, the ester, the prodrug, the solvate, and the deuterated compound thereof according to any one of claims 1-13, or the pharmaceutical composition according to claim 14 in preparing a medicament for reducing and/or inhibiting replication of a coronavirus.

19. A method for preventing and/or treating a disease or condition caused by or associated with viral infection, comprising administering to a patient the compound or the pharmaceutically acceptable salt, the stereoisomer, the ester, the prodrug, the solvate, and the deuterated compound thereof according to any one of claims 1-13, or the pharmaceutical composition according to claim 14.
